# EUROPEAN PATENT APPLICATION

(11) **EP 4 792 891 A2**
(43) Date of publication of application: **19.08.2026**
(21) Application number: 26189374.7
(22) Date of filing: 12.07.2024
(51) Int. Cl.: A61P 37/06

(54) **MULTISPECIFIC ANTIBODIES AND USES THEREOF**

(30) Priority: 13.07.2023 WO PCT/CN2023/107143; 14.09.2023 WO PCT/CN2023/118668; 31.10.2023 WO PCT/CN2023/128207; 16.11.2023 WO PCT/CN2023/131905; 09.04.2024 WO PCT/CN2024/086867
(62) Document number(s) of the earlier application(s) in accordance with Art. 76 EPC: 24840606.8 / 4 743 115
(71) Applicant: LTZ Therapeutics Inc., Redwood City, CA 94065 (US)
(72) Inventor: LI, Jinze, Danville, California, 94506 (US); ZHOU, Jianhui, Fremont, California, 94539 (US); CASBON, Amy-Jo, Redwood City, California, 94065 (US); PALMER, Rachael, San Francisco, California, 94115 (US); NING, Zhenfei, Yantai, Shandong (CN); FENG, Jintao, Shenzhen, Guangdong, 518057 (CN); KEDAGE, Vivekananda, Foster City, California, 94404 (US); HU, Jie, Mountain View, California, 94040 (US); SHENG, Zhentao, Sunnyvale, California, 94087 (US); YUAN, Ping, Richmond, California, 94803 (US); HUANG, Su, Rochester, Minnesota, 55901 (US); TREDER, Martin, 68799 Reilingen (DE); XIE, Liying, Palo Alto, California, 94306 (US); XIA, Jingying, Shenzhen, Guangdong, 518057 (CN); YANG, Xuejiao, Shenzhen, Guangdong, 518057 (CN)
(74) Representative: Fish & Richardson P.C.

(57) **Abstract**

This disclosure relates to anti-CLEC5A (C-type lectin domain family 5 member A) antibodies, antigen-binding fragments thereof. This disclosure relates to an antibody or antigen-binding fragment thereof, comprising a first antigen-binding domain that specifically binds to a tumor associated antigen (TAA) and a second antigen-binding domain that specifically binds to CLEC5A, and an Fc region. The present invention also relates to an antibody or an antigen-binding fragment thereof, comprising a second antigen-binding domain that specifically binds to an autoimmune disease target and to CLEC5A, and an Fc region.

## Description

### TECHNICAL FIELD

This disclosure relates to anti-CLEC5A (C-type lectin domain family 5 member A) antibodies, antigen-binding fragments thereof, antibody-drug conjugate (ADC) derived therefrom, and the uses thereof. This disclosure also relates to anti-CLEC5A multispecific antibodies (e.g., bispecific antibodies or antigen-binding fragments thereof), and antibody drug conjugates derived therefrom.

### BACKGROUND

Human C-type lectin domain family 5 member A (CLEC5A), also known as myeloid DAP12-associating lectin-1 (MDL-1), is a type II transmembrane protein and only expresses on the myeloid lineages including macrophages, monocytes, neutrophiles, and dendritic cells (DCs). As a pattern recognition receptor, CLEC5A transmits signals into cytoplasm through non-covalent binding to the adaptor protein DAP12. Phosphorylation of DAP12 then initiates a Syk kinase-based signaling cascade resulting in macrophages activation and release of chemokines and proinflammation cytokines, including IL-6, TNF, CCL3, and CXCL8.

CLEC5A can trigger myeloid cell-related immune response and correlates with diverse infection and inflammatory diseases. CLEC5A promotes the production of high levels of pro-inflammatory cytokines and chemokines in flavivirus infections, especially dengue and Japanese encephalitis virus infections, and anti-CLEC5A mAb or CLEC5A inhibitors can reverse disease progression, suggesting that CLEC5A is a promising therapeutic target for flavivirus infections. In addition, similar in several autoimmune diseases, a high level of CLEC5A is found in active rheumatoid arthritis and CLEC5A activator increases proinflammation cytokines level. CLEC5A is also a pivotal contributor to cancer development and progression. Aberrantly high CLEC5A expression significantly correlates with decreased overall survival in high-grade serious ovarian cancer (HGSOC), gastric cancer, and glioma.

A bispecific antibody is an artificial protein that can simultaneously bind to two different types of antigens or two different epitopes. This dual specificity opens up a wide range of applications, including redirecting T cells to tumor cells, dual targeting of different disease mediators, and delivering payloads to targeted sites. The approval of catumaxomab (anti-EpCAM and anti-CD3) and blinatumomab (anti-CD19 and anti-CD3) has become a major milestone in the development of bispecific antibodies.

As bispecific antibodies have various applications, there is a need to continue developing various therapeutics based on bispecific antibodies.

Considering the important role of CLEC5A in immune system, there is a need to develop a therapeutic agent targeting CLEC5A, particularly bispecific antibodies targeting CLEC5A.

### SUMMARY

This disclosure relates to anti-CLEC5A antibodies, antigen-binding fragment thereof, and the uses thereof. This disclosure also relates to multispecific (e.g., bispecific) antibodies or antigen-binding fragments thereof, wherein the antibodies or antigen-binding fragments thereof specifically bind to a tumor associated antigen (TAA) and human C-type lectin domain family 5 member A (CLEC5A). In some embodiments, the antibodies or antigen-binding fragments thereof have an enhanced Fc. In some embodiments, the antibody or antigen-binding fragment thereof has an increased binding affinity to the FcγRIIa receptor and/or the FcγRIIIa receptor. In some embodiments, the antibodies or antigen-binding fragments thereof have a non-functional Fc.

The anti-CLEC5A antibodies described herein are not "typical agonist" as compared to a reference antibody used in the disclosure (e.g., DX244). For example, the anti-CLEC5A antibodies described herein can mediate phagocytosis of macrophages (similar to DX244), but with more potency and very low levels of cytokine release. Further, the TAA/CLEC5A bispecific antibodies described herein were shown to mediate killing of different TAA-expressing targeting cells by myeloid cells (e.g., monocytes and macrophages), at a very low E:T ratio and produce a very low cytokine release. Without withing to be bound by theory, it is contemplated that the anti-CLEC5A antibodies and TAA/CLEC5A bispecific antibodies described herein can be used to make potential myeloid cell engagers with a high efficacy and a good safety profile.

In one aspect, the disclosure is related to an antibody or antigen-binding fragment thereof that binds to CLEC5A (C-type lectin domain family 5 member A) comprising: a heavy chain variable region (VH) comprising complementarity determining regions (CDRs) 1, 2, and 3, in some embodiments, the VH CDR1 region comprises an amino acid sequence that is at least 80% identical to a selected VH CDR1 amino acid sequence, the VH CDR2 region comprises an amino acid sequence that is at least 80% identical to a selected VH CDR2 amino acid sequence, and the VH CDR3 region comprises an amino acid sequence that is at least 80% identical to a selected VH CDR3 amino acid sequence; and a light chain variable region (VL) comprising CDRs 1, 2, and 3, in some embodiments, the VL CDR1 region comprises an amino acid sequence that is at least 80% identical to a selected VL CDR1 amino acid sequence, the VL CDR2 region comprises an amino acid sequence that is at least 80% identical to a selected VL CDR2 amino acid sequence, and the VL CDR3 region comprises an amino acid sequence that is at least 80% identical to a selected VL CDR3 amino acid sequence, in some embodiments, the selected VH CDRs 1, 2, and 3 amino acid sequences and the selected VL CDRs, 1, 2, and 3 amino acid sequences are one of the following:
(1) the selected VH CDRs 1, 2, 3 amino acid sequences are set forth in SEQ ID NOs: 3, 5, 7, respectively, and the selected VL CDRs 1, 2, 3 amino acid sequences are set forth in SEQ ID NOs: 8-10, respectively;
(2) the selected VH CDRs 1, 2, 3 amino acid sequences are set forth in SEQ ID NOs: 4, 6, 7, respectively, and the selected VL CDRs 1, 2, 3 amino acid sequences are set forth in SEQ ID NOs: 8-10, respectively;
(3) the selected VH CDRs 1, 2, 3 amino acid sequences are set forth in SEQ ID NOs: 13, 15, 17, respectively, and the selected VL CDRs 1, 2, 3 amino acid sequences are set forth in SEQ ID NOs: 18-20, respectively;
(4) the selected VH CDRs 1, 2, 3 amino acid sequences are set forth in SEQ ID NOs: 14, 16, 17, respectively, and the selected VL CDRs 1, 2, 3 amino acid sequences are set forth in SEQ ID NOs: 18-20, respectively;
(5) the selected VH CDRs 1, 2, 3 amino acid sequences are set forth in SEQ ID NOs: 23, 25, 27, respectively, and the selected VL CDRs 1, 2, 3 amino acid sequences are set forth in SEQ ID NOs: 28-30, respectively;
(6) the selected VH CDRs 1, 2, 3 amino acid sequences are set forth in SEQ ID NOs: 24, 26, 27, respectively, and the selected VL CDRs 1, 2, 3 amino acid sequences are set forth in SEQ ID NOs: 28-30, respectively;
(7) the selected VH CDRs 1, 2, 3 amino acid sequences are set forth in SEQ ID NOs: 33, 35, 37, respectively, and the selected VL CDRs 1, 2, 3 amino acid sequences are set forth in SEQ ID NOs: 38-40, respectively;
(8) the selected VH CDRs 1, 2, 3 amino acid sequences are set forth in SEQ ID NOs: 34, 36, 37, respectively, and the selected VL CDRs 1, 2, 3 amino acid sequences are set forth in SEQ ID NOs: 38-40, respectively;
(9) the selected VH CDRs 1, 2, 3 amino acid sequences are set forth in SEQ ID NOs: 43, 45, 47, respectively, and the selected VL CDRs 1, 2, 3 amino acid sequences are set forth in SEQ ID NOs: 48-50, respectively;
(10) the selected VH CDRs 1, 2, 3 amino acid sequences are set forth in SEQ ID NOs: 44, 46, 47, respectively, and the selected VL CDRs 1, 2, 3 amino acid sequences are set forth in SEQ ID NOs: 48-50, respectively;
(11) the selected VH CDRs 1, 2, 3 amino acid sequences are set forth in SEQ ID NOs: 53, 55, 57, respectively, and the selected VL CDRs 1, 2, 3 amino acid sequences are set forth in SEQ ID NOs: 58-60, respectively;
(12) the selected VH CDRs 1, 2, 3 amino acid sequences are set forth in SEQ ID NOs: 54, 56, 57, respectively, and the selected VL CDRs 1, 2, 3 amino acid sequences are set forth in SEQ ID NOs: 58-60, respectively;
(13) the selected VH CDRs 1, 2, 3 amino acid sequences are set forth in SEQ ID NOs: 63, 65, 67, respectively, and the selected VL CDRs 1, 2, 3 amino acid sequences are set forth in SEQ ID NOs: 68-70, respectively;
(14) the selected VH CDRs 1, 2, 3 amino acid sequences are set forth in SEQ ID NOs: 64, 66, 67, respectively, and the selected VL CDRs 1, 2, 3 amino acid sequences are set forth in SEQ ID NOs: 68-70, respectively;
(15) the selected VH CDRs 1, 2, 3 amino acid sequences are set forth in SEQ ID NOs: 73, 75, 77, respectively, and the selected VL CDRs 1, 2, 3 amino acid sequences are set forth in SEQ ID NOs: 78-80, respectively; and
(16) the selected VH CDRs 1, 2, 3 amino acid sequences are set forth in SEQ ID NOs: 74, 76, 77, respectively, and the selected VL CDRs 1, 2, 3 amino acid sequences are set forth in SEQ ID NOs: 78-80, respectively.

In some embodiments, the VH comprises CDRs 1, 2, 3 with the amino acid sequences set forth in SEQ ID NOs: 3, 5, 7, respectively, and the VL comprises CDRs 1, 2, 3 with the amino acid sequences set forth in SEQ ID NOs: 8-10, respectively, according to Kabat definition. In some embodiments, the VH comprises CDRs 1, 2, 3 with the amino acid sequences set forth in SEQ ID NOs: 13, 15, 17, respectively, and the VL comprises CDRs 1, 2, 3 with the amino acid sequences set forth in SEQ ID NOs: 18-20, respectively, according to Kabat definition. In some embodiments, the VH comprises CDRs 1, 2, 3 with the amino acid sequences set forth in SEQ ID NOs: 23, 25, 27, respectively, and the VL comprises CDRs 1, 2, 3 with the amino acid sequences set forth in SEQ ID NOs: 28-30, respectively, according to Kabat definition. In some embodiments, the VH comprises CDRs 1, 2, 3 with the amino acid sequences set forth in SEQ ID NOs: 33, 35, 37, respectively, and the VL comprises CDRs 1, 2, 3 with the amino acid sequences set forth in SEQ ID NOs: 38-40, respectively, according to Kabat definition. In some embodiments, the VH comprises CDRs 1, 2, 3 with the amino acid sequences set forth in SEQ ID NOs: 43, 45, 47, respectively, and the VL comprises CDRs 1, 2, 3 with the amino acid sequences set forth in SEQ ID NOs: 48-50, respectively, according to Kabat definition. In some embodiments, the VH comprises CDRs 1, 2, 3 with the amino acid sequences set forth in SEQ ID NOs: 53, 55, 57, respectively, and the VL comprises CDRs 1, 2, 3 with the amino acid sequences set forth in SEQ ID NOs: 58-60, respectively, according to Kabat definition. In some embodiments, the VH comprises CDRs 1, 2, 3 with the amino acid sequences set forth in SEQ ID NOs: 63, 65, 67, respectively, and the VL comprises CDRs 1, 2, 3 with the amino acid sequences set forth in SEQ ID NOs: 68-70, respectively, according to Kabat definition. In some embodiments, the VH comprises CDRs 1, 2, 3 with the amino acid sequences set forth in SEQ ID NOs: 73, 75, 77, respectively, and the VL comprises CDRs 1, 2, 3 with the amino acid sequences set forth in SEQ ID NOs: 78-80, respectively, according to Kabat definition. In some embodiments, the VH comprises CDRs 1, 2, 3 with the amino acid sequences set forth in SEQ ID NOs: 4, 6, 7, respectively, and the VL comprises CDRs 1, 2, 3 with the amino acid sequences set forth in SEQ ID NOs: 8-10, respectively, according to Chothia definition. In some embodiments, the VH comprises CDRs 1, 2, 3 with the amino acid sequences set forth in SEQ ID NOs: 14, 16, 17, respectively, and the VL comprises CDRs 1, 2, 3 with the amino acid sequences set forth in SEQ ID NOs: 18-20, respectively, according to Chothia definition. In some embodiments, the VH comprises CDRs 1, 2, 3 with the amino acid sequences set forth in SEQ ID NOs: 24, 26, 27, respectively, and the VL comprises CDRs 1, 2, 3 with the amino acid sequences set forth in SEQ ID NOs: 28-30, respectively, according to Chothia definition. In some embodiments, the VH comprises CDRs 1, 2, 3 with the amino acid sequences set forth in SEQ ID NOs: 34, 36, 37, respectively, and the VL comprises CDRs 1, 2, 3 with the amino acid sequences set forth in SEQ ID NOs: 38-40, respectively, according to Chothia definition. In some embodiments, the VH comprises CDRs 1, 2, 3 with the amino acid sequences set forth in SEQ ID NOs: 44, 46, 47, respectively, and the VL comprises CDRs 1, 2, 3 with the amino acid sequences set forth in SEQ ID NOs: 48-50, respectively, according to Chothia definition. In some embodiments, the VH comprises CDRs 1, 2, 3 with the amino acid sequences set forth in SEQ ID NOs: 54, 56, 57, respectively, and the VL comprises CDRs 1, 2, 3 with the amino acid sequences set forth in SEQ ID NOs: 58-60, respectively, according to Chothia definition. In some embodiments, the VH comprises CDRs 1, 2, 3 with the amino acid sequences set forth in SEQ ID NOs: 64, 66, 67, respectively, and the VL comprises CDRs 1, 2, 3 with the amino acid sequences set forth in SEQ ID NOs: 68-70, respectively, according to Chothia definition. In some embodiments, the VH comprises CDRs 1, 2, 3 with the amino acid sequences set forth in SEQ ID NOs: 74, 76, 77, respectively, and the VL comprises CDRs 1, 2, 3 with the amino acid sequences set forth in SEQ ID NOs: 78-80, respectively, according to Chothia definition.

In one aspect, the disclosure is related to an antibody or antigen-binding fragment thereof that binds to CLEC5A comprising a heavy chain variable region (VH) comprising an amino acid sequence that is at least 90% identical to a selected VH sequence, and a light chain variable region (VL) comprising an amino acid sequence that is at least 90% identical to a selected VL sequence, in some embodiments, the selected VH sequence and the selected VL sequence are one of the following:
(1) the selected VH sequence is SEQ ID NO: 11, and the selected VL sequence is SEQ ID NO: 12;
(2) the selected VH sequence is SEQ ID NO: 21, and the selected VL sequence is SEQ ID NO: 22;
(3) the selected VH sequence is SEQ ID NO: 31, and the selected VL sequence is SEQ ID NO: 32;
(4) the selected VH sequence is SEQ ID NO: 41, and the selected VL sequence is SEQ ID NO: 42;
(5) the selected VH sequence is SEQ ID NO: 51, and the selected VL sequence is SEQ ID NO: 52;
(6) the selected VH sequence is SEQ ID NO: 61, and the selected VL sequence is SEQ ID NO: 62;
(7) the selected VH sequence is SEQ ID NO: 71, and the selected VL sequence is SEQ ID NO: 72;
(8) the selected VH sequence is SEQ ID NO: 81, and the selected VL sequence is SEQ ID NO: 82;
(9) the selected VH sequence is SEQ ID NO: 83, and the selected VL sequence is SEQ ID NO: 84;
(10) the selected VH sequence is SEQ ID NO: 85, and the selected VL sequence is SEQ ID NO: 86;
(11) the selected VH sequence is SEQ ID NO: 87, and the selected VL sequence is SEQ ID NO: 88;
(12) the selected VH sequence is SEQ ID NO: 89, and the selected VL sequence is SEQ ID NO: 90; and
(13) the selected VH sequence is SEQ ID NO: 91, and the selected VL sequence is SEQ ID NO: 92.

In some embodiments, the VH comprises the sequence of SEQ ID NO: 11 and the VL comprises the sequence of SEQ ID NO: 12. In some embodiments, the VH comprises the sequence of SEQ ID NO: 21 and the VL comprises the sequence of SEQ ID NO: 22. In some embodiments, the VH comprises the sequence of SEQ ID NO: 31 and the VL comprises the sequence of SEQ ID NO: 32. In some embodiments, the VH comprises the sequence of SEQ ID NO: 41 and the VL comprises the sequence of SEQ ID NO: 42. In some embodiments, the VH comprises the sequence of SEQ ID NO: 51 and the VL comprises the sequence of SEQ ID NO: 52. In some embodiments, the VH comprises the sequence of SEQ ID NO: 61 and the VL comprises the sequence of SEQ ID NO: 62. In some embodiments, the VH comprises the sequence of SEQ ID NO: 71 and the VL comprises the sequence of SEQ ID NO: 72. In some embodiments, the VH comprises the sequence of SEQ ID NO: 81 and the VL comprises the sequence of SEQ ID NO: 82. In some embodiments, the VH comprises the sequence of SEQ ID NO: 83 and the VL comprises the sequence of SEQ ID NO: 84. In some embodiments, the VH comprises the sequence of SEQ ID NO: 85 and the VL comprises the sequence of SEQ ID NO: 86. In some embodiments, the VH comprises the sequence of SEQ ID NO: 87 and the VL comprises the sequence of SEQ ID NO: 88. In some embodiments, the VH comprises the sequence of SEQ ID NO: 89 and the VL comprises the sequence of SEQ ID NO: 90. In some embodiments, the VH comprises the sequence of SEQ ID NO: 91 and the VL comprises the sequence of SEQ ID NO: 92.

In some embodiments, the antibody or antigen-binding fragment thereof specifically binds to human, mouse, or monkey CLEC5A.

In some embodiments, the antibody or antigen-binding fragment thereof can mediate phagocytosis of macrophages (e.g., with a target cell killing efficacy that at least 60%, at least 70%, at least 80%, at least 90%, at least 100%, at least 110%, at least 120%, at least 130%, at least 140%, or at least 150% as compared to that of a reference antibody (e.g., DX244)), and/or
In some embodiments, the antibody or antigen-binding fragment thereof can induce low levels of cytokine (e.g., IL-6 or TNFα) release (e.g., less than 80%, less than 70%, less than 60%, less than 50%, less than 40%, less than 30%, less than 20%, less than 10%, less than 5%, or less than 1% as compared to that induced by a reference antibody (e.g., DX244)).

In one aspect, the disclosure is related to an antibody or antigen-binding fragment thereof that binds to CLEC5A, in some embodiments, the antibody or antigen-binding fragment thereof can mediate phagocytosis of macrophages (e.g., with a target cell killing efficacy that is at least 60%, at least 70%, at least 80%, at least 90%, at least 100%, at least 110%, at least 120%, at least 130%, at least 140%, or at least 150% as compared to that of a reference antibody (e.g., DX244)), and/or in some embodiments, the antibody or antigen-binding fragment thereof can induce a low level of cytokine (e.g., IL-6 or TNFα) release (e.g., less than 80%, less than 70%, less than 60%, less than 50%, less than 40%, less than 30%, less than 20%, less than 10%, less than 5%, or less than 1% as compared to that induced by a reference antibody (e.g., DX244)).

In one aspect, the disclosure is related to an antibody or antigen-binding fragment thereof, comprising: i) a first antigen-binding domain that specifically binds to a first antigen, in some embodiments, the first antigen is a tumor associated antigen (TAA); and ii) a second antigen-binding domain that specifically binds to C-type lectin domain family 5 member A (CLEC5A).

In one aspect, the disclosure is related to an antibody or antigen-binding fragment thereof, comprising: i) a first antigen-binding domain that specifically binds to a first antigen, in some embodiments, the first antigen is an autoimmune disease target; and ii) a second antigen-binding domain that specifically binds to CLEC5A.

In some embodiments, the antibody or antigen-binding fragment thereof has one or more of the following effects:
i) the antibody or antigen-binding fragment thereof can mediate target cell killing by myeloid cells (e.g., monocytes and/or macrophages (e.g., M0, M1, and/or M2 macrophages));
ii) the antibody or antigen-binding fragment thereof can mediate target cell killing by myeloid cells (e.g., monocytes and/or macrophages) at a low E:T (effector cell : target cell) ratio, optionally on some embodiments, the low E:T ratio is less than 1:10, less than 1:9, less than 1:8, less than 1:7, less than 1:6, less than 1:5, less than 1:4, less than 1:3, less than 1:2, less than 1:1, less than 2:1, less than 3:1, less than 4:1, less than 5:1, less than 6:1, less than 7:1, less than 8:1, less than 9:1, or less than 10:1); and
iii) the antibody or antigen-binding fragment thereof can induce a low level of cytokine (e.g., IL-6 or TNFα) release of myeloid cells (e.g., monocytes and/or macrophages), e.g., less than 80%, less than 70%, less than 60%, less than 50%, less than 40%, less than 30%, less than 20%, less than 10%, less than 5%, or less than 1% as compared to that induced by a reference antibody.

In some embodiments, the first antigen-binding domain comprises a first heavy chain variable region (VH1) and a first light chain variable region (VL1); and the second antigen-binding domain comprises a second heavy chain variable region (VH2) and a second light chain variable region (VL2). In some embodiments, the second antigen-binding domain is a single-chain fragment variable (scFv) domain, in some embodiments, the VH2 and VL2 are linked by a first linker. In some embodiments, the second antigen-binding domain is connected to the C-terminus of a light chain through a second linker. In some embodiments, the antibody or antigen-binding fragment thereof described herein further comprises an Fc region. In some embodiments, the C-terminus of the VH1 of the first antigen-binding domain is connected to the Fc region, optionally through a CH1 domain. In some embodiments, the C-terminus of the VH1 of the first antigen-binding domain is connected to the N-terminus of a Fc region, and the N-terminus of the second antigen-binding domain is connected to the C-terminus of the Fc region. In some embodiments, the antibody comprises a first heavy chain comprising the VH1 and a first light chain comprising the VL1; and a second heavy chain comprising the VH2 and a second light chain comprising the VL2. In some embodiments, the first heavy chain comprises one or more knob mutations; and the second heavy chain comprises one or more hole mutations. In some embodiments, the first heavy chain comprises one or more hole mutations; and the second heavy chain comprises one or more knob mutations.

In some embodiments, the Fc region is an Fc region of human IgG1, IgG2, IgG3, or IgG4. In some embodiments, the Fc region is an Fc region of human IgG1. In some embodiments, the Fc region comprises one or more the following amino acid residues (all numbering is according to EU numbering):
(1) an Alanine (A) at position 236; a Leucine (L) at position 330 and a Glutamic acid (E) at position 332;
(2) an Alanine (A) at position 236; an Aspartic acid (D) at position 293; a Leucine (L) at position 330 and a Glutamic acid (E) at position 332;
(3) an Alanine (A) at position 236;
(4) an Alanine (A) at position 236; an Aspartic acid (D) at position 293; and a Glutamic acid (E) at position 332;
(5) an Aspartic acid (D) at position 293 and a Glutamic acid (E) at position 332;
(6) an Aspartic acid (D) at position 293; a Leucine (L) at position 330 and a Glutamic acid (E) at position 332; and
(7) an Alanine (A) at position 234; an Alanine (A) at position 235 and a Glycine (G) at position 329;
optionally, the Fc region is afucosylated.

In some embodiments, the tumor associated antigen (TAA) is HER2, CD79b, EGFR, EpCAM, BCMA, CD38, GPRC5D, DLL3, CD70, GPC3, Mesothelin, Fas ligand (FasL), CD1d, Membrane glycolipids, globotriaosyl-ceramide (Gb3Cer/CD77), gangliosides (GD2, GD3, and GM2), CD34, CD45, human leukocyte antigen-DR (HLA-DR), CD123, CLL1, CD105, CD71, SSC, MAGE, MUC16, CD19, WT-l, B7H3, TEM8, CD22, LI-CAM, ROR-l, CEA, 4-1BB, ETA, 5T4, adenocarcinoma antigen, alpha- fetoprotein (AFP), BAFF, B- lymphoma cell, CA242 antigen, CA-125, carbonic anhydrase 9 (CA- IX), C-MET, CCR4, CD133, CD152, CD20, CD125, CD200, CD221, CD23 (IgE receptor), CD28, CD30 (TNFRSF8), CD33, CD4, CD40, CD44 v6, CD51, CD52, CD56, CD74, CD80, CEA, CNT0888, CTLA-4, DR5, CD3, FAP, fibronectin extra domain-B, folate receptor 1, GD2, GD3 ganglioside, glycoprotein 75, GPNMB, , HGF, human scatter factor receptor kinase, IGF-l receptor, IGF-I, IgG1, IL-5, IL-13, IL-6, IL-15, insulin-like growth factor I receptor, integrin a5b1, integrin avb3, MSLN, MS4A1, MUC1, mucin CanAg, N-glycolylneuraminic acid, NPC-1C, PD-1, PD-L1, PDGF-R a, TWEAK, phosphatidylserine, prostatic carcinoma cells, RANKL, RON, SCH 900105, SDC1, SLAMF7, TAG-72, tenascin C, TGF beta 2, TGF-b, TRAIL-R1, TRAIL-R2, tumor antigen CTAA16.88, VEGF-A, VEGFR-l, VEGFR2, or vimentin.

In some embodiments, autoimmune disease target is CD79b, CD38, ACHE, BAFF, BTK, CCL2, CD19, CD20, CD25, CD40, CD52, CD80, CD86, ETAR, ETBR, FCGRT, GM-CSF, JAK1, IFNAR, IFNB1, IFNG, IgE, IgG Fc, IL1A, IL1B, IL-2, IL-4, IL-5, IL-6, IL6R, IL7, IL-12, IL-13, IL-17, IL-18, IL-21, IL-22, IL-23, Integrin, ITG-A4B1, ITG-A4B7, ITG-AVB6, TL1A, TNF-α, TNF-β, TNFSF13B, TSLP, TYK2, or VEGFR.

In some embodiments, the TAA is HER2. In some embodiments,
(1) the selected VH1 CDRs 1, 2, 3 amino acid sequences are set forth in SEQ ID NOs: 100, 102, 104, respectively, and the selected VL1 CDRs 1, 2, 3 amino acid sequences are set forth in SEQ ID NOs: 105-107, respectively; and the selected VH2 CDRs 1, 2, 3 amino acid sequences and the selected VL2 CDRs 1, 2, 3 amino acid sequences are selected from one of the sequences described herein; or
(2) the selected VH1 CDRs 1, 2, 3 amino acid sequences are set forth in SEQ ID NOs: 101, 103, 104, respectively, and the selected VL1 CDRs 1, 2, 3 amino acid sequences are set forth in SEQ ID NOs: 105-107, respectively; and the selected VH2 CDRs 1, 2, 3 amino acid sequences and the selected VL2 CDRs 1, 2, 3 amino acid sequences are selected from one of the sequences described herein.

In some embodiments, the TAA or autoimmune disease target is CD79b. In some embodiments,
(1) the selected VH1 CDRs 1, 2, 3 amino acid sequences are set forth in SEQ ID NOs: 200, 202, 204, respectively, and the selected VL1 CDRs 1, 2, 3 amino acid sequences are set forth in SEQ ID NOs: 205-207, respectively; and the selected VH2 CDRs 1, 2, 3 amino acid sequences and the selected VL2 CDRs 1, 2, 3 amino acid sequences are selected from one of the sequences described herein; or
(2) the selected VH1 CDRs 1, 2, 3 amino acid sequences are set forth in SEQ ID NOs: 201, 203, 204, respectively, and the selected VL1 CDRs 1, 2, 3 amino acid sequences are set forth in SEQ ID NOs: 205-207, respectively; and the selected VH2 CDRs 1, 2, 3 amino acid sequences and the selected VL2 CDRs 1, 2, 3 amino acid sequences are selected from one of the sequences described herein.

In some embodiments, the TAA is EGFR. In some embodiments,
(1) the selected VH1 CDRs 1, 2, 3 amino acid sequences are set forth in SEQ ID NOs: 126, 128, 130, respectively, and the selected VL1 CDRs 1, 2, 3 amino acid sequences are set forth in SEQ ID NOs: 131-133, respectively; and the selected VH2 CDRs 1, 2, 3 amino acid sequences and the selected VL2 CDRs 1, 2, 3 amino acid sequences are selected from one of the sequences described herein;
(2) the selected VH1 CDRs 1, 2, 3 amino acid sequences are set forth in SEQ ID NOs: 127, 129, 130, respectively, and the selected VL1 CDRs 1, 2, 3 amino acid sequences are set forth in SEQ ID NOs: 131-133, respectively; and the selected VH2 CDRs 1, 2, 3 amino acid sequences and the selected VL2 CDRs 1, 2, 3 amino acid sequences are selected from one of the sequences described herein;
(3) the selected VH1 CDRs 1, 2, 3 amino acid sequences are set forth in SEQ ID NOs: 136, 138, 140, respectively, and the selected VL1 CDRs 1, 2, 3 amino acid sequences are set forth in SEQ ID NOs: 141-143, respectively; and the selected VH2 CDRs 1, 2, 3 amino acid sequences and the selected VL2 CDRs 1, 2, 3 amino acid sequences are selected from one of the sequences described herein; or
(4) the selected VH1 CDRs 1, 2, 3 amino acid sequences are set forth in SEQ ID NOs: 137, 139, 140, respectively, and the selected VL1 CDRs 1, 2, 3 amino acid sequences are set forth in SEQ ID NOs: 141-143, respectively; and the selected VH2 CDRs 1, 2, 3 amino acid sequences and the selected VL2 CDRs 1, 2, 3 amino acid sequences are selected from one of the sequences described herein.

In some embodiments, the TAA is EpCAM. In some embodiments,
(1) the selected VH1 CDRs 1, 2, 3 amino acid sequences are set forth in SEQ ID NOs: 150, 152, 154, respectively, and the selected VL1 CDRs 1, 2, 3 amino acid sequences are set forth in SEQ ID NOs: 155-157, respectively; and the selected VH2 CDRs 1, 2, 3 amino acid sequences and the selected VL2 CDRs 1, 2, 3 amino acid sequences are selected from one of the sequences described herein; or
(2) the selected VH1 CDRs 1, 2, 3 amino acid sequences are set forth in SEQ ID NOs: 151, 153, 154, respectively, and the selected VL1 CDRs 1, 2, 3 amino acid sequences are set forth in SEQ ID NOs: 155-157, respectively; and the selected VH2 CDRs 1, 2, 3 amino acid sequences and the selected VL2 CDRs 1, 2, 3 amino acid sequences are selected from one of the sequences described herein;

In some embodiments, the TAA or autoimmune disease target is GPRC5D. In some embodiments,
(1) the selected VH1 CDRs 1, 2, 3 amino acid sequences are set forth in SEQ ID NOs: 164, 166, 168, respectively, and the selected VL1 CDRs 1, 2, 3 amino acid sequences are set forth in SEQ ID NOs: 169-171, respectively; and the selected VH2 CDRs 1, 2, 3 amino acid sequences and the selected VL2 CDRs 1, 2, 3 amino acid sequences are selected from one of the sequences described herein; or
(2) the selected VH1 CDRs 1, 2, 3 amino acid sequences are set forth in SEQ ID NOs: 165, 167, 168, respectively, and the selected VL1 CDRs 1, 2, 3 amino acid sequences are set forth in SEQ ID NOs: 169-171, respectively; and the selected VH2 CDRs 1, 2, 3 amino acid sequences and the selected VL2 CDRs 1, 2, 3 amino acid sequences are selected from one of the sequences described herein.

In some embodiments, the TAA or autoimmune disease target is BCMA. In some embodiments,
(1) the selected VH1 CDRs 1, 2, 3 amino acid sequences are set forth in SEQ ID NOs: 176, 178, 180, respectively, and the selected VL1 CDRs 1, 2, 3 amino acid sequences are set forth in SEQ ID NOs: 181-183, respectively; and the selected VH2 CDRs 1, 2, 3 amino acid sequences and the selected VL2 CDRs 1, 2, 3 amino acid sequences are selected from one of the sequences described herein; or
(2) the selected VH1 CDRs 1, 2, 3 amino acid sequences are set forth in SEQ ID NOs: 177, 179, 180, respectively, and the selected VL1 CDRs 1, 2, 3 amino acid sequences are set forth in SEQ ID NOs: 181-183, respectively; and the selected VH2 CDRs 1, 2, 3 amino acid sequences and the selected VL2 CDRs 1, 2, 3 amino acid sequences are selected from one of the sequences described herein.

In some embodiments, the TAA or autoimmune disease target is CD38. In some embodiments,
(1) the selected VH1 CDRs 1, 2, 3 amino acid sequences are set forth in SEQ ID NOs: 188, 190, 192, respectively, and the selected VL1 CDRs 1, 2, 3 amino acid sequences are set forth in SEQ ID NOs: 193-195, respectively; and the selected VH2 CDRs 1, 2, 3 amino acid sequences and the selected VL2 CDRs 1, 2, 3 amino acid sequences are selected from one of the sequences described herein; or
(2) the selected VH1 CDRs 1, 2, 3 amino acid sequences are set forth in SEQ ID NOs: 189, 191, 192, respectively, and the selected VL1 CDRs 1, 2, 3 amino acid sequences are set forth in SEQ ID NOs: 193-195, respectively; and the selected VH2 CDRs 1, 2, 3 amino acid sequences and the selected VL2 CDRs 1, 2, 3 amino acid sequences are selected from one of the sequences described herein.

In one aspect, the disclosure is related to an antibody or antigen-binding fragment thereof that cross-competes with the antibody or antigen-binding fragment thereof described herein.

In one aspect, the disclosure is related to a nucleic acid comprising a polynucleotide encoding an antibody or antigen-binding fragment thereof described herein.

In one aspect, the disclosure is related to a vector comprising the nucleic acid described herein. In one aspect, the disclosure is related to a cell comprising the vector described herein.

In one aspect, the disclosure is related to a method of decreasing the rate of tumor growth, the method comprising contacting a tumor cell with an effective amount of a composition comprising the antibody or antigen-binding fragment thereof described herein.

In one aspect, the disclosure is related to a method of killing a tumor cell, the method comprising contacting a tumor cell with an effective amount of a composition comprising the antibody or antigen-binding fragment thereof described herein.

In one aspect, the disclosure is related to a method of increasing immune response in a subject, the method comprising administering to the subject an effective amount of a composition comprising the antibody or antigen-binding fragment thereof described herein.

In one aspect, the disclosure is related to a method of treating a subject having cancer, the method comprising administering a therapeutically effective amount of a composition comprising the antibody or antigen-binding fragment thereof described herein to the subject.

In some embodiments, the cancer is a solid tumor or a blood tumor. In some embodiments, the cancer is breast cancer, lung cancer, colorectal cancer, prostate cancer, ovarian cancer, esophageal cancer, tracheal cancer, gastric cancer bladder cancer, uterine cancer, rectal cancer, cancer of the small intestine, pancreatic cancer and/or liver cancer. In some embodiments, the cancer is multiple myeloma, B-cell lymphoma, diffuse Large B-Cell Lymphoma, acute B-cell leukemia, chronic lymphocytic leukemia, B-cell prelymphocytic leukemia, spleen with villous lymphocytes Lymphoma, hairy cell leukemia, follicular lymphoma, and/or mantle cell lymphoma. In some embodiments, the subject is further treated with an effective amount of an anti-4-1BB antibody, an anti-OX40 antibody, an anti-PD-1 antibody, an anti-CTLA4 antibody, an anti-CD40 antibody, and/or an anti-PD-L1 antibody.

In one aspect, the disclosure is related to a method of treating a subject having an autoimmune disease, the method comprising administering a therapeutically effective amount of a composition comprising the antibody or antigen-binding fragment thereof described herein to the subject. In some embodiments, the autoimmune disease is selected from rheumatoid arthritis, psoriasis, multiple sclerosis, immune thrombocytopenic purpura, myasthenia gravis, neuromyelitis optica, IgG4-related diseases, systemic Lupus Erythematosus, lupus nephritis, giant cell arteritis, takayasu disease, cold agglutinin disease, warm autoimmune hemolytic anemia, and anti-neutrophil cytoplasmic antibody (ANCA) associated vasculitides including for example, tranulomatosis with polyangiitis (GPA) (Wegener's Granulomatosis) and Microscopic Polyangiitis (MPA). In some embodiments, the autoimmune disease is multiple sclerosis, systemic lupus erythematosus and/or rheumatoid arthritis.

In one aspect, the disclosure is related to a pharmaceutical composition comprising the antibody or antigen-binding fragment thereof described herein, and a pharmaceutically acceptable carrier.

As used herein, the term "antigen-binding domain" refers to one or more protein domain(s) (e.g., formed from amino acids from a single polypeptide or formed from amino acids from two or more polypeptides (e.g., the same or different polypeptides) that is capable of specifically binding to one or more different antigen(s) (e.g., an effector antigen or tumor antigen). In some examples, an antigen-binding domain can bind to an antigen or epitope with specificity and affinity similar to that of naturally-occurring antibodies. In some embodiments, the antigen-binding domain can be an antibody or a fragment thereof. One example of an antigen-binding domain is an antigen-binding domain formed by VH-VL. In some embodiments, an antigen-binding domain can include an alternative scaffold. In some embodiments, the antigen-binding domain is a VHH. Non-limiting examples of antigen-binding domains are described herein. Additional examples of antigen-binding domains are known in the art. In some examples, an antigen-binding domain can bind to a single antigen (e.g., one of an effector antigen and a tumor antigen).

As used herein, the term "antibody" refers to any antigen-binding molecule that contains at least one (e.g., one, two, three, four, five, or six) complementary determining region (CDR) (e.g., any of the three CDRs from an immunoglobulin light chain or any of the three CDRs from an immunoglobulin heavy chain) and is capable of specifically binding to an antigen. Non-limiting examples of antibodies include: monoclonal antibodies, polyclonal antibodies, multi-specific antibodies (e.g., bi-specific antibodies), single-chain antibodies, chimeric antibodies, human antibodies, and humanized antibodies. In some embodiments, an antibody can contain an Fc region of a human antibody. The term antibody also includes derivatives, e.g., bi-specific antibodies, single-chain antibodies, diabodies, linear antibodies, and multi-specific antibodies formed from antibody fragments.

As used herein, the term "antigen-binding fragment" refers to a portion of a full-length antibody, wherein the portion of the antibody is capable of specifically binding to an antigen. In some embodiments, the antigen-binding fragment contains at least one variable domain (e.g., a variable domain of a heavy chain or a variable domain of light chain). Non-limiting examples of antibody fragments include, e.g., Fab, Fab', F(ab')₂, and Fv fragments.

As used herein, the term "multispecific antibody" is an antibody that includes two or more different antigen-binding domains that collectively specifically bind two or more different epitopes. The two or more different epitopes may be epitopes on the same antigen (e.g., a single polypeptide present on the surface of a cell) or on different antigens (e.g., different proteins present on the surface of the same cell or present on the surface of different cells). In some aspects, a multispecific antibody binds two different epitopes (i.e., a "bispecific antibody"). In some aspects, a multispecific antibody binds three different epitopes (i.e., a "trispecific antibody"). In some aspects, a multispecific antibody binds four different epitopes (i.e., a "quadspecific antibody"). In some aspects, a multispecific antibody binds five different epitopes (i.e., a "quintspecific antibody"). Each binding specificity may be present in any suitable valency. Non-limiting examples of multispecific antibodies are described herein.

As used herein, the term "bispecific antibody" refers to an antibody that binds to two different epitopes. The epitopes can be on the same antigen or on different antigens.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

### DESCRIPTION OF DRAWINGS

**FIG. 1A** shows the gating strategy to evaluate immune cell subsets in human PBMCs.
**FIG. 1B** shows CLEC5A expression in immune cell subsets in human PBMCs.
**FIG. 2A** shows the gating strategy utilized to evaluate immune cell subsets in human solid tumors.
**FIGs. 2B-2C** show CLEC5A expression in tumor-associated myeloid cells.
**FIG. 3A** shows the gating strategy utilized to characterize non-neutrophil myeloid cells.
**FIG. 3B** shows tumor-associated macrophages characterization in human solid tumors.
**FIGs. 3C-3D** show CLEC5A expression in tumor-associated macrophages in human solid tumors.
**FIG. 4** shows binding affinities of chimeric anti-CLEC5A antibodies to human macrophages.
**FIG. 5** shows TNFα release of M1 macrophages in chimeric anti-CLEC5A antibody-coated plates.
**FIG. 6** shows binding affinities of chimeric anti-CLEC5A antibodies to mouse CLEC5A.
**FIGs. 7A-7B** show BLI binding results of chimeric anti-CLEC5A antibodies to human CLEC5A.
**FIGs. 8A-8E** show humanized anti-CLEC5A antibodies binding to human CLEC5A, as determined by ELISA.
**FIGs. 9A-9F** show schematic illustrations of examples of TAA/CLEC5A bispecific antibodies.
**FIG. 10** shows HER2/CLEC5A bispecific antibodies binding to human macrophages.
**FIG. 11** shows TAA/CLEC5A bispecific antibodies binding to human macrophages.
**FIG. 12** shows CD79b /CLEC5A bispecific antibodies binding to human macrophages.
**FIG. 13** shows TAA/CLEC5A bispecific antibodies binding to human CLEC5A.
**FIG. 14** shows HER2/CLEC5A bispecific antibodies binding to human HER2.
**FIG. 15** shows EGFR/CLEC5A bispecific antibodies binding to human EGFR.
**FIG. 16** shows EpCAM/CLEC5A bispecific antibodies binding to DLD-1 cells.
**FIG. 17** shows CD79b/CLEC5A bispecific antibodies binding to Ramos cells.
**FIGs. 18A-18C** show HER2/CLEC5A bispecific antibody-mediated SK-BR-3 cell killing by M0 macrophages (E:T ratio of 5:1).
**FIG. 19** shows HER2/CLEC5A bispecific antibody-mediated SK-BR-3 cell killing by M1 and M2 macrophages (E:T ratio of 5:1).
**FIG. 20** shows IFN-γ release from HER2/CLEC5A antibody-mediated M1 and M2 macrophage killing of SK-BR-3 cells (E:T ratio of 5:1).
**FIGs. 21A-21C** show HER2/CLEC5A bispecific antibody-mediated SK-BR-3 cell killing by M0 macrophages in the presence of media (**FIG. 21A**), plasma (**FIG. 21B**), or hIgG1 (**FIG. 21C**).
**FIG. 22** shows EGFR/CLEC5A bispecific antibody-mediated DLD-1 cell killing by M0 macrophages.
**FIG. 23** shows EpCAM/CLEC5A bispecific antibody-mediated DLD-1 cell killing by M0 macrophages.
**FIG. 24** shows EpCAM expression level on various cancer cell lines.
**FIGs. 25A-25F** show EpCAM/CLEC5A bispecific antibodies mediated cancer cell killing by M0 macrophages.
**FIG. 26** shows different myeloid cell engagers mediated multiple myeloma cancer cell killing by PBMCs.
**FIG. 27** shows CD79b expression level on malignant lymphoma cell lines and B cell in healthy PBMC donors.
**FIGs. 28A-28C** show CD79b/CLEC5A bispecific antibody-mediated Ramos cell killing by PBMCs (test 1).
**FIGs. 29A-29B** show CD79b/CLEC5A bispecific antibody-mediated Ramos cell killing by PBMCs (test 2).
**FIGs. 30A-30C** show CD79b/CLEC5A bispecific antibody-mediated Ramos cell killing by PBMCs (test 3).
**FIGs. 31A-31B** show IL-6 level from CD79b/CLEC5A bispecific antibody-mediated killing of Ramos cells.
**FIG. 32** shows TNFα level from CD79b/CLEC5A bispecific antibody-mediated killing of Ramos cells.
**FIGs. 33A-33C** show CD79b/CLEC5A bispecific antibody-mediated endogenous B cell killing by PBMCs (test 1).
**FIGs. 34A-34C** show CD79b/CLEC5A bispecific antibody-mediated endogenous B cell killing by PBMCs (test 2).
**FIGs. 35A-35B** show CD79b/CLEC5A bispecific antibody-mediated endogenous B cell killing by PBMCs (test 3).
**FIG. 36** shows TNFα release from CD79b/CLEC5A bispecific antibody-mediated PBMC killing of endogenous B cells.
**FIGs. 37A-37F** show CD79b/CLEC5A bispecific antibody-mediated Ramos cell killing by M0 macrophages (test 1).
**FIGs. 38A-38D** show cytokine release from CD79b/CLEC5A bispecific antibody-mediated M0 macrophage killing of Ramos cells (test 1).
**FIGs. 39A-39B** show CD79b/CLEC5A bispecific antibody-mediated Ramos cell killing by M0 macrophages at different E:T ratios (test 2).
**FIGs. 40A-40B** show cytokine release from CD79b/CLEC5A bispecific antibody-mediated M0 macrophage killing of Ramos cells (test 2).
**FIGs. 41A-41C** show CD79b/CLEC5A bispecific antibody-mediated Ramos cell killing by M0 macrophages (test 3).
**FIG. 42** shows cytokine release from CD79b/CLEC5A bispecific antibody-mediated M0 macrophage killing of Ramos cells (test 3).
**FIG. 43** shows M0 macrophages survival rate from CD79b/CLEC5A bispecific antibody-mediated M0 macrophage killing of Ramos cells (test 3).
**FIGs. 44A-44B** show CD79b/CLEC5A bispecific antibody-mediated Daudi cell killing by M0 macrophages at different E:T ratios (test 1).
**FIGs. 45A-45B** show CD79b/CLEC5A bispecific antibody-mediated Daudi cell killing by M0 macrophages at different E:T ratios (test 2).
**FIGs. 46A-46D** show cytokine release from CD79b/CLEC5A bispecific antibody-mediated M0 macrophage killing of Daudi cells.
**FIGs. 47A-47B** show CD79b/CLEC5A bispecific antibody-mediated Ramos cell killing by M1 macrophages (test 1).
**FIGs. 48A-48B** show cytokine release from CD79b/CLEC5A bispecific antibody-mediated M1 macrophage killing of Ramos cells (test 1).
**FIG. 49** shows CD79b/CLEC5A bispecific antibody-mediated Ramos cell killing by M1 macrophages (test 2).
**FIG. 50** shows cytokine release from CD79b/CLEC5A bispecific antibody- mediated M1 macrophage killing of Ramos cells (test 2).
**FIG. 51** shows M1 macrophages survival rate from CD79b/CLEC5A bispecific antibody-mediated M1 macrophage killing of Ramos cells (test 2).
**FIG. 52** shows CD79b/CLEC5A bispecific antibody-mediated Daudi cell killing by M1 macrophages.
**FIG. 53** shows cytokine release from CD79b/CLEC5A bispecific antibody-mediated M1 macrophage killing of Daudi cells.
**FIGs. 54A-54D** show CD79b/CLEC5A bispecific antibody-mediated Ramos cell killing by M2 macrophages (test 1).
**FIGs. 55A-55D** show cytokine release from CD79b/CLEC5A bispecific antibody-mediated M2 macrophage killing of Ramos cells (test 1).
**FIG. 56** shows CD79b/CLEC5A bispecific antibody-mediated Ramos cell killing by M2 macrophages (test 2).
**FIG. 57** shows cytokine release from CD79b/CLEC5A bispecific antibody-mediated M2 macrophage killing of Ramos cells (test 2).
**FIG. 58** shows M2 macrophages survival rate from CD79b/CLEC5A bispecific antibody-mediated M2 macrophage killing of Ramos cells (test 2).
**FIG. 59** shows CD79b/CLEC5A bispecific antibody-mediated Daudi cell killing by M2 macrophages at different E:T ratios.
**FIG. 60** shows cytokine release from CD79b/CLEC5A bispecific antibody-mediated M2 macrophage killing of Daudi cells.
**FIGs. 61A-61B** show CD79b/CLEC5A bispecific antibody-mediated Ramos cell killing by monocytes.
**FIGs. 62A-62B** show cytokine release from CD79b/CLEC5A bispecific antibody-mediated monocytes killing of Ramos cells.
**FIGs. 63A-63B** show monocytes survival rate from CD79b/CLEC5A bispecific antibody-mediated monocytes killing of Ramos cells.
**FIGs. 64A-64B** show CD79b/CLEC5A bispecific antibody-mediated B cell killing by monocytes.
**FIGs. 65A-65B** show cytokine release from CD79b/CLEC5A bispecific antibody-mediated monocytes killing of B cells.
**FIGs. 66A-66B** show monocytes survival rate from CD79b/CLEC5A bispecific antibody-mediated monocytes killing of B cells.

### DETAILED DESCRIPTION

A multispecific antibody or antigen-binding fragment thereof is an artificial protein that can simultaneously bind to two or more than two different epitopes (e.g., on two different antigens). In some embodiments, the multispecific antibody is a bispecific antibody. A bispecific antibody or antigen-binding fragment thereof can have two arms. Each arm can have one heavy chain variable region and one light chain variable region, forming an antigen-binding domain (or an antigen-binding region). The two arms can bind to two different antigens. In some embodiments, additional antigen-binding domains can be added to a monoclonal antibody (e.g., to the C terminus of the light chain or the heavy chain).

The present disclosure provides several anti-CLEC5A antibodies, antigen-binding fragments thereof, and methods of using these anti-CLEC5A antibodies and antigen-binding fragments to inhibit tumor growth, treat cancers, and to treat autoimmune diseases.

This disclosure relates to multispecific (e.g., bispecific) antibodies or antigen-binding fragments thereof, comprising a first antigen-binding domain that specifically binds to a tumor associated antigen (TAA) and a second antigen-binding domain that specifically binds to CLEC5A.

### Human C-type Lectin Domain Family 5 Member A (CLEC5A)

Human C-type lectin domain family 5 member A (CLEC5A), also known as myeloid DAP12-associating lectin-1 (MDL-1), is a type-II transmembrane protein. C-type lectins are characterized by a common structural C-type lectin domain (CTLD) that can bind glycan and non-glycan ligands in a Ca²⁺-independent manner. CTLDs that bind glycans in Ca²⁺-dependent manner are known as "carbohydrate recognition domains" (CRD). The myeloid C-type lectin CLEC5A is a spleen tyrosine kinase (Syk)-coupled type-II membrane protein comprising a C-terminal CTLD and a short N-terminal cytoplasmic domain. Among the 15 groups of C-type lectins, CLEC5A falls into Group V (the NK cell receptor family), which includes CLEC7A (Dectin-1), CLEC5A, CLEC2, CLEC1, NK receptors (such as NKG2D, the NKRP1 family, the NKG2 family, CD69 and CD94), mast cell-associated functional antigen (MAFA), osteoclast inhibitory lectin (OCIL), and CD72. Similar to NKG2D, CLEC5A signals via the ITAM-containing DNAX-activating protein 12 (DAP12) when it is phosphorylated by Src upon activation.

The human CLEC5A mRNA encodes a 165-residue polypeptide with an N-terminal signal peptide (a.a. 1-22), followed by a short intracellular cytoplasmic domain (a.a. 23-56), a transmembrane domain (a.a. 57-70) and an extracellular domain (a.a. 71-165). The transmembrane domain contains a positively charged amino acid, Lys-58, which recruits DAP10 and DAP12 to associate with CLEC5A after activation. CLEC5A is mainly expressed by myeloid cells, including monocytes, macrophages, neutrophils, and dendritic cells, and is further upregulated by interferon-gamma (IFN-γ). In addition, CLEC5A expression is under the control of the PU.1 transcription factor, which is a central regulator of myeloid cell differentiation. CLEC5A expression is upregulated by the nuclear factor erythroid 2-related factor 2 (Nrf2), suggesting CLEC5A is regulated by oxidative stress.

An X-ray crystal structure has revealed that CLEC5A is a homodimeric protein when it binds to dengue virus serotype. Moreover, the CLEC5A crystal structure revealed conformational flexibility, suggesting that CLEC5A can adopt various conformations in vivo and that its conformation is ligand-dependent.

While NK receptors recognize stress-associated autologous antigens and are crucial for immunosurveillance, group V spleen tyrosine kinase (Syk)-coupled C-type lectins in myeloid cells recognize diverse exogenous and endogenous antigens and are involved in host defense, aseptic inflammation, platelet activation and development. CLEC5A was shown to interact with glycan moieties on dengue virus (DV), Japanese encephalitis virus (JEV) and type A influenza virus (IAV). In addition, CLEC5A has been found to interact with N-acetylglucosamine (GlcNAc) and N-acetylmuramic acid (MurNAc) disaccharides on gram-positive bacteria (Listeria monocytogenes and Staphylococcus aureus). Moreover, CLEC5A has a critical role in the Inflammatory responses associated with collagen-induced rheumatoid arthritis and Concanavalin A-induced liver inflammation. CLEC5A also interacts with exosomes released from activated platelets.

A detailed review of CLEC5A and its functions can be found in Sung, Pei-Shan, Wei-Chiao Chang, and Shie-Liang Hsieh."CLEC5A: a promiscuous pattern recognition receptor to microbes and beyond" Lectin in Host Defense Against Microbial Infections (2020): 57-73; Chen, Rui, et al., "A pan-cancer analysis reveals CLEC5A as a biomarker for cancer immunity and prognosis" Frontiers in Immunology 13 (2022): 831542; and Wang, Quhui, et al., "CLEC5A promotes the proliferation of gastric cancer cells by activating the PI3K/AKT/mTOR pathway" Biochemical and Biophysical Research Communications 524.3 (2020): 656-662; each of which is incorporated by reference in its entirety.

### Anti-CLEC5A Antibodies and Antigen-Binding Fragments

The disclosure provides antibodies and antigen-binding fragments thereof that specifically bind to CLEC5A (e.g., human CLEC5A). The antibodies and antigen-binding fragments described herein are capable of binding to CLEC5A. These antibodies can be agonists or antagonists to CLEC5A mediated signaling. In some embodiments, the antibodies and antigen-binding fragments can bind to the extracellular domains of human CLEC5A.

The disclosure provides e.g., anti-CLEC5A antibodies 6A5, 6G9, 14A2, 5C7, 7G10, 3A7, 13E6, 9E11, the chimeric antibodies thereof, and the humanized antibodies thereof.

The CDR sequences for 3A7, and 3A7 derived antibodies include CDRs of the heavy chain variable domain, SEQ ID NOs: 3, 5, 7, and CDRs of the light chain variable domain, SEQ ID NOs: 8-10, as defined by Kabat definition. Under Chothia definition, the CDR sequences of the heavy chain variable domain are set forth in SEQ ID NOs: 4, 6, 7, and CDRs of the light chain variable domain are set forth in SEQ ID NOs: 8-10.

The CDR sequences for 5C7, and 5C7 derived antibodies include CDRs of the heavy chain variable domain, SEQ ID NOs: 13, 15, 17, and CDRs of the light chain variable domain, SEQ ID NOs: 18-20, as defined by Kabat definition. Under Chothia definition, the CDR sequences of the heavy chain variable domain are set forth in SEQ ID NOs: 14, 16, 17, and CDRs of the light chain variable domain are set forth in SEQ ID NOs: 18-20.

The CDR sequences for 6A5, and 6A5 derived antibodies (e.g., humanized antibodies) include CDRs of the heavy chain variable domain, SEQ ID NOs: 23, 25, 27, and CDRs of the light chain variable domain, SEQ ID NOs: 28-30 as defined by Kabat definition. The CDRs can also be defined by Chothia definition. Under the Chothia definition, the CDR sequences of the heavy chain variable domain are set forth in SEQ ID NOs: 24, 26, 27 and CDR sequences of the light chain variable domain are set forth in SEQ ID NOs: 28-30.

The CDR sequences for 6G9, and 6G9 derived antibodies include CDRs of the heavy chain variable domain, SEQ ID NOs: 33, 35, 37, and CDRs of the light chain variable domain, SEQ ID NOs: 38-40, as defined by Kabat definition. Under Chothia definition, the CDR sequences of the heavy chain variable domain are set forth in SEQ ID NOs: 34, 36, 37, and CDRs of the light chain variable domain are set forth in SEQ ID NOs: 38-40.

The CDR sequences for 7G10, and 7G10 derived antibodies include CDRs of the heavy chain variable domain, SEQ ID NOs: 43, 45, 47, and CDRs of the light chain variable domain, SEQ ID NOs: 48-50, as defined by Kabat definition. Under Chothia definition, the CDR sequences of the heavy chain variable domain are set forth in SEQ ID NOs: 44, 46, 47, and CDRs of the light chain variable domain are set forth in SEQ ID NOs: 48-50.

The CDR sequences for 9E11, and 9E11 derived antibodies include CDRs of the heavy chain variable domain, SEQ ID NOs: 53, 55, 57, and CDRs of the light chain variable domain, SEQ ID NOs: 58-60, as defined by Kabat definition. Under Chothia definition, the CDR sequences of the heavy chain variable domain are set forth in SEQ ID NOs: 54, 56, 57, and CDRs of the light chain variable domain are set forth in SEQ ID NOs: 58-60.

The CDR sequences for 13E6, and 13E6 derived antibodies include CDRs of the heavy chain variable domain, SEQ ID NOs: 63, 65, 67, and CDRs of the light chain variable domain, SEQ ID NOs: 68-70, as defined by Kabat definition. Under Chothia definition, the CDR sequences of the heavy chain variable domain are set forth in SEQ ID NOs: 64, 66, 67, and CDRs of the light chain variable domain are set forth in SEQ ID NOs: 68-70.

The CDR sequences for 14A2, and 14A2 derived antibodies include CDRs of the heavy chain variable domain, SEQ ID NOs: 73, 75, 77, and CDRs of the light chain variable domain, SEQ ID NOs: 78-80, as defined by Kabat definition. Under Chothia definition, the CDR sequences of the heavy chain variable domain are set forth in SEQ ID NOs: 74, 76, 77, and CDRs of the light chain variable domain are set forth in SEQ ID NOs: 78-80.

The amino acid sequence for the heavy chain variable region of 3A7 antibody is set forth in SEQ ID NO: 11. The amino acid sequence for the light chain variable region of 3A7 antibody is set forth in SEQ ID NO: 12.

The amino acid sequence for the heavy chain variable region of 5C7 antibody is set forth in SEQ ID NO: 21. The amino acid sequence for the light chain variable region of 5C7 antibody is set forth in SEQ ID NO: 22.

The amino acid sequence for the heavy chain variable region of 6A5 antibody is set forth in SEQ ID NO: 31. The amino acid sequence for the light chain variable region of 6A5 antibody is set forth in SEQ ID NO: 32.

The amino acid sequence for the heavy chain variable region of 6G9 antibody is set forth in SEQ ID NO: 41. The amino acid sequence for the light chain variable region of 6G9 antibody is set forth in SEQ ID NO: 42.

The amino acid sequence for the heavy chain variable region of 7G10 antibody is set forth in SEQ ID NO: 51. The amino acid sequence for the light chain variable region of 7G10 antibody is set forth in SEQ ID NO: 52.

The amino acid sequence for the heavy chain variable region of 9E11 antibody is set forth in SEQ ID NO: 61. The amino acid sequence for the light chain variable region of 9E11 antibody is set forth in SEQ ID NO: 62.

The amino acid sequence for the heavy chain variable region of 13E6 antibody is set forth in SEQ ID NO: 71. The amino acid sequence for the light chain variable region of 13E6 antibody is set forth in SEQ ID NO: 72.

The amino acid sequence for the heavy chain variable region of 14A2 antibody is set forth in SEQ ID NO: 81. The amino acid sequence for the light chain variable region of 14A2 antibody is set forth in SEQ ID NO: 82.

Humanization percentage means the percentage identity of the heavy chain or light chain variable region sequence as compared to human antibody sequences in International Immunogenetics Information System (IMGT) database. In some embodiments, humanization percentage is greater than 80%, 81%, 82%, 83%, 84%, 85%, 86%, 87%, 88%, 89%, 90%, 91%, 92%, 93%, 94%, or 95%. A detailed description regarding how to determine humanization percentage and how to determine top hits is known in the art, and is described, e.g., in Jones, et al. "The INNs and outs of antibody nonproprietary names." MAbs. Vol. 8. No. 1. Taylor & Francis, 2016, which is incorporated herein by reference in its entirety. A high humanization percentage often has various advantages, e.g., more safe and more effective in humans, more likely to be tolerated by a human subject, and/or less likely to have side effects. In some embodiments, the variable regions are fully human, e.g., derived from human heavy chain immunoglobulin locus sequences (e.g., recombination of human IGHV, human IGHD, and human IGHJ genes), and/or human kappa chain immunoglobulin locus sequences (e.g., recombination of human IGKV and human IGKJ genes).

The amino acid sequence for the heavy chain variable region of humanized 3A7 antibody is set forth in SEQ ID NO: 83. The amino acid sequence for the light chain variable region of humanized 3A7 antibody is set forth in SEQ ID NO: 84.

The amino acid sequence for the heavy chain variable region of humanized 5C7 antibody is set forth in SEQ ID NO: 85. The amino acid sequence for the light chain variable region of humanized 5C7 antibody is set forth in SEQ ID NO: 86.

The amino acid sequence for the heavy chain variable region of humanized 6A5 antibody is set forth in SEQ ID NO: 87. The amino acid sequence for the light chain variable region of humanized 6A5 antibody is set forth in SEQ ID NO: 88.

The amino acid sequence for the heavy chain variable region of humanized 7G10 antibody is set forth in SEQ ID NO: 89. The amino acid sequence for the light chain variable region of humanized 7G10 antibody is set forth in SEQ ID NO: 90.

The amino acid sequence for the heavy chain variable region of humanized 13E6 antibody is set forth in SEQ ID NO: 91. The amino acid sequence for the light chain variable region of humanized 13E6 antibody is set forth in SEQ ID NO: 92.

The amino acid sequences for heavy chain variable regions and light variable regions of the modified antibodies are also provided. In some embodiments, the heavy chain variable region is at least 80%, 85%, 90%, 91%, 92%, 93%, 94%, 95%, 96%, 97%, 98%, or 99% identical to any one of SEQ ID NOs: 11, 21, 31, 41, 51, 61, 71, 81, 83, 85, 87, 89, and 91. In some embodiments, the light chain variable region is at least 80%, 85%, 90%, 91%, 92%, 93%, 94%, 95%, 96%, 97%, 98%, or 99% identical to any one of SEQ ID NOs: 12, 22, 32, 42, 52, 62, 72, 82, 84, 86, 88, 90, and 92. The heavy chain variable region sequence can be paired with the corresponding light chain variable region sequence, and together they bind to CLEC5A.

Furthermore, in some embodiments, the antibodies or antigen-binding fragments thereof described herein can also contain one, two, or three heavy chain variable region CDRs selected from the group of SEQ ID NOs: 3, 5, 7, SEQ ID NOs: 4, 6, 7, SEQ ID NOs: 13, 15, 17, SEQ ID NOs: 14, 16, 17, SEQ ID NOs: 23, 25, 27, SEQ ID NOs: 24, 26, 27, SEQ ID NOs: 33, 35, 37, SEQ ID NOs: 34, 36, 37, SEQ ID NOs: 43, 45, 47, SEQ ID NOs: 44, 46, 47, SEQ ID NOs: 53, 55, 57, SEQ ID NOs: 54, 56, 57, SEQ ID NOs: 63, 65, 67, SEQ ID NOs: 64, 66, 67, SEQ ID NOs: 73, 75, 77, and SEQ ID NOs: 74, 76, 77; and/or one, two, or three light chain variable region CDRs selected from the group of SEQ ID NOs: 8-10, SEQ ID NOs: 18-20, SEQ ID NOs: 28-30, SEQ ID NOs: 38-40, SEQ ID NOs: 48-50, SEQ ID NOs: 58-60, SEQ ID NOs: 68-70, and SEQ ID NOs: 78-80.

In some embodiments, the antibodies can have a heavy chain variable region (VH) comprising complementarity determining regions (CDRs) 1, 2, 3, wherein the CDR1 region comprises or consists of an amino acid sequence that is at least 80%, 85%, 90%, or 95% identical to a selected VH CDR1 amino acid sequence, the CDR2 region comprises or consists of an amino acid sequence that is at least 80%, 85%, 90%, or 95% identical to a selected VH CDR2 amino acid sequence, and the CDR3 region comprises or consists of an amino acid sequence that is at least 80%, 85%, 90%, or 95% identical to a selected VH CDR3 amino acid sequence. In some embodiments, the antibody can have a light chain variable region (VL) comprising CDRs 1, 2, 3, wherein the CDR1 region comprises or consists of an amino acid sequence that is at least 80%, 85%, 90%, or 95% identical to a selected VL CDR1 amino acid sequence, the CDR2 region comprises or consists of an amino acid sequence that is at least 80%, 85%, 90%, or 95% identical to a selected VL CDR2 amino acid sequence, and the CDR3 region comprises or consists of an amino acid sequence that is at least 80%, 85%, 90%, or 95% identical to a selected VL CDR3 amino acid sequence. The selected VH CDRs 1, 2, 3 amino acid sequences and the selected VL CDRs, 1, 2, 3 amino acid sequences are shown in **Table 22**.

In some embodiments, the antibody or an antigen-binding fragment described herein can contain a heavy chain variable domain containing one, two, or three of the CDRs of SEQ ID NO: 3 with zero, one or two amino acid insertions, deletions, or substitutions; SEQ ID NO: 5 with zero, one or two amino acid insertions, deletions, or substitutions; SEQ ID NO: 7 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 4 with zero, one or two amino acid insertions, deletions, or substitutions; SEQ ID NO: 6 with zero, one or two amino acid insertions, deletions, or substitutions; SEQ ID NO: 7 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 13 with zero, one or two amino acid insertions, deletions, or substitutions; SEQ ID NO: 15 with zero, one or two amino acid insertions, deletions, or substitutions; SEQ ID NO: 17 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 14 with zero, one or two amino acid insertions, deletions, or substitutions; SEQ ID NO: 16 with zero, one or two amino acid insertions, deletions, or substitutions; SEQ ID NO: 17 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 23 with zero, one or two amino acid insertions, deletions, or substitutions; SEQ ID NO: 25 with zero, one or two amino acid insertions, deletions, or substitutions; SEQ ID NO: 27 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 24 with zero, one or two amino acid insertions, deletions, or substitutions; SEQ ID NO: 26 with zero, one or two amino acid insertions, deletions, or substitutions; SEQ ID NO: 27 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 33 with zero, one or two amino acid insertions, deletions, or substitutions; SEQ ID NO: 35 with zero, one or two amino acid insertions, deletions, or substitutions; SEQ ID NO: 37 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 34 with zero, one or two amino acid insertions, deletions, or substitutions; SEQ ID NO: 36 with zero, one or two amino acid insertions, deletions, or substitutions; SEQ ID NO: 37 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 43 with zero, one or two amino acid insertions, deletions, or substitutions; SEQ ID NO: 45 with zero, one or two amino acid insertions, deletions, or substitutions; SEQ ID NO: 47 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 44 with zero, one or two amino acid insertions, deletions, or substitutions; SEQ ID NO: 46 with zero, one or two amino acid insertions, deletions, or substitutions; SEQ ID NO: 47 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 53 with zero, one or two amino acid insertions, deletions, or substitutions; SEQ ID NO: 55 with zero, one or two amino acid insertions, deletions, or substitutions; SEQ ID NO: 57 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 54 with zero, one or two amino acid insertions, deletions, or substitutions; SEQ ID NO: 56 with zero, one or two amino acid insertions, deletions, or substitutions; SEQ ID NO: 57 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 63 with zero, one or two amino acid insertions, deletions, or substitutions; SEQ ID NO: 65 with zero, one or two amino acid insertions, deletions, or substitutions; SEQ ID NO: 67 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 64 with zero, one or two amino acid insertions, deletions, or substitutions; SEQ ID NO: 66 with zero, one or two amino acid insertions, deletions, or substitutions; SEQ ID NO: 67 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 73 with zero, one or two amino acid insertions, deletions, or substitutions; SEQ ID NO: 75 with zero, one or two amino acid insertions, deletions, or substitutions; SEQ ID NO: 77 with zero, one or two amino acid insertions, deletions, or substitutions.

In some embodiments, the antibody or an antigen-binding fragment described herein can contain a heavy chain variable domain containing one, two, or three of the CDRs of SEQ ID NO: 74 with zero, one or two amino acid insertions, deletions, or substitutions; SEQ ID NO: 76 with zero, one or two amino acid insertions, deletions, or substitutions; SEQ ID NO: 77 with zero, one or two amino acid insertions, deletions, or substitutions.

In some embodiments, the antibody or an antigen-binding fragment described herein can contain a light chain variable domain containing one, two, or three of the CDRs of SEQ ID NO: 8 with zero, one or two amino acid insertions, deletions, or substitutions; SEQ ID NO: 9 with zero, one or two amino acid insertions, deletions, or substitutions; SEQ ID NO: 10 with zero, one or two amino acid insertions, deletions, or substitutions.

In some embodiments, the antibody or an antigen-binding fragment described herein can contain a light chain variable domain containing one, two, or three of the CDRs of SEQ ID NO: 18 with zero, one or two amino acid insertions, deletions, or substitutions; SEQ ID NO: 19 with zero, one or two amino acid insertions, deletions, or substitutions; SEQ ID NO: 20 with zero, one or two amino acid insertions, deletions, or substitutions.

In some embodiments, the antibody or an antigen-binding fragment described herein can contain a light chain variable domain containing one, two, or three of the CDRs of SEQ ID NO: 28 with zero, one or two amino acid insertions, deletions, or substitutions; SEQ ID NO: 29 with zero, one or two amino acid insertions, deletions, or substitutions; SEQ ID NO: 30 with zero, one or two amino acid insertions, deletions, or substitutions.

In some embodiments, the antibody or an antigen-binding fragment described herein can contain a light chain variable domain containing one, two, or three of the CDRs of SEQ ID NO: 38 with zero, one or two amino acid insertions, deletions, or substitutions; SEQ ID NO: 39 with zero, one or two amino acid insertions, deletions, or substitutions; SEQ ID NO: 40 with zero, one or two amino acid insertions, deletions, or substitutions.

In some embodiments, the antibody or an antigen-binding fragment described herein can contain a light chain variable domain containing one, two, or three of the CDRs of SEQ ID NO: 48 with zero, one or two amino acid insertions, deletions, or substitutions; SEQ ID NO: 49 with zero, one or two amino acid insertions, deletions, or substitutions; SEQ ID NO: 50 with zero, one or two amino acid insertions, deletions, or substitutions.

In some embodiments, the antibody or an antigen-binding fragment described herein can contain a light chain variable domain containing one, two, or three of the CDRs of SEQ ID NO: 58 with zero, one or two amino acid insertions, deletions, or substitutions; SEQ ID NO: 59 with zero, one or two amino acid insertions, deletions, or substitutions; SEQ ID NO: 60 with zero, one or two amino acid insertions, deletions, or substitutions.

In some embodiments, the antibody or an antigen-binding fragment described herein can contain a light chain variable domain containing one, two, or three of the CDRs of SEQ ID NO: 68 with zero, one or two amino acid insertions, deletions, or substitutions; SEQ ID NO: 69 with zero, one or two amino acid insertions, deletions, or substitutions; SEQ ID NO: 70 with zero, one or two amino acid insertions, deletions, or substitutions.

In some embodiments, the antibody or an antigen-binding fragment described herein can contain a light chain variable domain containing one, two, or three of the CDRs of SEQ ID NO: 78 with zero, one or two amino acid insertions, deletions, or substitutions; SEQ ID NO: 79 with zero, one or two amino acid insertions, deletions, or substitutions; SEQ ID NO: 80 with zero, one or two amino acid insertions, deletions, or substitutions.

The insertions, deletions, and substitutions can be within the CDR sequence, or at one or both terminal ends of the CDR sequence. In some embodiments, the CDR is determined based on Kabat definition. In some embodiments, the CDR is determined based on Chothia definition. In some embodiments, the CDR is determined based on a combination of Kabat definition and Chothia definition.

The disclosure also provides antibodies or antigen-binding fragments thereof that bind to CLEC5A. The antibodies or antigen-binding fragments thereof contain a heavy chain variable region (VH) comprising or consisting of an amino acid sequence that is at least 80%, 85%, 90%, or 95% identical to a selected VH sequence, and a light chain variable region (VL) comprising or consisting of an amino acid sequence that is at least 80%, 85%, 90%, or 95% identical to a selected VL sequence. In some embodiments, the selected VH sequence is SEQ ID NO: 11, and the selected VL sequence is SEQ ID NO: 12. In some embodiments, the selected VH sequence is SEQ ID NO: 21 and the selected VL sequence is SEQ ID NO: 22. In some embodiments, the selected VH sequence is SEQ ID NO: 31, and the selected VL sequence is SEQ ID NO: 32. In some embodiments, the selected VH sequence is SEQ ID NO: 41, and the selected VL sequence is SEQ ID NO: 42. In some embodiments, the selected VH sequence is SEQ ID NO: 51, and the selected VL sequence is SEQ ID NO: 52. In some embodiments, the selected VH sequence is SEQ ID NO: 61, and the selected VL sequence is SEQ ID NO: 62. In some embodiments, the selected VH sequence is SEQ ID NO: 71, and the selected VL sequence is SEQ ID NO: 72. In some embodiments, the selected VH sequence is SEQ ID NO: 81, and the selected VL sequence is SEQ ID NO: 82. In some embodiments, the selected VH sequence is SEQ ID NO: 83, and the selected VL sequence is SEQ ID NO: 84. In some embodiments, the selected VH sequence is SEQ ID NO: 85, and the selected VL sequence is SEQ ID NO: 86. In some embodiments, the selected VH sequence is SEQ ID NO: 87, and the selected VL sequence is SEQ ID NO: 88. In some embodiments, the selected VH sequence is SEQ ID NO: 89, and the selected VL sequence is SEQ ID NO: 90. In some embodiments, the selected VH sequence is SEQ ID NO: 91, and the selected VL sequence is SEQ ID NO: 92.

The disclosure also provides antibodies or antigen-binding fragments thereof that can compete with the antibodies described herein. In some aspects, the antibodies or antigen-binding fragments can bind to the same epitope as the antibodies described herein.

The present disclosure also provides an antibody or antigen-binding fragment thereof that cross-competes with any antibody or antigen-binding fragment as described herein. The cross-competing assay is known in the art, and is described e.g., in Moore et al., "Antibody cross-competition analysis of the human immunodeficiency virus type 1 gp120 exterior envelope glycoprotein." Journal of virology 70.3 (1996): 1863-1872, which is incorporated herein reference in its entirety. In one aspect, the present disclosure also provides an antibody or antigen-binding fragment thereof that binds to the same epitope or region as any antibody or antigen-binding fragment as described herein. The epitope binning assay is known in the art, and is described e.g., in Estep et al. "High throughput solution-based measurement of antibody-antigen affinity and epitope binning." MAbs. Vol. 5. No. 2. Taylor & Francis, 2013, which is incorporated herein reference in its entirety.

The disclosure also provides nucleic acid comprising a polynucleotide encoding a polypeptide comprising an immunoglobulin heavy chain or an immunoglobulin light chain. The immunoglobulin heavy chain or immunoglobulin light chain comprises CDRs or have sequences as shown in **Table 22**. When the polypeptides are paired with corresponding polypeptide (e.g., a corresponding heavy chain variable region or a corresponding light chain variable region), the paired polypeptides bind to CLEC5A (e.g., human CLEC5A).

The anti-CLEC5A antibodies and antigen-binding fragments can also be antibody variants (including derivatives and conjugates) of antibodies or antibody fragments and multi-specific (e.g., bi-specific) antibodies or antibody fragments. Additional antibodies provided herein are polyclonal, monoclonal, multimeric, multi-specific (e.g., bi-specific), humanized antibodies, chimeric antibodies (e.g., human-mouse chimera), single-chain antibodies, intracellularly-made antibodies (i.e., intrabodies), and antigen-binding fragments thereof. The antibodies or antigen-binding fragments thereof can be of any type (e.g., IgG, IgE, IgM, IgD, IgA, and IgY), class (e.g., IgG1, IgG2, IgG3, IgG4, IgA1, and IgA2), or subclass. In some embodiments, the antibody or antigen-binding fragment thereof is an IgG antibody or antigen-binding fragment thereof.

Fragments of antibodies are suitable for use in the methods provided so long as they retain the desired affinity and specificity of the full-length antibody. Thus, a fragment of an antibody that binds to CLEC5A will retain an ability to bind to CLEC5A. An Fv fragment is an antibody fragment which contains a complete antigen recognition and binding site. This region consists of a dimer of one heavy and one light chain variable domain in tight association, which can be covalent in nature, for example in scFv. It is in this configuration that the three CDRs of each variable domain interact to define an antigen binding site on the surface of the VH-VL dimer. Collectively, the six CDRs or a subset thereof confer antigen binding specificity to the antibody. However, even a single variable domain (or half of an Fv comprising only three CDRs specific for an antigen) can have the ability to recognize and bind antigen, although usually at a lower affinity than the entire binding site.

Single-chain Fv or scFv antibody fragments comprise the VH and VL domains (or regions) of antibody, wherein these domains are present in a single polypeptide chain. Generally, the scFv polypeptide further comprises a polypeptide linker between the VH and VL domains, which enables the scFv to form the desired structure for antigen binding.

The Fab fragment contains a variable and constant domain of the light chain and a variable domain and the first constant domain (CH1) of the heavy chain. F(ab')2 antibody fragments comprise a pair of Fab fragments which are generally covalently linked near their carboxy termini by hinge cysteines between them. Other chemical couplings of antibody fragments are also known in the art.

Antibodies and antibody fragments of the present disclosure can be modified in the Fc region to provide desired effector functions or serum half-life. In some embodiments, the Fc region can be modified to silence or decrease complement-dependent cytotoxicity (CDC) or antibody-dependent cellular cytotoxicity (ADCC).

In some embodiments, the antibody or antigen-binding fragment thereof described herein recognizes an endogenous CLEC5A or a recombinant CLEC5A. In some embodiments, the antibody or antigen-binding fragment thereof described herein recognizes human CLEC5A (e.g., the extracellular region of human CLEC5A).

### Multispecific Antibodies or Antigen-Binding Fragments Thereof

In one aspect, provided herein are antibodies or antigen-binding fragments thereof, comprising: a first antigen-binding domain that specifically binds to a first antigen, wherein the first antigen is a tumor associated antigen (TAA); and a second antigen-binding domain that specifically binds to C-type lectin domain family 5 member A (CLEC5A). In some embodiments, the antibody or antigen-binding fragments thereof comprises a fragment crystallizable region (Fc region). In some embodiments, the antibody or antigen-binding fragments thereof is a bispecific antibody.

In some embodiments, the bispecific antibodies or antigen-binding fragments thereof (e.g., anti-TAA/CLEC5A antibodies) specifically bind to a tumor associated antigen (e.g., HER2) and CLEC5A, and such bispecific antibodies have modified or enhanced Fc regions (e.g., an Fc region with the GAALIE mutations, LALAPG mutations, S293D+I332E mutations, knobs-into-holes (KIH) mutations or afucosylation).

In some embodiments, the antibody or antigen-binding fragment thereof described herein comprises a first heavy chain comprising a first heavy chain variable region (VH1) and a first light chain comprising a first light chain variable region (VL1); and a second heavy chain comprising a second heavy chain variable region (VH2) and a second light chain comprising a second light chain variable region (VL2).

In some embodiments, the first antigen-binding domain is a human or humanized antigen-binding domain; and/or the second antigen-binding domain is a human or humanized antigen-binding domain.

In some embodiments, the first antigen-binding domain is a single-chain variable fragment (scFV) or VHH. In some embodiments, the second antigen-binding domain is an scFv or VHH.

In some embodiments, the multispecific antibody described herein is designed to have an Fc region that comprises the LALAPG mutations: an Alanine (A) at position 234; an Alanine (A) at position 235; and a Glycine (G) at position 329 in EU numbering. In some embodiments, the multispecific antibody described herein is designed to have an IgG1 subtype structure with the LALAPG mutations (L234A, L235A, and P329G mutations in EU numbering). In some embodiments, the multispecific antibody described herein is designed to have an IgG1 Fc region having an Alanine (A) at position 234; an Alanine (A) at position 235; and a Glycine (G) at position 329 in EU numbering. In some embodiments, the Fc region comprises an amino acid sequence that is about or at least 60%, 65%, 70%, 75%, 80%, 85%, 90%, 95%, or 100% identical to SEQ ID NO: 96.

In some embodiments, the multispecific antibody described herein is designed to have an Fc region that comprises the GAALIE mutations: an Alanine (A) at position 236, a Leucine (L) at position 330, and a Glutamic acid (E) at position 332 in EU numbering. In some embodiments, the multispecific antibody described herein is designed to have an IgG1 Fc region having an Alanine (A) at position 236, a Leucine (L) at position 330, and a Glutamic acid (E) at position 332 in EU numbering. In some embodiments, The Fc region comprises an amino acid sequence that is about or at least 60%, 65%, 70%, 75%, 80%, 85%, 90%, 95%, or 100% identical to SEQ ID NO: 97.

In some embodiments, the multispecific antibody described herein is designed to have an Fc region that comprises an Aspartic acid (D) at position 239 and a glutamic acid (E) at position 332 in EU numbering. In some embodiments, the multispecific antibody described herein is designed to have an IgG1 subtype structure with the S239D+I332E mutations in EU numbering. In some embodiments, the multispecific antibody described herein is designed to have an IgG1 Fc region having an Aspartic acid (D) at position 239 and a glutamic acid (E) at position 332 in EU numbering.

In some embodiments, the multispecific antibody described herein can be designed to have an IgG1 subtype structure with knobs-into-holes (KIH) mutations, which can promote heterodimerization and avoid mispairing between the two heavy chains. In some embodiments, the multispecific antibody has a higher endocytosis rate than the corresponding monoclonal antibodies or the control multispecific antibodies. In some embodiments, the antibody or antigen-binding fragment thereof has an increased binding affinity to the FcγRIIa receptor and/or the FcγRIIIa receptor.

In some embodiments, the multispecific antibody described herein is designed to have an Fc region that is afucosylated. Afucosylated antibodies are engineered so that the oligosaccharides in the Fc region of the antibody do not have any fucose sugar units. When antibodies are afucosylated, antibody-dependent cellular cytotoxicity (ADCC) is increased.

The first antigen-binding fragment and the second antigen-binding fragment of the bi-specific antibodies or antigen-binding fragments described herein can be in any suitable format. In some embodiments, the second antigen-binding domain is a single-chain fragment variable (scFv) domain comprising a light chain variable domain (VL) and a heavy chain variable domain (VH) linked by a first linker.

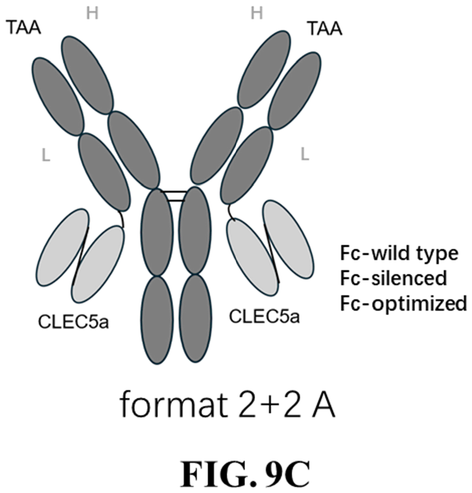

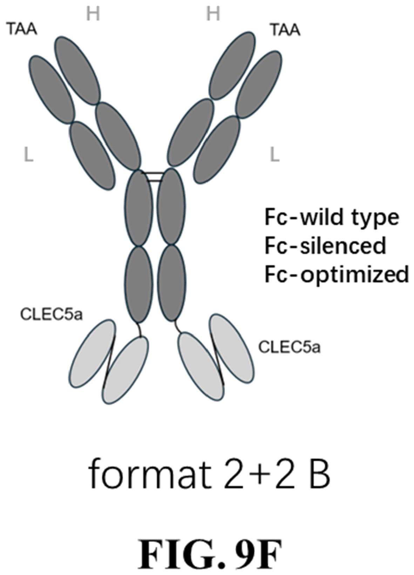

In some embodiments, the second antigen-binding domain is linked to the C-terminus of the light chain of the first antigen-binding domain through a second linker. In some embodiments, the heavy chain variable domain of the first antigen-binding domain is linked to the Fc region. In some embodiments, the VH1 is linked to a CH1 domain, and the VL1 is linked to a CL domain. A schematic illustration of this configuration is shown in **FIG. 9C** and **FIG. 9F**.

Also provided herein are antibodies or antigen-binding fragments thereof, comprising: a first antigen-binding domain that specifically binds to a first antigen; and a second antigen-binding domain that specifically binds to C-type lectin domain family 5 member A (CLEC5A). In some embodiments, the second antigen-binding domain that specifically binds to CLEC5A is linked to the light chain of the antibody or antigen binding fragments thereof. In some embodiments, the second antigen-binding domain that specifically binds to CLEC5A is linked to the C-terminus of the light chain of the antibody or antigen binding fragments thereof. In some embodiments, the second antigen-binding domain that specifically binds to CLEC5A is linked to the light chain of the antibody or antigen binding fragments thereof via a linker described herein. In some embodiments, the first antigen is a TAA (e.g., HER2). A schematic illustration of this configuration is shown in **FIG. 9C**.

Also provided herein are antibodies or antigen-binding fragments thereof, comprising: a first antigen-binding domain that specifically binds to a first antigen; and a second antigen-binding domain that specifically binds to C-type lectin domain family 5 member A (CLEC5A). In some embodiments, the second antigen-binding domain that specifically binds to CLEC5A is linked to the C-terminus of the heavy chain of the antibody or antigen-binding fragment thereof. In some embodiments, the second antigen-binding domain that specifically binds to CLEC5A is linked to the C-terminus of the Fc region of the antibody or antigen-binding fragment thereof. In some embodiments, the second antigen-binding domain that specifically binds to CLEC5A is linked to the C-terminus of the Fc region of the antibody or antigen-binding fragment thereof via a linker described herein. In some embodiments, the first antigen is a TAA (e.g., HER2). A schematic illustration of this configuration is shown in **FIG. 9F**.

Also provided herein are antibodies or antigen-binding fragments thereof, comprising: a first chain comprising a first light chain and a first scFv or VHH; a second chain comprising a first heavy chain; a third chain comprising a second heavy chain; and a fourth chain comprising a second light chain and a second scFv or VHH, wherein the first light chain and second light chain each comprises a VL that has the same sequence (VL1), and the first heavy chain and second heavy chain each comprises a VH that has the same sequence (VH1). In some embodiments, the first and second scFv or VHH comprise the same amino acid sequence. In some embodiments, each of the first and second scFv or VHH comprises a VH2 and/or a VL2. In some embodiments, the antibody or antigen-binding fragment thereof comprises an Fc region. In some embodiments, the first and second heavy chains comprise an Fc region. In some embodiments, the VH1 and VL1 bind to a first antigen. In some embodiments, the first antigen is a TAA. In some embodiments, the VH2 and VL2 bind to a second antigen. In some embodiments, the second antigen is CLEC5A. A schematic illustration of this configuration is shown in **FIG. 9C**.

Also provided herein are antibodies or antigen-binding fragments thereof, comprising: a first chain comprising a first light chain; a second chain comprising a first heavy chain and a first scFv or VHH; a third chain comprising a second heavy chain and a second scFv or VHH; and a fourth chain comprising a second light chain, wherein the first light chain and second light chain each comprises a VL that has the same sequence (VL1), and the first heavy chain and second heavy chain each comprises a VH that has the same sequence (VH1). In some embodiments, the first and second scFv or VHH comprise the same amino acid sequence. In some embodiments, each of the first and second scFv or VHH comprises a VH2 and/or a VL2. In some embodiments, the antibody or antigen-binding fragment thereof comprises an Fc region. In some embodiments, the first and second heavy chains comprise an Fc region. In some embodiments, the first scFv or VHH is linked to the C-terminus of the Fc region of the first heavy chain. In some embodiments, the second scFv or VHH is linked to the C-terminus of the Fc region of the second heavy chain. In some embodiments, the VH1 and VL1 bind to a first antigen. In some embodiments, the first antigen is a TAA. In some embodiments, the VH2 and VL2 bind to a second antigen. In some embodiments, the second antigen is CLEC5A. A schematic illustration of this configuration is shown in **FIG. 9F**.

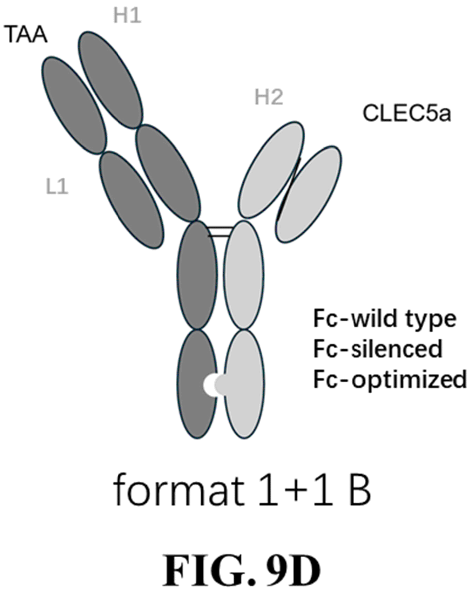

Also provided herein are antibodies or antigen-binding fragments thereof, comprising: a first antigen-binding domain that specifically binds to a first antigen; and a second antigen-binding domain that specifically binds to C-type lectin domain family 5 member A (CLEC5A). In some embodiments, the second antigen-binding domain that specifically binds to CLEC5A is linked to the N-terminus of the heavy chain of the antibody or antigen binding fragments thereof. In some embodiments, the second antigen-binding domain that specifically binds to CLEC5A is linked to the N-terminus of the Fc region of the antibody or antigen binding fragments thereof. In some embodiments, the second antigen-binding domain that specifically binds to CLEC5A is linked to the N-terminus of the Fc region of the antibody or antigen binding fragments thereof via a linker described herein. In some embodiments, the first antigen is a TAA (e.g., HER2). A schematic illustration of this configuration is shown in **FIG. 9D**.

Also provided herein are antibodies or antigen-binding fragments thereof, comprising: a first chain comprising a first light chain, wherein the first light chain comprises a VL1; a second chain comprising a first heavy chain, wherein the first heavy chain comprises a VH1 and a first Fc region; and a third chain comprising a scFv or VHH, and a second Fc region. In some embodiments, the scFv or VHH comprises a VH2 and/or a VL2. In some embodiments, the first Fc region comprises one or more knob mutations, and the second Fc region includes one or more hole mutations. In some embodiments, the first Fc region comprises one or more hole mutations, and the second Fc region includes one or more knob mutations. In some embodiments, the VH1 and VL1 bind to a first antigen. In some embodiments, the first antigen is a TAA. In some embodiments, the VH2 and VL2 bind to a second antigen. In some embodiments, the second antigen is CLEC5A. A schematic illustration of this configuration is shown in **FIG. 9D**.

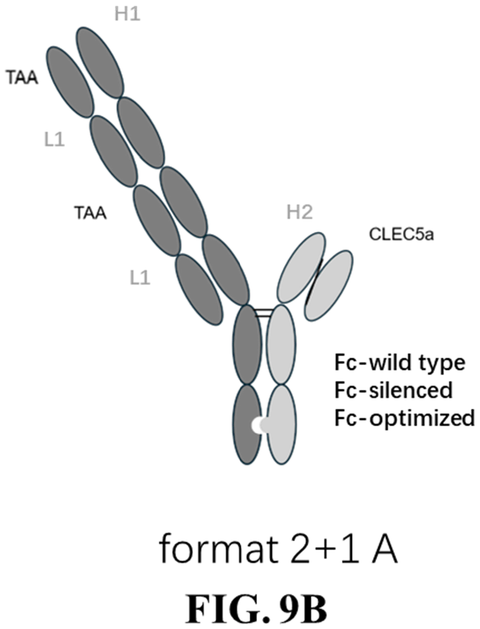

Also provided herein are antibodies or antigen-binding fragments thereof, comprising: a first antigen-binding domain that specifically binds to a first antigen; a second antigen-binding domain that specifically binds to a first antigen; and a third antigen-binding domain that specifically binds to C-type lectin domain family 5 member A (CLEC5A). In some embodiments, the third antigen-binding domain that specifically binds to CLEC5A is linked to the N-terminus of the heavy chain of the antibody or antigen binding fragments thereof. In some embodiments, the third antigen-binding domain that specifically binds to CLEC5A is linked to the N-terminus of the Fc region of the antibody or antigen binding fragments thereof. In some embodiments, the second antigen-binding domain that specifically binds to CLEC5A is linked to the N-terminus of the Fc region of the antibody or antigen binding fragments thereof via a linker described herein. In some embodiments, the first antigen and/or the second antigen are TAAs (e.g., HER2). In some embodiments, the first antigen and the second antigen are the same TAA. In some embodiments, the first antigen and the second antigen are different TAAs. A schematic illustration of this configuration is shown in **FIG. 9B**.

Also provided herein are antibodies or antigen-binding fragments thereof, comprising: a first chain comprising a first light chain, wherein the first light chain comprises a VL1 and a VL2; a second chain comprising a first heavy chain, wherein the first heavy chain comprises a VH1, a VH2, and a first Fc region; and a third chain comprising a scFv or VHH, and a second Fc region. In some embodiments, the scFv or VHH comprises a VH3 and/or a VL3. In some embodiments, the first Fc region comprises one or more knob mutations, and the second Fc region includes one or more hole mutations. In some embodiments, the first Fc region comprises one or more hole mutations, and the second Fc region includes one or more knob mutations. In some embodiments, the VH1 and VL1 bind to a first antigen. In some embodiments, the VH2 and VL2 bind to a second antigen. In some embodiments, the first antigen and/or the second antigen are TAAs. In some embodiments, the VH3 and VL3 bind to a third antigen. In some embodiments, the third antigen is CLEC5A. A schematic illustration of this configuration is shown in **FIG. 9B**.

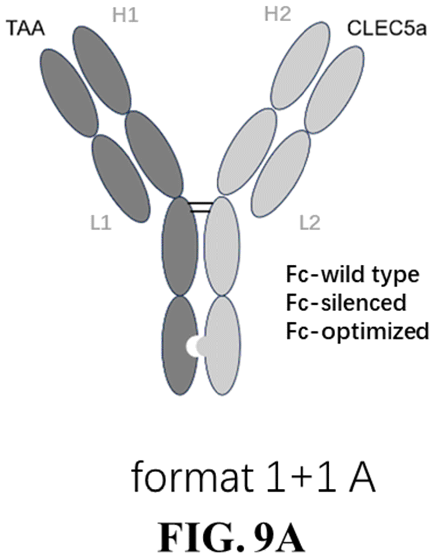

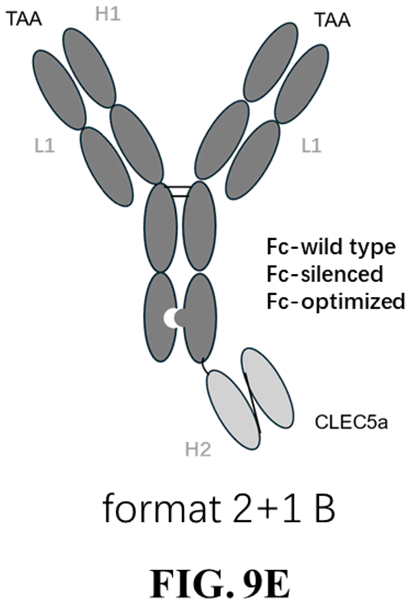

In some embodiments, the antibody or antigen-binding fragment thereof comprises a first heavy chain comprising the VH1 and a first light chain comprising the VL1; and a second heavy chain comprising the VH2 and a second light chain comprising the VL2. A schematic illustration of this configuration is shown in **FIG. 9A** or **FIG. 9E**. In some embodiments, the Fc region comprises knobs-into-holes (KIH) mutations. In some embodiments, the first heavy chain includes one or more knob mutations, and the second heavy chain includes one or more hole mutations. In some embodiments, the first heavy chain includes one or more hole mutations, and the second heavy chain includes one or more knob mutations.

Also provided herein are antibodies or antigen-binding fragments thereof, comprising: a first chain comprising a first light chain, wherein the first light chain comprises a VL1; a second chain comprising a first heavy chain, wherein the first heavy chain comprises a VH1; a third chain comprising a second heavy chain, wherein the second heavy chain comprises a VH2; and a fourth chain comprising a second light chain, wherein the second light chain comprises a VL2. A schematic illustration of this configuration is shown in **FIG. 9A**. In some embodiments, the VH1 and VL1 bind to a first antigen. In some embodiments, the first antigen is a TAA. In some embodiments, the VH2 and VL2 bind to a second antigen. In some embodiments, the second antigen is CLEC5A. In some embodiments, the first heavy chain includes one or more knob mutations, and the second heavy chain includes one or more hole mutations. In some embodiments, the first heavy chain includes one or more hole mutations, and the second heavy chain includes one or more knob mutations.

Also provided herein are antibodies or antigen-binding fragments thereof, comprising: a first chain comprising a first light chain, wherein the first light chain comprises a VL1; a second chain comprising a first heavy chain, wherein the first heavy chain comprises a VH1; a third chain comprising a second heavy chain and a scFv or VHH, wherein the second heavy chain comprises a VH2; and a fourth chain comprising a second light chain, wherein the second light chain comprises a VL2. In some embodiments, the scFv or VHH comprises a VH3 and/or a VL3. A schematic illustration of this configuration is shown in **FIG. 9E**. In some embodiments, the VH1 and VL1 bind to a first antigen. In some embodiments, the VH2 and VL2 bind to a second antigen. In some embodiments, the first antigen and/or the second antigen are TAAs. In some embodiments, the first antigen and the second antigen are the same TAA. In some embodiments, the first antigen and the second antigen are different TAAs. In some embodiments, the VH3 and VL3 bind to a third antigen. In some embodiments, the third antigen is CLEC5A. In some embodiments, the first heavy chain includes one or more knob mutations, and the second heavy chain includes one or more hole mutations. In some embodiments, the first heavy chain includes one or more hole mutations, and the second heavy chain includes one or more knob mutations.

In some embodiments, CrossMab substitutions are introduced into the first light chain and the first heavy chain. In some embodiments, the CH1 domain of the first heavy chain is replaced with the light chain constant region (CL) of the first light chain, and the CL of the first light chain is replaced with the CH1 domain of the first heavy chain.

In some embodiments, CrossMab substitutions are introduced into the second light chain and the second heavy chain. In some embodiments, the CH1 domain of the second heavy chain is replaced with the light chain constant region (CL) of the second light chain, and the CL of the second light chain is replaced with the CH1 domain of the second heavy chain.

In some embodiments, the multispecific antibody has a heavy chain sequence comprising a wild-type IgG1 Fc region (SEQ ID NO: 95). In some embodiments, the multispecific antibody hass a heavy chain sequence comprising an Fc region that is about or at least 80%, 85%, 86%, 87%, 88%, 89%, 90%, 91%, 92%, 93%, 94%, 95%, 96%, 97%, 98%, 99% or 100% identical to SEQ ID NO: 95.

In some embodiments, the multispecific antibody has a heavy chain sequence comprising an IgG1 Fc region with the LALAPG mutations (SEQ ID NO: 96). In some embodiments, the multispecific antibody has a heavy chain sequence comprising an Fc region that is about or at least 80%, 85%, 86%, 87%, 88%, 89%, 90%, 91%, 92%, 93%, 94%, 95%, 96%, 97%, 98%, 99% or 100% identical to SEQ ID NO: 96.

In some embodiments, the multispecific antibody has a heavy chain sequence comprising an IgG1 Fc region with the optimized mutations (SEQ ID NO: 97). In some embodiments, the multispecific antibody has a heavy chain sequence comprising an Fc region that is about or at least 80%, 85%, 86%, 87%, 88%, 89%, 90%, 91%, 92%, 93%, 94%, 95%, 96%, 97%, 98%, 99% or 100% identical to SEQ ID NO: 97.

The disclosure provides multispecific (e.g., bispecific) anti-TAA/CLEC5A (e.g., anti-HER2/CLEC5A) antibodies, the modified antibodies thereof, including, e.g., chimeric antibodies, humanized antibodies, and human antibodies.

In some embodiments, the multispecific (e.g., bispecific) anti-TAA/CLEC5A (e.g., anti-HER2/CLEC5A) antibodies, or antibody fragments thereof include the combinations of anti-HER2 and anti-CLEC5A antigen-binding domains shown in **Table 22**.

In some embodiments, the anti-TAA (e.g., anti-HER2/CLEC5A) antibody is a bispecific antibody. Bispecific antibodies can be made by engineering the interface between a pair of antibody molecules to maximize the percentage of heterodimers that are recovered from recombinant cell culture. For example, the interface can contain at least a part of the CH3 domain of an antibody constant domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers. This method is described, e.g., in WO 96/27011, which is incorporated by reference in its entirety.

Any of the multispecific (e.g., bispecific) anti-TAA/CLEC5A (e.g., anti-HER2/CLEC5A) antibodies or antigen-binding fragments thereof described herein may be conjugated to a stabilizing molecule (e.g., a molecule that increases the half-life of the antibody or antigen-binding fragment thereof in a subject or in solution). Non-limiting examples of stabilizing molecules include: a polymer (e.g., a polyethylene glycol) or a protein (e.g., serum albumin, such as human serum albumin). The conjugation of a stabilizing molecule can increase the half-life or extend the biological activity of an anti-TAA/CLEC5A (e.g., anti-HER2/CLEC5A) antibody or an antigen-binding fragment in vitro (e.g., in tissue culture or when stored as a pharmaceutical composition) or in vivo (e.g., in a human).

The multispecific (e.g., bispecific) anti-TAA/CLEC5A (e.g., anti-HER2/CLEC5A) antibodies can also be antibody variants (including derivatives and conjugates) of multispecific (e.g., bispecific) anti-TAA/CLEC5A (e.g., anti-HER2/CLEC5A) antibodies or antibody fragments. Additional multispecific (e.g., bispecific) anti-TAA/CLEC5A (e.g., anti-HER2/CLEC5A) antibodies provided herein are polyclonal, monoclonal, multispecific (multimeric, e.g., bispecific), human antibodies, chimeric antibodies (e.g., human-mouse chimera), single-chain antibodies, intracellularly-made antibodies (i.e., intrabodies), and antigen-binding fragments thereof. The multispecific (e.g., bispecific) anti-TAA/CLEC5A (e.g., anti-HER2/CLEC5A) antibodies can be of any type (e.g., IgG, IgE, IgM, IgD, IgA, and IgY), class (e.g., IgG1, IgG2, IgG3, IgG4, IgA1, and IgA2), or subclass. In some embodiments, the multispecific (e.g., bispecific) anti-TAA/CLEC5A (e.g., anti-HER2/CLEC5A) antibody or antigen-binding fragment is an IgG (e.g., IgG1 Fc region is shown in SEQ ID NO: 95) antibody or antigen-binding fragment thereof.

Fragments of multispecific (e.g., bispecific) anti-TAA/CLEC5A (e.g., anti-HER2/CLECSA) antibodies are suitable for use in the methods provided so long as they retain the desired affinity and specificity to both the TAA (e.g., HER2) and CLEC5A. Thus, a fragment of a multispecific (e.g., bispecific) anti-TAA/CLEC5A (e.g., anti-HER2/CLEC5A) antibody will retain an ability to bind to TAA (e.g., HER2) and CLEC5A.

### Tumor Associated Antigens

A tumor associated antigen (TAA) is an antigenic substance produced in tumor cells. It can trigger an immune response in the host. Tumor antigens are useful tumor markers in identifying tumor cells with diagnostic tests and are potential candidates for use in cancer therapy. Numerous tumor-associated antigens are known in the art (see, e.g., Yu et al., Cancers (Basel). 2023 Apr; 15(8): 2323; and Tong et al., Mol Cancer, 2022 Nov 1;21(1):206, each of which is incorporated herein by reference in its entirety).

In some embodiments, the TAA is selected from the group consisting of: HER2, CD79b, BCMA, CD38, GPRC5D, DLL3, CD70, GPC3, Fas ligand (FasL), CD1d, Membrane glycolipids, globotriaosyl-ceramide (Gb3Cer/CD77), gangliosides (GD2, GD3, and GM2), GPRC5D, CD34, CD45, human leukocyte antigen-DR (HLA-DR), CD123, CLL1, CD105, CD71, SSC, MAGE, MUC16, CD19, WT-1, B7H3, TEM8, CD22, LI-CAM, ROR-1, CEA, 4-1BB, ETA, 5T4, adenocarcinoma antigen, alpha- fetoprotein (AFP), BAFF, B- lymphoma cell, CA242 antigen, CA-125, carbonic anhydrase 9 (CA- IX), C-MET, CCR4, CD133, CD152, CD20, CD125, CD200, CD221, CD23 (IgE receptor), CD28, CD30 (TNFRSF8), CD33, CD4, CD40, CD44 v6, CD51, CD52, CD56, CD70, CD74, CD80, CEA, CNT0888, CTLA-4, DLL3, DR5, EGFR, EpCAM, CD3, FAP, fibronectin extra domain-B, folate receptor 1, GD2, GD3 ganglioside, glycoprotein 75, GPNMB, HGF, human scatter factor receptor kinase, IGF-1 receptor, IGF-I, IgG1, IL-5, IL-13, IL-6, IL-15, insulin-like growth factor I receptor, integrin a5b1, integrin avb3, MSLN, MS4A1, MUC1, mucin CanAg, N-glycolylneuraminic acid, NPC-1C, PD-1, PD-L1, PDGF-R a, TWEAK, phosphatidylserine, prostatic carcinoma cells, RANKL, RON, SCH 900105, SDC1, SLAMF7, TAG-72, tenascin C, TGF beta 2, TGF-b, TRAIL-R1, TRAIL-R2, tumor antigen CTAA16.88, VEGF-A, VEGFR-1, VEGFR2, OX40 and vimentin.

In some embodiments, the multispecific (e.g., bispecific) antibodies or antigen-binding fragments thereof, comprises a first antigen-binding domain that specifically binds to a tumor associated antigen (TAA) and a second antigen-binding domain that specifically binds to CLEC5A. In some embodiments, the first antigen-binding domain specifically binds to HER2. In some embodiments, the first antigen-binding domain specifically binds to a TAA selected from HER2, EGFR, EpCAM, CD79b, GPRC5D, BCMA, CD38, DLL3, CD70, GPC3, and Mesothelin.

### Anti-TAA/CLEC5A Antibodies and Antigen-binding Fragments Thereof

In some embodiments, the multispecific (e.g., bispecific) anti-TAA/CLEC5A (e.g., anti-HER2/CLEC5A) antibody described herein has an agonistic activity on the activation of macrophages. In some embodiments, the activation of macrophages is increased by about or at least 5%, 10%, 15%, 20%, 25%, 30%, 35%, 40%, 45%, 50%, 55%, 60%, 65%, 70%, 75%, 80%, 85%, 90%, 95%, or 100% upon contact with the anti-TAA/CLEC5A (e.g., anti-HER2/CLEC5A) antibody described herein, compared to the activation of macrophages without contact with such antibody. In some embodiments, the anti-TAA/CLEC5A (e.g., anti-HER2/CLEC5A) antibody described herein binding to macrophages, is with the LALAPG mutations on the Fc region (SEQ ID NO: 96). In some embodiments, the anti-TAA/CLEC5A (e.g., anti-HER2/CLEC5A) antibody described herein binding to macrophages, is with the optimized mutations on the Fc region (SEQ ID NO: 97).

In some embodiments, the multispecific (e.g., bispecific) anti-TAA/CLEC5A (e.g., anti-HER2/CLEC5A) antibody described herein binding to macrophages, is with silent mutations (e.g., LALAPG mutations) on the Fc region. In some embodiments, the anti-TAA/CLEC5A (e.g., anti-HER2/CLEC5A) antibody described herein binds to target cells (e.g., HER2+ cancer cells) with silent mutations (e.g., LALAPG mutations) on the Fc region.

In some embodiments, the multispecific (e.g., bispecific) anti-TAA/CLEC5A (e.g., anti-HER2/CLEC5A) antibody described herein activates macrophages (e.g., through the engagement of CLEC5A), which mediate the killing of target cells (e.g., HER2+ cancer cells). In some embodiments, the anti-TAA/CLEC5A (e.g., anti-HER2/CLEC5A) antibody described herein activating macrophages, is with silent mutations (e.g., LALAPG mutations, SEQ ID NO: 96) on the Fc region. In some embodiments, the anti-TAA/CLEC5A (e.g., anti-HER2/CLEC5A) antibody described herein includes a wild-type human IgG1 Fc region. In some embodiments, the anti-TAA/CLEC5A (e.g., anti-HER2/CLEC5A) described herein binding to macrophages, is with the optimized mutations (SEQ ID NO: 97) on the Fc region.

In some embodiments, the multispecific (e.g., bispecific) anti-TAA/CLEC5A (e.g., anti-HER2/CLEC5A) antibody described herein activates macrophages (e.g., through the engagement of CLEC5A) and induces macrophage-mediated target cell killing. In some embodiments, the macrophage-mediated killing results in the killing of target cells (e.g., HER2+ cancer cells). In some embodiments, about 5%, 10%, 15%, 20%, 25%, 30%, 35%, 40%, 45%, 50%, 55%, 60%, 65%, 70%, 75%, 80%, 85%, 90%, 95% or more of the total number of the target cells are killed through macrophage-mediated phagocytosis.

In some embodiments, the TAA described herein is selected from HER2, EGFR, EpCAM, CD79b, GPRC5D, BCMA, CD38, DLL3, CD70, GPC3, and Mesothelin.

### Anti-HER2/CLEC5A Antibodies and Antigen-binding Fragments Thereof

Receptor tyrosine-protein kinase erbB-2 (HER2) is a protein that in humans is encoded by the ERBB2 gene. ERBB is abbreviated from erythroblastic oncogene B, a gene originally isolated from the avian genome. The human protein is also frequently referred to as HER2 (human epidermal growth factor receptor 2) or CD340 (cluster of differentiation 340).

HER2 is a member of the human epidermal growth factor receptor (HER/EGFR/ERBB) family. But contrary to other member of the ERBB family, HER2 does not directly bind ligand. HER2 activation results from heterodimerization with another ERBB member or by homodimerization when HER2 concentration are high, for instance in cancer. Amplification or over-expression of this oncogene has been shown to play an important role in the development and progression of certain aggressive types of breast cancer. In recent years the protein has become an important biomarker and target of therapy for approximately 30% of breast cancer patients.

Detailed reviews of HER2 can be found, for example, at "ERBB2 erb-b2 receptor tyrosine kinase 2 [Homo sapiens (human)] - Gene - NCBI" at the NCBI website; "ERBB2". Genetics Home Reference; Barh D, Gunduz M (2015-01-22). Noninvasive Molecular Markers in Gynecologic Cancers. CRC Press. p. 427. ISBN 9781466569393; and Hsu JL, Hung MC (2016). "The role of HER2, EGFR, and other receptor tyrosine kinases in breast cancer". Cancer and Metastasis Reviews. 35 (4): 575-588. doi:10.1007/s10555-016-9649-6, the entire content of each of which is incorporated herein by reference.

The disclosure provides multispecific (e.g., bispecific) antibodies and antigen-binding fragments thereof that specifically bind to HER2/CLEC5A (e.g., human HER2/CLEC5A). In one aspect, the disclosure provides an anti-HER2/CLEC5A multispecific (e.g., bispecific) antibody or antigen-binding fragment thereof, comprising: a first antigen-binding domain that specifically binds to HER2; and a second antigen-binding domain that specifically binds to CLEC5A.

In some embodiments, the first antigen-binding domain comprises a first heavy chain variable region (VH1) and a first light chain variable region (VL1); and the second antigen-binding domain comprises a second heavy chain variable region (VH2) and a second light chain variable region (VL2).

In some embodiments, the first heavy chain variable region (VH1) comprises complementarity determining regions (CDRs) 1, 2, and 3, wherein the VH1 CDR1 region comprises an amino acid sequence that is at least 80% identical to a selected VH1 CDR1 amino acid sequence, the VH1 CDR2 region comprises an amino acid sequence that is at least 80% identical to a selected VH1 CDR2 amino acid sequence, and the VH1 CDR3 region comprises an amino acid sequence that is at least 80% identical to a selected VH1 CDR3 amino acid sequence; and
the first light chain variable region (VL1) comprises CDRs 1, 2, and 3, wherein the VL1 CDR1 region comprises an amino acid sequence that is at least 80% identical to a selected VL1 CDR1 amino acid sequence, the VL1 CDR2 region comprises an amino acid sequence that is at least 80% identical to a selected VL1 CDR2 amino acid sequence, and the VL1 CDR3 region comprises an amino acid sequence that is at least 80% identical to a selected VL1 CDR3 amino acid sequence, wherein the selected VH1 CDRs 1, 2, 3 amino acid sequences, and the selected VL1 CDRs 1, 2, and 3 amino acid sequences are one of the following:
   (1) the selected VH1 CDRs 1, 2, 3 amino acid sequences are set forth in SEQ ID NOs: 100, 102, and 104, respectively, and the selected VL1 CDRs 1, 2, 3 amino acid sequences are set forth in SEQ ID NOs: 105, 106, and 107, respectively; and
   (2) the selected VH1 CDRs 1, 2, 3 amino acid sequences are set forth in SEQ ID NOs: 101, 103, and 104, respectively, and the selected VL1 CDRs 1, 2, 3 amino acid sequences are set forth in SEQ ID NOs: 105, 106, and 107, respectively;
the second heavy chain variable region (VH2) comprises complementarity determining regions (CDRs) 1, 2, and 3, wherein the VH2 CDR1 region comprises an amino acid sequence that is at least 80% identical to a selected VH2 CDR1 amino acid sequence, the VH2 CDR2 region comprises an amino acid sequence that is at least 80% identical to a selected VH2 CDR2 amino acid sequence, and the VH2 CDR3 region comprises an amino acid sequence that is at least 80% identical to a selected VH2 CDR3 amino acid sequence;
and the second light chain variable region (VL2) comprises CDRs 1, 2, and 3, wherein the VL2 CDR1 region comprises an amino acid sequence that is at least 80% identical to a selected VL2 CDR1 amino acid sequence, the VL2 CDR2 region comprises an amino acid sequence that is at least 80% identical to a selected VL2 CDR2 amino acid sequence, and the VL2 CDR3 region comprises an amino acid sequence that is at least 80% identical to a selected VL2 CDR3 amino acid sequence,
wherein the selected VH2 CDRs 1, 2, and 3 amino acid sequences, and the selected VL2 CDRs 1, 2, and 3 amino acid sequences are one of the following:
   (1) the selected VH2 CDRs 1, 2, 3 amino acid sequences are set forth in SEQ ID NOs: 3, 5, and 7, respectively, and the selected VL2 CDRs 1, 2, 3 amino acid sequences are set forth in SEQ ID NOs: 8, 9, and 10, respectively;
   (2) the selected VH2 CDRs 1, 2, 3 amino acid sequences are set forth in SEQ ID NOs: 4, 6, and 7, respectively, and the selected VL2 CDRs 1, 2, 3 amino acid sequences are set forth in SEQ ID NOs: 8, 9 and 10, respectively;
   (3) the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively; and
   (4) the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the selected VH1 CDRs 1, 2, 3 amino acid sequences are set forth in SEQ ID NOs: 100, 102, and 104, respectively, the selected VL1 CDRs 1, 2, 3 amino acid sequences are set forth in SEQ ID NOs: 105, 106, and 107, respectively; the selected VH2 CDRs 1, 2, 3 amino acid sequences are set forth in SEQ ID NOs: 3, 5, and 7, respectively, and the selected VL2 CDRs 1, 2, 3 amino acid sequences are set forth in SEQ ID NOs: 8, 9, and 10, respectively.

In some embodiments, the selected VH1 CDRs 1, 2, 3 amino acid sequences are set forth in SEQ ID NOs: 101, 103, and 104, respectively, the selected VL1 CDRs 1, 2, 3 amino acid sequences are set forth in SEQ ID NOs: 105, 106, and 107, respectively; the selected VH2 CDRs 1, 2, 3 amino acid sequences are set forth in SEQ ID NOs: 4, 6, and 7, respectively, and the selected VL2 CDRs 1, 2, 3 amino acid sequences are set forth in SEQ ID NOs: 8, 9, and 10, respectively.

In some embodiments, the selected VH1 CDRs 1, 2, 3 amino acid sequences are set forth in SEQ ID NOs: 100, 102, and 104, respectively, the selected VL1 CDRs 1, 2, 3 amino acid sequences are set forth in SEQ ID NOs: 105, 106, and 107, respectively; the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively.

In some embodiments, the selected VH1 CDRs 1, 2, 3 amino acid sequences are set forth in SEQ ID NOs: 101, 103, and 104, respectively, the selected VL1 CDRs 1, 2, 3 amino acid sequences are set forth in SEQ ID NOs: 105, 106, and 107, respectively; the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19, and 20, respectively.

In some embodiments, the first heavy chain variable region comprises a sequence that is at least 80%, 85%, 90%, 95%, 99%, or 100% identical to SEQ ID NO: 108, the first light chain variable region comprises a sequence that is at least 80%, 85%, 90%, 95%, 99%, or 100% identical to SEQ ID NO: 109, the second heavy chain variable region comprises a sequence that is at least 80%, 85%, 90%, 95%, 99%, or 100% identical to SEQ ID NO: 11, and the second light chain variable region comprises a sequence that is at least 80%, 85%, 90%, 95%, 99%, or 100% identical to SEQ ID NO: 12.

In some embodiments, the first heavy chain variable region comprises a sequence that is at least 80%, 85%, 90%, 95%, 99%, or 100% identical to SEQ ID NO: 108, the first light chain variable region comprises a sequence that is at least 80%, 85%, 90%, 95%, 99%, or 100% identical to SEQ ID NO: 109, the second heavy chain variable region comprises a sequence that is at least 80%, 85%, 90%, 95%, 99%, or 100% identical to SEQ ID NO: 83, and the second light chain variable region comprises a sequence that is at least 80%, 85%, 90%, 95%, 99%, or 100% identical to SEQ ID NO: 84.

In some embodiments, the first heavy chain variable region comprises a sequence that is at least 80%, 85%, 90%, 95%, 99%, or 100% identical to SEQ ID NO: 108, the first light chain variable region comprises a sequence that is at least 80%, 85%, 90%, 95%, 99%, or 100% identical to SEQ ID NO: 109, the second heavy chain variable region comprises a sequence that is at least 80%, 85%, 90%, 95%, 99%, or 100% identical to SEQ ID NO: 21, and the second light chain variable region comprises a sequence that is at least 80%, 85%, 90%, 95%, 99%, or 100% identical to SEQ ID NO: 22.

In some embodiments, the first heavy chain variable region comprises a sequence that is at least 80%, 85%, 90%, 95%, 99%, or 100% identical to SEQ ID NO: 108, the first light chain variable region comprises a sequence that is at least 80%, 85%, 90%, 95%, 99%, or 100% identical to SEQ ID NO: 109, the second heavy chain variable region comprises a sequence that is at least 80%, 85%, 90%, 95%, 99%, or 100% identical to SEQ ID NO: 85, and the second light chain variable region comprises a sequence that is at least 80%, 85%, 90%, 95%, 99%, or 100% identical to SEQ ID NO: 86.

In some embodiments, the VH1 comprises an amino acid sequence that is at least 80%, 85%, 90%, 95%, 99%, or 100% identical to a selected VH sequence, and the VL1 comprises an amino acid sequence that is at least 80%, 85%, 90%, 95%, 99%, or 100% identical to a selected VL sequence, wherein the selected VH sequence is SEQ ID NO: 108, and the selected VL sequence is SEQ ID NO: 109.

In some embodiments, the VH2 comprises an amino acid sequence that is at least 80%, 85%, 90%, 95%, 99%, or 100% identical to a selected VH sequence, and the VL2 comprises an amino acid sequence that is at least 80%, 85%, 90%, 95%, 99%, or 100% identical to a selected VL sequence, wherein the selected VH sequence and the selected VL sequence are one of the following:
(1) the selected VH sequence is SEQ ID NO: 11, and the selected VL sequence is SEQ ID NO: 12;
(2) the selected VH sequence is SEQ ID NO: 21, and the selected VL sequence is SEQ ID NO: 22;
(3) the selected VH sequence is SEQ ID NO: 83, and the selected VL sequence is SEQ ID NO: 84; and
(4) the selected VH sequence is SEQ ID NO: 86, and the selected VL sequence is SEQ ID NO: 87.

In some embodiments, the VH1 comprises VH1 CDR1, VH1 CDR2, and VH1 CDR3 that are identical to VH CDR1, VH CDR2, and VH CDR3 of a selected VH sequence; and the VL1 comprising VL1 CDR1, VL1 CDR2, and VL1 CDR3 that are identical to VL CDR1, VL CDR2, and VL CDR3 of a selected VL sequence, wherein the selected VH sequence is SEQ ID NO: 108, and the selected VL sequence is SEQ ID NO: 109.

In some embodiments, the VH2 comprises VH2 CDR1, VH2CDR2, and VH2 CDR3 that are identical to VH CDR1, VH CDR2, and VH CDR3 of a selected VH sequence; and the VL2 comprising VL2 CDR1, VL2 CDR2, and VL2 CDR3 that are identical to VL CDR1, VL CDR2, and VL CDR3 of a selected VL sequence, wherein the selected VH sequence and the selected VL sequence are one of the following:
(1) the selected VH sequence is SEQ ID NO: 11, and the selected VL sequence is SEQ ID NO: 12;
(2) the selected VH sequence is SEQ ID NO: 21, and the selected VL sequence is SEQ ID NO: 22;
(3) the selected VH sequence is SEQ ID NO: 83, and the selected VL sequence is SEQ ID NO: 84; and
(4) the selected VH sequence is SEQ ID NO: 86, and the selected VL sequence is SEQ ID NO: 87.

In some embodiments, the first antigen-binding domain is the antigen-binding domain of anti-HER2 antibody Trastuzumab. Trastuzumab (CAS 180288-69-1, HERCEPTIN^{®}, huMAb4D5-8, rhuMAb HER2, Genentech) is a recombinant DNA-derived, IgG1 kappa, monoclonal antibody that is a humanized format of a murine anti-HER2 antibody (4D5) that selectively binds with high affinity in a cell-based assay (Kd=5 nM) to the extracellular domain of HER2 (see, e.g., U.S. Pat. Nos. 5,677,171; 5,821,337; 6,054,297; 6,165,464; 6,339,142; 6,407,213; 6,639,055; 6,719,971; 6,800,738; 7,074,404; Coussens et al (1985) Science 230:1132-9; Slamon et al (1989) Science 244:707-12; Slamon et al (2001) New Engl. J. Med. 344:783-792).

In some embodiments, the second antigen-binding domain is any of the antigen-binding domain of anti-CLEC5A antibodies, the chimeric antibodies thereof, and the humanized antibodies thereof described herein. In some embodiments, the second antigen-binding domain is the antigen-binding domain of anti-CLEC5A antibody 5C7 or 3A7, the chimeric antibodies thereof, and the humanized antibodies thereof.

The CDR sequences for 3A7, and 3A7 derived antibodies include CDRs of the heavy chain variable domain, SEQ ID NOs: 3, 5, and 7, and CDRs of the light chain variable domain, SEQ ID NOs: 8, 9, and 10, as defined by Kabat definition. Under Chothia definition, the CDR sequences of the heavy chain variable domain are set forth in SEQ ID NOs: 4, 6, and 7, and CDRs of the light chain variable domain are set forth in SEQ ID NOs: 8, 9, and 10.

The CDR sequences for 5C7, and 5C7 derived antibodies include CDRs of the heavy chain variable domain, SEQ ID NOs: 13, 15, and 17, and CDRs of the light chain variable domain, SEQ ID NOs: 18, 19, and 20, as defined by Kabat definition. Under Chothia definition, the CDR sequences of the heavy chain variable domain are set forth in SEQ ID NOs: 14, 16, and 17, and CDRs of the light chain variable domain are set forth in SEQ ID NOs: 18, 19, and 20.

In some embodiments, the first antigen-binding domain specifically binds to human, rabbit, mouse, monkey, or dog HER2; and/or the second antigen-binding domain specifically binds to human, rabbit, mouse, monkey, or dog CLEC5A.

In some embodiments, the first antigen-binding domain is a human or humanized antigen-binding domain; and/or the second antigen-binding domain is a human or humanized antigen-binding domain.

In some embodiments, the first antigen-binding domain is a single-chain variable fragment (scFv); and/or the second antigen-binding domain is a scFv.

In some embodiments, the antibody or antigen-binding fragment thereof comprises a fragment crystallizable region (Fc region).

In some embodiments, the multispecific (e.g., bispecific) anti-HER2/CLEC5A antibody described herein is designed to have an IgG1 subtype structure with the LALAPG mutations (L234A, L235A, and P329G mutations in EU numbering). In some embodiments, the multispecific (e.g., bispecific) anti-HER2/CLEC5A antibody described herein is designed to have an IgG1 Fc region having an Alanine (A) at position 234; an Alanine (A) at position 235; and a Glycine (G) at position 329 in EU numbering. In some embodiments, The Fc region comprises an amino acid sequence that is about or at least 60%, 65%, 70%, 75%, 80%, 85%, 90%, 95%, or 100% identical to SEQ ID NO: 96.

In some embodiments, the anti-HER2/CLEC5A antibody described herein can be designed to have an IgG1 Fc region having an Alanine (A) at position 236, a Leucine (L) at position 330, and a Glutamic acid (E) at position 332 in EU numbering. In some embodiments, The Fc region comprises an amino acid sequence that is about or at least 60%, 65%, 70%, 75%, 80%, 85%, 90%, 95%, or 100% identical to SEQ ID NO: 97.

In some embodiments, the multispecific (e.g., bispecific) anti-HER2/CLEC5A antibody described herein is designed to have an Fc region that comprises an Aspartic acid (D) at position 239 and a glutamic acid (E) at position 332 in EU numbering. In some embodiments, the multispecific (e.g., bispecific) anti-HER2/CLEC5A antibody described herein is designed to have an IgG1 subtype structure with the S239D+I332E mutations in EU numbering. In some embodiments, the multispecific antibody described herein is designed to have an IgG1 Fc region having an Aspartic acid (D) at position 239 and a glutamic acid (E) at position 332 in EU numbering.

In some embodiments, the multispecific (e.g., bispecific) anti-HER2/CLEC5A antibody described herein can be designed to have an IgG1 subtype structure with knobs-into-holes (KIH) mutations, which can promote heterodimerization and avoid mispairing between the two heavy chains. In some embodiments, the anti-HER2/CLEC5A antibody has a higher endocytosis rate than the corresponding monoclonal antibodies or the control bispecific antibodies.

The first antigen-binding fragment and the second antigen-binding fragment of the bi-specific antibodies or antigen-binding fragments described herein can be in any suitable configurations. In some embodiments, wherein the second antigen-binding domain is a single-chain fragment variable (scFv) domain comprising a light chain variable domain (VL) and a heavy chain variable domain (VH) linked by a first linker.

In some embodiments, the second antigen-binding domain is linked to the C-terminus of the light chain of the first antigen-binding domain through a second linker. In some embodiments, the heavy chain variable domain of the first antigen-binding domain is linked to the Fc region. In some embodiments, the VH1 is linked to a CH1 domain, and the VL1 is linked to a CL domain. A schematic illustration of this configuration is shown in **FIG. 9C**. In some embodiments, the antibody or antigen-binding fragment thereof described herein comprises a first heavy chain and a first light chain; and a second heavy chain and a second light chain. A schematic illustration of this configuration is shown in **FIG. 9A**. In some embodiments, the Fc region comprises knobs-into-holes (KIH) mutations. In some embodiments, the first heavy chain includes one or more knob mutations, and the second heavy chain includes one or more hole mutations. In some embodiments, the first heavy chain includes one or more hole mutations, and the second heavy chain includes one or more knob mutations. In some embodiments, the multispecific (e.g., bispecific) anti-HER2/CLEC5A antibody described herein can have a structure shown in any one of **FIGs. 9A-****9F**.

In some embodiments, the multispecific (e.g., bispecific) anti-HER2/CLEC5A antibody includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 118. In some embodiments, the heavy chain includes an IgG1 Fc region comprising the LALAPG mutations (SEQ ID NO: 96).

In some embodiments, the multispecific (e.g., bispecific) anti-HER2/CLEC5A antibody includes a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 119.

In some embodiments, the multispecific (e.g., bispecific) anti-HER2/CLEC5A antibody is referred to as "HER2/3A7 (2+2) Fc-LALAPG" or "HER2/3A7 (2+2 A) Fc-silenced" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 118; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 119.

In some embodiments, the multispecific (e.g., bispecific) anti-HER2/CLEC5A antibody includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 120. In some embodiments, the heavy chain includes an IgG1 Fc region comprising the LALAPG mutations (SEQ ID NO: 96).

In some embodiments, the multispecific (e.g., bispecific) anti-HER2/CLEC5A antibody includes a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 121.

In some embodiments, the multispecific (e.g., bispecific) anti-HER2/CLEC5A antibody is referred to as "HER2/5C7 (2+2) Fc-LALAPG" or "HER2/5C7 (2+2 A) Fc-silenced" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 120; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 121.

In some embodiments, the multispecific (e.g., bispecific) anti-HER2/CLEC5A antibody includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 122. In some embodiments, the heavy chain includes an IgG1 Fc region comprising the optimized mutations (SEQ ID NO: 97).

In some embodiments, the multispecific (e.g., bispecific) anti-HER2/CLEC5A antibody includes a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 123.

In some embodiments, the multispecific (e.g., bispecific) anti-HER2/CLEC5A antibody is referred to as "HER2/5C7 (2+2 A) Fc-optimized" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 122; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 123.

In some embodiments, the multispecific (e.g., bispecific) anti-HER2/CLEC5A antibody includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 110. In some embodiments, the first heavy chain includes an IgG1 Fc region comprising the LALAPG mutations (SEQ ID NO: 96). In some embodiments, the first heavy chain includes one or more hole mutations.

In some embodiments, the multispecific (e.g., bispecific) anti-HER2/CLEC5A antibody includes a first light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 112.

In some embodiments, the multispecific (e.g., bispecific) anti-HER2/CLEC5A antibody includes having a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 111. In some embodiments, the second heavy chain includes an IgG1 Fc region comprising the LALAPG mutations (SEQ ID NO: 96). In some embodiments, the second heavy chain includes one or more knob mutations.

In some embodiments, the multispecific (e.g., bispecific) anti-HER2/CLEC5A antibody includes a second light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 113.

In some embodiments, the multispecific (e.g., bispecific) anti-HER2/CLEC5A antibody is referred to as "HER2/3A7 (1+1 A) Fc-LALAPG" or "HER2/3A7 (1+1 A) Fc-silenced" and includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 110; and a first light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 112; and a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 111; and a second light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 113.

In some embodiments, the multispecific (e.g., bispecific) anti-HER2/CLEC5A antibody includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 114. In some embodiments, the first heavy chain includes an IgG1 Fc region comprising the LALAPG mutations (SEQ ID NO: 96). In some embodiments, the first heavy chain includes one or more hole mutations.

In some embodiments, the multispecific (e.g., bispecific) anti-HER2/CLEC5A antibody includes a first light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 116.

In some embodiments, the multispecific (e.g., bispecific) anti-HER2/CLEC5A antibody includes a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 115. In some embodiments, the second heavy chain includes an IgG1 Fc region comprising the LALAPG mutations (SEQ ID NO: 96). In some embodiments, the second heavy chain includes one or more knob mutations.

In some embodiments, the multispecific (e.g., bispecific) anti-HER2/CLEC5A antibody includes a second light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 117.

In some embodiments, the multispecific (e.g., bispecific) anti-HER2/CLEC5A antibody is referred to as "HER2/5C7 (1+1 A) Fc-LALAPG" or "HER2/5C7 (1+1 A) Fc-silenced" and includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 114; and a first light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 116; and a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 115; and a second light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 117.

The linkers described herein can be any suitable linkers known in the art. In some embodiments, the linker can comprise a spacer sequence. Various spacer sequences are known in the art, including, without limitation, glycine serine (GS) spacers (also known as GS linkers) such as (GS)n, (SG)n, and (GGGGS)n (SEQ ID NO: 99), where n represents an integer of at least 1. Those of skill in the art would be able to select the appropriate spacer sequence.

In some embodiments, knobs-into-holes mutations were introduced in the Fc regions of the multispecific (e.g., bispecific) antibodies to reduce the chance of wrong pairing between the two heavy chains.

The disclosure also provides nucleic acid comprising a polynucleotide encoding an anti-HER2/CLEC5A antibody. The immunoglobulin heavy chain or immunoglobulin light chain in the anti-HER2/CLEC5A antibody comprises CDRs as shown in **Table 22**. When the polypeptides are paired with corresponding polypeptide (e.g., a corresponding heavy chain variable region or a corresponding light chain variable region), the paired polypeptides bind to HER2 and/or CLEC5A.

### Anti-EGFR/CLEC5A Antibodies and Antigen-binding Fragments Thereof

The epidermal growth factor receptor (EGFR; ErbB-1; HER1 in humans) is a transmembrane protein that is a receptor for members of the epidermal growth factor family (EGF family) of extracellular protein ligands. The epidermal growth factor receptor is a member of the ErbB family of receptors, a subfamily of four closely related receptor tyrosine kinases: EGFR (ErbB-1), HER2/neu (ErbB-2), Her 3 (ErbB-3), and Her 4 (ErbB-4), In many cancer types, mutations affecting EGFR expression or activity could result in more progressive cancer. Deficient signaling of the EGFR and other receptor tyrosine kinases in humans is associated with diseases such as Alzheimer's, while over-expression is associated with the development of a wide variety of tumors. Interruption of EGFR signalling, either by blocking EGFR binding sites on the extracellular domain of the receptor or by inhibiting intracellular tyrosine kinase activity, can prevent the growth of EGFR-expressing tumors and improve the patient's condition.

EGFR is a transmembrane protein that is activated by binding of its specific ligands, including epidermal growth factor and transforming growth factor a (TGFa). ErbB2 has no known direct activating ligand and may be in an activated state constitutively or become active upon heterodimerization with other family members such as EGFR. Upon activation by its growth factor ligands, EGFR undergoes a transition from an inactive monomeric form to an active homodimer.

EGFR dimerization stimulates its intrinsic intracellular protein-tyrosine kinase activity. As a result, autophosphorylation of several tyrosine (Y) residues in the C-terminal domain of EGFR occurs. These include Y992, Y1045, Y1068, Y1148, and Y1173.

Autophosphorylation elicits downstream activation and signaling by several other proteins that associate with the phosphorylated tyrosines through their own phosphotyrosine-binding SH2 domains. These downstream signaling proteins initiate several signal transduction cascades, principally the MAPK, Akt, and JNK pathways, leading to DNA synthesis and cell proliferation. Such proteins modulate phenotypes such as cell migration, adhesion, and proliferation. The kinase domain of EGFR can also cross-phosphorylate tyrosine residues of other receptors it is aggregated with, and can itself be activated in that manner.

Mutations that lead to EGFR overexpression (known as upregulation or amplification) have been associated with a number of cancers, including adenocarcinoma of the lung (40% of cases), anal cancers, glioblastoma (50%), and epithelian tumors of the head and neck (80-100%). These somatic mutations involving EGFR lead to its constant activation, which produces uncontrolled cell division. In glioblastoma a specific mutation of EGFR, called EGFRvIII, is often observed. Mutations, amplifications, or misregulations of EGFR or family members are implicated in about 30% of all epithelial cancer.

The disclosure provides multispecific (e.g., bispecific) antibodies and antigen-binding fragments thereof that specifically bind to EGFR/CLEC5A (e.g., human EGFR/CLEC5A). In one aspect, the disclosure provides an anti-EGFR/CLEC5A multispecific (e.g., bispecific) antibody or antigen-binding fragment thereof, comprising: a first antigen-binding domain that specifically binds to EGFR; and a second antigen-binding domain that specifically binds to CLEC5A.

In some embodiments, the first antigen-binding domain comprises a first heavy chain variable region (VH1) and a first light chain variable region (VL1); and the second antigen-binding domain comprises a second heavy chain variable region (VH2) and a second light chain variable region (VL2).

In some embodiments, the first heavy chain variable region (VH1) comprises complementarity determining regions (CDRs) 1, 2, and 3, wherein the VH1 CDR1 region comprises an amino acid sequence that is at least 80% identical to a selected VH1 CDR1 amino acid sequence, the VH1 CDR2 region comprises an amino acid sequence that is at least 80% identical to a selected VH1 CDR2 amino acid sequence, and the VH1 CDR3 region comprises an amino acid sequence that is at least 80% identical to a selected VH1 CDR3 amino acid sequence; and
the first light chain variable region (VL1) comprises CDRs 1, 2, and 3, wherein the VL1 CDR1 region comprises an amino acid sequence that is at least 80% identical to a selected VL1 CDR1 amino acid sequence, the VL1 CDR2 region comprises an amino acid sequence that is at least 80% identical to a selected VL1 CDR2 amino acid sequence, and the VL1 CDR3 region comprises an amino acid sequence that is at least 80% identical to a selected VL1 CDR3 amino acid sequence, wherein the selected VH1 CDRs 1, 2, 3 amino acid sequences, and the selected VL1 CDRs 1, 2, and 3 amino acid sequences are one of the following:
   (1) the selected VH1 CDRs 1, 2, 3 amino acid sequences are set forth in SEQ ID NOs: 126, 128, and 130, respectively, and the selected VL1 CDRs 1, 2, 3 amino acid sequences are set forth in SEQ ID NOs: 131, 132, and 133, respectively;
   (2) the selected VH1 CDRs 1, 2, 3 amino acid sequences are set forth in SEQ ID NOs: 127, 129, and 130, respectively, and the selected VL1 CDRs 1, 2, 3 amino acid sequences are set forth in SEQ ID NOs: 131, 132, and 133, respectively;
   (3) the selected VH2 CDRs 1, 2, 3 amino acid sequences are set forth in SEQ ID NOs: 136, 138, and 140, respectively, and the selected VL2 CDRs 1, 2, 3 amino acid sequences are set forth in SEQ ID NOs: 141, 142, and 143, respectively; and
   (4) the selected VH2 CDRs 1, 2, 3 amino acid sequences are set forth in SEQ ID NOs: 137, 139, and 140, respectively, and the selected VL2 CDRs 1, 2, 3 amino acid sequences are set forth in SEQ ID NOs: 141, 142, and 143, respectively;
the second heavy chain variable region (VH2) comprises complementarity determining regions (CDRs) 1, 2, and 3, wherein the VH2 CDR1 region comprises an amino acid sequence that is at least 80% identical to a selected VH2 CDR1 amino acid sequence, the VH2 CDR2 region comprises an amino acid sequence that is at least 80% identical to a selected VH2 CDR2 amino acid sequence, and the VH2 CDR3 region comprises an amino acid sequence that is at least 80% identical to a selected VH2 CDR3 amino acid sequence;
and the second light chain variable region (VL2) comprises CDRs 1, 2, and 3, wherein the VL2 CDR1 region comprises an amino acid sequence that is at least 80% identical to a selected VL2 CDR1 amino acid sequence, the VL2 CDR2 region comprises an amino acid sequence that is at least 80% identical to a selected VL2 CDR2 amino acid sequence, and the VL2 CDR3 region comprises an amino acid sequence that is at least 80% identical to a selected VL2 CDR3 amino acid sequence,
wherein the selected VH2 CDRs 1, 2, and 3 amino acid sequences, and the selected VL2 CDRs 1, 2, and 3 amino acid sequences are one of the following:
   (1) the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively; and
   (2) the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the selected VH1 CDRs 1, 2, 3 amino acid sequences are set forth in SEQ ID NOs: 126, 128, and 130, respectively, the selected VL1 CDRs 1, 2, 3 amino acid sequences are set forth in SEQ ID NOs: 131, 132, and 133, respectively; the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively.

In some embodiments, the selected VH1 CDRs 1, 2, 3 amino acid sequences are set forth in SEQ ID NOs: 127, 129, and 130, respectively, the selected VL1 CDRs 1, 2, 3 amino acid sequences are set forth in SEQ ID NOs: 131, 132, and 133, respectively; the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the selected VH1 CDRs 1, 2, 3 amino acid sequences are set forth in SEQ ID NOs: 136, 138, and 140, respectively, the selected VL1 CDRs 1, 2, 3 amino acid sequences are set forth in SEQ ID NOs: 141, 142, and 143, respectively; the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively.

In some embodiments, the selected VH1 CDRs 1, 2, 3 amino acid sequences are set forth in SEQ ID NOs: 137, 139, and 140, respectively, the selected VL1 CDRs 1, 2, 3 amino acid sequences are set forth in SEQ ID NOs: 141, 142, and 143, respectively; the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19, and 20, respectively.

In some embodiments, the first heavy chain variable region comprises a sequence that is at least 80%, 85%, 90%, 95%, 99%, or 100% identical to SEQ ID NO: 134, the first light chain variable region comprises a sequence that is at least 80%, 85%, 90%, 95%, 99%, or 100% identical to SEQ ID NO: 135, the second heavy chain variable region comprises a sequence that is at least 80%, 85%, 90%, 95%, 99%, or 100% identical to SEQ ID NO: 21, and the second light chain variable region comprises a sequence that is at least 80%, 85%, 90%, 95%, 99%, or 100% identical to SEQ ID NO: 22.

In some embodiments, the first heavy chain variable region comprises a sequence that is at least 80%, 85%, 90%, 95%, 99%, or 100% identical to SEQ ID NO: 134, the first light chain variable region comprises a sequence that is at least 80%, 85%, 90%, 95%, 99%, or 100% identical to SEQ ID NO: 135, the second heavy chain variable region comprises a sequence that is at least 80%, 85%, 90%, 95%, 99%, or 100% identical to SEQ ID NO: 85, and the second light chain variable region comprises a sequence that is at least 80%, 85%, 90%, 95%, 99%, or 100% identical to SEQ ID NO: 86.

In some embodiments, the first heavy chain variable region comprises a sequence that is at least 80%, 85%, 90%, 95%, 99%, or 100% identical to SEQ ID NO: 144, the first light chain variable region comprises a sequence that is at least 80%, 85%, 90%, 95%, 99%, or 100% identical to SEQ ID NO: 145, the second heavy chain variable region comprises a sequence that is at least 80%, 85%, 90%, 95%, 99%, or 100% identical to SEQ ID NO: 21, and the second light chain variable region comprises a sequence that is at least 80%, 85%, 90%, 95%, 99%, or 100% identical to SEQ ID NO: 22.

In some embodiments, the first heavy chain variable region comprises a sequence that is at least 80%, 85%, 90%, 95%, 99%, or 100% identical to SEQ ID NO: 144, the first light chain variable region comprises a sequence that is at least 80%, 85%, 90%, 95%, 99%, or 100% identical to SEQ ID NO: 145, the second heavy chain variable region comprises a sequence that is at least 80%, 85%, 90%, 95%, 99%, or 100% identical to SEQ ID NO: 85, and the second light chain variable region comprises a sequence that is at least 80%, 85%, 90%, 95%, 99%, or 100% identical to SEQ ID NO: 86.

In some embodiments, the VH1 comprises an amino acid sequence that is at least 80%, 85%, 90%, 95%, 99%, or 100% identical to a selected VH sequence, and the VL1 comprises an amino acid sequence that is at least 80%, 85%, 90%, 95%, 99%, or 100% identical to a selected VL sequence. In some embodiments, the selected VH sequence is SEQ ID NO: 134, and the selected VL sequence is SEQ ID NO: 135. In some embodiments, the selected VH sequence is SEQ ID NO: 144, and the selected VL sequence is SEQ ID NO: 145.

In some embodiments, the VH2 comprises an amino acid sequence that is at least 80%, 85%, 90%, 95%, 99%, or 100% identical to a selected VH sequence, and the VL2 comprises an amino acid sequence that is at least 80%, 85%, 90%, 95%, 99%, or 100% identical to a selected VL sequence, wherein the selected VH sequence and the selected VL sequence are one of the following:
(1) the selected VH sequence is SEQ ID NO: 21, and the selected VL sequence is SEQ ID NO: 22; and
(2) the selected VH sequence is SEQ ID NO: 85, and the selected VL sequence is SEQ ID NO: 86.

In some embodiments, the VH1 comprises VH1 CDR1, VH1 CDR2, and VH1 CDR3 that are identical to VH CDR1, VH CDR2, and VH CDR3 of a selected VH sequence; and the VL1 comprising VL1 CDR1, VL1 CDR2, and VL1 CDR3 that are identical to VL CDR1, VL CDR2, and VL CDR3 of a selected VL sequence. In some embodiments, the selected VH sequence is SEQ ID NO: 134, and the selected VL sequence is SEQ ID NO: 135. In some embodiments, the selected VH sequence is SEQ ID NO: 144, and the selected VL sequence is SEQ ID NO: 145.

In some embodiments, the VH2 comprises VH2 CDR1, VH2CDR2, and VH2 CDR3 that are identical to VH CDR1, VH CDR2, and VH CDR3 of a selected VH sequence; and the VL2 comprising VL2 CDR1, VL2 CDR2, and VL2 CDR3 that are identical to VL CDR1, VL CDR2, and VL CDR3 of a selected VL sequence, wherein the selected VH sequence and the selected VL sequence are one of the following:
(1) the selected VH sequence is SEQ ID NO: 21, and the selected VL sequence is SEQ ID NO: 22; and
(2) the selected VH sequence is SEQ ID NO: 85, and the selected VL sequence is SEQ ID NO: 86.

In some embodiments, the first antigen-binding domain is the antigen-binding domain of anti-EGFR antibody Cetuximab, Panitumumab, Necitumumab, or Eg-B4-VHH. In some embodiments, the first antigen-binding domain is the antigen-binding domain of anti-EGFR antibody Amivantamab (EGFR1). In some embodiments, the first antigen-binding domain is the antigen-binding domain of anti-EGFR antibody Nimotuzumab (EGFR2).

In some embodiments, the second antigen-binding domain is any of the antigen-binding domain of anti-CLEC5A antibodies, the chimeric antibodies thereof, and the humanized antibodies thereof described herein. In some embodiments, the second antigen-binding domain is the antigen-binding domain of anti-CLEC5A antibody 5C7, the chimeric antibodies thereof, and the humanized antibodies thereof.

The CDR sequences for 5C7, and 5C7 derived antibodies include CDRs of the heavy chain variable domain, SEQ ID NOs: 13, 15, and 17, and CDRs of the light chain variable domain, SEQ ID NOs: 18, 19, and 20, as defined by Kabat definition. Under Chothia definition, the CDR sequences of the heavy chain variable domain are set forth in SEQ ID NOs: 14, 16, and 17, and CDRs of the light chain variable domain are set forth in SEQ ID NOs: 18, 19, and 20.

In some embodiments, the first antigen-binding domain specifically binds to human, rabbit, mouse, monkey, or dog EGFR; and/or the second antigen-binding domain specifically binds to human, rabbit, mouse, monkey, or dog CLEC5A.

In some embodiments, the first antigen-binding domain is a human or humanized antigen-binding domain; and/or the second antigen-binding domain is a human or humanized antigen-binding domain.

In some embodiments, the first antigen-binding domain is a single-chain variable fragment (scFv); and/or the second antigen-binding domain is a scFv.

In some embodiments, the antibody or antigen-binding fragment thereof comprises a fragment crystallizable region (Fc region).

In some embodiments, the multispecific (e.g., bispecific) anti-EGFR/CLEC5A antibody described herein is designed to have an IgG1 subtype structure with the LALAPG mutations (L234A, L235A, and P329G mutations in EU numbering). In some embodiments, the multispecific (e.g., bispecific) anti-EGFR/CLEC5A antibody described herein is designed to have an IgG1 Fc region having an Alanine (A) at position 234; an Alanine (A) at position 235; and a Glycine (G) at position 329 in EU numbering. In some embodiments, The Fc region comprises an amino acid sequence that is about or at least 60%, 65%, 70%, 75%, 80%, 85%, 90%, 95%, or 100% identical to SEQ ID NO: 96.

In some embodiments, the anti-EGFR/CLEC5A antibody described herein can be designed to have an IgG1 Fc region having an Alanine (A) at position 236, a Leucine (L) at position 330, and a Glutamic acid (E) at position 332 in EU numbering. In some embodiments, The Fc region comprises an amino acid sequence that is about or at least 60%, 65%, 70%, 75%, 80%, 85%, 90%, 95%, or 100% identical to SEQ ID NO: 97.

In some embodiments, the multispecific (e.g., bispecific) anti-EGFR/CLEC5A antibody described herein is designed to have an Fc region that comprises an Aspartic acid (D) at position 239 and a glutamic acid (E) at position 332 in EU numbering. In some embodiments, the multispecific (e.g., bispecific) anti-EGFR/CLEC5A antibody described herein is designed to have an IgG1 subtype structure with the S239D+I332E mutations in EU numbering. In some embodiments, the multispecific antibody described herein is designed to have an IgG1 Fc region having an Aspartic acid (D) at position 239 and a glutamic acid (E) at position 332 in EU numbering.

In some embodiments, the multispecific (e.g., bispecific) anti-EGFR/CLEC5A antibody described herein can be designed to have an IgG1 subtype structure with knobs-into-holes (KIH) mutations, which can promote heterodimerization and avoid mispairing between the two heavy chains. In some embodiments, the anti-EGFR/CLEC5A antibody has a higher endocytosis rate than the corresponding monoclonal antibodies or the control bispecific antibodies.

The first antigen-binding fragment and the second antigen-binding fragment of the bi-specific antibodies or antigen-binding fragments described herein can be in any suitable configurations. In some embodiments, wherein the second antigen-binding domain is a single-chain fragment variable (scFv) domain comprising a light chain variable domain (VL) and a heavy chain variable domain (VH) linked by a first linker.

In some embodiments, the second antigen-binding domain is linked to the C-terminus of the light chain of the first antigen-binding domain through a second linker. In some embodiments, the heavy chain variable domain of the first antigen-binding domain is linked to the Fc region. In some embodiments, the VH1 is linked to a CH1 domain, and the VL1 is linked to a CL domain. A schematic illustration of this configuration is shown in **FIG. 9C**. In some embodiments, the antibody or antigen-binding fragment thereof described herein comprises a first heavy chain and a first light chain; and a second heavy chain and a second light chain. A schematic illustration of this configuration is shown in **FIG. 9A**. In some embodiments, the Fc region comprises knobs-into-holes (KIH) mutations. In some embodiments, the first heavy chain includes one or more knob mutations, and the second heavy chain includes one or more hole mutations. In some embodiments, the first heavy chain includes one or more hole mutations, and the second heavy chain includes one or more knob mutations. In some embodiments, the multispecific (e.g., bispecific) anti-EGFR/CLEC5A antibody described herein can have a structure shown in any one of **FIGs. 9A-****9F**.

In some embodiments, the multispecific (e.g., bispecific) anti-EGFR/CLEC5A antibody includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 146 or 148. In some embodiments, the heavy chain includes an IgG1 Fc region comprising the optimized mutations (SEQ ID NO: 97).

In some embodiments, the multispecific (e.g., bispecific) anti-EGFR/CLEC5A antibody includes a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 147 or 149.

In some embodiments, the multispecific (e.g., bispecific) anti-EGFR/CLEC5A antibody is referred to as "EGFR1/5C7 (2+2 A) Fc-optimized" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 146; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 147. In some embodiments, the multispecific (e.g., bispecific) anti-EGFR/CLEC5A antibody is referred to as "EGFR2/5C7 (2+2 A) Fc-optimized" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 148; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 149.

The linkers described herein can be any suitable linkers known in the art. In some embodiments, the linker can comprise a spacer sequence. Various spacer sequences are known in the art, including, without limitation, glycine serine (GS) spacers (also known as GS linkers) such as (GS)n, (SG)n, and (GGGGS)n (SEQ ID NO: 99), where n represents an integer of at least 1. Those of skill in the art would be able to select the appropriate spacer sequence.

In some embodiments, knobs-into-holes mutations were introduced in the Fc regions of the multispecific (e.g., bispecific) antibodies to reduce the chance of wrong pairing between the two heavy chains.

The disclosure also provides nucleic acid comprising a polynucleotide encoding an anti-EGFR/CLEC5A antibody. The immunoglobulin heavy chain or immunoglobulin light chain in the anti-EGFR/CLEC5A antibody comprises CDRs as shown in **Table 22**. When the polypeptides are paired with corresponding polypeptide (e.g., a corresponding heavy chain variable region or a corresponding light chain variable region), the paired polypeptides bind to EGFR and/or CLEC5A.

### Anti-EpCAM/CLEC5A Antibodies and Antigen-binding Fragments Thereof

Epithelial cell adhesion molecule (EpCAM) - also known as tumor-associated calcium signal transducer 1 (TACST-1), 17-1A and CD326- is a 40-kDa transmembrane glycoprotein that is highly expressed in epithelial cancers, and at lower levels in normal simple epithelia. The structure and function of EpCAM is reviewed, for example, in Schnell et al., Biochimica et Biophysica Acta - Biomembranes (2013), 1828(8): 1989-2001; Trzpis et al. Am J Pathol. (2007) 171(2): 386-395 and Baeuerle and Gires, Br. J. Cancer, (2007) 96:417-423.

EpCAM is expressed at the basolateral membrane and plays a role in calcium-independent homophilic cell adhesion. The mature EpCAM molecule (after processing to remove the 23 amino acid signal peptide) comprises an N-terminal, 242 amino acid extracellular domain comprising an epidermal growth factor-like repeat region, a human thyroglobulin (TY) repeat region and a cysteine-poor region, a single-pass 23 amino acid transmembrane domain and a Cterminal, 26 amino acid cytoplasmic domain comprising two binding sites for a-actinin and a NPXY internalization motif. EpCAM is frequently overexpressed in cancers of epithelial origin and is expressed by cancer stem cells and is therefore a molecule of significant interest for therapy and diagnosis. Owing to its frequent and high expression on carcinomas and their metastases, EpCAM serves as a prognostic marker, a therapeutic target, and an anchor molecule on circulating and disseminated tumor cells (CTCs/DTCs), which are considered the major source for metastatic cancer cells. The extracellular domain EpCAM can be cleaved to yield the soluble extracellular domain molecule EpEX, and the intracellular molecule EpICD. EpICD has been shown to associate with other proteins to form a nuclear complex which upregulates the expression of genes promoting cell proliferation. EpCAM may also be involved in the epithelial to mesenchymal cell transition (EMT) and may contribute to the formation of large metastases.

The disclosure provides multispecific (e.g., bispecific) antibodies and antigen-binding fragments thereof that specifically bind to EpCAM/CLEC5A (e.g., human EpCAM/CLEC5A). In one aspect, the disclosure provides an anti-EpCAM/CLEC5A multispecific (e.g., bispecific) antibody or antigen-binding fragment thereof, comprising: a first antigen-binding domain that specifically binds to EpCAM; and a second antigen-binding domain that specifically binds to CLEC5A.

In some embodiments, the first antigen-binding domain comprises a first heavy chain variable region (VH1) and a first light chain variable region (VL1); and the second antigen-binding domain comprises a second heavy chain variable region (VH2) and a second light chain variable region (VL2).

In some embodiments, the first heavy chain variable region (VH1) comprises complementarity determining regions (CDRs) 1, 2, and 3, wherein the VH1 CDR1 region comprises an amino acid sequence that is at least 80% identical to a selected VH1 CDR1 amino acid sequence, the VH1 CDR2 region comprises an amino acid sequence that is at least 80% identical to a selected VH1 CDR2 amino acid sequence, and the VH1 CDR3 region comprises an amino acid sequence that is at least 80% identical to a selected VH1 CDR3 amino acid sequence; and
the first light chain variable region (VL1) comprises CDRs 1, 2, and 3, wherein the VL1 CDR1 region comprises an amino acid sequence that is at least 80% identical to a selected VL1 CDR1 amino acid sequence, the VL1 CDR2 region comprises an amino acid sequence that is at least 80% identical to a selected VL1 CDR2 amino acid sequence, and the VL1 CDR3 region comprises an amino acid sequence that is at least 80% identical to a selected VL1 CDR3 amino acid sequence, wherein the selected VH1 CDRs 1, 2, 3 amino acid sequences, and the selected VL1 CDRs 1, 2, and 3 amino acid sequences are one of the following:
   (1) the selected VH1 CDRs 1, 2, 3 amino acid sequences are set forth in SEQ ID NOs: 150, 152, and 154, respectively, and the selected VL1 CDRs 1, 2, 3 amino acid sequences are set forth in SEQ ID NOs: 155, 156, and 157, respectively; and
   (2) the selected VH1 CDRs 1, 2, 3 amino acid sequences are set forth in SEQ ID NOs: 151, 153, and 154, respectively, and the selected VL1 CDRs 1, 2, 3 amino acid sequences are set forth in SEQ ID NOs: 155, 156, and 157, respectively;
the second heavy chain variable region (VH2) comprises complementarity determining regions (CDRs) 1, 2, and 3, wherein the VH2 CDR1 region comprises an amino acid sequence that is at least 80% identical to a selected VH2 CDR1 amino acid sequence, the VH2 CDR2 region comprises an amino acid sequence that is at least 80% identical to a selected VH2 CDR2 amino acid sequence, and the VH2 CDR3 region comprises an amino acid sequence that is at least 80% identical to a selected VH2 CDR3 amino acid sequence;
and the second light chain variable region (VL2) comprises CDRs 1, 2, and 3, wherein the VL2 CDR1 region comprises an amino acid sequence that is at least 80% identical to a selected VL2 CDR1 amino acid sequence, the VL2 CDR2 region comprises an amino acid sequence that is at least 80% identical to a selected VL2 CDR2 amino acid sequence, and the VL2 CDR3 region comprises an amino acid sequence that is at least 80% identical to a selected VL2 CDR3 amino acid sequence,
wherein the selected VH2 CDRs 1, 2, and 3 amino acid sequences, and the selected VL2 CDRs 1, 2, and 3 amino acid sequences are one of the following:
   (1) the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively; and
   (2) the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the selected VH1 CDRs 1, 2, 3 amino acid sequences are set forth in SEQ ID NOs: 150, 152, and 154, respectively, the selected VL1 CDRs 1, 2, 3 amino acid sequences are set forth in SEQ ID NOs: 155, 156, and 157, respectively; the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively.

In some embodiments, the selected VH1 CDRs 1, 2, 3 amino acid sequences are set forth in SEQ ID NOs: 151, 153, and 154, respectively, the selected VL1 CDRs 1, 2, 3 amino acid sequences are set forth in SEQ ID NOs: 155, 156, and 157, respectively; the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the first heavy chain variable region comprises a sequence that is at least 80%, 85%, 90%, 95%, 99%, or 100% identical to SEQ ID NO: 158, the first light chain variable region comprises a sequence that is at least 80%, 85%, 90%, 95%, 99%, or 100% identical to SEQ ID NO: 159, the second heavy chain variable region comprises a sequence that is at least 80%, 85%, 90%, 95%, 99%, or 100% identical to SEQ ID NO: 21, and the second light chain variable region comprises a sequence that is at least 80%, 85%, 90%, 95%, 99%, or 100% identical to SEQ ID NO: 22.

In some embodiments, the first heavy chain variable region comprises a sequence that is at least 80%, 85%, 90%, 95%, 99%, or 100% identical to SEQ ID NO: 158, the first light chain variable region comprises a sequence that is at least 80%, 85%, 90%, 95%, 99%, or 100% identical to SEQ ID NO: 159, the second heavy chain variable region comprises a sequence that is at least 80%, 85%, 90%, 95%, 99%, or 100% identical to SEQ ID NO: 85, and the second light chain variable region comprises a sequence that is at least 80%, 85%, 90%, 95%, 99%, or 100% identical to SEQ ID NO: 86.

In some embodiments, the VH1 comprises an amino acid sequence that is at least 80%, 85%, 90%, 95%, 99%, or 100% identical to a selected VH sequence, and the VL1 comprises an amino acid sequence that is at least 80%, 85%, 90%, 95%, 99%, or 100% identical to a selected VL sequence. In some embodiments, the selected VH sequence is SEQ ID NO: 158, and the selected VL sequence is SEQ ID NO: 159.

In some embodiments, the VH2 comprises an amino acid sequence that is at least 80%, 85%, 90%, 95%, 99%, or 100% identical to a selected VH sequence, and the VL2 comprises an amino acid sequence that is at least 80%, 85%, 90%, 95%, 99%, or 100% identical to a selected VL sequence, wherein the selected VH sequence and the selected VL sequence are one of the following:
(1) the selected VH sequence is SEQ ID NO: 21, and the selected VL sequence is SEQ ID NO: 22; and
(2) the selected VH sequence is SEQ ID NO: 85, and the selected VL sequence is SEQ ID NO: 86.

In some embodiments, the VH1 comprises VH1 CDR1, VH1 CDR2, and VH1 CDR3 that are identical to VH CDR1, VH CDR2, and VH CDR3 of a selected VH sequence; and the VL1 comprising VL1 CDR1, VL1 CDR2, and VL1 CDR3 that are identical to VL CDR1, VL CDR2, and VL CDR3 of a selected VL sequence. In some embodiments, the selected VH sequence is SEQ ID NO: 158, and the selected VL sequence is SEQ ID NO: 159.

In some embodiments, the VH2 comprises VH2 CDR1, VH2 CDR2, and VH2 CDR3 that are identical to VH CDR1, VH CDR2, and VH CDR3 of a selected VH sequence; and the VL2 comprising VL2 CDR1, VL2 CDR2, and VL2 CDR3 that are identical to VL CDR1, VL CDR2, and VL CDR3 of a selected VL sequence, wherein the selected VH sequence and the selected VL sequence are one of the following:
(1) the selected VH sequence is SEQ ID NO: 21, and the selected VL sequence is SEQ ID NO: 22; and
(2) the selected VH sequence is SEQ ID NO: 85, and the selected VL sequence is SEQ ID NO: 86.

In some embodiments, the first antigen-binding domain is the antigen-binding domain of anti-EpCAM antibody Solitomab.

In some embodiments, the second antigen-binding domain is any of the antigen-binding domain of anti-CLEC5A antibodies, the chimeric antibodies thereof, and the humanized antibodies thereof described herein. In some embodiments, the second antigen-binding domain is the antigen-binding domain of anti-CLEC5A antibody 5C7, the chimeric antibodies thereof, and the humanized antibodies thereof.

The CDR sequences for 5C7, and 5C7 derived antibodies include CDRs of the heavy chain variable domain, SEQ ID NOs: 13, 15, and 17, and CDRs of the light chain variable domain, SEQ ID NOs: 18, 19, and 20, as defined by Kabat definition. Under Chothia definition, the CDR sequences of the heavy chain variable domain are set forth in SEQ ID NOs: 14, 16, and 17, and CDRs of the light chain variable domain are set forth in SEQ ID NOs: 18, 19, and 20.

In some embodiments, the first antigen-binding domain specifically binds to human, rabbit, mouse, monkey, or dog EpCAM; and/or the second antigen-binding domain specifically binds to human, rabbit, mouse, monkey, or dog CLEC5A.

In some embodiments, the first antigen-binding domain is a human or humanized antigen-binding domain; and/or the second antigen-binding domain is a human or humanized antigen-binding domain.

In some embodiments, the first antigen-binding domain is a single-chain variable fragment (scFv); and/or the second antigen-binding domain is a scFv.

In some embodiments, the antibody or antigen-binding fragment thereof comprises a fragment crystallizable region (Fc region).

In some embodiments, the multispecific (e.g., bispecific) anti-EpCAM/CLEC5A antibody described herein is designed to have an IgG1 subtype structure with the LALAPG mutations (L234A, L235A, and P329G mutations in EU numbering). In some embodiments, the multispecific (e.g., bispecific) anti-EpCAM/CLEC5A antibody described herein is designed to have an IgG1 Fc region having an Alanine (A) at position 234; an Alanine (A) at position 235; and a Glycine (G) at position 329 in EU numbering. In some embodiments, The Fc region comprises an amino acid sequence that is about or at least 60%, 65%, 70%, 75%, 80%, 85%, 90%, 95%, or 100% identical to SEQ ID NO: 96.

In some embodiments, the anti-EpCAM/CLEC5A antibody described herein can be designed to have an IgG1 Fc region having an Alanine (A) at position 236, a Leucine (L) at position 330, and a Glutamic acid (E) at position 332 in EU numbering. In some embodiments, The Fc region comprises an amino acid sequence that is about or at least 60%, 65%, 70%, 75%, 80%, 85%, 90%, 95%, or 100% identical to SEQ ID NO: 97.

In some embodiments, the multispecific (e.g., bispecific) anti-EpCAM/CLEC5A antibody described herein is designed to have an Fc region that comprises an Aspartic acid (D) at position 239 and a glutamic acid (E) at position 332 in EU numbering. In some embodiments, the multispecific (e.g., bispecific) anti-EpCAM/CLEC5A antibody described herein is designed to have an IgG1 subtype structure with the S239D+I332E mutations in EU numbering. In some embodiments, the multispecific antibody described herein is designed to have an IgG1 Fc region having an Aspartic acid (D) at position 239 and a glutamic acid (E) at position 332 in EU numbering.

In some embodiments, the multispecific (e.g., bispecific) anti-EpCAM/CLEC5A antibody described herein can be designed to have an IgG1 subtype structure with knobs-into-holes (KIH) mutations, which can promote heterodimerization and avoid mispairing between the two heavy chains. In some embodiments, the anti-EpCAM/CLEC5A antibody has a higher endocytosis rate than the corresponding monoclonal antibodies or the control bispecific antibodies.

The first antigen-binding fragment and the second antigen-binding fragment of the bi-specific antibodies or antigen-binding fragments described herein can be in any suitable configurations. In some embodiments, wherein the second antigen-binding domain is a single-chain fragment variable (scFv) domain comprising a light chain variable domain (VL) and a heavy chain variable domain (VH) linked by a first linker.

In some embodiments, the second antigen-binding domain is linked to the C-terminus of the light chain of the first antigen-binding domain through a second linker. In some embodiments, the heavy chain variable domain of the first antigen-binding domain is linked to the Fc region. In some embodiments, the VH1 is linked to a CH1 domain, and the VL1 is linked to a CL domain. A schematic illustration of this configuration is shown in **FIG. 9C**. In some embodiments, the antibody or antigen-binding fragment thereof described herein comprises a first heavy chain and a first light chain; and a second heavy chain and a second light chain. A schematic illustration of this configuration is shown in **FIG. 9A**. In some embodiments, the Fc region comprises knobs-into-holes (KIH) mutations. In some embodiments, the first heavy chain includes one or more knob mutations, and the second heavy chain includes one or more hole mutations. In some embodiments, the first heavy chain includes one or more hole mutations, and the second heavy chain includes one or more knob mutations. In some embodiments, the multispecific (e.g., bispecific) anti-EpCAM/CLEC5A antibody described herein can have a structure shown in any one of **FIGs. 9A-****9F**.

In some embodiments, the multispecific (e.g., bispecific) anti-EpCAM/CLEC5A antibody includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 160 or 162. In some embodiments, the heavy chain includes an IgG1 Fc region comprising the optimized mutations (SEQ ID NO: 97).

In some embodiments, the multispecific (e.g., bispecific) anti-EpCAM/CLEC5A antibody includes a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 161 or 163.

In some embodiments, the multispecific (e.g., bispecific) anti-EpCAM/CLEC5A antibody is referred to as "EpCAM/5C7 (2+2 A) Fc-optimized" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 160; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 161. In some embodiments, the heavy chain sequence is about or at least 80%, 85%, 86%, 87%, 88%, 89%, 90%, 91%, 92%, 93%, 94%, 95%, 96%, 97%, 98%, 99% or 100% identical to SEQ ID NO: 162; and the light chain sequence is about or at least 80%, 85%, 86%, 87%, 88%, 89%, 90%, 91%, 92%, 93%, 94%, 95%, 96%, 97%, 98%, 99% or 100% identical to SEQ ID NO: 163.

The linkers described herein can be any suitable linkers known in the art. In some embodiments, the linker can comprise a spacer sequence. Various spacer sequences are known in the art, including, without limitation, glycine serine (GS) spacers (also known as GS linkers) such as (GS)n, (SG)n, and (GGGGS)n (SEQ ID NO: 99), where n represents an integer of at least 1. Those of skill in the art would be able to select the appropriate spacer sequence.

In some embodiments, knobs-into-holes mutations were introduced in the Fc regions of the multispecific (e.g., bispecific) antibodies to reduce the chance of wrong pairing between the two heavy chains.

The disclosure also provides nucleic acid comprising a polynucleotide encoding an anti-EpCAM/CLEC5A antibody. The immunoglobulin heavy chain or immunoglobulin light chain in the anti-EpCAM/CLEC5A antibody comprises CDRs as shown in **Table 22**. When the polypeptides are paired with corresponding polypeptide (e.g., a corresponding heavy chain variable region or a corresponding light chain variable region), the paired polypeptides bind to EpCAM and/or CLEC5A.

### Anti-GPRC5D/CLEC5A Antibodies and Antigen-binding Fragments Thereof

G-protein coupled receptor family C group 5 member D (GPRC5D) is a protein that in humans is encoded by the GPRC5D gene. The protein encoded by this gene is a member of the G protein-coupled receptor family.

The disclosure provides multispecific (e.g., bispecific) antibodies and antigen-binding fragments thereof that specifically bind to GPRC5D/CLEC5A (e.g., human GPRC5D/CLEC5A). In one aspect, the disclosure provides an anti-GPRC5D/CLEC5A multispecific (e.g., bispecific) antibody or antigen-binding fragment thereof, comprising: a first antigen-binding domain that specifically binds to GPRC5D; and a second antigen-binding domain that specifically binds to CLEC5A.

In some embodiments, the first antigen-binding domain comprises a first heavy chain variable region (VH1) and a first light chain variable region (VL1); and the second antigen-binding domain comprises a second heavy chain variable region (VH2) and a second light chain variable region (VL2).

In some embodiments, the first heavy chain variable region (VH1) comprises complementarity determining regions (CDRs) 1, 2, and 3, wherein the VH1 CDR1 region comprises an amino acid sequence that is at least 80% identical to a selected VH1 CDR1 amino acid sequence, the VH1 CDR2 region comprises an amino acid sequence that is at least 80% identical to a selected VH1 CDR2 amino acid sequence, and the VH1 CDR3 region comprises an amino acid sequence that is at least 80% identical to a selected VH1 CDR3 amino acid sequence; and
the first light chain variable region (VL1) comprises CDRs 1, 2, and 3, wherein the VL1 CDR1 region comprises an amino acid sequence that is at least 80% identical to a selected VL1 CDR1 amino acid sequence, the VL1 CDR2 region comprises an amino acid sequence that is at least 80% identical to a selected VL1 CDR2 amino acid sequence, and the VL1 CDR3 region comprises an amino acid sequence that is at least 80% identical to a selected VL1 CDR3 amino acid sequence, wherein the selected VH1 CDRs 1, 2, 3 amino acid sequences, and the selected VL1 CDRs 1, 2, and 3 amino acid sequences are one of the following:
   (1) the selected VH1 CDRs 1, 2, 3 amino acid sequences are set forth in SEQ ID NOs: 164, 166, and 168, respectively, and the selected VL1 CDRs 1, 2, 3 amino acid sequences are set forth in SEQ ID NOs: 169, 170, and 171, respectively; and
   (2) the selected VH1 CDRs 1, 2, 3 amino acid sequences are set forth in SEQ ID NOs: 165, 167, and 168, respectively, and the selected VL1 CDRs 1, 2, 3 amino acid sequences are set forth in SEQ ID NOs: 169, 170, and 171, respectively;
the second heavy chain variable region (VH2) comprises complementarity determining regions (CDRs) 1, 2, and 3, wherein the VH2 CDR1 region comprises an amino acid sequence that is at least 80% identical to a selected VH2 CDR1 amino acid sequence, the VH2 CDR2 region comprises an amino acid sequence that is at least 80% identical to a selected VH2 CDR2 amino acid sequence, and the VH2 CDR3 region comprises an amino acid sequence that is at least 80% identical to a selected VH2 CDR3 amino acid sequence;
and the second light chain variable region (VL2) comprises CDRs 1, 2, and 3, wherein the VL2 CDR1 region comprises an amino acid sequence that is at least 80% identical to a selected VL2 CDR1 amino acid sequence, the VL2 CDR2 region comprises an amino acid sequence that is at least 80% identical to a selected VL2 CDR2 amino acid sequence, and the VL2 CDR3 region comprises an amino acid sequence that is at least 80% identical to a selected VL2 CDR3 amino acid sequence,
wherein the selected VH2 CDRs 1, 2, and 3 amino acid sequences, and the selected VL2 CDRs 1, 2, and 3 amino acid sequences are one of the following:
   (1) the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively; and
   (2) the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the selected VH1 CDRs 1, 2, 3 amino acid sequences are set forth in SEQ ID NOs: 164, 166, and 168, respectively, the selected VL1 CDRs 1, 2, 3 amino acid sequences are set forth in SEQ ID NOs: 169, 170, and 171, respectively; the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively.

In some embodiments, the selected VH1 CDRs 1, 2, 3 amino acid sequences are set forth in SEQ ID NOs: 165, 167, and 168, respectively, the selected VL1 CDRs 1, 2, 3 amino acid sequences are set forth in SEQ ID NOs: 169, 170, and 171, respectively; the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the first heavy chain variable region comprises a sequence that is at least 80%, 85%, 90%, 95%, 99%, or 100% identical to SEQ ID NO: 172, the first light chain variable region comprises a sequence that is at least 80%, 85%, 90%, 95%, 99%, or 100% identical to SEQ ID NO: 173, the second heavy chain variable region comprises a sequence that is at least 80%, 85%, 90%, 95%, 99%, or 100% identical to SEQ ID NO: 21, and the second light chain variable region comprises a sequence that is at least 80%, 85%, 90%, 95%, 99%, or 100% identical to SEQ ID NO: 22.

In some embodiments, the first heavy chain variable region comprises a sequence that is at least 80%, 85%, 90%, 95%, 99%, or 100% identical to SEQ ID NO: 172, the first light chain variable region comprises a sequence that is at least 80%, 85%, 90%, 95%, 99%, or 100% identical to SEQ ID NO: 173, the second heavy chain variable region comprises a sequence that is at least 80%, 85%, 90%, 95%, 99%, or 100% identical to SEQ ID NO: 85, and the second light chain variable region comprises a sequence that is at least 80%, 85%, 90%, 95%, 99%, or 100% identical to SEQ ID NO: 86.

In some embodiments, the VH1 comprises an amino acid sequence that is at least 80%, 85%, 90%, 95%, 99%, or 100% identical to a selected VH sequence, and the VL1 comprises an amino acid sequence that is at least 80%, 85%, 90%, 95%, 99%, or 100% identical to a selected VL sequence. In some embodiments, the selected VH sequence is SEQ ID NO: 172, and the selected VL sequence is SEQ ID NO: 173.

In some embodiments, the VH2 comprises an amino acid sequence that is at least 80%, 85%, 90%, 95%, 99%, or 100% identical to a selected VH sequence, and the VL2 comprises an amino acid sequence that is at least 80%, 85%, 90%, 95%, 99%, or 100% identical to a selected VL sequence, wherein the selected VH sequence and the selected VL sequence are one of the following:
(1) the selected VH sequence is SEQ ID NO: 21, and the selected VL sequence is SEQ ID NO: 22; and
(2) the selected VH sequence is SEQ ID NO: 85, and the selected VL sequence is SEQ ID NO: 86.

In some embodiments, the VH1 comprises VH1 CDR1, VH1 CDR2, and VH1 CDR3 that are identical to VH CDR1, VH CDR2, and VH CDR3 of a selected VH sequence; and the VL1 comprising VL1 CDR1, VL1 CDR2, and VL1 CDR3 that are identical to VL CDR1, VL CDR2, and VL CDR3 of a selected VL sequence. In some embodiments, the selected VH sequence is SEQ ID NO: 172, and the selected VL sequence is SEQ ID NO: 173.

In some embodiments, the VH2 comprises VH2 CDR1, VH2CDR2, and VH2 CDR3 that are identical to VH CDR1, VH CDR2, and VH CDR3 of a selected VH sequence; and the VL2 comprising VL2 CDR1, VL2 CDR2, and VL2 CDR3 that are identical to VL CDR1, VL CDR2, and VL CDR3 of a selected VL sequence, wherein the selected VH sequence and the selected VL sequence are one of the following:
(1) the selected VH sequence is SEQ ID NO: 21, and the selected VL sequence is SEQ ID NO: 22; and
(2) the selected VH sequence is SEQ ID NO: 85, and the selected VL sequence is SEQ ID NO: 86.

In some embodiments, the second antigen-binding domain is any of the antigen-binding domain of anti-CLEC5A antibodies, the chimeric antibodies thereof, and the humanized antibodies thereof described herein. In some embodiments, the second antigen-binding domain is the antigen-binding domain of anti-CLEC5A antibody 5C7, the chimeric antibodies thereof, and the humanized antibodies thereof.

The CDR sequences for 5C7, and 5C7 derived antibodies include CDRs of the heavy chain variable domain, SEQ ID NOs: 13, 15, and 17, and CDRs of the light chain variable domain, SEQ ID NOs: 18, 19, and 20, as defined by Kabat definition. Under Chothia definition, the CDR sequences of the heavy chain variable domain are set forth in SEQ ID NOs: 14, 16, and 17, and CDRs of the light chain variable domain are set forth in SEQ ID NOs: 18, 19, and 20.

In some embodiments, the first antigen-binding domain specifically binds to human, rabbit, mouse, monkey, or dog GPRC5D; and/or the second antigen-binding domain specifically binds to human, rabbit, mouse, monkey, or dog CLEC5A.

In some embodiments, the first antigen-binding domain is a human or humanized antigen-binding domain; and/or the second antigen-binding domain is a human or humanized antigen-binding domain.

In some embodiments, the first antigen-binding domain is a single-chain variable fragment (scFv); and/or the second antigen-binding domain is a scFv.

In some embodiments, the antibody or antigen-binding fragment thereof comprises a fragment crystallizable region (Fc region).

In some embodiments, the multispecific (e.g., bispecific) anti-GPRC5D/CLEC5A antibody described herein is designed to have an IgG1 subtype structure with the LALAPG mutations (L234A, L235A, and P329G mutations in EU numbering). In some embodiments, the multispecific (e.g., bispecific) anti-GPRC5D/CLEC5A antibody described herein is designed to have an IgG1 Fc region having an Alanine (A) at position 234; an Alanine (A) at position 235; and a Glycine (G) at position 329 in EU numbering. In some embodiments, The Fc region comprises an amino acid sequence that is about or at least 60%, 65%, 70%, 75%, 80%, 85%, 90%, 95%, or 100% identical to SEQ ID NO: 96.

In some embodiments, the anti-GPRC5D/CLEC5A antibody described herein can be designed to have an IgG1 Fc region having an Alanine (A) at position 236, a Leucine (L) at position 330, and a Glutamic acid (E) at position 332 in EU numbering. In some embodiments, The Fc region comprises an amino acid sequence that is about or at least 60%, 65%, 70%, 75%, 80%, 85%, 90%, 95%, or 100% identical to SEQ ID NO: 97.

In some embodiments, the multispecific (e.g., bispecific) anti-GPRC5D/CLEC5A antibody described herein is designed to have an Fc region that comprises an Aspartic acid (D) at position 239 and a glutamic acid (E) at position 332 in EU numbering. In some embodiments, the multispecific (e.g., bispecific) anti-GPRC5D/CLEC5A antibody described herein is designed to have an IgG1 subtype structure with the S239D+I332E mutations in EU numbering. In some embodiments, the multispecific antibody described herein is designed to have an IgG1 Fc region having an Aspartic acid (D) at position 239 and a glutamic acid (E) at position 332 in EU numbering.

In some embodiments, the multispecific (e.g., bispecific) anti-GPRC5D/CLEC5A antibody described herein can be designed to have an IgG1 subtype structure with knobs-into-holes (KIH) mutations, which can promote heterodimerization and avoid mispairing between the two heavy chains. In some embodiments, the anti-GPRC5D/CLEC5A antibody has a higher endocytosis rate than the corresponding monoclonal antibodies or the control bispecific antibodies.

The first antigen-binding fragment and the second antigen-binding fragment of the bi-specific antibodies or antigen-binding fragments described herein can be in any suitable configurations. In some embodiments, wherein the second antigen-binding domain is a single-chain fragment variable (scFv) domain comprising a light chain variable domain (VL) and a heavy chain variable domain (VH) linked by a first linker.

In some embodiments, the second antigen-binding domain is linked to the C-terminus of the light chain of the first antigen-binding domain through a second linker. In some embodiments, the heavy chain variable domain of the first antigen-binding domain is linked to the Fc region. In some embodiments, the VH1 is linked to a CH1 domain, and the VL1 is linked to a CL domain. A schematic illustration of this configuration is shown in **FIG. 9C**. In some embodiments, the antibody or antigen-binding fragment thereof described herein comprises a first heavy chain and a first light chain; and a second heavy chain and a second light chain. A schematic illustration of this configuration is shown in **FIG. 9A**. In some embodiments, the Fc region comprises knobs-into-holes (KIH) mutations. In some embodiments, the first heavy chain includes one or more knob mutations, and the second heavy chain includes one or more hole mutations. In some embodiments, the first heavy chain includes one or more hole mutations, and the second heavy chain includes one or more knob mutations. In some embodiments, the multispecific (e.g., bispecific) anti-GPRC5D/CLEC5A antibody described herein can have a structure shown in any one of **FIGs. 9A-****9F**.

In some embodiments, the multispecific (e.g., bispecific) anti-GPRC5D/CLEC5A antibody includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 174. In some embodiments, the heavy chain includes an IgG1 Fc region comprising the optimized mutations (SEQ ID NO: 97).

In some embodiments, the multispecific (e.g., bispecific) anti-GPRC5D/CLEC5A antibody includes a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 175.

In some embodiments, the multispecific (e.g., bispecific) anti-GPRC5D/CLEC5A antibody is referred to as "GPRC5D/5C7 (2+2 A) Fc-optimized" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 174; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 175.

The linkers described herein can be any suitable linkers known in the art. In some embodiments, the linker can comprise a spacer sequence. Various spacer sequences are known in the art, including, without limitation, glycine serine (GS) spacers (also known as GS linkers) such as (GS)n, (SG)n, and (GGGGS)n (SEQ ID NO: 99), where n represents an integer of at least 1. Those of skill in the art would be able to select the appropriate spacer sequence.

In some embodiments, knobs-into-holes mutations were introduced in the Fc regions of the multispecific (e.g., bispecific) antibodies to reduce the chance of wrong pairing between the two heavy chains.

The disclosure also provides nucleic acid comprising a polynucleotide encoding an anti-GPRC5D/CLEC5A antibody. The immunoglobulin heavy chain or immunoglobulin light chain in the anti-GPRC5D/CLEC5A antibody comprises CDRs as shown in Table 22. When the polypeptides are paired with corresponding polypeptide (e.g., a corresponding heavy chain variable region or a corresponding light chain variable region), the paired polypeptides bind to GPRC5D and/or CLEC5A.

### Anti-BCMA/CLEC5A Antibodies and Antigen-binding Fragments Thereof

B-cell maturation antigen (BCMA or BCM), also known as tumor necrosis factor receptor superfamily member 17 (TNFRSF17), is a protein that in humans is encoded by the TNFRSF17 gene. BCMA is a cell surface receptor of the TNF receptor superfamily which recognizes B-cell activating factor (BAFF). Serum B-cell maturation antigen (sBCMA) is the cleaved form of BCMA, found at low levels in the serum of normal patients and generally elevated in patients with multiple myeloma (MM).

The disclosure provides multispecific (e.g., bispecific) antibodies and antigen-binding fragments thereof that specifically bind to BCMA/CLEC5A (e.g., human BCMA/CLEC5A). In one aspect, the disclosure provides an anti-BCMA/CLEC5A multispecific (e.g., bispecific) antibody or antigen-binding fragment thereof, comprising: a first antigen-binding domain that specifically binds to BCMA; and a second antigen-binding domain that specifically binds to CLEC5A.

In some embodiments, the first antigen-binding domain comprises a first heavy chain variable region (VH1) and a first light chain variable region (VL1); and the second antigen-binding domain comprises a second heavy chain variable region (VH2) and a second light chain variable region (VL2).

In some embodiments, the first heavy chain variable region (VH1) comprises complementarity determining regions (CDRs) 1, 2, and 3, wherein the VH1 CDR1 region comprises an amino acid sequence that is at least 80% identical to a selected VH1 CDR1 amino acid sequence, the VH1 CDR2 region comprises an amino acid sequence that is at least 80% identical to a selected VH1 CDR2 amino acid sequence, and the VH1 CDR3 region comprises an amino acid sequence that is at least 80% identical to a selected VH1 CDR3 amino acid sequence; and
the first light chain variable region (VL1) comprises CDRs 1, 2, and 3, wherein the VL1 CDR1 region comprises an amino acid sequence that is at least 80% identical to a selected VL1 CDR1 amino acid sequence, the VL1 CDR2 region comprises an amino acid sequence that is at least 80% identical to a selected VL1 CDR2 amino acid sequence, and the VL1 CDR3 region comprises an amino acid sequence that is at least 80% identical to a selected VL1 CDR3 amino acid sequence, wherein the selected VH1 CDRs 1, 2, 3 amino acid sequences, and the selected VL1 CDRs 1, 2, and 3 amino acid sequences are one of the following:
   (1) the selected VH1 CDRs 1, 2, 3 amino acid sequences are set forth in SEQ ID NOs: 176, 178, and 180, respectively, and the selected VL1 CDRs 1, 2, 3 amino acid sequences are set forth in SEQ ID NOs: 181, 182, and 183, respectively; and
   (2) the selected VH1 CDRs 1, 2, 3 amino acid sequences are set forth in SEQ ID NOs: 177, 179, and 180, respectively, and the selected VL1 CDRs 1, 2, 3 amino acid sequences are set forth in SEQ ID NOs: 181, 182, and 183, respectively;
the second heavy chain variable region (VH2) comprises complementarity determining regions (CDRs) 1, 2, and 3, wherein the VH2 CDR1 region comprises an amino acid sequence that is at least 80% identical to a selected VH2 CDR1 amino acid sequence, the VH2 CDR2 region comprises an amino acid sequence that is at least 80% identical to a selected VH2 CDR2 amino acid sequence, and the VH2 CDR3 region comprises an amino acid sequence that is at least 80% identical to a selected VH2 CDR3 amino acid sequence;
and the second light chain variable region (VL2) comprises CDRs 1, 2, and 3, wherein the VL2 CDR1 region comprises an amino acid sequence that is at least 80% identical to a selected VL2 CDR1 amino acid sequence, the VL2 CDR2 region comprises an amino acid sequence that is at least 80% identical to a selected VL2 CDR2 amino acid sequence, and the VL2 CDR3 region comprises an amino acid sequence that is at least 80% identical to a selected VL2 CDR3 amino acid sequence,
wherein the selected VH2 CDRs 1, 2, and 3 amino acid sequences, and the selected VL2 CDRs 1, 2, and 3 amino acid sequences are one of the following:
   (1) the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively; and
   (2) the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the selected VH1 CDRs 1, 2, 3 amino acid sequences are set forth in SEQ ID NOs: 176, 178, and 180, respectively, the selected VL1 CDRs 1, 2, 3 amino acid sequences are set forth in SEQ ID NOs: 181, 182, and 183, respectively; the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively.

In some embodiments, the selected VH1 CDRs 1, 2, 3 amino acid sequences are set forth in SEQ ID NOs: 177, 179, and 180, respectively, the selected VL1 CDRs 1, 2, 3 amino acid sequences are set forth in SEQ ID NOs: 181, 182, and 183, respectively; the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the first heavy chain variable region comprises a sequence that is at least 80%, 85%, 90%, 95%, 99%, or 100% identical to SEQ ID NO: 184, the first light chain variable region comprises a sequence that is at least 80%, 85%, 90%, 95%, 99%, or 100% identical to SEQ ID NO: 185, the second heavy chain variable region comprises a sequence that is at least 80%, 85%, 90%, 95%, 99%, or 100% identical to SEQ ID NO: 21, and the second light chain variable region comprises a sequence that is at least 80%, 85%, 90%, 95%, 99%, or 100% identical to SEQ ID NO: 22.

In some embodiments, the first heavy chain variable region comprises a sequence that is at least 80%, 85%, 90%, 95%, 99%, or 100% identical to SEQ ID NO: 184, the first light chain variable region comprises a sequence that is at least 80%, 85%, 90%, 95%, 99%, or 100% identical to SEQ ID NO: 185, the second heavy chain variable region comprises a sequence that is at least 80%, 85%, 90%, 95%, 99%, or 100% identical to SEQ ID NO: 85, and the second light chain variable region comprises a sequence that is at least 80%, 85%, 90%, 95%, 99%, or 100% identical to SEQ ID NO: 86.

In some embodiments, the VH1 comprises an amino acid sequence that is at least 80%, 85%, 90%, 95%, 99%, or 100% identical to a selected VH sequence, and the VL1 comprises an amino acid sequence that is at least 80%, 85%, 90%, 95%, 99%, or 100% identical to a selected VL sequence. In some embodiments, the selected VH sequence is SEQ ID NO: 184, and the selected VL sequence is SEQ ID NO: 185.

In some embodiments, the VH2 comprises an amino acid sequence that is at least 80%, 85%, 90%, 95%, 99%, or 100% identical to a selected VH sequence, and the VL2 comprises an amino acid sequence that is at least 80%, 85%, 90%, 95%, 99%, or 100% identical to a selected VL sequence, wherein the selected VH sequence and the selected VL sequence are one of the following:
(1) the selected VH sequence is SEQ ID NO: 21, and the selected VL sequence is SEQ ID NO: 22; and
(2) the selected VH sequence is SEQ ID NO: 85, and the selected VL sequence is SEQ ID NO: 86.

In some embodiments, the VH1 comprises VH1 CDR1, VH1 CDR2, and VH1 CDR3 that are identical to VH CDR1, VH CDR2, and VH CDR3 of a selected VH sequence; and the VL1 comprising VL1 CDR1, VL1 CDR2, and VL1 CDR3 that are identical to VL CDR1, VL CDR2, and VL CDR3 of a selected VL sequence. In some embodiments, the selected VH sequence is SEQ ID NO: 184, and the selected VL sequence is SEQ ID NO: 185.

In some embodiments, the VH2 comprises VH2 CDR1, VH2 CDR2, and VH2 CDR3 that are identical to VH CDR1, VH CDR2, and VH CDR3 of a selected VH sequence; and the VL2 comprising VL2 CDR1, VL2 CDR2, and VL2 CDR3 that are identical to VL CDR1, VL CDR2, and VL CDR3 of a selected VL sequence, wherein the selected VH sequence and the selected VL sequence are one of the following:
(1) the selected VH sequence is SEQ ID NO: 21, and the selected VL sequence is SEQ ID NO: 22; and
(2) the selected VH sequence is SEQ ID NO: 85, and the selected VL sequence is SEQ ID NO: 86.

In some embodiments, the second antigen-binding domain is any of the antigen-binding domain of anti-CLEC5A antibodies, the chimeric antibodies thereof, and the humanized antibodies thereof described herein. In some embodiments, the second antigen-binding domain is the antigen-binding domain of anti-CLEC5A antibody 5C7, the chimeric antibodies thereof, and the humanized antibodies thereof.

The CDR sequences for 5C7, and 5C7 derived antibodies include CDRs of the heavy chain variable domain, SEQ ID NOs: 13, 15, and 17, and CDRs of the light chain variable domain, SEQ ID NOs: 18, 19, and 20, as defined by Kabat definition. Under Chothia definition, the CDR sequences of the heavy chain variable domain are set forth in SEQ ID NOs: 14, 16, and 17, and CDRs of the light chain variable domain are set forth in SEQ ID NOs: 18, 19, and 20.

In some embodiments, the first antigen-binding domain specifically binds to human, rabbit, mouse, monkey, or dog BCMA; and/or the second antigen-binding domain specifically binds to human, rabbit, mouse, monkey, or dog CLEC5A.

In some embodiments, the first antigen-binding domain is a human or humanized antigen-binding domain; and/or the second antigen-binding domain is a human or humanized antigen-binding domain.

In some embodiments, the first antigen-binding domain is a single-chain variable fragment (scFv); and/or the second antigen-binding domain is a scFv.

In some embodiments, the antibody or antigen-binding fragment thereof comprises a fragment crystallizable region (Fc region).

In some embodiments, the multispecific (e.g., bispecific) anti-BCMA/CLEC5A antibody described herein is designed to have an IgG1 subtype structure with the LALAPG mutations (L234A, L235A, and P329G mutations in EU numbering). In some embodiments, the multispecific (e.g., bispecific) anti-BCMA/CLEC5A antibody described herein is designed to have an IgG1 Fc region having an Alanine (A) at position 234; an Alanine (A) at position 235; and a Glycine (G) at position 329 in EU numbering. In some embodiments, The Fc region comprises an amino acid sequence that is about or at least 60%, 65%, 70%, 75%, 80%, 85%, 90%, 95%, or 100% identical to SEQ ID NO: 96.

In some embodiments, the anti-BCMA/CLEC5A antibody described herein can be designed to have an IgG1 Fc region having an Alanine (A) at position 236, a Leucine (L) at position 330, and a Glutamic acid (E) at position 332 in EU numbering. In some embodiments, The Fc region comprises an amino acid sequence that is about or at least 60%, 65%, 70%, 75%, 80%, 85%, 90%, 95%, or 100% identical to SEQ ID NO: 97.

In some embodiments, the multispecific (e.g., bispecific) anti-BCMA/CLEC5A antibody described herein is designed to have an Fc region that comprises an Aspartic acid (D) at position 239 and an glutamic acid (E) at position 332 in EU numbering. In some embodiments, the multispecific (e.g., bispecific) anti-BCMA/CLEC5A antibody described herein is designed to have an IgG1 subtype structure with the S239D+I332E mutations in EU numbering. In some embodiments, the multispecific antibody described herein is designed to have an IgG1 Fc region having an Aspartic acid (D) at position 239 and an glutamic acid (E) at position 332 in EU numbering.

In some embodiments, the multispecific (e.g., bispecific) anti-BCMA/CLEC5A antibody described herein can be designed to have an IgG1 subtype structure with knobs-into-holes (KIH) mutations, which can promote heterodimerization and avoid mispairing between the two heavy chains. In some embodiments, the anti-BCMA/CLEC5A antibody has a higher endocytosis rate than the corresponding monoclonal antibodies or the control bispecific antibodies.

The first antigen-binding fragment and the second antigen-binding fragment of the bi-specific antibodies or antigen-binding fragments described herein can be in any suitable configurations. In some embodiments, wherein the second antigen-binding domain is a single-chain fragment variable (scFv) domain comprising a light chain variable domain (VL) and a heavy chain variable domain (VH) linked by a first linker.

In some embodiments, the second antigen-binding domain is linked to the C-terminus of the light chain of the first antigen-binding domain through a second linker. In some embodiments, the heavy chain variable domain of the first antigen-binding domain is linked to the Fc region. In some embodiments, the VH1 is linked to a CH1 domain, and the VL1 is linked to a CL domain. A schematic illustration of this configuration is shown in **FIG. 9C**. In some embodiments, the antibody or antigen-binding fragment thereof described herein comprises a first heavy chain and a first light chain; and a second heavy chain and a second light chain. A schematic illustration of this configuration is shown in **FIG. 9A**. In some embodiments, the Fc region comprises knobs-into-holes (KIH) mutations. In some embodiments, the first heavy chain includes one or more knob mutations, and the second heavy chain includes one or more hole mutations. In some embodiments, the first heavy chain includes one or more hole mutations, and the second heavy chain includes one or more knob mutations. In some embodiments, the multispecific (e.g., bispecific) anti-BCMA/CLEC5A antibody described herein can have a structure shown in any one of **FIGs. 9A-****9F**.

In some embodiments, the multispecific (e.g., bispecific) anti-BCMA/CLEC5A antibody includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 186. In some embodiments, the heavy chain includes an IgG1 Fc region comprising the optimized mutations (SEQ ID NO: 97).

In some embodiments, the multispecific (e.g., bispecific) anti-BCMA/CLEC5A antibody includes a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 187.

In some embodiments, the multispecific (e.g., bispecific) anti-BCMA/CLEC5A antibody is referred to as "BCMA/5C7 (2+2 A) Fc-optimized" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 186; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 187.

The linkers described herein can be any suitable linkers known in the art. In some embodiments, the linker can comprise a spacer sequence. Various spacer sequences are known in the art, including, without limitation, glycine serine (GS) spacers (also known as GS linkers) such as (GS)n, (SG)n, and (GGGGS)n (SEQ ID NO: 99), where n represents an integer of at least 1. Those of skill in the art would be able to select the appropriate spacer sequence.

In some embodiments, knobs-into-holes mutations were introduced in the Fc regions of the multispecific (e.g., bispecific) antibodies to reduce the chance of wrong pairing between the two heavy chains.

The disclosure also provides nucleic acid comprising a polynucleotide encoding an anti-BCMA/CLEC5A antibody. The immunoglobulin heavy chain or immunoglobulin light chain in the anti-BCMA/CLEC5A antibody comprises CDRs as shown in **Table 22**. When the polypeptides are paired with corresponding polypeptide (e.g., a corresponding heavy chain variable region or a corresponding light chain variable region), the paired polypeptides bind to BCMA and/or CLEC5A.

### Anti-CD38/CLEC5A Antibodies and Antigen-binding Fragments Thereof

CD38 (cluster of differentiation 38), also known as cyclic ADP ribose hydrolase is a glycoprotein found on the surface of many immune cells (white blood cells), including CD4+, CD8+, B lymphocytes and natural killer cells. CD38 also functions in cell adhesion, signal transduction and calcium signaling. In humans, the CD38 protein is encoded by the CD38 gene which is located on chromosome 4. CD38 is a paralog of CD157, which is also located on chromosome 4 (4p15) in humans.

The disclosure provides multispecific (e.g., bispecific) antibodies and antigen-binding fragments thereof that specifically bind to CD38/CLEC5A (e.g., human CD38/CLEC5A). In one aspect, the disclosure provides an anti-CD38/CLEC5A multispecific (e.g., bispecific) antibody or antigen-binding fragment thereof, comprising: a first antigen-binding domain that specifically binds to CD38; and a second antigen-binding domain that specifically binds to CLEC5A.

In some embodiments, the first antigen-binding domain comprises a first heavy chain variable region (VH1) and a first light chain variable region (VL1); and the second antigen-binding domain comprises a second heavy chain variable region (VH2) and a second light chain variable region (VL2).

In some embodiments, the first heavy chain variable region (VH1) comprises complementarity determining regions (CDRs) 1, 2, and 3, wherein the VH1 CDR1 region comprises an amino acid sequence that is at least 80% identical to a selected VH1 CDR1 amino acid sequence, the VH1 CDR2 region comprises an amino acid sequence that is at least 80% identical to a selected VH1 CDR2 amino acid sequence, and the VH1 CDR3 region comprises an amino acid sequence that is at least 80% identical to a selected VH1 CDR3 amino acid sequence; and
the first light chain variable region (VL1) comprises CDRs 1, 2, and 3, wherein the VL1 CDR1 region comprises an amino acid sequence that is at least 80% identical to a selected VL1 CDR1 amino acid sequence, the VL1 CDR2 region comprises an amino acid sequence that is at least 80% identical to a selected VL1 CDR2 amino acid sequence, and the VL1 CDR3 region comprises an amino acid sequence that is at least 80% identical to a selected VL1 CDR3 amino acid sequence, wherein the selected VH1 CDRs 1, 2, 3 amino acid sequences, and the selected VL1 CDRs 1, 2, and 3 amino acid sequences are one of the following:
   (1) the selected VH1 CDRs 1, 2, 3 amino acid sequences are set forth in SEQ ID NOs: 188, 190, and 192, respectively, and the selected VL1 CDRs 1, 2, 3 amino acid sequences are set forth in SEQ ID NOs: 193, 194, and 195, respectively; and
   (2) the selected VH1 CDRs 1, 2, 3 amino acid sequences are set forth in SEQ ID NOs: 189, 191, and 192, respectively, and the selected VL1 CDRs 1, 2, 3 amino acid sequences are set forth in SEQ ID NOs: 193, 194, and 195, respectively;
the second heavy chain variable region (VH2) comprises complementarity determining regions (CDRs) 1, 2, and 3, wherein the VH2 CDR1 region comprises an amino acid sequence that is at least 80% identical to a selected VH2 CDR1 amino acid sequence, the VH2 CDR2 region comprises an amino acid sequence that is at least 80% identical to a selected VH2 CDR2 amino acid sequence, and the VH2 CDR3 region comprises an amino acid sequence that is at least 80% identical to a selected VH2 CDR3 amino acid sequence;
and the second light chain variable region (VL2) comprises CDRs 1, 2, and 3, wherein the VL2 CDR1 region comprises an amino acid sequence that is at least 80% identical to a selected VL2 CDR1 amino acid sequence, the VL2 CDR2 region comprises an amino acid sequence that is at least 80% identical to a selected VL2 CDR2 amino acid sequence, and the VL2 CDR3 region comprises an amino acid sequence that is at least 80% identical to a selected VL2 CDR3 amino acid sequence,
wherein the selected VH2 CDRs 1, 2, and 3 amino acid sequences, and the selected VL2 CDRs 1, 2, and 3 amino acid sequences are one of the following:
   (1) the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively; and
   (2) the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the selected VH1 CDRs 1, 2, 3 amino acid sequences are set forth in SEQ ID NOs: 188, 190, and 192, respectively, the selected VL1 CDRs 1, 2, 3 amino acid sequences are set forth in SEQ ID NOs: 193, 194, and 195, respectively; the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively.

In some embodiments, the selected VH1 CDRs 1, 2, 3 amino acid sequences are set forth in SEQ ID NOs: 189, 191, and 192, respectively, the selected VL1 CDRs 1, 2, 3 amino acid sequences are set forth in SEQ ID NOs: 193, 194, and 195, respectively; the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the first heavy chain variable region comprises a sequence that is at least 80%, 85%, 90%, 95%, 99%, or 100% identical to SEQ ID NO: 196, the first light chain variable region comprises a sequence that is at least 80%, 85%, 90%, 95%, 99%, or 100% identical to SEQ ID NO: 197, the second heavy chain variable region comprises a sequence that is at least 80%, 85%, 90%, 95%, 99%, or 100% identical to SEQ ID NO: 21, and the second light chain variable region comprises a sequence that is at least 80%, 85%, 90%, 95%, 99%, or 100% identical to SEQ ID NO: 22.

In some embodiments, the first heavy chain variable region comprises a sequence that is at least 80%, 85%, 90%, 95%, 99%, or 100% identical to SEQ ID NO: 196, the first light chain variable region comprises a sequence that is at least 80%, 85%, 90%, 95%, 99%, or 100% identical to SEQ ID NO: 197, the second heavy chain variable region comprises a sequence that is at least 80%, 85%, 90%, 95%, 99%, or 100% identical to SEQ ID NO: 85, and the second light chain variable region comprises a sequence that is at least 80%, 85%, 90%, 95%, 99%, or 100% identical to SEQ ID NO: 86.

In some embodiments, the VH1 comprises an amino acid sequence that is at least 80%, 85%, 90%, 95%, 99%, or 100% identical to a selected VH sequence, and the VL1 comprises an amino acid sequence that is at least 80%, 85%, 90%, 95%, 99%, or 100% identical to a selected VL sequence. In some embodiments, the selected VH sequence is SEQ ID NO: 196, and the selected VL sequence is SEQ ID NO: 197.

In some embodiments, the VH2 comprises an amino acid sequence that is at least 80%, 85%, 90%, 95%, 99%, or 100% identical to a selected VH sequence, and the VL2 comprises an amino acid sequence that is at least 80%, 85%, 90%, 95%, 99%, or 100% identical to a selected VL sequence, wherein the selected VH sequence and the selected VL sequence are one of the following:
(1) the selected VH sequence is SEQ ID NO: 21, and the selected VL sequence is SEQ ID NO: 22; and
(2) the selected VH sequence is SEQ ID NO: 85, and the selected VL sequence is SEQ ID NO: 86.

In some embodiments, the VH1 comprises VH1 CDR1, VH1 CDR2, and VH1 CDR3 that are identical to VH CDR1, VH CDR2, and VH CDR3 of a selected VH sequence; and the VL1 comprising VL1 CDR1, VL1 CDR2, and VL1 CDR3 that are identical to VL CDR1, VL CDR2, and VL CDR3 of a selected VL sequence. In some embodiments, the selected VH sequence is SEQ ID NO: 196, and the selected VL sequence is SEQ ID NO: 197.

In some embodiments, the VH2 comprises VH2 CDR1, VH2 CDR2, and VH2 CDR3 that are identical to VH CDR1, VH CDR2, and VH CDR3 of a selected VH sequence; and the VL2 comprising VL2 CDR1, VL2 CDR2, and VL2 CDR3 that are identical to VL CDR1, VL CDR2, and VL CDR3 of a selected VL sequence, wherein the selected VH sequence and the selected VL sequence are one of the following:
(1) the selected VH sequence is SEQ ID NO: 21, and the selected VL sequence is SEQ ID NO: 22; and
(2) the selected VH sequence is SEQ ID NO: 85, and the selected VL sequence is SEQ ID NO: 86.

In some embodiments, the second antigen-binding domain is any of the antigen-binding domain of anti-CLEC5A antibodies, the chimeric antibodies thereof, and the humanized antibodies thereof described herein. In some embodiments, the second antigen-binding domain is the antigen-binding domain of anti-CLEC5A antibody 5C7, the chimeric antibodies thereof, and the humanized antibodies thereof.

The CDR sequences for 5C7, and 5C7 derived antibodies include CDRs of the heavy chain variable domain, SEQ ID NOs: 13, 15, and 17, and CDRs of the light chain variable domain, SEQ ID NOs: 18, 19, and 20, as defined by Kabat definition. Under Chothia definition, the CDR sequences of the heavy chain variable domain are set forth in SEQ ID NOs: 14, 16, and 17, and CDRs of the light chain variable domain are set forth in SEQ ID NOs: 18, 19, and 20.

In some embodiments, the first antigen-binding domain specifically binds to human, rabbit, mouse, monkey, or dog CD38; and/or the second antigen-binding domain specifically binds to human, rabbit, mouse, monkey, or dog CLEC5A.

In some embodiments, the first antigen-binding domain is a human or humanized antigen-binding domain; and/or the second antigen-binding domain is a human or humanized antigen-binding domain.

In some embodiments, the first antigen-binding domain is a single-chain variable fragment (scFv); and/or the second antigen-binding domain is a scFv.

In some embodiments, the antibody or antigen-binding fragment thereof comprises a fragment crystallizable region (Fc region).

In some embodiments, the multispecific (e.g., bispecific) anti-CD38/CLEC5A antibody described herein is designed to have an IgG1 subtype structure with the LALAPG mutations (L234A, L235A, and P329G mutations in EU numbering). In some embodiments, the multispecific (e.g., bispecific) anti-CD38/CLEC5A antibody described herein is designed to have an IgG1 Fc region having an Alanine (A) at position 234; an Alanine (A) at position 235; and a Glycine (G) at position 329 in EU numbering. In some embodiments, The Fc region comprises an amino acid sequence that is about or at least 60%, 65%, 70%, 75%, 80%, 85%, 90%, 95%, or 100% identical to SEQ ID NO: 96.

In some embodiments, the anti-CD38/CLEC5A antibody described herein can be designed to have an IgG1 Fc region having an Alanine (A) at position 236, a Leucine (L) at position 330, and a Glutamic acid (E) at position 332 in EU numbering. In some embodiments, The Fc region comprises an amino acid sequence that is about or at least 60%, 65%, 70%, 75%, 80%, 85%, 90%, 95%, or 100% identical to SEQ ID NO: 97.

In some embodiments, the multispecific (e.g., bispecific) anti-CD38/CLEC5A antibody described herein is designed to have an Fc region that comprises an Aspartic acid (D) at position 239 and an glutamic acid (E) at position 332 in EU numbering. In some embodiments, the multispecific (e.g., bispecific) anti-CD38/CLEC5A antibody described herein is designed to have an IgG1 subtype structure with the S239D+I332E mutations in EU numbering. In some embodiments, the multispecific antibody described herein is designed to have an IgG1 Fc region having an Aspartic acid (D) at position 239 and an glutamic acid (E) at position 332 in EU numbering.

In some embodiments, the multispecific (e.g., bispecific) anti-CD38/CLEC5A antibody described herein can be designed to have an IgG1 subtype structure with knobs-into-holes (KIH) mutations, which can promote heterodimerization and avoid mispairing between the two heavy chains. In some embodiments, the anti-CD38/CLEC5A antibody has a higher endocytosis rate than the corresponding monoclonal antibodies or the control bispecific antibodies.

The first antigen-binding fragment and the second antigen-binding fragment of the bi-specific antibodies or antigen-binding fragments described herein can be in any suitable configurations. In some embodiments, wherein the second antigen-binding domain is a single-chain fragment variable (scFv) domain comprising a light chain variable domain (VL) and a heavy chain variable domain (VH) linked by a first linker.

In some embodiments, the second antigen-binding domain is linked to the C-terminus of the light chain of the first antigen-binding domain through a second linker. In some embodiments, the heavy chain variable domain of the first antigen-binding domain is linked to the Fc region. In some embodiments, the VH1 is linked to a CH1 domain, and the VL1 is linked to a CL domain. A schematic illustration of this configuration is shown in **FIG. 9C**. In some embodiments, the antibody or antigen-binding fragment thereof described herein comprises a first heavy chain and a first light chain; and a second heavy chain and a second light chain. A schematic illustration of this configuration is shown in **FIG. 9A**. In some embodiments, the Fc region comprises knobs-into-holes (KIH) mutations. In some embodiments, the first heavy chain includes one or more knob mutations, and the second heavy chain includes one or more hole mutations. In some embodiments, the first heavy chain includes one or more hole mutations, and the second heavy chain includes one or more knob mutations. In some embodiments, the multispecific (e.g., bispecific) anti-CD38/CLEC5A antibody described herein can have a structure shown in any one of **FIGs. 9A-9F**.

In some embodiments, the multispecific (e.g., bispecific) anti-CD38/CLEC5A antibody includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 198. In some embodiments, the heavy chain includes an IgG1 Fc region comprising the optimized mutations (SEQ ID NO: 97).

In some embodiments, the multispecific (e.g., bispecific) anti-CD38/CLEC5A antibody includes a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 199.

In some embodiments, the multispecific (e.g., bispecific) anti-CD38/CLEC5A antibody is referred to as "CD38/5C7 (2+2 A) Fc-optimized" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 198; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 199.

The linkers described herein can be any suitable linkers known in the art. In some embodiments, the linker can comprise a spacer sequence. Various spacer sequences are known in the art, including, without limitation, glycine serine (GS) spacers (also known as GS linkers) such as (GS)n, (SG)n, and (GGGGS)n (SEQ ID NO: 99), where n represents an integer of at least 1. Those of skill in the art would be able to select the appropriate spacer sequence.

In some embodiments, knobs-into-holes mutations were introduced in the Fc regions of the multispecific (e.g., bispecific) antibodies to reduce the chance of wrong pairing between the two heavy chains.

The disclosure also provides nucleic acid comprising a polynucleotide encoding an anti-CD38/CLEC5A antibody. The immunoglobulin heavy chain or immunoglobulin light chain in the anti-CD38/CLEC5A antibody comprises CDRs as shown in **Table 22**. When the polypeptides are paired with corresponding polypeptide (e.g., a corresponding heavy chain variable region or a corresponding light chain variable region), the paired polypeptides bind to CD38 and/or CLEC5A.

### Anti-CD79b/CLEC5A Antibodies and Antigen-binding Fragments Thereof

CD79b molecule, immunoglobulin-associated beta, also known as CD79B (Cluster of Differentiation 79B), is a human gene. It is associated with agammaglobulinemia-6. The B lymphocyte antigen receptor is a multimeric complex that includes the antigen-specific component, surface immunoglobulin (Ig). Surface Ig non-covalently associates with two other proteins, Ig-alpha and Ig-beta, which are necessary for expression and function of the B-cell antigen receptor. This gene encodes the Ig-beta protein of the B-cell antigen component. Alternatively spliced transcript variants encoding different isoforms have been described.

The disclosure provides multispecific (e.g., bispecific) antibodies and antigen-binding fragments thereof that specifically bind to CD79b/CLEC5A (e.g., human CD79b/CLEC5A). In one aspect, the disclosure provides an anti-CD79b/CLEC5A multispecific (e.g., bispecific) antibody or antigen-binding fragment thereof, comprising: a first antigen-binding domain that specifically binds to CD79b; and a second antigen-binding domain that specifically binds to CLEC5A.

In some embodiments, the first antigen-binding domain comprises a first heavy chain variable region (VH1) and a first light chain variable region (VL1); and the second antigen-binding domain comprises a second heavy chain variable region (VH2) and a second light chain variable region (VL2).

In some embodiments, the first heavy chain variable region (VH1) comprises complementarity determining regions (CDRs) 1, 2, and 3, wherein the VH1 CDR1 region comprises an amino acid sequence that is at least 80% identical to a selected VH1 CDR1 amino acid sequence, the VH1 CDR2 region comprises an amino acid sequence that is at least 80% identical to a selected VH1 CDR2 amino acid sequence, and the VH1 CDR3 region comprises an amino acid sequence that is at least 80% identical to a selected VH1 CDR3 amino acid sequence; and
the first light chain variable region (VL1) comprises CDRs 1, 2, and 3, wherein the VL1 CDR1 region comprises an amino acid sequence that is at least 80% identical to a selected VL1 CDR1 amino acid sequence, the VL1 CDR2 region comprises an amino acid sequence that is at least 80% identical to a selected VL1 CDR2 amino acid sequence, and the VL1 CDR3 region comprises an amino acid sequence that is at least 80% identical to a selected VL1 CDR3 amino acid sequence, wherein the selected VH1 CDRs 1, 2, 3 amino acid sequences, and the selected VL1 CDRs 1, 2, and 3 amino acid sequences are one of the following:
   (1) the selected VH1 CDRs 1, 2, 3 amino acid sequences are set forth in SEQ ID NOs: 200, 202, and 204, respectively, and the selected VL1 CDRs 1, 2, 3 amino acid sequences are set forth in SEQ ID NOs: 205, 206, and 207, respectively; and
   (2) the selected VH1 CDRs 1, 2, 3 amino acid sequences are set forth in SEQ ID NOs: 201, 203, and 204, respectively, and the selected VL1 CDRs 1, 2, 3 amino acid sequences are set forth in SEQ ID NOs: 205, 206, and 207, respectively;
the second heavy chain variable region (VH2) comprises complementarity determining regions (CDRs) 1, 2, and 3, wherein the VH2 CDR1 region comprises an amino acid sequence that is at least 80% identical to a selected VH2 CDR1 amino acid sequence, the VH2 CDR2 region comprises an amino acid sequence that is at least 80% identical to a selected VH2 CDR2 amino acid sequence, and the VH2 CDR3 region comprises an amino acid sequence that is at least 80% identical to a selected VH2 CDR3 amino acid sequence;
and the second light chain variable region (VL2) comprises CDRs 1, 2, and 3, wherein the VL2 CDR1 region comprises an amino acid sequence that is at least 80% identical to a selected VL2 CDR1 amino acid sequence, the VL2 CDR2 region comprises an amino acid sequence that is at least 80% identical to a selected VL2 CDR2 amino acid sequence, and the VL2 CDR3 region comprises an amino acid sequence that is at least 80% identical to a selected VL2 CDR3 amino acid sequence,
wherein the selected VH2 CDRs 1, 2, and 3 amino acid sequences, and the selected VL2 CDRs 1, 2, and 3 amino acid sequences are one of the following:
   (1) the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively;
   (2) the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively;
   (3) the selected VH2 CDRs 1, 2, 3 amino acid sequences are set forth in SEQ ID NOs: 3, 5, and 7, respectively, and the selected VL2 CDRs 1, 2, 3 amino acid sequences are set forth in SEQ ID NOs: 8, 9, and 10, respectively;
   (4) the selected VH2 CDRs 1, 2, 3 amino acid sequences are set forth in SEQ ID NOs: 4, 6, and 7, respectively, and the selected VL2 CDRs 1, 2, 3 amino acid sequences are set forth in SEQ ID NOs: 8, 9 and 10, respectively;
   (5) the selected VH2 CDRs 1, 2, 3 amino acid sequences are set forth in SEQ ID NOs: 63, 65, and 67, respectively, and the selected VL2 CDRs 1, 2, 3 amino acid sequences are set forth in SEQ ID NOs: 68, 69, and 70, respectively; and
   (6) the selected VH2 CDRs 1, 2, 3 amino acid sequences are set forth in SEQ ID NOs: 64, 66, and 67, respectively, and the selected VL2 CDRs 1, 2, 3 amino acid sequences are set forth in SEQ ID NOs: 68, 69 and 70, respectively.

In some embodiments, the selected VH1 CDRs 1, 2, 3 amino acid sequences are set forth in SEQ ID NOs: 200, 202, and 204, respectively, the selected VL1 CDRs 1, 2, 3 amino acid sequences are set forth in SEQ ID NOs: 205, 206, and 207, respectively; the selected VH2 CDRs 1, 2, 3 amino acid sequences are set forth in SEQ ID NOs: 13, 15, and 17, respectively, and the selected VL2 CDRs 1, 2, 3 amino acid sequences are set forth in SEQ ID NOs: 18, 19, and 20, respectively.

In some embodiments, the selected VH1 CDRs 1, 2, 3 amino acid sequences are set forth in SEQ ID NOs: 201, 203, and 204, respectively, the selected VL1 CDRs 1, 2, 3 amino acid sequences are set forth in SEQ ID NOs: 205, 206, and 207, respectively; the selected VH2 CDRs 1, 2, 3 amino acid sequences are set forth in SEQ ID NOs: 14, 16, and 17, respectively, and the selected VL2 CDRs 1, 2, 3 amino acid sequences are set forth in SEQ ID NOs: 18, 19 and 20, respectively.

In some embodiments, the selected VH1 CDRs 1, 2, 3 amino acid sequences are set forth in SEQ ID NOs: 200, 202, and 204, respectively, the selected VL1 CDRs 1, 2, 3 amino acid sequences are set forth in SEQ ID NOs: 205, 206, and 207, respectively; the selected VH2 CDRs 1, 2, 3 amino acid sequences are set forth in SEQ ID NOs: 3, 5, and 7, respectively, and the selected VL2 CDRs 1, 2, 3 amino acid sequences are set forth in SEQ ID NOs: 8, 9, and 10, respectively.

In some embodiments, the selected VH1 CDRs 1, 2, 3 amino acid sequences are set forth in SEQ ID NOs: 201, 203, and 204, respectively, the selected VL1 CDRs 1, 2, 3 amino acid sequences are set forth in SEQ ID NOs: 205, 206, and 207, respectively; the selected VH2 CDRs 1, 2, 3 amino acid sequences are set forth in SEQ ID NOs: 4, 6, and 7, respectively, and the selected VL2 CDRs 1, 2, 3 amino acid sequences are set forth in SEQ ID NOs: 8, 9 and 10, respectively.

In some embodiments, the selected VH1 CDRs 1, 2, 3 amino acid sequences are set forth in SEQ ID NOs: 200, 202, and 204, respectively, the selected VL1 CDRs 1, 2, 3 amino acid sequences are set forth in SEQ ID NOs: 205, 206, and 207, respectively; the selected VH2 CDRs 1, 2, 3 amino acid sequences are set forth in SEQ ID NOs: 63, 65, and 67, respectively, and the selected VL2 CDRs 1, 2, 3 amino acid sequences are set forth in SEQ ID NOs: 68, 69, and 70, respectively.

In some embodiments, the selected VH1 CDRs 1, 2, 3 amino acid sequences are set forth in SEQ ID NOs: 201, 203, and 204, respectively, the selected VL1 CDRs 1, 2, 3 amino acid sequences are set forth in SEQ ID NOs: 205, 206, and 207, respectively; the selected VH2 CDRs 1, 2, 3 amino acid sequences are set forth in SEQ ID NOs: 64, 66, and 67, respectively, and the selected VL2 CDRs 1, 2, 3 amino acid sequences are set forth in SEQ ID NOs: 68, 69 and 70, respectively.

In some embodiments, the first heavy chain variable region comprises a sequence that is at least 80%, 85%, 90%, 95%, 99%, or 100% identical to SEQ ID NO: 208, the first light chain variable region comprises a sequence that is at least 80%, 85%, 90%, 95%, 99%, or 100% identical to SEQ ID NO: 209, the second heavy chain variable region comprises a sequence that is at least 80%, 85%, 90%, 95%, 99%, or 100% identical to SEQ ID NO: 21, and the second light chain variable region comprises a sequence that is at least 80%, 85%, 90%, 95%, 99%, or 100% identical to SEQ ID NO: 22.

In some embodiments, the first heavy chain variable region comprises a sequence that is at least 80%, 85%, 90%, 95%, 99%, or 100% identical to SEQ ID NO: 208, the first light chain variable region comprises a sequence that is at least 80%, 85%, 90%, 95%, 99%, or 100% identical to SEQ ID NO: 209, the second heavy chain variable region comprises a sequence that is at least 80%, 85%, 90%, 95%, 99%, or 100% identical to SEQ ID NO: 85, and the second light chain variable region comprises a sequence that is at least 80%, 85%, 90%, 95%, 99%, or 100% identical to SEQ ID NO: 86.

In some embodiments, the first heavy chain variable region comprises a sequence that is at least 80%, 85%, 90%, 95%, 99%, or 100% identical to SEQ ID NO: 208, the first light chain variable region comprises a sequence that is at least 80%, 85%, 90%, 95%, 99%, or 100% identical to SEQ ID NO: 209, the second heavy chain variable region comprises a sequence that is at least 80%, 85%, 90%, 95%, 99%, or 100% identical to SEQ ID NO: 11, and the second light chain variable region comprises a sequence that is at least 80%, 85%, 90%, 95%, 99%, or 100% identical to SEQ ID NO: 12.

In some embodiments, the first heavy chain variable region comprises a sequence that is at least 80%, 85%, 90%, 95%, 99%, or 100% identical to SEQ ID NO: 208, the first light chain variable region comprises a sequence that is at least 80%, 85%, 90%, 95%, 99%, or 100% identical to SEQ ID NO: 209, the second heavy chain variable region comprises a sequence that is at least 80%, 85%, 90%, 95%, 99%, or 100% identical to SEQ ID NO: 83, and the second light chain variable region comprises a sequence that is at least 80%, 85%, 90%, 95%, 99%, or 100% identical to SEQ ID NO: 84.

In some embodiments, the first heavy chain variable region comprises a sequence that is at least 80%, 85%, 90%, 95%, 99%, or 100% identical to SEQ ID NO: 208, the first light chain variable region comprises a sequence that is at least 80%, 85%, 90%, 95%, 99%, or 100% identical to SEQ ID NO: 209, the second heavy chain variable region comprises a sequence that is at least 80%, 85%, 90%, 95%, 99%, or 100% identical to SEQ ID NO: 71, and the second light chain variable region comprises a sequence that is at least 80%, 85%, 90%, 95%, 99%, or 100% identical to SEQ ID NO: 72.

In some embodiments, the first heavy chain variable region comprises a sequence that is at least 80%, 85%, 90%, 95%, 99%, or 100% identical to SEQ ID NO: 208, the first light chain variable region comprises a sequence that is at least 80%, 85%, 90%, 95%, 99%, or 100% identical to SEQ ID NO: 209, the second heavy chain variable region comprises a sequence that is at least 80%, 85%, 90%, 95%, 99%, or 100% identical to SEQ ID NO: 91, and the second light chain variable region comprises a sequence that is at least 80%, 85%, 90%, 95%, 99%, or 100% identical to SEQ ID NO: 92.

In some embodiments, the VH1 comprises an amino acid sequence that is at least 80%, 85%, 90%, 95%, 99%, or 100% identical to a selected VH sequence, and the VL1 comprises an amino acid sequence that is at least 80%, 85%, 90%, 95%, 99%, or 100% identical to a selected VL sequence. In some embodiments, the selected VH sequence is SEQ ID NO: 208, and the selected VL sequence is SEQ ID NO: 209.

In some embodiments, the VH2 comprises an amino acid sequence that is at least 80%, 85%, 90%, 95%, 99%, or 100% identical to a selected VH sequence, and the VL2 comprises an amino acid sequence that is at least 80%, 85%, 90%, 95%, 99%, or 100% identical to a selected VL sequence, wherein the selected VH sequence and the selected VL sequence are one of the following:
(1) the selected VH sequence is SEQ ID NO: 21, and the selected VL sequence is SEQ ID NO: 22;
(2) the selected VH sequence is SEQ ID NO: 85, and the selected VL sequence is SEQ ID NO: 86;
(3) the selected VH sequence is SEQ ID NO: 11, and the selected VL sequence is SEQ ID NO: 12;
(4) the selected VH sequence is SEQ ID NO: 83, and the selected VL sequence is SEQ ID NO: 84;
(5) the selected VH sequence is SEQ ID NO: 71, and the selected VL sequence is SEQ ID NO: 72; and
(6) the selected VH sequence is SEQ ID NO: 91, and the selected VL sequence is SEQ ID NO: 92.

In some embodiments, the VH1 comprises VH1 CDR1, VH1 CDR2, and VH1 CDR3 that are identical to VH CDR1, VH CDR2, and VH CDR3 of a selected VH sequence; and the VL1 comprising VL1 CDR1, VL1 CDR2, and VL1 CDR3 that are identical to VL CDR1, VL CDR2, and VL CDR3 of a selected VL sequence. In some embodiments, the selected VH sequence is SEQ ID NO: 208, and the selected VL sequence is SEQ ID NO: 209.

In some embodiments, the VH2 comprises VH2 CDR1, VH2 CDR2, and VH2 CDR3 that are identical to VH CDR1, VH CDR2, and VH CDR3 of a selected VH sequence; and the VL2 comprising VL2 CDR1, VL2 CDR2, and VL2 CDR3 that are identical to VL CDR1, VL CDR2, and VL CDR3 of a selected VL sequence, wherein the selected VH sequence and the selected VL sequence are one of the following:
(1) the selected VH sequence is SEQ ID NO: 21, and the selected VL sequence is SEQ ID NO: 22;
(2) the selected VH sequence is SEQ ID NO: 85, and the selected VL sequence is SEQ ID NO: 86;
(3) the selected VH sequence is SEQ ID NO: 11, and the selected VL sequence is SEQ ID NO: 12;
(4) the selected VH sequence is SEQ ID NO: 83, and the selected VL sequence is SEQ ID NO: 84;
(5) the selected VH sequence is SEQ ID NO: 71, and the selected VL sequence is SEQ ID NO: 72; and
(6) the selected VH sequence is SEQ ID NO: 91, and the selected VL sequence is SEQ ID NO: 92.

In some embodiments, the second antigen-binding domain is any of the antigen-binding domain of anti-CLEC5A antibodies, the chimeric antibodies thereof, and the humanized antibodies thereof described herein. In some embodiments, the second antigen-binding domain is the antigen-binding domain of anti-CLEC5A antibody 5C7, 3A7, 13E6, the chimeric antibodies thereof, and the humanized antibodies thereof.

The CDR sequences for 5C7, and 5C7 derived antibodies include CDRs of the heavy chain variable domain, SEQ ID NOs: 13, 15, and 17, and CDRs of the light chain variable domain, SEQ ID NOs: 18, 19, and 20, as defined by Kabat definition. Under Chothia definition, the CDR sequences of the heavy chain variable domain are set forth in SEQ ID NOs: 14, 16, and 17, and CDRs of the light chain variable domain are set forth in SEQ ID NOs: 18, 19, and 20.

The CDR sequences for 3A7, and 3A7 derived antibodies include CDRs of the heavy chain variable domain, SEQ ID NOs: 3, 5, and 7, and CDRs of the light chain variable domain, SEQ ID NOs: 8, 9, and 10, as defined by Kabat definition. Under Chothia definition, the CDR sequences of the heavy chain variable domain are set forth in SEQ ID NOs: 4, 6, and 7, and CDRs of the light chain variable domain are set forth in SEQ ID NOs: 8, 9, and 10.

The CDR sequences for 13E6, and 13E6 derived antibodies include CDRs of the heavy chain variable domain, SEQ ID NOs: 63, 65, and 67, and CDRs of the light chain variable domain, SEQ ID NOs: 68, 69, and 70, as defined by Kabat definition. Under Chothia definition, the CDR sequences of the heavy chain variable domain are set forth in SEQ ID NOs: 64, 66, and 67, and CDRs of the light chain variable domain are set forth in SEQ ID NOs: 68, 69, and 70.

In some embodiments, the first antigen-binding domain specifically binds to human, rabbit, mouse, monkey, or dog CD79b; and/or the second antigen-binding domain specifically binds to human, rabbit, mouse, monkey, or dog CLEC5A.

In some embodiments, the first antigen-binding domain is a human or humanized antigen-binding domain; and/or the second antigen-binding domain is a human or humanized antigen-binding domain.

In some embodiments, the first antigen-binding domain is a single-chain variable fragment (scFv); and/or the second antigen-binding domain is a scFv.

In some embodiments, the antibody or antigen-binding fragment thereof comprises a fragment crystallizable region (Fc region).

In some embodiments, the multispecific (e.g., bispecific) anti-CD79b/CLEC5A antibody described herein is designed to have an IgG1 subtype structure with the LALAPG mutations (L234A, L235A, and P329G mutations in EU numbering). In some embodiments, the multispecific (e.g., bispecific) anti-CD79b/CLEC5A antibody described herein is designed to have an IgG1 Fc region having an Alanine (A) at position 234; an Alanine (A) at position 235; and a Glycine (G) at position 329 in EU numbering. In some embodiments, The Fc region comprises an amino acid sequence that is about or at least 60%, 65%, 70%, 75%, 80%, 85%, 90%, 95%, or 100% identical to SEQ ID NO: 96.

In some embodiments, the anti-CD79b/CLEC5A antibody described herein can be designed to have an IgG1 Fc region having an Alanine (A) at position 236, a Leucine (L) at position 330, and a Glutamic acid (E) at position 332 in EU numbering. In some embodiments, The Fc region comprises an amino acid sequence that is about or at least 60%, 65%, 70%, 75%, 80%, 85%, 90%, 95%, or 100% identical to SEQ ID NO: 97.

In some embodiments, the multispecific (e.g., bispecific) anti-CD79b/CLEC5A antibody described herein is designed to have an Fc region that comprises an Aspartic acid (D) at position 239 and an glutamic acid (E) at position 332 in EU numbering. In some embodiments, the multispecific (e.g., bispecific) anti-CD79b/CLEC5A antibody described herein is designed to have an IgG1 subtype structure with the S239D+I332E mutations in EU numbering. In some embodiments, the multispecific antibody described herein is designed to have an IgG1 Fc region having an Aspartic acid (D) at position 239 and an glutamic acid (E) at position 332 in EU numbering.

In some embodiments, the multispecific (e.g., bispecific) anti-CD79b/CLEC5A antibody described herein can be designed to have an IgG1 subtype structure with knobs-into-holes (KIH) mutations, which can promote heterodimerization and avoid mispairing between the two heavy chains. In some embodiments, the anti-CD79b/CLEC5A antibody has a higher endocytosis rate than the corresponding monoclonal antibodies or the control bispecific antibodies.

The first antigen-binding fragment and the second antigen-binding fragment of the bi-specific antibodies or antigen-binding fragments described herein can be in any suitable configurations. In some embodiments, wherein the second antigen-binding domain is a single-chain fragment variable (scFv) domain comprising a light chain variable domain (VL) and a heavy chain variable domain (VH) linked by a first linker.

In some embodiments, the second antigen-binding domain is linked to the C-terminus of the light chain of the first antigen-binding domain through a second linker. In some embodiments, the heavy chain variable domain of the first antigen-binding domain is linked to the Fc region. In some embodiments, the VH1 is linked to a CH1 domain, and the VL1 is linked to a CL domain. A schematic illustration of this configuration is shown in **FIG. 9C**. In some embodiments, the antibody or antigen-binding fragment thereof described herein comprises a first heavy chain and a first light chain; and a second heavy chain and a second light chain. A schematic illustration of this configuration is shown in **FIG. 9A**. In some embodiments, the Fc region comprises knobs-into-holes (KIH) mutations. In some embodiments, the first heavy chain includes one or more knob mutations, and the second heavy chain includes one or more hole mutations. In some embodiments, the first heavy chain includes one or more hole mutations, and the second heavy chain includes one or more knob mutations. In some embodiments, the multispecific (e.g., bispecific) anti-CD79b/CLEC5A antibody described herein can have a structure shown in any one of **FIGs. 9A-****9F**.

In some embodiments, the multispecific (e.g., bispecific) anti-CD79b/CLEC5A antibody includes a first heavy chain (shown as "H1" in **FIG. 9A**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 210, and a second heavy chain (shown as "H2" in **FIG. 9A**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 211. In some embodiments, the first and/or second heavy chains include an IgG1 Fc region comprising the LALAPG mutations (SEQ ID NO: 96). In some embodiments, the multispecific (e.g., bispecific) anti-CD79b/CLEC5A antibody includes a first light chain (shown as "L1" in **FIG. 9A**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 212, and a second light chain (shown as "L2" in **FIG. 9A**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 213.

In some embodiments, the multispecific (e.g., bispecific) anti-CD79b/CLEC5A antibody is referred to as "CD79b/5C7 (1+1 A) Fc-silenced" and includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 210; a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 211; a first light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 212; and a second light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 213.

In some embodiments, the multispecific (e.g., bispecific) anti-CD79b/CLEC5A antibody includes a first heavy chain (shown as "H1" in **FIG. 9A**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 227, and a second heavy chain (shown as "H2" in **FIG. 9A**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 228. In some embodiments, the first and/or second heavy chains include an IgG1 Fc region comprising the optimized mutations (SEQ ID NO: 97). In some embodiments, the multispecific (e.g., bispecific) anti-CD79b/CLEC5A antibody includes a first light chain (shown as "L1" in **FIG. 9A**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 229, and a second light chain (shown as "L2" in **FIG. 9A**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 230.

In some embodiments, the multispecific (e.g., bispecific) anti-CD79b/CLEC5A antibody is referred to as "CD79b/5C7 (1+1 A) Fc-optimized" and includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 227; a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 228; a first light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 229; and a second light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 230.

In some embodiments, the multispecific (e.g., bispecific) anti-CD79b/CLEC5A antibody includes a first heavy chain (shown as "H1" in **FIG. 9B**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 217, and a second heavy chain (shown as "H2" in **FIG. 9B**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 218. In some embodiments, the first and/or second heavy chains include an IgG1 Fc region comprising the LALAPG mutations (SEQ ID NO: 96). In some embodiments, the multispecific (e.g., bispecific) anti-CD79b/CLEC5A antibody includes a light chain (shown as "L1" in **FIG. 9B**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 219.

In some embodiments, the multispecific (e.g., bispecific) anti-CD79b/CLEC5A antibody is referred to as "CD79b/5C7 (2+1 A) Fc-silenced" and includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 217; a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 218; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 219.

In some embodiments, the multispecific (e.g., bispecific) anti-CD79b/CLEC5A antibody includes a first heavy chain (shown as "H1" in **FIG. 9B**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 234, and a second heavy chain (shown as "H2" in **FIG. 9B**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 235. In some embodiments, the first and/or second heavy chains include an IgG1 Fc region comprising the optimized mutations (SEQ ID NO: 97). In some embodiments, the multispecific (e.g., bispecific) anti-CD79b/CLEC5A antibody includes a light chain (shown as "L1" in **FIG. 9B**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 236.

In some embodiments, the multispecific (e.g., bispecific) anti-CD79b/CLEC5A antibody is referred to as "CD79b/5C7 (2+1 A) Fc-optimized" and includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 234; a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 235; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 236.

In some embodiments, the multispecific (e.g., bispecific) anti- CD79b/CLEC5A antibody includes a heavy chain (shown as "H" in **FIG. 9C**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 223. In some embodiments, the heavy chain includes an IgG1 Fc region comprising the LALAPG mutations (SEQ ID NO: 96). In some embodiments, the multispecific (e.g., bispecific) anti- CD79b/CLEC5A antibody includes a light chain (shown as "L" in **FIG. 9C**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 224.

In some embodiments, the multispecific (e.g., bispecific) anti-CD79b/CLEC5A antibody is referred to as "CD79b/5C7 (2+2 A) Fc-silenced" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 223; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 224.

In some embodiments, the multispecific (e.g., bispecific) anti-CD79b/CLEC5A antibody includes a heavy chain (shown as "H" in **FIG. 9C**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 240. In some embodiments, the heavy chain includes an IgG1 Fc region comprising the optimized mutations (SEQ ID NO: 97). In some embodiments, the multispecific (e.g., bispecific) anti-CD79b/CLEC5A antibody includes a light chain (shown as "L" in **FIG. 9C**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 241.

In some embodiments, the multispecific (e.g., bispecific) anti-CD79b/CLEC5A antibody is referred to as "CD79b/5C7 (2+2 A) Fc-optimized" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 240; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 241.

In some embodiments, the multispecific (e.g., bispecific) anti-CD79b/CLEC5A antibody includes a first heavy chain (shown as "H1" in **FIG. 9D**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 214, and a second heavy chain (shown as "H2" in FIG. 9D) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 215. In some embodiments, the first and/or second heavy chains include an IgG1 Fc region comprising the LALAPG mutations (SEQ ID NO: 96). In some embodiments, the multispecific (e.g., bispecific) anti-CD79b/CLEC5A antibody includes a light chain (shown as "L1" in FIG. 9D) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 216.

In some embodiments, the multispecific (e.g., bispecific) anti-CD79b/CLEC5A antibody is referred to as "CD79b/5C7 (1+1 B) Fc-silenced" and includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 214; a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 215; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 216.

In some embodiments, the multispecific (e.g., bispecific) anti-CD79b/CLEC5A antibody includes a first heavy chain (shown as "H1" in **FIG. 9D**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 231, and a second heavy chain (shown as "H2" in **FIG. 9D**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 232. In some embodiments, the first and/or second heavy chains include an IgG1 Fc region comprising the optimized mutations (SEQ ID NO: 97). In some embodiments, the multispecific (e.g., bispecific) anti-CD79b/CLEC5A antibody includes a light chain (shown as "L1" in **FIG. 9D**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 233.

In some embodiments, the multispecific (e.g., bispecific) anti-CD79b/CLEC5A antibody is referred to as "CD79b/5C7 (1+1 B) Fc-optimized" and includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 231; a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 232; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 233.

In some embodiments, the multispecific (e.g., bispecific) anti-CD79b/CLEC5A antibody includes a first heavy chain (shown as "H1" in **FIG. 9E**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 220, and a second heavy chain (shown as "H2" in **FIG. 9E**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 221. In some embodiments, the first and/or second heavy chains include an IgG1 Fc region comprising the LALAPG mutations (SEQ ID NO: 96). In some embodiments, the multispecific (e.g., bispecific) anti-CD79b/CLEC5A antibody includes a light chain (shown as "L1" in **FIG. 9E**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 222.

In some embodiments, the multispecific (e.g., bispecific) anti-CD79b/CLEC5A antibody is referred to as "CD79b/5C7 (2+1 B) Fc-silenced" and includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 220; a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 221; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 222.

In some embodiments, the multispecific (e.g., bispecific) anti-CD79b/CLEC5A antibody includes a first heavy chain (shown as "H1" in **FIG. 9E**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 237, and a second heavy chain (shown as "H2" in **FIG. 9E**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 238. In some embodiments, the first and/or second heavy chains include an IgG1 Fc region comprising the optimized mutations (SEQ ID NO: 97). In some embodiments, the multispecific (e.g., bispecific) anti-CD79b/CLEC5A antibody includes a light chain (shown as "L1" in **FIG. 9E**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 239.

In some embodiments, the multispecific (e.g., bispecific) anti-CD79b/CLEC5A antibody is referred to as "CD79b/5C7 (2+1 B) Fc-optimized" and includes a first heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 237; a second heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 238; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 239.

In some embodiments, the multispecific (e.g., bispecific) anti-CD79b/CLEC5A antibody includes a heavy chain (shown as "H" in **FIG. 9F**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 225. In some embodiments, the heavy chain includes an IgG1 Fc region comprising the LALAPG mutations (SEQ ID NO: 96). In some embodiments, the multispecific (e.g., bispecific) anti-CD79b/CLEC5A antibody includes a light chain (shown as "L" in **FIG. 9F**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 226.

In some embodiments, the multispecific (e.g., bispecific) anti-CD79b/CLEC5A antibody is referred to as "CD79b/5C7 (2+2 B) Fc-silenced" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 225; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 226.

In some embodiments, the multispecific (e.g., bispecific) anti-CD79b/CLEC5A antibody includes a heavy chain (shown as "H" in **FIG. 9F**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 244. In some embodiments, the heavy chain includes an IgG1 Fc region comprising the LALAPG mutations (SEQ ID NO: 96). In some embodiments, the multispecific (e.g., bispecific) anti-CD79b/CLEC5A antibody includes a light chain (shown as "L" in **FIG. 9F**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 245.

In some embodiments, the multispecific (e.g., bispecific) anti-CD79b/CLEC5A antibody is referred to as "CD79b/3A7 (2+2 B) Fc-silenced" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 244; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 245.

In some embodiments, the multispecific (e.g., bispecific) anti-CD79b/CLEC5A antibody includes a heavy chain (shown as "H" in **FIG. 9F**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 246. In some embodiments, the heavy chain includes an IgG1 Fc region comprising the LALAPG mutations (SEQ ID NO: 96). In some embodiments, the multispecific (e.g., bispecific) anti-CD79b/CLEC5A antibody includes a light chain (shown as "L" in **FIG. 9F**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 247.

In some embodiments, the multispecific (e.g., bispecific) anti-CD79b/CLEC5A antibody is referred to as "CD79b/13E6 (2+2 B) Fc-silenced" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 246; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 247.

In some embodiments, the multispecific (e.g., bispecific) anti-CD79b/CLEC5A antibody includes a heavy chain (shown as "H" in **FIG. 9F**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 242. In some embodiments, the heavy chain includes an IgG1 Fc region comprising the optimized mutations (SEQ ID NO: 97). In some embodiments, the multispecific (e.g., bispecific) anti-CD79b/CLEC5A antibody includes a light chain (shown as "L" in **FIG. 9F**) sequence that is about or at least 80%, 85%, 86%, 87%, 88%, 89%, 90%, 91%, 92%, 93%, 94%, 95%, 96%, 97%, 98%, 99% or 100% identical to SEQ ID NO: 243.

In some embodiments, the multispecific (e.g., bispecific) anti-CD79b/CLEC5A antibody is referred to as "CD79b/5C7 (2+2 B) Fc-optimized" and includes a heavy chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 242; and a light chain sequence that is about or at least 80%, 85%, 86%, 87%, 88%, 89%, 90%, 91%, 92%, 93%, 94%, 95%, 96%, 97%, 98%, 99% or 100% identical to SEQ ID NO: 243.

The linkers described herein can be any suitable linkers known in the art. In some embodiments, the linker can comprise a spacer sequence. Various spacer sequences are known in the art, including, without limitation, glycine serine (GS) spacers (also known as GS linkers) such as (GS)n, (SG)n, and (GGGGS)n (SEQ ID NO: 99), where n represents an integer of at least 1. Those of skill in the art would be able to select the appropriate spacer sequence.

In some embodiments, knobs-into-holes mutations were introduced in the Fc regions of the multispecific (e.g., bispecific) antibodies to reduce the chance of wrong pairing between the two heavy chains.

The disclosure also provides nucleic acid comprising a polynucleotide encoding an anti-CD79b/CLEC5A antibody. The immunoglobulin heavy chain or immunoglobulin light chain in the anti-CD79b/CLEC5A antibody comprises CDRs as shown in **Table 22**. When the polypeptides are paired with corresponding polypeptide (e.g., a corresponding heavy chain variable region or a corresponding light chain variable region), the paired polypeptides bind to CD79b and/or CLEC5A.

### Antibody Drug Conjugates (ADC)

The antibodies, the antigen-binding fragments thereof, or the antigen-binding protein constructs (e.g., bispecific antibodies) described herein can be conjugated to a therapeutic agent (a drug). The therapeutic agent can be covalently or non-covalently bind to the antibody or antigen-binding fragment or the antigen binding protein construct (e.g., a bispecific antibody).

In some embodiments, the therapeutic agent is a cytotoxic or cytostatic agent (e.g., monomethyl auristatin E, monomethyl auristatin F, cytochalasin B, gramicidin D, ethidium bromide, emetine, mitomycin, etoposide, tenoposide, vincristine, vinblastine, colchicin, doxorubicin, daunorubicin, dihydroxy anthracin, maytansinoids such as DM-1 and DM-4, dione, mitoxantrone, mithramycin, actinomycin D, 1-dehydrotestosterone, glucocorticoids, procaine, tetracaine, lidocaine, propranolol, puromycin, epirubicin, and cyclophosphamide and analogs). Useful classes of cytotoxic, cytostatic, or immunomodulatory agents include, for example, antitubulin agents, DNA minor groove binders, DNA replication inhibitors, and alkylating agents.

In some embodiments, the therapeutic agent can include, but not limited to, cytotoxic reagents, such as chemo-therapeutic agents, immunotherapeutic agents and the like, antiviral agents or antimicrobial agents. In some embodiments, the therapeutic agent to be conjugated can be selected from, but not limited to, MMAE (monomethyl auristatin E), MMAD (monomethyl auristatin D), or MMAF (monomethyl auristatin F).

In some embodiments, the therapeutic agent is an auristatin, such as auristatin E (also known in the art as a derivative of dolastatin-10) or a derivative thereof. The auristatin can be, for example, an ester formed between auristatin E and a keto acid. For example, auristatin E can be reacted with paraacetyl benzoic acid or benzoylvaleric acid to produce AEB and AEVB, respectively. Other typical auristatins include AFP, MMAF, and MMAE. The synthesis and structure of exemplary auristatins are described in U.S. Patent Application Publication No. 2003-0083263; International Patent Publication No. WO 04/010957, International Patent Publication No. WO 02/088172, and U.S. Pat. Nos. 7,498,298, 6,884,869, 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414, each of which is incorporated by reference herein in its entirety and for all purposes.

Auristatins have been shown to interfere with microtubule dynamics and nuclear and cellular division and have anticancer activity. Auristatins bind tubulin and can exert a cytotoxic or cytostatic effect on cancer cell. There are a number of different assays, known in the art, which can be used for determining whether an auristatin or resultant antibody-drug conjugate exerts a cytostatic or cytotoxic effect on a desired cell.

In some embodiments, the therapeutic agent is a chemotherapeutic agent. Examples of chemotherapeutic agents include alkylating agents such as thiotepa and cyclosphosphamide (CYTOXAN^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK7; razoxane; sizofiran; spirogermanium; tenuazonic acid; triaziquone; 2',2',2'-trichlorotriethylamine; urethan; vindesine; dacarbazine; mannomustine; mitobronitol; mitolactol; pipobroman; gacytosine; arabinoside ("Ara-C"); cyclophosphamide; taxanes, e.g. paclitaxel (TAXOL^{®}, Bristol-Myers Squibb Oncology, Princeton, N.J.) and doxetaxel (TAXOTERE^{®}, Rhone-Poulenc Rorer, Antony, France); chlorambucil; gemcitabine; 6-thioguanine; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above. A detailed description of the chemotherapeutic agents can be found in, e.g., US20180193477A1, which is incorporated by reference in its entirety.

In some embodiments, the antigen-binding construct is coupled to the drug via a cleavable linker e.g. a SPBD linker or a maleimidocaproyl-valine-citrulline-p-aminobenzyloxycarbonyl (VC) linker. In some embodiments, the antigen-binding construct is coupled to the drug via a non-cleavable linker e.g. a MCC linker formed using SMCC or sulfo-SMCC. Selection of an appropriate linker for a given ADC can be readily made by the skilled person having knowledge of the art and taking into account relevant factors, such as the site of attachment to the antigen binding construct, any structural constraints of the drug and the hydrophobicity of the drug (see, for example, review in Nolting, Chapter 5, Antibody-Drug Conjugates: Methods in Molecular Biology, 2013, Ducry (Ed.), Springer). A number of specific linker-toxin combinations have been described and may be used with the antigen binding constructs described herein to prepare ADCs in certain embodiments. Examples include, but are not limited to, cleavable peptide-based linkers with auristatins such as MMAE and MMAF, camptothecins such as SN-38, duocarmycins and PBD dimers; non-cleavable MC-based linkers with auristatins MMAF and MMAE; acid-labile hydrazone-based linkers with calicheamicins and doxorubicin; disulfide-based linkers with maytansinoids such as DM1 and DM4, and bis-maleimido-trioxyethylene glycol (BMPEO)-based linkers with maytansinoid DM1. Some these therapeutic agents and linkers are described, e.g., in Peters & Brown, (2015) Biosci. Rep. e00225; Dosio et al., (2014) Recent Patents on Anti-Cancer Drug Discovery 9:35-65; US Patent Publication No. US 2015/0374847, and US20180193477A1; which are incorporated herein by reference in the entirety.

Depending on the desired drug and selected linker, those skilled in the art can select suitable method for coupling them together. For example, some conventional coupling methods, such as amine coupling methods, can be used to form the desired drug-linker complex which still contains reactive groups for conjugating to the antibodies through covalent linkage. In some embodiments, a drug-maleimide complex (i.e., maleimide linking drug) can be used for the payload bearing reactive group in the present disclosure. Most common reactive group capable of bonding to thiol group in ADC preparation is maleimide. Additionally, organic bromides, iodides also are frequently used.

The ADC can be prepared by one of several routes known in the art, employing organic chemistry reactions, conditions, and reagents known to those skilled in the art (see, for example, Bioconjugate Techniques (G. T. Hermanson, 2013, Academic Press). For example, conjugation can be achieved by (1) reaction of a nucleophilic group or an electrophilic group of an antibody with a bivalent linker reagent, to form antibody-linker intermediate Ab-L, via a covalent bond, followed by reaction with an activated drug moiety D; or (2) reaction of a nucleophilic group or an electrophilic group of a drug moiety with a linker reagent, to form drug-linker intermediate D-L, via a covalent bond, followed by reaction with the nucleophilic group or an electrophilic group of an antibody. Conjugation methods (1) and (2) can be employed with a variety of antibodies, drug moieties, and linkers to prepare the ADCs described here. Various prepared linkers, linker components and toxins are commercially available or may be prepared using standard synthetic organic chemistry techniques. These methods are described e.g., in March's Advanced Organic Chemistry (Smith & March, 2006, Sixth Ed., Wiley); Toki et al., (2002) J. Org. Chem. 67:1866-1872; Frisch et al., (1997) Bioconj. Chem. 7:180-186; Bioconjugate Techniques (G. T. Hermanson, 2013, Academic Press); US20210379193A1, and US20180193477A1, which are incorporated herein by reference in the entirety. In addition, a number of pre-formed drug-linkers suitable for reaction with a selected antigen binding construct are also available commercially, for example, linker-toxins comprising DM1, DM4, MMAE, MMAF or Duocarmycin SA are available from Creative BioLabs (Shirley, N.Y.).

Several specific examples of methods of preparing ADCs are known in the art and are described in U.S. Pat. No. 8,624,003 (pot method), U.S. Pat. No. 8,163,888 (one-step), and U.S. Pat. No. 5,208,020 (two-step method), and US20180193477A1, which are incorporated herein by reference in the entirety. Other methods are known in the art and include those described in Antibody-Drug Conjugates: Methods in Molecular Biology, 2013, Ducry (Ed.), Springer.

Drug loading is represented by the number of drug moieties per antibody in a molecule of ADC. For some antibody-drug conjugates, the drug loading may be limited by the number of attachment sites on the antibody. For example, where the attachment is a cysteine thiol, as in certain exemplary embodiments described herein, the drug loading may range from 0 to 8 drug moieties per antibody. In certain embodiments, higher drug loading, e.g. p≥5, may cause aggregation, insolubility, toxicity, or loss of cellular permeability of certain antibody-drug conjugates. In certain embodiments, the average drug loading for an antibody-drug conjugate ranges from 1 to about 8; from about 2 to about 6; or from about 3 to about 5. Indeed, it has been shown that for certain antibody-drug conjugates, the optimal ratio of drug moieties per antibody can be around 4. In some embodiments, the drug-to-antibody ratio (DAR) is about or at least 1, 2, 3, 4, 5, 6, 7, or 8. In some embodiments, the average DAR in the composition is about 1~ about 2, about 2~ about 3, about 3~ about 4, about 3~ about 5, about 4~ about 5, about 5~ about 6, about 6~ about 7, or about 7~ about 8.

### Antibodies and Antigen Binding Fragments thereof

In some embodiments, the multispecific antibodies (e.g., bispecific antibodies) and antigen-binding fragments thereof can have various forms.

In general, antibodies (also called immunoglobulins) can be made up of two classes of polypeptide chains, light chains and heavy chains. A non-limiting anti-HER2/CLEC5A antibody of the present disclosure can be an intact, four immunoglobulin chain antibody comprising two heavy chains and two light chains. The heavy chain of the anti-HER2/CLEC5A antibody can be of any isotype including IgM, IgG, IgE, IgA, or IgD or sub-isotype including IgG1, IgG2, IgG2a, IgG2b, IgG3, IgG4, IgE1, IgE2, etc. The light chain can be a kappa light chain or a lambda light chain.

The hypervariable regions, known as the complementary determining regions (CDRs), form loops that comprise the principle antigen binding surface of the antibody. The four framework regions largely adopt a beta-sheet conformation and the CDRs form loops connecting, and in some cases forming part of, the beta-sheet structure. The CDRs in each chain are held in close proximity by the framework regions and, with the CDRs from the other chain, contribute to the formation of the antigen-binding domain.

Methods for identifying the CDR regions of an antibody by analyzing the amino acid sequence of the antibody are well known, and a number of definitions of the CDRs are commonly used. The Kabat definition is based on sequence variability, and the Chothia definition is based on the location of the structural loop regions. These methods and definitions are described in, e.g., Martin, "Protein sequence and structure analysis of antibody variable domains," Antibody engineering, Springer Berlin Heidelberg, 2001. 422-439; Abhinandan, et al. "Analysis and improvements to Kabat and structurally correct numbering of antibody variable domains," Molecular immunology 45.14 (2008): 3832-3839; Wu, T.T. and Kabat, E.A. (1970) J. Exp. Med. 132: 211-250; Martin et al., Methods Enzymol. 203:121-53 (1991); Morea et al., Biophys Chem. 68(1-3):9-16 (Oct. 1997); Morea et al., J Mol Biol. 275(2):269-94 (Jan .1998); Chothia et al., Nature 342(6252):877-83 (Dec. 1989); Ponomarenko and Bourne, BMC Structural Biology 7:64 (2007); each of which is incorporated herein by reference in its entirety.

The CDRs are important for recognizing an epitope of an antigen. As used herein, an "epitope" is the smallest portion of a target molecule capable of being specifically bound by the antigen-binding domain of an antibody. The minimal size of an epitope may be about three, four, five, six, or seven amino acids, but these amino acids need not be in a consecutive linear sequence of the antigen's primary structure, as the epitope may depend on an antigen's three-dimensional configuration based on the antigen's secondary and tertiary structure.

In some embodiments, the antibody is an intact immunoglobulin molecule (e.g., IgG1, IgG2a, IgG2b, IgG3, IgM, IgD, IgE, IgA). The IgG subclasses (IgG1, IgG2, IgG3, and IgG4) are highly conserved, differ in their constant region, particularly in their hinges and upper CH2 domains. The sequences and differences of the IgG subclasses are known in the art, and are described, e.g., in Vidarsson, et al, "IgG subclasses and allotypes: from structure to effector functions." Frontiers in immunology 5 (2014); Irani, et al. "Molecular properties of human IgG subclasses and their implications for designing therapeutic monoclonal antibodies against infectious diseases." Molecular immunology 67.2 (2015): 171-182; Shakib, Farouk, ed. The human IgG subclasses: molecular analysis of structure, function and regulation. Elsevier, 2016; each of which is incorporated herein by reference in its entirety.

The antibody can also be an immunoglobulin molecule that is derived from any species (e.g., human, rodent, mouse, rat, or camelid). The antigen-binding domain or antigen binding fragment is a portion of an antibody that retains specific binding activity of the intact antibody, i.e., any portion of an antibody that is capable of specific binding to an epitope on the intact antibody's target molecule. It includes, e.g., Fab, Fab', F(ab')₂, and variants of these fragments. Thus, in some embodiments, an antibody or antigen binding fragment thereof can comprise e.g., a scFv, a Fv, a Fd, a dAb, a bispecific antibody, a bispecific scFv, a diabody, a linear antibody, a single-chain antibody molecule, a multispecific antibody formed from antibody fragments, and any polypeptide that includes a binding domain which is, or is homologous to, an antibody binding domain. Non-limiting examples of antigen-binding domains include, e.g., the heavy chain and/or light chain CDRs of an intact antibody, the heavy and/or light chain variable regions of an intact antibody, full length heavy or light chains of an intact antibody, or an individual CDR from either the heavy chain or the light chain of an intact antibody.

In some embodiments, the scFv in a multispecific (e.g., bispecific) antibody has two heavy chain variable domains, and two light chain variable domains. In some embodiments, the scFv has two antigen binding regions (Antigen binding regions: A and B), and the two antigen binding regions can bind to the respective target antigens with different affinities.

In some embodiments, the multispecific (e.g., bispecific) antibodies or antigen-binding fragments thereof can comprises one, two, or three heavy chain variable region CDRs selected from **Table 22**.

In some embodiments, the multispecific (e.g., bispecific) antibodies described herein can be conjugated to a therapeutic agent. The antibody-drug conjugate comprising the antibody or antigen-binding fragment thereof can covalently or non-covalently bind to a therapeutic agent. In some embodiments, the therapeutic agent is a cytotoxic or cytostatic agent (e.g., monomethyl auristatin E, monomethyl auristatin F, cytochalasin B, gramicidin D, ethidium bromide, emetine, mitomycin, etoposide, tenoposide, vincristine, vinblastine, colchicin, doxorubicin, daunorubicin, dihydroxy anthracin, maytansinoids such as DM-1 and DM-4, dione, mitoxantrone, mithramycin, actinomycin D, 1-dehydrotestosterone, glucocorticoids, procaine, tetracaine, lidocaine, propranolol, puromycin, epirubicin, and cyclophosphamide and analogs). In some embodiments, the therapeutic agent is MMAE or MMAF. In some embodiments, the therapeutic agent is conjugated via a linker, e.g., a VC linker. Details of the linkers used for ADCs can be found "e.g., in Su, Z. et al. "Antibody-drug conjugates: Recent advances in linker chemistry." Acta Pharmaceutica Sinica B (2021), which is incorporated herein by reference in its entirety.

Multispecific (e.g., bispecific) antibodies can be made by engineering the interface between a pair of antibody molecules to maximize the percentage of heterodimers that are recovered from recombinant cell culture. For example, the interface can contain at least a part of the CH3 domain of an antibody constant domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers. This method is described, e.g., in WO 96/27011, which is incorporated by reference in its entirety.

Any of the multispecific (e.g., bispecific) antibodies or antigen-binding fragments thereof described herein may be conjugated to a stabilizing molecule (e.g., a molecule that increases the half-life of the antibody or antigen-binding fragment thereof in a subject or in solution). Non-limiting examples of stabilizing molecules include: a polymer (e.g., a polyethylene glycol) or a protein (e.g., serum albumin, such as human serum albumin). The conjugation of a stabilizing molecule can increase the half-life or extend the biological activity of an anti-HER2/CLEC5A antibody or an antigen-binding fragment in vitro (e.g., in tissue culture or when stored as a pharmaceutical composition) or in vivo (e.g., in a human).

The bispecific antibodies or antigen-binding fragments thereof can also have various forms. Many different formats of bispecific antibodies or antigen-binding fragments thereof are known in the art, and are described e.g., in Suurs, et al. "A review of bispecific antibodies and antibody constructs in oncology and clinical challenges," Pharmacology & therapeutics (2019), which is incorporated herein by reference in the entirety.

In some embodiments, the bispecific antibody is a BiTE, a (scFv)₂, a nanobody, a nanobody-HSA, a DART, a TandAb, a scDiabody, a scDiabody-CH3, scFv-CH-CL-scFv, a HSAbody, scDiabody-HAS, or a tandem-scFv. In some embodiments, the bispecific antibody is a VHH-scAb, a VHH-Fab, a Dual scFab, a F(ab')₂, a diabody, a crossMab, a DAF (two-in-one), a DAF (four-in-one), a DutaMab, a DT-IgG, a knobs-in-holes common light chain, a knobs-in-holes assembly, a charge pair, a Fab-arm exchange, a SEEDbody, a LUZ-Y, a Fcab, a κλ-body, an orthogonal Fab, a DVD-IgG, a IgG(H)-scFv, a scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-IgG, IgG-2scFv, scFv4-Ig, Zybody, DVI-IgG, Diabody-CH3, a triple body, a miniantibody, a minibody, a TriBi minibody, scFv-CH3 KIH, Fab-scFv, a F(ab')₂-scFv2, a scFv-KIH, a Fab-scFv-Fc, a tetravalent HCAb, a scDiabody-Fc, a Diabody-Fc, a tandem scFv-Fc, an Intrabody, a dock and lock, a lmmTAC, an IgG-IgG conjugate, a Cov-X-Body, or a scFv1-PEG-scFv2.

In some embodiments, the bispecific antibody can be a TrioMab. In a TrioMab, the two heavy chains are from different species, wherein different sequences restrict the heavy-light chain pairing.

In some embodiments, the bispecific antibody has two different heavy chains and one common light chain. In some embodiments, the bispecific antibody has two different heavy chains and two different light chains. Heterodimerization of heavy chains can be based on the knobs-into-holes or some other heavy chain pairing technique.

In some embodiments, CrossMAb technique can be used produce bispecific antibodies. CrossMAb technique can be used enforce correct light chain association in bispecific heterodimeric IgG antibodies, this technique allows the generation of various bispecific antibody formats, including bi-(1+1), tri-(2+1) and tetra-(2+2) valent bispecific antibodies, as well as non-Fc tandem antigen-binding fragment (Fab)-based antibodies. These formats can be derived from any existing antibody pair using domain crossover, without the need for the identification of common light chains, post-translational processing/in vitro chemical assembly or the introduction of a set of mutations enforcing correct light chain association. The method is described in Klein et al., "The use of CrossMAb technology for the generation of bi- and multispecific antibodies." mAbs. Vol. 8. No. 6. Taylor & Francis, 2016, which is incorporated by reference in its entirety. In some embodiments, the CH1 in the heavy chain and the CL domain in the light chain are swapped.

The bispecific antibody can be a Duobody. The Fab-exchange mechanism naturally occurring in IgG4 antibodies is mimicked in a controlled matter in IgG1 antibodies, a mechanism called controlled Fab exchange. This format can ensure specific pairing between the heavy-light chains.

In Dual-variable-domain antibody (DVD-Ig), additional VH and variable light chain (VL) domain are added to each N-terminus for bispecific targeting. This format resembles the IgG-scFv, but the added binding domains are bound individually to their respective N-termini instead of a scFv to each heavy chain N-terminus.

In scFv-IgG, the two scFv are connected to the C-terminus of the heavy chain (CH3). The scFv-IgG format has two different bivalent binding sites and is consequently also called tetravalent. There are no heavy-chain and light-chain pairing problem in the scFv-IgG.

In some embodiments, the bispecific antibody can have a IgG-IgG format. Two intact IgG antibodies are conjugated by chemically linking the C-terminals of the heavy chains.

The bispecific antibody can also have a Fab-scFv-Fc format. In Fab-scFv-Fc format, a light chain, heavy chain and a third chain containing the Fc region and the scFv are assembled. It can ensure efficient manufacturing and purification.

In some embodiments, the bispecific antibody can be a TF. Three Fab fragments are linked by disulfide bridges. Two fragments target the tumor associated antigen (TAA) and one fragment targets a hapten. The TF format does not have an Fc region.

ADAPTIR has two scFvs bound to each side of an Fc region. It abandons the intact IgG as a basis for its construct, but conserves the Fc region to extend the half-life and facilitate purification.

Dual affinity retargeting (DART) has two peptide chains connecting the opposite fragments, thus VLA with VHB and VLB with VHA, and a sulfur bond at their C-termini fusing them together. In DART, the sulfur bond can improve stability over BiTEs.

In DART-Fc, an Fc region is attached to the DART structure. It can be generated by assembling three chains, two via a disulfide bond, as with the DART. One chain contains half of the Fc region which will dimerize with the third chain, only expressing the Fc region. The addition of Fc region enhances half-life leading to longer effective concentrations, avoiding continuous IV.

In tetravalent DART, four peptide chains are assembled. Basically, two DART molecules are created with half an Fc region and will dimerize. This format has bivalent binding to both targets, thus it is a tetravalent molecule.

Tandem diabody (TandAb) comprises two diabodies. Each diabody consists of an VHA and VLB fragment and a VHA and VLB fragment that are covalently associated. The two diabodies are linked with a peptide chain. It can improve stability over the diabody consisting of two scFvs. It has two bivalent binding sites.

The scFv-scFv-toxin includes toxin and two scFv with a stabilizing linker. It can be used for specific delivery of payload.

In some embodiments, the bispecific antibody in present disclosure is designed to be 1+1 (monovalent for each target) and has an IgG1 subtype structure. This can reduce the avidity to cells with low expression levels of the first and second epitope, and increase the avidity to cells that co-express the first and second epitope, to achieve enhanced targeting function.

In some embodiments, the bispecific antibodies include KIH mutations. In some embodiments, the bispecific antibody includes a first arm comprising a first antigen-binding domain that specifically binds to the first epitope, and a second arm comprising a second antigen-binding domain that specifically binds to the second epitope. In some embodiments, the first antigen-binding domain includes a heavy chain that including one or more knob mutations (a knob heavy chain), and the second antigen-binding domain includes a heavy chain including one or more hole mutations (a hole heavy chain). In some embodiments, the first antigen-binding domain includes a heavy chain that includes one or more hole mutations (a hole heavy chain), and the second antigen-binding domain that includes a heavy chain including one or more knob mutations (a knob heavy chain).

### Antibody Characteristics

In some embodiments, the multispecific (e.g., bispecific) antibodies, or antigen-binding fragments thereof can initiate macrophage-mediated target cell (tumor cell) killing, CDC or ADCC.

The disclosure provides multispecific (e.g., bispecific) antibodies and antigen-binding fragments thereof that can specifically bind to a TAA (e.g., HER2) and CLEC5A. The antibodies or antigen-binding fragments thereof described herein can bind to antigens on target cells (e.g., TAA-expressing cells such as HER2+ cells) and recruit effector cells such as macrophages for the killing of the target cells.

General techniques can be used to measure the affinity of an antibody for an antigen include, e.g., ELISA, RIA, and surface plasmon resonance (SPR). Affinities can be deduced from the quotient of the kinetic rate constants (KD=koff/kon). In some implementations, the antibodies or antigen-binding fragments thereof can bind to the TAA with a dissociation rate (koff) of less than 0.1 s⁻¹, less than 0.01 s⁻¹, less than 0.001 s⁻¹, less than 0.0001 s⁻¹, or less than 0.00001 s⁻¹. In some embodiments, the dissociation rate (koff) is greater than 0.01 s⁻¹, greater than 0.001 s⁻¹, greater than 0.0001 s⁻¹, greater than 0.00001 s⁻¹, or greater than 0.000001 s⁻¹.

In some embodiments, kinetic association rates (kon) is greater than 1 × 10²/Ms, greater than 1 × 10³/Ms, greater than 1 × 10⁴/Ms, greater than 1 × 10⁵/Ms, or greater than 1 × 10⁶/Ms. In some embodiments, kinetic association rates (kon) is less than 1 × 10⁵/Ms, less than 1 × 10⁶/Ms, or less than 1 × 10⁷/Ms.

In some embodiments, the antibodies or antigen-binding fragments thereof can bind to the TAA with a KD of less than 1 × 10⁻⁶ M, less than 1 × 10⁻⁷ M, less than 1 × 10⁻⁸ M, less than 1 × 10⁻⁹ M, or less than 1 × 10⁻¹⁰ M. In some embodiments, the KD is less than 50 nM, 40 nM, 30 nM, 20 nM, 10 nM, 9 nM, 8 nM, 7 nM, 6 nM, 5 nM, 4 nM, 3 nM, 2 nM, or 1 nM. In some embodiments, KD is greater than 1 × 10⁻⁷ M, greater than 1 × 10⁻⁸ M, greater than 1 × 10⁻⁹ M, or greater than 1 × 10⁻¹⁰ M.

The multispecific (e.g., bispecific) antibodies or antigen-binding fragments thereof can also include an antigen-binding domain that can specifically bind to CLEC5A. In some embodiments, the antibodies or antigen-binding fragments thereof described herein activates macrophages and enhances macrophage-mediated cell killing (e.g., tumor cell killing). In some embodiments, the antibody can reduce tumor volume in an animal. In some embodiments, the antibody can reduce or slow down the progression of an autoimmune disease.

In some embodiments, the antibody or antigen-binding fragment thereof described herein has an agonistic activity on macrophage activation. In some embodiments, the activation of macrophages is increased by about or at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100%, compared to the activation of macrophages without the contact with the antibody or antigen-binding fragment thereof described herein.

In some embodiments, the multispecific (e.g., bispecific) antibodies or antigen-binding fragments thereof can bind to the TAA and/or CLEC5A with a dissociation rate (koff) of less than 0.1 s⁻¹, less than 0.01 s⁻¹, less than 0.001 s⁻¹, less than 0.0001 s⁻¹, or less than 0.00001 s⁻¹. In some embodiments, the dissociation rate (koff) is greater than 0.01 s⁻¹, greater than 0.001 s⁻¹, greater than 0.0001 s⁻¹, greater than 0.00001 s⁻¹, or greater than 0.000001 s⁻¹.

In some embodiments, kinetic association rates (kon) is greater than 1 × 10²/Ms, greater than 1 × 10³/Ms, greater than 1 × 10⁴/Ms, greater than 1 × 10⁵/Ms, or greater than 1 × 10⁶/Ms. In some embodiments, kinetic association rates (kon) is less than 1 × 10⁵/Ms, less than 1 × 10⁶/Ms, or less than 1 × 10⁷/Ms.

Affinities can be deduced from the quotient of the kinetic rate constants (KD=koff/kon). In some embodiments, KD is less than 1 × 10⁻⁶ M, less than 1 × 10⁻⁷ M, less than 1 × 10⁻⁸ M, less than 1 × 10⁻⁹ M, or less than 1 × 10⁻¹⁰ M. In some embodiments, the KD is less than 50 nM, 40 nM, 30 nM, 20 nM, 15 nM, 10 nM, 9 nM, 8 nM, 7 nM, 6 nM, 5 nM, 4 nM, 3 nM, 2 nM, or 1 nM. In some embodiments, KD is greater than 1 × 10⁻⁷ M, greater than 1 × 10⁻⁸ M, greater than 1 × 10⁻ 9 M, or greater than 1 × 10⁻¹⁰ M.

Because the multispecific antibody (e.g., bispecific antibody) binds to both the TAA and CLEC5A, for cells that express both these molecules, the antibody has a higher binding affinity to these cells. Avidity can be used to measure the binding affinity of an antibody to these cells. Avidity is the accumulated strength of multiple affinities of individual non-covalent binding interactions.

In some embodiments, the multispecific (e.g., bispecific) antibody, antigen-binding fragment, or ADC has a tumor growth inhibition rate or percentage (TGI%) that is greater than 10%, 20%, 30%, 40%, 50%, 60%, 70%, 80%, 90%, 100%, 110%, 120%, 130%, 140%, 150%, 160%, 170%, 180%, 190%, or 200%. In some embodiments, the multispecific (e.g., bispecific) antibody, antigen-binding fragment, or ADC has a tumor growth inhibition percentage that is less than 60%, 70%, 80%, 90%, 100%, 110%, 120%, 130%, 140%, or 150%. The TGI (%) can be determined, e.g., at 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or 41 days after the treatment starts. As used herein, the tumor growth inhibition rate or percentage (TGI%) is calculated using the following formula: TGI (%) = [1-(Ti-T0)/(Vi-V0)]×100% Ti is the average tumor volume in the treatment group on day i. T0 is the average tumor volume in the treatment group on day zero. Vi is the average tumor volume in the control group on day i. V0 is the average tumor volume in the control group on day zero.

In some embodiments, the multispecific (e.g., bispecific) antibody, antigen-binding fragment, or ADC has a functional Fc region. In some embodiments, effector function of a functional Fc region is antibody-dependent cell-mediated cytotoxicity (ADCC). In some embodiments, effector function of a functional Fc region is phagocytosis. In some embodiments, effector function of a functional Fc region is ADCC and phagocytosis. In some embodiments, the Fc region is human IgG1, human IgG2, human IgG3, or human IgG4.

In some embodiments, the multispecific (e.g., bispecific) antibody, antigen-binding fragment, or ADC does not have a functional Fc region. For example, the antibodies or antigen-binding fragments thereof are Fab, Fab', F(ab')₂, and Fv fragments. In some embodiments, the multispecific (e.g., bispecific) antibody or antigen-binding fragments thereof as described herein have an Fc region without effector function. In some embodiments, the Fc is a human IgG1 Fc. In some embodiments, the Fc does not have a functional Fc region. For example, the Fc region has LALA mutations (L234A and L235A mutations in EU numbering), or LALAPG mutations (L234A, L235A, P329G mutations in EU numbering). The amino acid sequence of the LALAPG mutant of human IgG1 is shown in SEQ ID NO: 42.

In some embodiments, the multispecific (e.g., bispecific) antibody or antigen-binding fragment thereof comprises a fragment crystallizable region (Fc region). In some embodiments, the antibody described herein can be designed to have an IgG1 Fc region having an Alanine (A) at position 236, a Leucine (L) at position 330, and a Glutamic acid (E) at position 332 in EU numbering. In some embodiments, The Fc region comprises an amino acid sequence that is about or at least 60%, 65%, 70%, 75%, 80%, 85%, 90%, 95%, or 100% identical to SEQ ID NO: 43.

Some other modifications to the Fc region can be made. For example, a cysteine residue(s) can be introduced into the Fc region, thereby allowing interchain disulfide bond formation in this region. The homodimeric fusion protein thus generated may have any increased half-life in vitro and/or in vivo.

In some embodiments, multispecific (e.g., bispecific) antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn297 (e.g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues; or position 314 in Kabat numbering); however, Asn297 may also be located about ±3 amino acids upstream or downstream of position 297, i.e., between positions 294 and 300, due to minor sequence variations in antibodies. Such fucosylation variants may have improved ADCC function. In some embodiments, to reduce glycan heterogeneity, the Fc region of the antibody can be further engineered to replace the Asparagine at position 297 with Alanine (N297A).

### Recombinant Vectors

The present disclosure also provides recombinant vectors (e.g., expression vectors) that include an isolated polynucleotide disclosed herein (e.g., a polynucleotide that encodes a polypeptide disclosed herein), host cells into which are introduced the recombinant vectors (i.e., such that the host cells contain the polynucleotide and/or a vector comprising the polynucleotide), and the production of multispecific (e.g., bispecific) antibody polypeptides or fragments thereof by recombinant techniques.

In some embodiments, the multispecific (e.g., bispecific) antibody is an anti-TAA/CLEC5A (e.g., anti-HER2/CLEC5A antibody). The full length sequence of human HER2 and/or CLEC5A is known in the art. In some embodiments, a His-tagged human HER2 and/or CLEC5A protein is used as the immunogen.

As used herein, a "vector" is any construct capable of delivering one or more polynucleotide(s) of interest to a host cell when the vector is introduced to the host cell. An "expression vector" is capable of delivering and expressing the one or more polynucleotide(s) of interest as an encoded polypeptide in a host cell into which the expression vector has been introduced. Thus, in an expression vector, the polynucleotide of interest is positioned for expression in the vector by being operably linked with regulatory elements such as a promoter, enhancer, and/or a poly-A tail, either within the vector or in the genome of the host cell at or near or flanking the integration site of the polynucleotide of interest such that the polynucleotide of interest will be translated in the host cell introduced with the expression vector.

A vector can be introduced into the host cell by methods known in the art, e.g., electroporation, chemical transfection (e.g., DEAE-dextran), transformation, transfection, and infection and/or transduction (e.g., with recombinant virus). Thus, non-limiting examples of vectors include viral vectors (which can be used to generate recombinant virus), naked DNA or RNA, plasmids, cosmids, phage vectors, and DNA or RNA expression vectors associated with cationic condensing agents.

In some implementations, a polynucleotide disclosed herein (e.g., a polynucleotide that encodes a polypeptide disclosed herein) is introduced using a viral expression system (e.g., vaccinia or other pox virus, retrovirus, or adenovirus), which may involve the use of a non-pathogenic (defective), replication competent virus, or may use a replication defective virus. In the latter case, viral propagation generally will occur only in complementing virus packaging cells. Suitable systems are disclosed, for example, in Fisher-Hoch et al., 1989, Proc. Natl. Acad. Sci. USA 86:317-321; Flexner et al., 1989, Ann. N.Y. Acad Sci. 569:86-103; Flexner et al., 1990, Vaccine, 8:17-21; U.S. Pat. Nos. 4,603,112, 4,769,330, and 5,017,487; WO 89/01973; U.S. Pat. No. 4,777,127; GB 2,200,651; EP 0,345,242; WO 91/02805; Berkner-Biotechniques, 6:616-627, 1988; Rosenfeld et al., 1991, Science, 252:431-434; Kolls et al., 1994, Proc. Natl. Acad. Sci. USA, 91:215-219; Kass-Eisler et al., 1993, Proc. Natl. Acad. Sci. USA, 90:11498-11502; Guzman et al., 1993, Circulation, 88:2838-2848; and Guzman et al., 1993, Cir. Res., 73:1202-1207. Techniques for incorporating DNA into such expression systems are well known to those of ordinary skill in the art. The DNA may also be "naked," as described, for example, in Ulmer et al., 1993, Science, 259:1745-1749, and Cohen, 1993, Science, 259:1691-1692. The uptake of naked DNA may be increased by coating the DNA onto biodegradable beads that are efficiently transported into the cells.

For expression, the DNA insert comprising a polypeptide-encoding polynucleotide disclosed herein can be operatively linked to an appropriate promoter (e.g., a heterologous promoter), such as the phage lambda PL promoter, the E. coli lac, trp and tac promoters, the SV40 early and late promoters and promoters of retroviral LTRs, to name a few. Other suitable promoters are known to the skilled artisan. The expression constructs can further contain sites for transcription initiation, termination and, in the transcribed region, a ribosome binding site for translation. The coding portion of the mature transcripts expressed by the constructs may include a translation initiating at the beginning and a termination codon (UAA, UGA, or UAG) appropriately positioned at the end of the polypeptide to be translated.

As indicated, the expression vectors can include at least one selectable marker. Such markers include dihydrofolate reductase or neomycin resistance for eukaryotic cell culture and tetracycline or ampicillin resistance genes for culturing in E. coli and other bacteria. Representative examples of appropriate hosts include, but are not limited to, bacterial cells, such as E. coli, Streptomyces, and Salmonella typhimurium cells; fungal cells, such as yeast cells; insect cells such as Drosophila S2 and Spodoptera Sf9 cells; animal cells such as CHO, COS, Bowes melanoma, and HK 293 cells; and plant cells. Appropriate culture mediums and conditions for the host cells described herein are known in the art.

Non-limiting vectors for use in bacteria include pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Non-limiting eukaryotic vectors include pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Non-limiting bacterial promoters suitable for use include the E. coli lacI and lacZ promoters, the T3 and T7 promoters, the gpt promoter, the lambda PR and PL promoters and the trp promoter. Suitable eukaryotic promoters include the CMV immediate early promoter, the HSV thymidine kinase promoter, the early and late SV40 promoters, the promoters of retroviral LTRs, such as those of the Rous sarcoma virus (RSV), and metallothionein promoters, such as the mouse metallothionein-I promoter.

In the yeast Saccharomyces cerevisiae, a number of vectors containing constitutive or inducible promoters such as alpha factor, alcohol oxidase, and PGH may be used. For reviews, see Ausubel et al. (1989) Current Protocols in Molecular Biology, John Wiley & Sons, New York, N.Y, and Grant et al., Methods Enzymol., 153: 516-544 (1997).

Introduction of the construct into the host cell can be affected by calcium phosphate transfection, DEAE-dextran mediated transfection, cationic lipid-mediated transfection, electroporation, transduction, infection or other methods. Such methods are described in many standard laboratory manuals, such as Davis et al., Basic Methods In Molecular Biology (1986), which is incorporated herein by reference in its entirety.

Transcription of DNA encoding an antibody of the present disclosure by higher eukaryotes may be increased by inserting an enhancer sequence into the vector. Enhancers are cis-acting elements of DNA, usually about from 10 to 300 bp that act to increase transcriptional activity of a promoter in a given host cell-type. Examples of enhancers include the SV40 enhancer, which is located on the late side of the replication origin at base pairs 100 to 270, the cytomegalovirus early promoter enhancer, the polyoma enhancer on the late side of the replication origin, and adenovirus enhancers.

For secretion of the translated protein into the lumen of the endoplasmic reticulum, into the periplasmic space or into the extracellular environment, appropriate secretion signals may be incorporated into the expressed polypeptide. The signals may be endogenous to the polypeptide, or they may be heterologous signals.

The polypeptide can be expressed in a modified form, such as a fusion protein (e.g., a GST-fusion) or with a histidine-tag, and may include not only secretion signals, but also additional heterologous functional regions. For instance, a region of additional amino acids, particularly charged amino acids, may be added to the N-terminus of the polypeptide to improve stability and persistence in the host cell, during purification, or during subsequent handling and storage. Also, peptide moieties can be added to the polypeptide to facilitate purification. Such regions can be removed prior to final preparation of the polypeptide. The addition of peptide moieties to polypeptides to engender secretion or excretion, to improve stability and to facilitate purification, among others, are familiar and routine techniques in the art.

The disclosure also provides a nucleic acid sequence that is at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% identical to any nucleotide sequence as described herein, and an amino acid sequence that is at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% identical to any amino acid sequence as described herein.

The disclosure also provides a nucleic acid sequence that has a homology of at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% to any nucleotide sequence as described herein, and an amino acid sequence that has a homology of at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% to any amino acid sequence as described herein.

In some embodiments, the disclosure relates to nucleotide sequences encoding any peptides that are described herein, or any amino acid sequences that are encoded by any nucleotide sequences as described herein. In some embodiments, the nucleic acid sequence is less than 10, 20, 30, 40, 50, 60, 70, 80, 90, 100, 110, 120, 130, 150, 200, 250, 300, 350, 400, 500, or 600 nucleotides. In some embodiments, the amino acid sequence is less than 5, 6, 7, 8, 9, 10, 20, 30, 40, 50, 60, 70, 80, 90, 100, 110, 120, 130, 140, 150, 160, 170, 180, 190, 200, 250, 300, 350, or 400 amino acid residues.

In some embodiments, the amino acid sequence (i) comprises an amino acid sequence; or (ii) consists of an amino acid sequence, wherein the amino acid sequence is any one of the sequences as described herein.

In some embodiments, the nucleic acid sequence (i) comprises a nucleic acid sequence; or (ii) consists of a nucleic acid sequence, wherein the nucleic acid sequence is any one of the sequences as described herein.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences. For example, the comparison of sequences and determination of percent identity between two sequences can be accomplished using a Blossum 62 scoring matrix with a gap penalty of 12, a gap extend penalty of 4, and a frameshift gap penalty of 5.

The disclosure provides one or more nucleic acid encoding any of the polypeptides as described herein. In some embodiments, the nucleic acid (e.g., cDNA) includes a polynucleotide encoding a polypeptide of a heavy chain as described herein. In some embodiments, the nucleic acid includes a polynucleotide encoding a polypeptide of a light chain as described herein. In some embodiments, the nucleic acid includes a polynucleotide encoding a scFv polypeptide as described herein.

In some embodiments, the vector can have two of the nucleic acids as described herein, wherein the vector encodes the VL region and the VH region that together bind to the TAA (e.g., HER2). In some embodiments, a pair of vectors is provided, wherein each vector comprises one of the nucleic acids as described herein, wherein together the pair of vectors encodes the VL region and the VH region that together bind to the TAA (e.g., HER2).

In some embodiments, the vector includes two of the nucleic acids as described herein, wherein the vector encodes the VL region and the VH region that together bind to CLEC5A. In some embodiments, a pair of vectors is provided, wherein each vector comprises one of the nucleic acids as described herein, wherein together the pair of vectors encodes the VL region and the VH region that together bind to CLEC5A.

### Methods of Treatment

The methods described herein include methods for the treatment of disorders associated with cancer. Generally, the methods include administering a therapeutically effective amount of the multispecific (e.g., bispecific) antibodies (e.g., anti-TAA/CLEC5A antibodies such as anti-HER2/CLEC5A antibodies) as described herein, to a subject who is in need of, or who has been determined to be in need of, such treatment.

As used in this context, to "treat" means to ameliorate at least one symptom of the disorder associated with cancer. Often, cancer results in death; thus, a treatment can result in an increased life expectancy (e.g., by at least 1, 2, 3, 4, 5, 6, 7, 8, 9, 10, 11, 12 months, or by at least 1, 2, 3, 4, 5, 6, 7, 8, 9, 10 years). Administration of a therapeutically effective amount of an agent described herein for the treatment of a condition associated with cancer will result in decreased number of cancer cells and/or alleviated symptoms.

As used herein, the term "cancer" refers to cells having the capacity for autonomous growth, i.e., an abnormal state or condition characterized by rapidly proliferating cell growth. The term is meant to include all types of cancerous growths or oncogenic processes, metastatic tissues or malignantly transformed cells, tissues, or organs, irrespective of histopathologic type or stage of invasiveness. The term "tumor" as used herein refers to cancerous cells, e.g., a mass of cancerous cells. Cancers that can be treated or diagnosed using the methods described herein include malignancies of the various organ systems, such as affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus. In some embodiments, the agents described herein are designed for treating or diagnosing a carcinoma in a subject. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. In some embodiments, the cancer is renal carcinoma or melanoma. Exemplary carcinomas include those forming from tissue of the cervix, lung, prostate, breast, head and neck, colon and ovary. The term also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation.

In some embodiments, the cancer is a chemotherapy resistant cancer.

In one aspect, the disclosure also provides methods for treating a cancer in a subject, methods of reducing the rate of the increase of volume of a tumor in a subject over time, methods of reducing the risk of developing a metastasis, or methods of reducing the risk of developing an additional metastasis in a subject. In some embodiments, the treatment can halt, slow, retard, or inhibit progression of a cancer. In some embodiments, the treatment can result in the reduction of in the number, severity, and/or duration of one or more symptoms of the cancer in a subject.

In one aspect, the disclosure features methods that include administering a therapeutically effective amount of the multispecific (e.g., bispecific) antibodies (e.g., anti-TAA/CLEC5A antibodies such as anti-HER2/CLEC5A antibodies or anti-HER2/CLEC5A antibody-drug conjugates) as described herein to a subject in need thereof, e.g., a subject having, or identified or diagnosed as having, a cancer, e.g., B cell lymphoma, bladder cancer, breast cancer, cervical cancer, colorectal cancer, gastric cancer, non-small cell lung cancer (NSCLC), mesothelioma, ovarian cancer, pancreatic cancer, prostate cancer, oral cancer, or renal cancer. In some embodiments, the cancer is a solid tumor such as breast cancer, lung cancer, head and neck cancer, thyroid cancer, central nervous system (CNS) cancer, liver cancer, or brain cancer.

As used herein, the terms "subject" and "patient" are used interchangeably throughout the specification and describe an animal, human or non-human, to whom treatment according to the methods of the present invention is provided. Veterinary and non-veterinary applications are contemplated by the present invention. Human patients can be adult humans or juvenile humans (e.g., humans below the age of 18 years old). In addition to humans, patients include but are not limited to mice, rats, hamsters, guinea-pigs, rabbits, ferrets, cats, dogs, and primates. Included are, for example, non-human primates (e.g., monkey, chimpanzee, gorilla, and the like), rodents (e.g., rats, mice, gerbils, hamsters, ferrets, rabbits), lagomorphs, swine (e.g., pig, miniature pig), equine, canine, feline, bovine, and other domestic, farm, and zoo animals.

In some embodiments, the compositions and methods disclosed herein can be used for treatment of patients at risk for a cancer. Patients with cancer can be identified with various methods known in the art.

As used herein, by an "effective amount" is meant an amount or dosage sufficient to effect beneficial or desired results including halting, slowing, retarding, or inhibiting progression of a disease, e.g., a cancer. An effective amount will vary depending upon, e.g., an age and a body weight of a subject to which the antibody, antigen binding fragment, antibody-drug conjugates, antibody-encoding polynucleotide (e.g., anti-TAA/CLEC5A antibody such as anti-HER2/CLEC5A antibody, anti-HER2/CLEC5A antigen binding fragment, anti-HER2/CLEC5A antibody-encoding polynucleotide), vector comprising the polynucleotide, and/or compositions thereof is to be administered, a severity of symptoms and a route of administration, and thus administration can be determined on an individual basis.

An effective amount can be administered in one or more administrations. By way of example, an effective amount of the antibody, or antigen binding fragment (e.g., anti-TAA/CLEC5A antibody such as anti-HER2/CLEC5A antibody, anti-HER2/CLEC5A antigen binding fragment) is an amount sufficient to ameliorate, stop, stabilize, reverse, inhibit, slow and/or delay progression of an autoimmune disease or a cancer in a patient or is an amount sufficient to ameliorate, stop, stabilize, reverse, slow and/or delay proliferation of a cell (e.g., a biopsied cell, any of the cancer cells described herein, or cell line (e.g., a cancer cell line)) in vitro.

Effective amounts and schedules for administering the antibody, antigen binding fragment, antibody-drug conjugates, and/or compositions disclosed herein may be determined empirically, and making such determinations is within the skill in the art. Those skilled in the art will understand that the dosage that must be administered will vary depending on, for example, the mammal that will receive the antibody, antigen binding fragment, antibody-drug conjugates, and/or compositions disclosed herein, the route of administration, the particular type of the agent or compositions disclosed herein used and other drugs being administered to the mammal.

A typical daily dosage of an effective amount of the antibody, antigen binding fragment, antibody-drug conjugates (e.g., anti-TAA/CLEC5A antibody such as anti-HER2/CLEC5A antibody, anti-HER2/CLEC5A antigen binding fragment, anti-HER2/CLEC5A antibody-drug conjugates) is 0.01 mg/kg to 100 mg/kg. In some embodiments, the dosage can be less than 100 mg/kg, 30 mg/kg, 20 mg/kg, 10 mg/kg, 9 mg/kg, 8 mg/kg, 7 mg/kg, 6 mg/kg, 5 mg/kg, 4 mg/kg, 3 mg/kg, 2 mg/kg, 1 mg/kg, 0.5 mg/kg, or 0.1 mg/kg. In some embodiments, the dosage can be greater than 10 mg/kg, 9 mg/kg, 8 mg/kg, 7 mg/kg, 6 mg/kg, 5 mg/kg, 4 mg/kg, 3 mg/kg, 2 mg/kg, 1 mg/kg, 0.5 mg/kg, 0.1 mg/kg, 0.05 mg/kg, or 0.01 mg/kg. In some embodiments, the dosage is about or at least 10 mg/kg, 9 mg/kg, 8 mg/kg, 7 mg/kg, 6 mg/kg, 5 mg/kg, 4 mg/kg, 3 mg/kg, 2 mg/kg, 1 mg/kg, 0.9 mg/kg, 0.8 mg/kg, 0.7 mg/kg, 0.6 mg/kg, 0.5 mg/kg, 0.4 mg/kg, 0.3 mg/kg, 0.2 mg/kg, or 0.1 mg/kg.

In some embodiments, one or more additional therapeutic agents can be administered to the subject. The additional therapeutic agent can be an inhibitor of a TAA. The additional therapeutic agent can comprise one or more inhibitors selected from the group consisting of an inhibitor of B-Raf, a HER2 inhibitor, an inhibitor of a MEK, an inhibitor of ERK, an inhibitor of K-Ras, an inhibitor of c-Met, an inhibitor of CLEC5A, an inhibitor of anaplastic lymphoma kinase (ALK), an inhibitor of a phosphatidylinositol 3-kinase (PI3K), an inhibitor of an Akt, an inhibitor of mTOR, a dual PI3K/mTOR inhibit'r, an inhibitor of Bruton's tyrosine kinase (BTK), and an inhibitor of Isocitrate dehydrogenase 1 (IDH1) and/or Isocitrate dehydrogenase 2 (IDH2). In some embodiments, the additional therapeutic agent is an inhibitor of indoleamine 2,3-dioxygenase-1 (IDO1) (e.g., epacadostat).

In some embodiments, the additional therapeutic agent can comprise one or more inhibitors selected from the group consisting of an inhibitor of HER2, an inhibitor of CLEC5A, an inhibitor of LSD1, an inhibitor of MDM2, an inhibitor of BCL2, an inhibitor of CHK1, an inhibitor of activated hedgehog signaling pathway, and an agent that selectively degrades the estrogen receptor.

In some embodiments, the additional therapeutic agent can comprise one or more therapeutic agents selected from the group consisting of Trabectedin, nab-paclitaxel, Trebananib, Pazopanib, Cediranib, Palbociclib, everolimus, fluoropyrimidine, IFL, regorafenib, Reolysin, Alimta, Zykadia, Sutent, temsirolimus, axitinib, everolimus, sorafenib, Votrient, Pazopanib, IMA-901, AGS-003, cabozantinib, Vinflunine, an Hsp90 inhibitor, Ad-GM-CSF, Temazolomide, IL-2, IFNa, vinblastine, Thalomid, dacarbazine, cyclophosphamide, lenalidomide, azacytidine, lenalidomide, bortezomid, amrubicine, carfilzomib, pralatrexate, and enzastaurin.

In some embodiments, the additional therapeutic agent can comprise one or more therapeutic agents selected from the group consisting of an adjuvant, a TLR agonist, tumor necrosis factor (TNF) alpha, IL-1, HMGB1, an IL-10 antagonist, an IL-4 antagonist, an IL-13 antagonist, an IL-17 antagonist, an HVEM antagonist, an ICOS agonist, a treatment targeting CX3CL1, a treatment targeting CXCL9, a treatment targeting CXCL10, a treatment targeting CCL5, an LFA-1 agonist, an ICAM1 agonist, and a Selectin agonist.

In some embodiments, carboplatin, nab-paclitaxel, paclitaxel, cisplatin, pemetrexed, gemcitabine, FOLFOX, or FOLFIRI are administered to the subject.

In some embodiments, the additional therapeutic agent is an anti-PD-1 antibody, an anti-PD-L1 antibody, anti-PD-L2 antibody, an anti-LAG-3 antibody, an anti-TIGIT antibody, an anti-BTLA antibody, an anti-CTLA4 antibody, an anti-CD40 antibody, an anti-OX40 antibody, an anti-4-1BB antibody, an anti-TIM3 antibody, or an anti-GITR antibody.

### Pharmaceutical Compositions and Routes of Administration

Also provided herein are pharmaceutical compositions that contain at least one (e.g., one, two, three, or four) of the multispecific (e.g., bispecific) antibodies or antigen-binding fragments, or antibody-drug conjugates (e.g., anti-TAA/CLEC5A antibodies such as anti-HER2/CLEC5A antibodies, anti-HER2/CLEC5A antigen-binding fragments, or anti-HER2/CLEC5A antibody-drug conjugates) described herein. The pharmaceutical compositions may be formulated in any manner known in the art.

Pharmaceutical compositions are formulated to be compatible with their intended route of administration (e.g., intravenous, intraarterial, intramuscular, intradermal, subcutaneous, or intraperitoneal). The compositions can include a sterile diluent (e.g., sterile water or saline), a fixed oil, polyethylene glycol, glycerine, propylene glycol or other synthetic solvents, antibacterial or antifungal agents, such as benzyl alcohol or methyl parabens, chlorobutanol, phenol, ascorbic acid, thimerosal, and the like, antioxidants, such as ascorbic acid or sodium bisulfite, chelating agents, such as ethylenediaminetetraacetic acid, buffers, such as acetates, citrates, or phosphates, and isotonic agents, such as sugars (e.g., dextrose), polyalcohols (e.g., mannitol or sorbitol), or salts (e.g., sodium chloride), or any combination thereof. Liposomal suspensions can also be used as pharmaceutically acceptable carriers (see, e.g., U.S. Patent No. 4,522,811). Preparations of the compositions can be formulated and enclosed in ampules, disposable syringes, or multiple dose vials. Where required (as in, for example, injectable formulations), proper fluidity can be maintained by, for example, the use of a coating, such as lecithin, or a surfactant.

Compositions containing one or more of any of the antibodies or antigen-binding fragments, or antibody-drug conjugates (e.g., anti-TAA/CLEC5A antibodies such as anti-HER2/CLEC5A antibodies, anti-HER2/CLEC5A antigen-binding fragments, or anti-HER2/CLEC5A antibody-drug conjugates) described herein can be formulated for parenteral (e.g., intravenous, intraarterial, intramuscular, intradermal, subcutaneous, or intraperitoneal) administration in dosage unit form (i.e., physically discrete units containing a predetermined quantity of active compound for ease of administration and uniformity of dosage).

Toxicity and therapeutic efficacy of compositions can be determined by standard pharmaceutical procedures in cell cultures or experimental animals (e.g., monkeys). One can determine the LD50 (the dose lethal to 50% of the population) and the ED50 (the dose therapeutically effective in 50% of the population): the therapeutic index being the ratio of LD50:ED50. Agents that exhibit high therapeutic indices are preferred. Where an agent exhibits an undesirable side effect, care should be taken to minimize potential damage (i.e., reduce unwanted side effects). Toxicity and therapeutic efficacy can be determined by other standard pharmaceutical procedures.

Data obtained from cell culture assays and animal studies can be used in formulating an appropriate dosage of any given agent for use in a subject (e.g., a human). A therapeutically effective amount of the antibodies or antigen-binding fragments, or antibody-drug conjugates (e.g., anti-TAA/CLEC5A antibodies such as anti-HER2/CLEC5A antibodies, anti-HER2/CLEC5A antigen-binding fragments, or anti-HER2/CLEC5A antibody-drug conjugates) will be an amount that treats the disease (e.g., kills cancer cells ) in a subject (e.g., a human subject identified as having cancer), or a subject identified as being at risk of developing the disease (e.g., a subject who has previously developed cancer but now has been cured), decreases the severity, frequency, and/or duration of one or more symptoms of a disease in a subject (e.g., a human). The effectiveness and dosing of any of the antibodies or antigen-binding fragments, or antibody-drug conjugates (e.g., anti-TAA/CLEC5A antibodies such as anti-HER2/CLEC5A antibodies, anti-HER2/CLEC5A antigen-binding fragments, or anti-HER2/CLEC5A antibody-drug conjugates) described herein can be determined by a health care professional or veterinary professional using methods known in the art, as well as by the observation of one or more symptoms of disease in a subject (e.g., a human). Certain factors may influence the dosage and timing required to effectively treat a subject (e.g., the severity of the disease or disorder, previous treatments, the general health and/or age of the subject, and the presence of other diseases).

Exemplary doses include milligram or microgram amounts of any of the antibodies or antigen-binding fragments, or antibody-drug conjugates (e.g., anti-TAA/CLEC5A antibodies such as anti-HER2/CLEC5A antibodies, anti-HER2/CLEC5A antigen-binding fragments, or anti-HER2/CLEC5A antibody-drug conjugates) described herein per kilogram of the subject's weight (e.g., about 1 µg/kg to about 500 mg/kg; about 100 µg/kg to about 500 mg/kg; about 100 µg/kg to about 50 mg/kg; about 10 µg/kg to about 5 mg/kg; about 10 µg/kg to about 0.5 mg/kg; or about 0.1 mg/kg to about 0.5 mg/kg). While these doses cover a broad range, one of ordinary skill in the art will understand that therapeutic agents vary in their potency, and effective amounts can be determined by methods known in the art. Typically, relatively low doses are administered at first, and the attending health care professional or veterinary professional (in the case of therapeutic application) or a researcher (when still working at the development stage) can subsequently and gradually increase the dose until an appropriate response is obtained. In addition, it is understood that the specific dose level for any particular subject will depend upon a variety of factors including the activity of the specific compound employed, the age, body weight, general health, gender, and diet of the subject, the time of administration, the route of administration, the rate of excretion, and the half-life of the therapeutic agent in vivo.

The pharmaceutical compositions can be included in a container, pack, or dispenser together with instructions for administration. The disclosure also provides methods of manufacturing the antibodies or antigen-binding fragments, or antibody-drug conjugates (e.g., anti-TAA/CLEC5A antibodies such as anti-HER2/CLEC5A antibodies, anti-HER2/CLEC5A antigen-binding fragments, or anti-HER2/CLEC5A antibody-drug conjugates) for various uses as described herein.

### EXAMPLES

The invention is further described in the following examples, which do not limit the scope of the invention described in the claims.

### Example 1: CLEC5A expression in immune cell subsets in human PBMCs

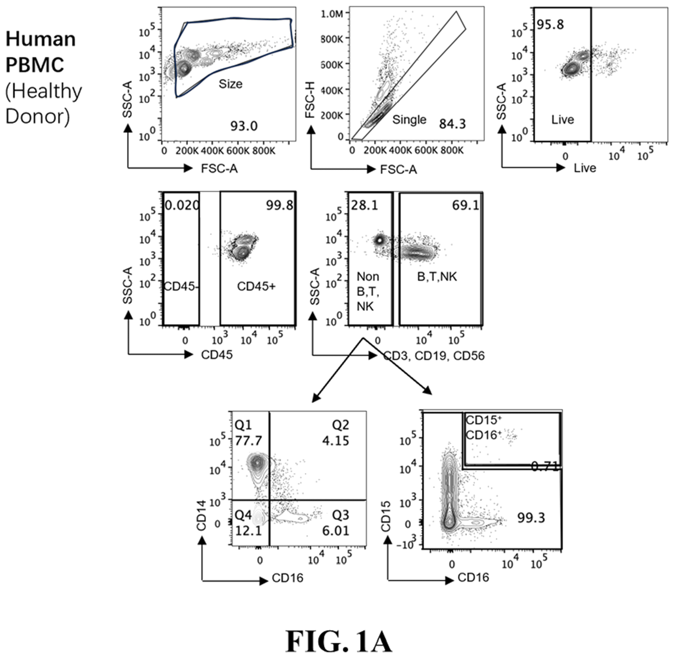

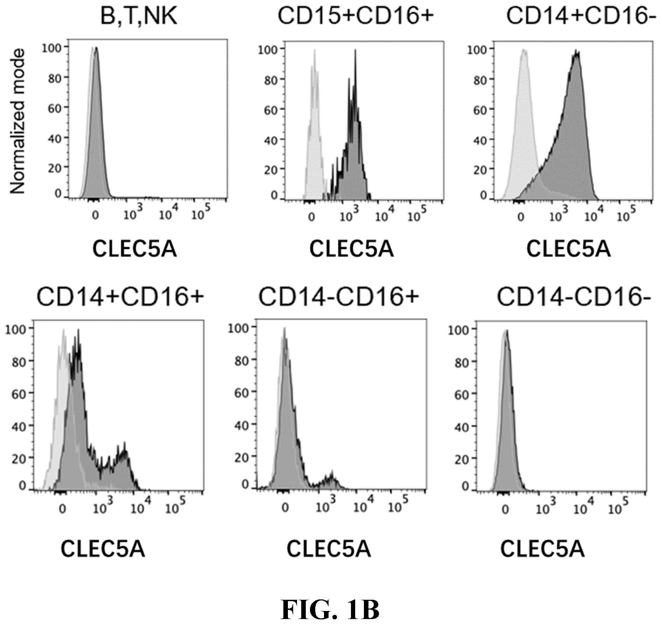

A gating strategy was used to evaluate immune cell subsets in human PBMCs (**FIG. 1A**). Frozen PBMCs from healthy donors (Stanford Blood Center) were thawed, stained with a fixable viability dye (Zombie Aqua^{™}, BioLegend), and then labeled with a panel of antibodies specific to immune cell subsets: CD45 (H130, BioLegend), CD3 (UCHT1, BioLegend), CD19 (HIB19, BioLegend), CD56 (5.1H11, BioLegend), CD14 (M5E2, BD Biosciences), CD16 (3G8, BioLegend), CD15 (HI98, BD Biosciences), and CLEC5A (283834, R&D Systems). Following staining, cells were fixed in 4% paraformaldehyde for 15 minutes at room temperature, washed, resuspended in FACS buffer (2% heat-inactivated FBS (fetal bovine serum) in PBS (phosphate-buffered saline) with 0.05% BSA (bovine serum albumin)), and then evaluated by flow cytometry (Attune^{™} CytPik^{™}, Invitrogen). As shown in **FIG. 1B**, CLEC5A was found to be highly expressed in CD15+CD16+ neutrophils and CD14+CD16- classical monocytes. Some CLEC5A was also expressed in intermediate CD14+CD16+ and CD14-CD16+ non-classical monocytes.

### Example 2: CLEC5A expression in tumor-associated myeloid cells in human solid tumors

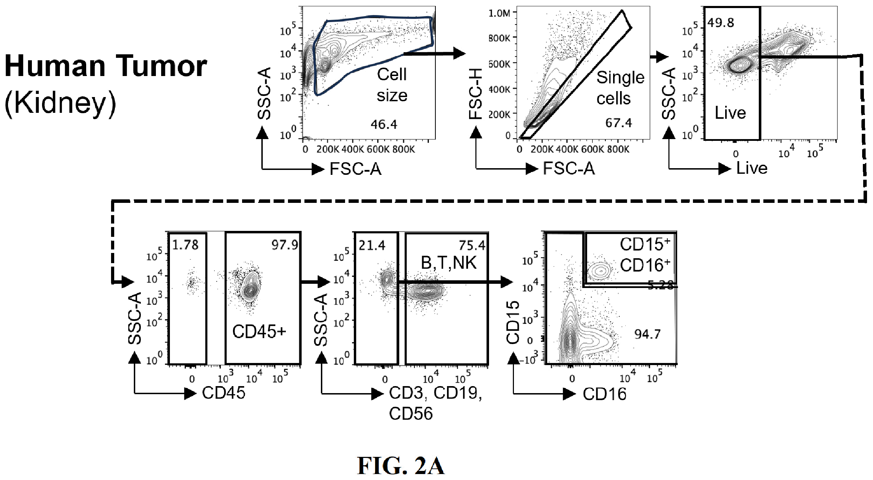

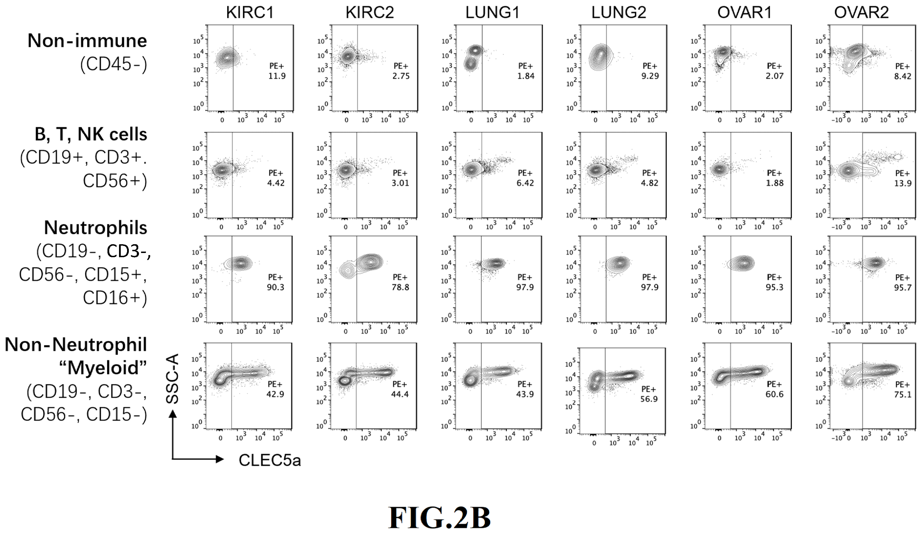

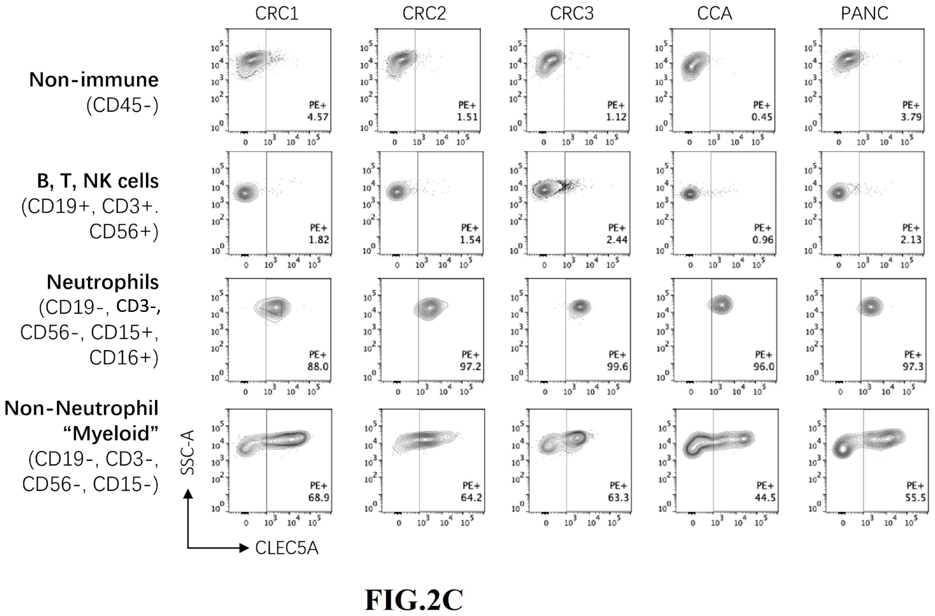

CLEC5A expression was evaluated in tumor-associated myeloid cells in human solid tumors. Frozen human dissociated tumor cells (Discovery Life Sciences) from 6 solid tumor indications (Kidney, Lung Ovarian, Colorectal, Bile Duct, and Pancreatic cancers) were thawed, stained with a fixable viability dye (Zombie Aqua^{™}, BioLegend), and then labeled with a panel of antibodies specific to immune cell subsets: CD45 (H130, BioLegend), CD3 (UCHT1, BioLegend), CD19 (HIB19, BioLegend), CD56 (5.1H11, BioLegend), CD14 (M5E2, BD Biosciences), CD16 (3G8, BioLegend), CD15 (HI98, BD Biosciences), CD11b (IRFCF44, Invitrogen), TREM2 (237920, R&D Systems), CD206 (15-2, BioLegend), and CLEC5A (283834, R&D Systems). Following staining, cells were fixed in 4% paraformaldehyde for 15 minutes at room temperature, washed, resuspended in FACS buffer (2% heat-inactivated FBS in PBS with 0.05% BSA), and then evaluated by flow cytometry (Attune^{™} CytPik^{™}, Invitrogen). A gating strategy was utilized to evaluate immune cell subsets in human solid tumors (**FIG. 2A**). As shown in **FIGs. 2B-2C**, the majority of neutrophils (80-98%, denoted by live/CD45+/CD19-/CD3-/CD56-/CD15+/CD16+) and non-neutrophil myeloid cells (>43%, denoted by live/CD45+/CD19-/CD3-/CD56-/CD15-) were positive for CLEC5A in human tumors. Highest expression of CLEC5A was observed in non-neutrophil myeloid cells. Little to no expression of CLEC5A expression was observed in non-immune cells (live/CD45-) and some expression was observed in a subset of cells (<14%) in a cell mixture of the B, T, and NK cells (live/CD45+/CD19+/CD3+/CD56+).

### Example 3: CLEC5A expression in tumor-associated macrophages (TAMs) in human solid tumors

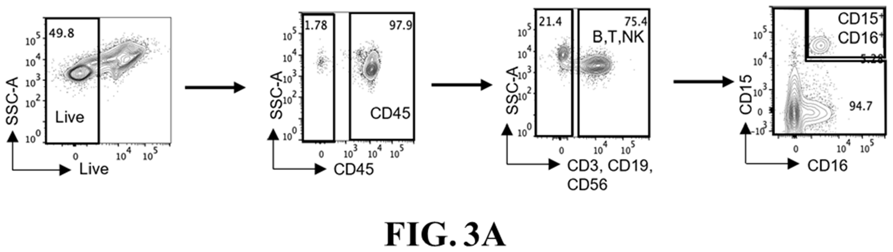

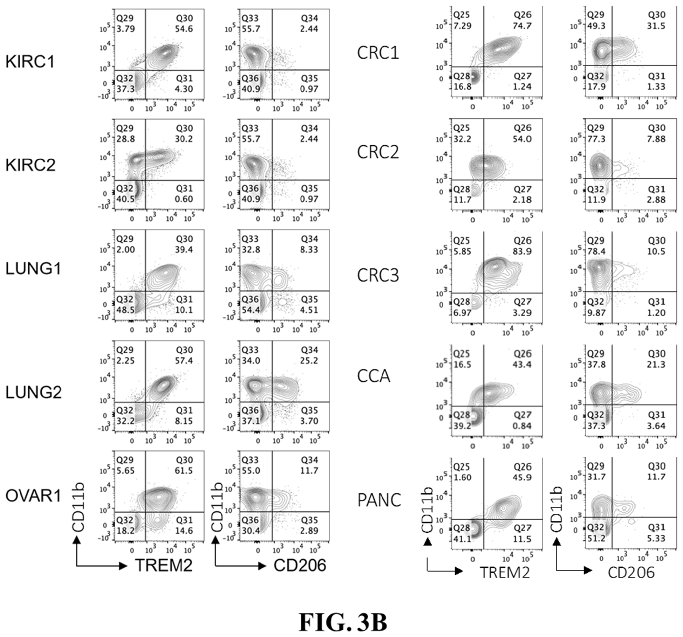

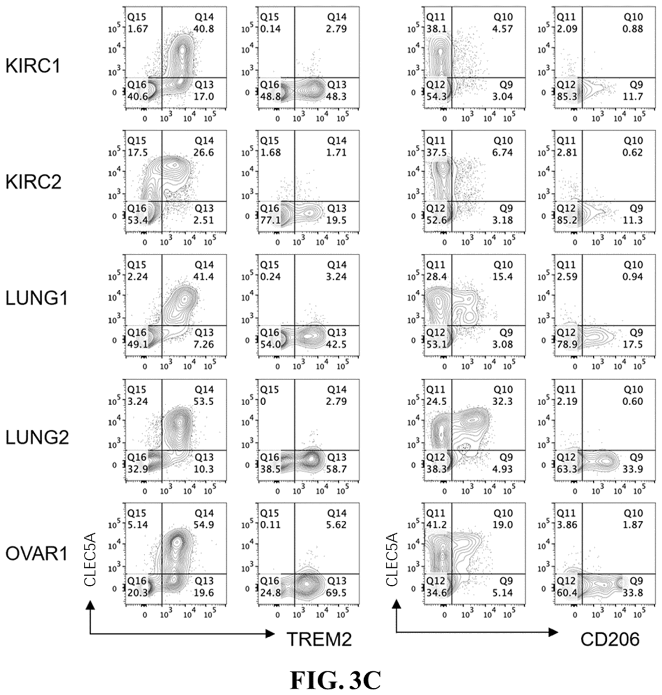

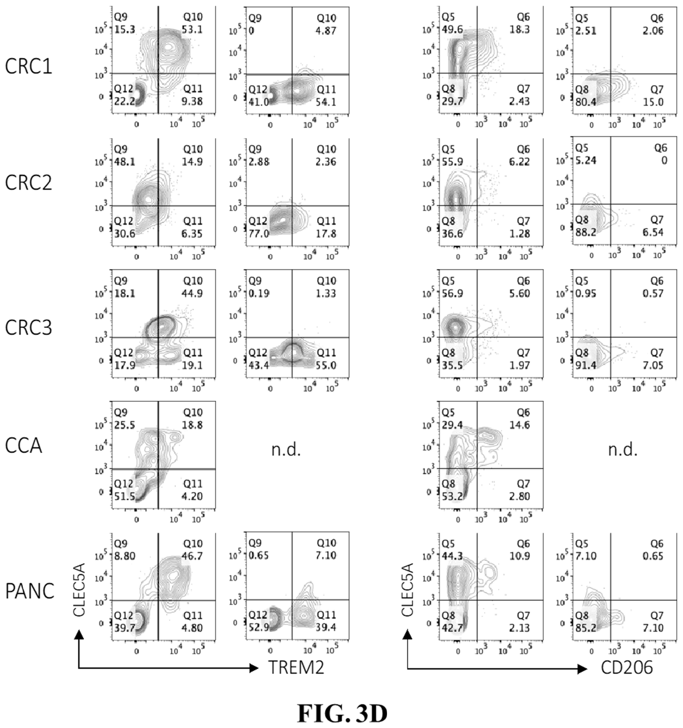

CLEC5A expression was evaluated in tumor-associated macrophages (TAMs) in human solid tumors. Specifically, frozen human dissociated tumor cells (Discovery Life Sciences) from 6 solid tumor indications (Kidney, Lung, Ovarian, Colorectal, Bile Duct, and Pancreatic cancers) were thawed, stained with a fixable viability dye (Zombie Aqua^{™}, BioLegend), and then labeled with a panel of antibodies specific to immune cell subsets: CD45 (H130, BioLegend), CD3 (UCHT1, BioLegend), CD19 (HIB19, BioLegend), CD56 (5.1H11, BioLegend), CD14 (M5E2, BD Biosciences), CD16 (3G8, BioLegend), CD15 (HI98, BD Biosciences), CD11b (IRFCF44, Invitrogen), TREM2 (237920, R&D Systems), CD206 (15-2, BioLegend), and CLEC5A (283834, R&D Systems). Following staining, cells were fixed in 4% paraformaldehyde for 15 minutes at room temperature, washed, resuspended in FACS buffer (2% heat-inactivated FBS in PBS with 0.05% BSA), and then evaluated by flow cytometry (Attune^{™} CytPik^{™}, Invitrogen). A gating strategy was utilized to characterize non-neutrophil myeloid cells (live/CD45+/CD19-/CD3-/CD56-/CD15-) in human dissociated tumor cells (**FIG. 3A**). As shown in **FIG. 3B**, the majority (~30-60%) of non-neutrophil myeloid cells were found to be TAMs due to their dual expression of CD11b and TREM2. Only a portion of the TAMs expressed CD206 (>45% of CD11b+ cells). As shown in **FIGs. 3C-3D**, CLEC5A was highly expressed in the majority (>70%) of TREM2+ TAMs and CD206+ TAMs.

### Example 4. Generation of CLEC5A antibodies

### Immunization

To generate monoclonal antibodies against CLEC5A, two New Zealand white rabbits were immunized with Human CLEC5A His Tag protein (Acro Biosystems, Cat#: CLA-H5243) following a 63-day rabbit immunization protocol. Specifically, 400 µg of antigen emulsified with CFA (complete Freun''s adjuvant) was injected on Day 1 subcutaneously during the first injection, followed by 3 additional subcutaneous injections (200 µg of antigen emulsified with IFA (incomplete Freun''s adjuvant)) on Day 7, Day 21, Day 42, and a final intravenous boost on Day 63.

### Antibody titer

5 ml of serum was collected from each rabbit on Day 28 and Day 49 for titer test. Antibody titer following immunization was determined by ELISA binding of serially diluted sera to human CLEC5A. Briefly, 96-well plates were coated with human CLEC5A (1 µg/ml) overnight at 4°C. The plates were blocked by PBS (pH 7.4, Fisher Scientific, Cat#: 21-040-CM) containing 1% BSA for 1 hour at room temperature. Samples (starting at 1:1000 dilution of sera and titrated 3-fold) were added to the plates and incubated at room temperature for 1 hour, followed by PBS washes. HRP Donkey anti-rabbit IgG secondary antibody (BioLegend, Cat#: 406401) was then added to the plates and incubated for 1 hour at room temperature, followed by PBS washes. The substrate was added to the plates and incubated for 5 minutes. OD450 was measured using a plate reader (CLARIOstar^{®}).

### Rabbit splenocyte isolation and B cell sorting

Rabbit spleens were harvested 5 days after the final booster shot. Splenocytes were prepared in a sterile cell strainer placed in the bottom of a 100 mm sterile petri dish containing 20 mL RPMI + 1% of penicillin/streptomycin (P/S). Using sterile forceps, spleen tissues were transferred to a cell strainer. Specifically, while a spleen was held with forceps, the spleen was cut into small pieces, which were then pressed through the mesh of the cell strainer. Tissue debris was washed with 10 mL RPMI + 1% P/S. Spleen cells were transferred from the petri dish to a new 50 mL conical tube. RPMI + 1% P/S was added to a final volume of 50 ml. Cells were centrifuged at 400 × g for 5 minutes and supernatant was aspirated. 13 mL ACK buffer (Gibco Cat#: A1049201) was added to resuspend the cells. The suspended cells were incubated at room temperature for 1 minute. RPMI + 1% P/S was added to reach a final volume of 50 ml. Cells were centrifuged at 400 × g for 5 minutes and supernatant was aspirated. The cell pellet was resuspended in RPMI + 10% FBS + 1 % P/S. The cells were centrifuged at 400 × g for 5 minutes and supernatant was aspirated. The pellet was resuspended in 15 ml RPMI + 10% FBS + 1 % P/S. The cells were pipetted through a 100 µm cell strainer into a 50 mL conical tube to remove cell clumps. Splenocytes were then seeded at the desired density (e.g., 4 × 10⁷ cells /ml) with a proper medium for sorting. The remaining splenocytes were frozen in 90% serum + 10% DMSO at 6 × 10⁷ cells/vial (~ 1.8 ml) at -80°C overnight. The frozen cells were transferred into a liquid nitrogen tank for long-term storage.

For B cell sorting, freshly isolated or thawed splenocytes (~ 2 × 10⁸ splenocytes) were cultured in B cell culture media (RPMI-1640, 15% FBS, 1 × HEPES, 1 × 2-ME (2-mercaptoethanol), 1% penicillin/streptomycin) overnight before sorting. 96-well B cell feeding plates were prepared accordingly one day before the sorting. On the day of sorting, suspended and loosely attached splenocytes were collected by gently pipetting the medium against the culturing surface of flask. The cells were then transferred to a conical tube and centrifuged at 400 × g for 3 minutes. The cell pellet was washed with fluorescence-activated cell sorting (FACS) buffer (1 × PBS + 0.5% BSA) twice. A biotinylated antigen was added at 5 µg/ml (final concentration). The mixture was incubated at room temperature (RT) for 20 minutes. The staining mixture was then centrifuged at 400 × g for 3 minutes and the cells were resuspended in FACS buffer. The cells were transferred into a 1.5 ml amber Eppendorf^{™} tube. The staining antibody mixtures were then added to the cells. The staining mixture was incubated at 4°C for 15-30 minutes, and then centrifuged at 400 ×g for 3 minutes. The cell pellet was washed twice with FACS buffer. The washed cell pellet was resuspended at ~10⁷ cells/ml in 1 × PBS + 1% FBS. Using FACS, antigen-specific single B cells were sorted into 96-well plates (20 plates for each rabbit). 96-well B cell culture plates with sorted B cells were cultured in 37°C with 5% CO₂ for 12 days.

### ELISA screening of single B cell culture, LEM supernatants and purified antibody

On Day 8 post sorting, 15 µL of B cell culture supernatant was collected from each well for antigen-specific ELISA. Briefly, the B cell culture supernatants were transferred to human CLEC5A-coated ELISA plates (384-well plates coated with CLEC5A and blocked with BSA). The cells were incubated at room temperature for 1 hour and washed 3 times with PBS plus 0.05% Tween^{®} 20. Antibodies were detected using goat anti-rabbit IgG HRP + TMB substrate (VWR, Cat#: 5120-007). B cell supernatants that met the cut off OD450 (>0.5 or 3× higher than the pre-immune serum) were then selected.

B cells sorted by an initial ELISA screening of 1920 supernatants from 40 plates (96-well plates) were screened for human CLEC5A-specific antibodies. Approximately more than 200 human CLEC5A antigen-binding (ELISA OD cutoff = 0.9) B cell clones were identified. On Day 12 post sorting, the B cell culture plates were centrifuged at 400 × g for 3 minutes. Supernatants from positive clones (with OD greater than the selected cutoff of 0.9 from antigen-specific ELISA) were collected and the cell pellets were preserved in 100 µL DNA/RNA shield (Zymo, Cat#: R1100-250) in 250 µL PCR tubes. Collected supernatants were subjected to additional tests to confirm the ELISA results and cell surface binding.

The heavy and light chain genes from the positive clones were cloned into a linear expression module (LEM). The LEM was then transfected into production cells and the culture supernatants were tested by ELISA as described above.

### Screening of CLEC5A-specific antibodies on cell surface

CLEC5A is mainly expressed on myeloid cells (monocytes, macrophages, neutrophiles, and dendritic cells). Peripheral monocytes were isolated from purified PBMCs with EasySep^{™} Human Monocyte Enrichment Kit without CD16 Depletion (StemCell Technologies, Cat#: 19058). Monocyte isolation was performed according to the manufacturer's instructions. About 50,000 monocytes were incubated with no dilution of B cell LEM supernatants or serially diluted purified antibodies in FACS buffer (PBS + 0.5% BSA) for 60 minutes on ice, respectively. After incubation, cells were washed twice and detected with FITC-labeled Donkey anti-rabbit IgG (BioLegend, Cat#: 406403) secondary antibody. For analysis, the mean fluorescence intensity (MFI of FITC) of each antibody was determined and plotted using GraphPad Prism software (Version 9.4.1; GraphPad Software Inc.). Top 20 clones that showed binding to human CLEC5A on cell surface were selected and cloned into a LEM. After testing, 8 clones (6A5, 6G9, 14A2, 5C7, 7G10, 3A7, 13E6 and 9E11) that showed binding to human CLEC5A on cell surface were selected. Finally, recombinant anti-CLEC5A antibodies were expressed for further functional assays.

### Example 5. Chimeric anti-CLEC5A antibodies and molecule constructions

To express chimeric rabbit/human IgG1 antibodies, constant regions of the heavy chain (hIgG1-Hc-LALAPG variant) of human IgG1 and the constant region of human light chain Kappa (CL-kappa) were synthesized and cloned into pcDNA3.4, respectively (by GeneScript). pcDNA3.4 containing hIgG1-Hc-LALAPG of human IgG1 (pcDNA3.4-huIgG1-Hc-LALAPG) was further digested with EcoRI/NheI for the cloning of VH sequences. EcoRI/BsiWI were used to digest VL sequences and the receiving vector - pcDNA3.4 expressing human CL-kappa (pcDNA3.4-huKappa-Lc). VH and VL sequences of 8 selected rabbit anti-CLEC5A antibodies, 6A5, 6G9, 14A2, 5C7, 7G10, 3A7, 13E6 and 9E11, and those of the reference antibody DX244 (VH: SEQ ID NO: 93; VL: SEQ ID NO: 94), were synthesized (by IDT) with designed sequences that overlapped at both 5' and 3' ends, which can anneal and assemble (NEB NEBuilder^{®} HiFi DNA Assembly) with the corresponding ends of receiving vectors. Competent E. coli (NEB^{®} 5-alpha) cells were transformed with the assembled plasmid encoding the clone with correct sequences, based on sequencing results (Elim Biopharm). The transformed cells were further cultured with LB containing carbenicillin (100 µg/ml) for plasmid purification (QIAGEN^{®} Plasmid Plus Kits). Plasmids were eluted in nuclease-free water (Sigma) and stored at -80°C.

### Example 6. Transfection and purification of chimeric anti-CLEC5A antibodies

All antibody light chain- and heavy chain-coding sequences were generated by direct DNA synthesis and cloned into a mammalian expression vector. The cloned sequences were verified by Sanger sequencing. Chinese hamster ovary (CHO) cells were grown in the CHOgro^{™} medium (Mirus) supplemented with 4 mM L-Glutamine and 0.33% Poloxamer-188, which was diluted to 4 × 10⁶ cells/ml with fresh cell culture media at the time of transfection. Cells were transfected by lipofection with 1 µg of plasmid DNA per 4 × 10⁶ cells. Transfected cells were kept in an incubator with shaking at 125 rpm in 5% CO₂ and 70% humidity environment at 32°C.

Protein expression titers were measured every 5 days. Cell supernatant was collected by centrifugation after 10-14 days post-transfection and filtered through a 0.22 µm PES (polyethersulfone) membrane filter. Expressed antibodies were purified by Protein A chromatography (TOYOPEARL^{®} AF-rProtein A HC-650M resin, Tosoh) and aggregates were removed by anion exchange resins (TOYOPEARL NH2-750F, Tosoh).

Purified antibodies were analyzed by HPLC (Dionex UltiMate^{®} 3000-RS UHPLC, ThermoFisher) controlled by Chromeleon^{®} software. A size exclusion column TSKgel UP-SW3000, 2 µm, 4.6 mm ID×15 cm (Tosoh) was used to resolve antibodies purity and aggregation. All purified antibodies had 97% or greater monomer purity, which were sterilized through a 0.22 µm PES membrane filter, and used for binding and functional assays.

### Example 7. Binding of chimeric anti-CLEC5A antibodies to human macrophages

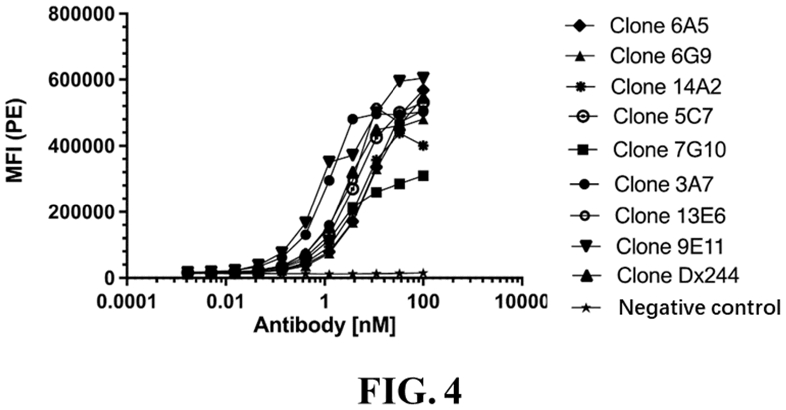

Binding abilities of anti-CLEC5A antibodies (Fc-silenced antibodies) to human macrophages were evaluated. CD14+ monocytes (purified from human PBMC donor #651 using the EasySep^{™} Human Monocyte Enrichment Kit without CD16 Depletion, StemCell Technologies, Cat#: 19058) were differentiated to M0 macrophages with 50 ng/mL of macrophage colony-stimulating factor (M-CSF, StemCell Technologies, Cat#: 78057) for 7 days. About 50,000 M0 macrophages were incubated with serially diluted antibodies in FACS buffer (PBS + 0.5% BSA) for 30 minutes on ice. After incubation, cells were washed twice and detected with anti-human IgG PE secondary antibody (Jackson ImmunoResearch, Cat#: 109-116-170). For analysis, the mean fluorescence intensity (MFI of PE) of each antibody was determined and plotted using GraphPad Prism software (Version 9.4.1; GraphPad Software Inc.). As shown in **FIG. 4** and the table below, all 8 anti-CLEC5A antibodies can bind to M0 macrophages (CLEC5A+) cells with various affinities.

**Table 1. Anti-CLEC5A antibodies binding to human macrophages**

| Anti-CLEC5AAb | binding EC50 (nM) |
|---|---|
| 6A5 | 9.80 |
| 6G9 | 6.84 |
| 14A2 | 3.99 |
| 5C7 | 3.93 |
| 7G10 | 2.06 |
| 3A7 | 0.96 |
| 13E6 | 2.57 |
| 9E11 | 1.59 |
| DX244 | 2.80 |
| Negative control | - |

### Example 8. TNFα release of chimeric anti-CLEC5A antibodies

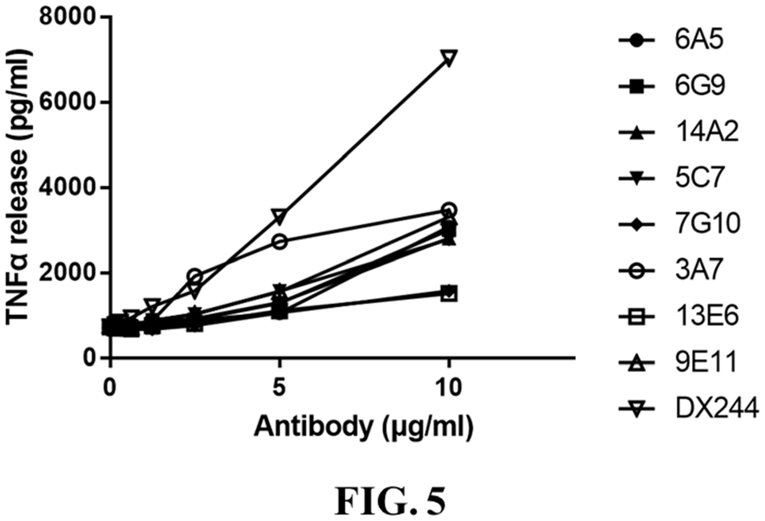

The TNFα release of anti-CLEC5A antibodies was evaluated. CD14+ monocytes (purified from human PBMC donor #651 using the EasySep^{™} Human Monocyte Enrichment Kit without CD16 Depletion, StemCell Technologies, Cat#: 19058) were differentiated with 50 ng/mL of macrophage colony-stimulating factor (M-CSF, StemCell Technologies, Cat#: 78057) for 6 days followed by polarizing it to M1 with 50 ng/mL M-CSF and 50 ng/mL IFN-γ (StemCell Technologies, Cat#: 78020) for 24 hours. Anti-CLEC5A antibodies were serially diluted and coated on a 96-well plate for 24 hours at 4°C. After incubation, the plate was washed with PBS. About 100,000 M1 macrophages were added to the anti-CLEC5A antibody-coated wells in complete RPMI media (with 10% heat-inactivated FBS and 5% penicillin/streptomycin). After 24 hours, supernatants were collected and TNFα was measured using a human TNFα ELISA kit(R&D systems, Cat#: DY210-05) according to manufacturer's instructions. For analysis, the TNFα levels (in pg/mL) were determined and plotted using GraphPad Prism software (Version 9.4.1; GraphPad Software Inc.). As shown in **FIG 5****,** all 8 anti-CLEC5A antibodies showed a moderate level of TNFα release, which was lower than that of benchmark DX244, suggesting that the anti-CLEC5A antibodies may have a safe profile.

### Example 9. Binding of chimeric anti-CLEC5A antibodies to mouse and human CLEC5A

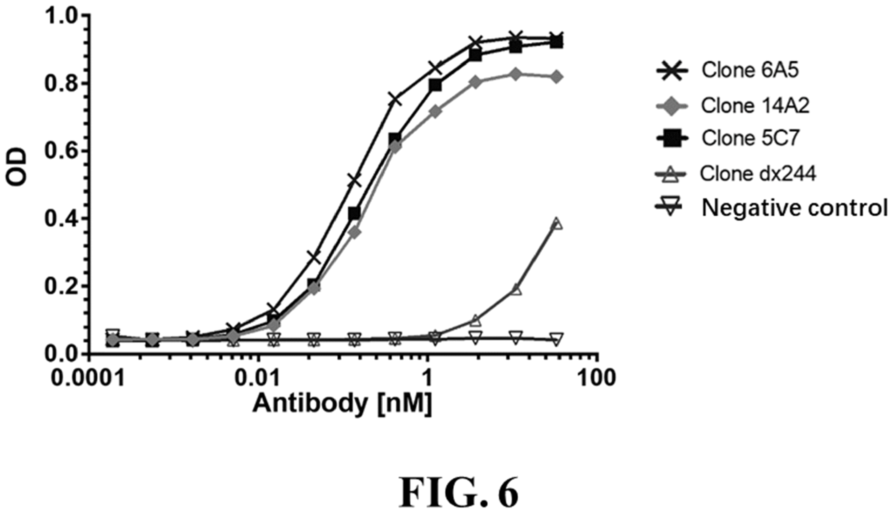

ELISA binding was used to evaluate if CLEC5A Fc-silenced antibodies can cross-react with recombinant mouse CLEC5A protein. Corning high binding flat bottom 96-well plates (Cat#: 3361) were coated with 1 µg/mL of mouse CLEC5A (R&D Systems, Cat#: 8438-CL-050) for 48 hours at 4°C. Protein antigen was aspirated, and wells were washed 3 times with 400 µL of wash buffer (0.05% Tween^{®} 20 in PBS) using a plate washer. CLEC5A Fc-silenced antibodies were prepared as the primary antibody at 5 µg/mL and serially diluted 3-fold through 11 points (diluted concentrations) with reagent buffer (PBS + 0.05% BSA). 100 µL of primary antibody was added to appropriate wells and incubated for 1 hour at room temperature. Primary antibody was aspirated, and wells were washed 3 times with 400 µL of wash buffer (0.05% Tween^{®} 20 in PBS) using a plate washer. Secondary antibody BioLegend donkey anti-human HRP (Cat#: 410902) was diluted 1:10,000 in reagent buffer (PBS + 0.5% BSA), and 100 µL was added per well, followed by an incubation for 30 minutes at room temperature. Secondary antibody was aspirated, and wells were washed 3 times with 400 µL of wash buffer (0.05% Tween^{®} 20 in PBS) using a plate washer. 100 µL of Thermo 1-step TMB Turbo (Cat#: 34022) was added to each well and incubated for 10 minutes at room temperature in the dark. 100 µL of Fisher 1N Sulfuric Acid (Cat#: SA212-2) stop solution was added to each well and the plate was read in a plate reader at 450 nm. For analysis, OD450 of each antibody was plotted using GraphPad Prism software (Version 9.4.1; GraphPad Software Inc.). As shown in **FIG. 6** and the table below, anti-CLEC5A antibodies 6A5, 14A2 and 5C7 exhibited cross-reactivities to mouse CLEC5A.

**Table 2. Anti-CLEC5A antibodies binding to mouse CLEC5A**

| Anti-CLEC5A Ab | binding EC50 (nM) |
|---|---|
| 6A5 | 0.1205 |
| 14A2 | 0.1844 |
| 5C7 | 0.1936 |
| DX244 | 51.18 |
| Negative control | - |

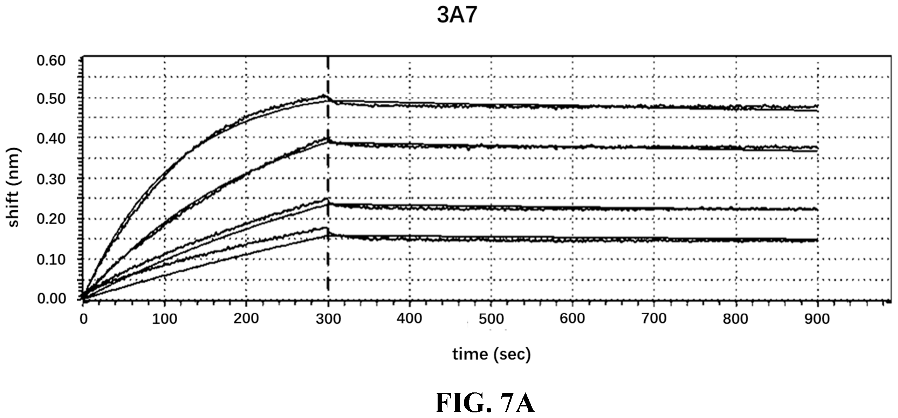

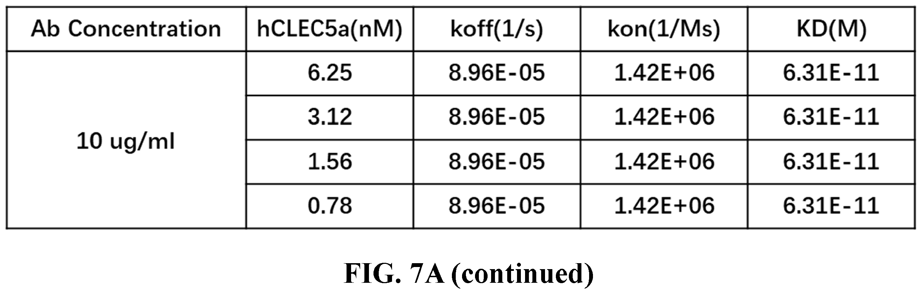

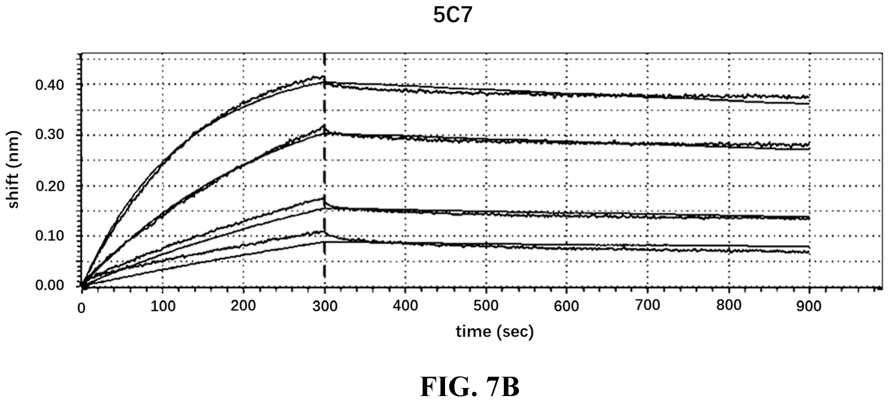

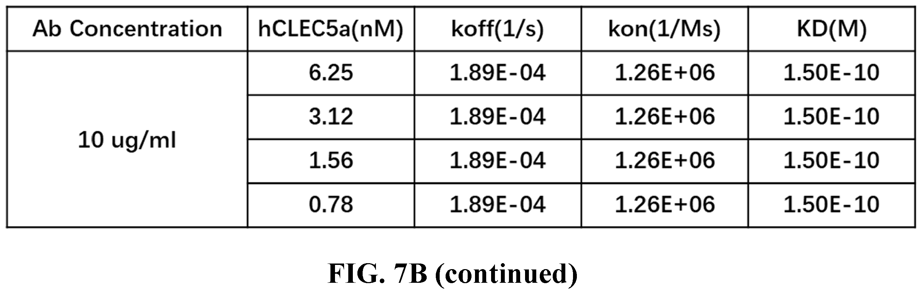

For BLI (Bio-Layer Interferometry) binding, 10 µg/ml of anti-CLEC5A antibodies were captured on an anti-human IgG Fc probe (Gator Bio, Cat#: 160024), followed by measurement of association and dissociation with recombinant human CLEC5A, His Tag (ACROBiosystems, Cat#: NC1-H52H4) at a single concentration of 11 nM in PBS + 0.05% Tween^{®} 20. Absolute KD was determined by applying a 1:1 binding model. Results of BLI binding assays are showed in **FIGs. 7A-7B**. The results indicate that anti-CLEC5A antibodies 3A7 and 5C7 can bind to human CLEC5A with high affinities.

### Example 10. Humanization of chimeric anti-CLEC5A antibodies and ELISA binding

Four rabbit anti-human CLEC5A monoclonal antibody clones, 3A7, 5C7, 6A5, 7G10 and 13E6 were humanized by grafting the CDRs of the leading antibodies into selected human germline frameworks closest to the rabbit frameworks identified by IgBLAST (www.ncbi.nlm.nih.gov/igblast/) and/or IMGT/DomainGapAlig (www.imgt.org/3Dstructure-DB/cgi/DomainGapAlign.cgi). Human germline IGHV, IGHD, IGKV, IGHJ and IGKJ were selected based on sequence similarity within both frameworks and CDRs. Certain human germline framework residues were back mutated to corresponding rabbit residues to maintain the canonical loop structure and light chain/heavy chain interface (Padlan E.A., Mol. Immunol., 1994, 31:169; Foote J. and Winter G., JMB, 1992, 224:487). By humanization, h3A7, h5C7, h6A5, h7G10 and h13E6 humanized antibodies were generated.

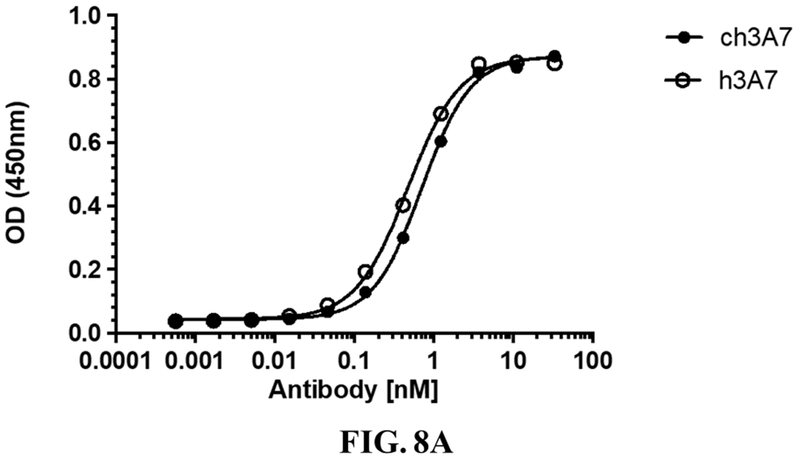

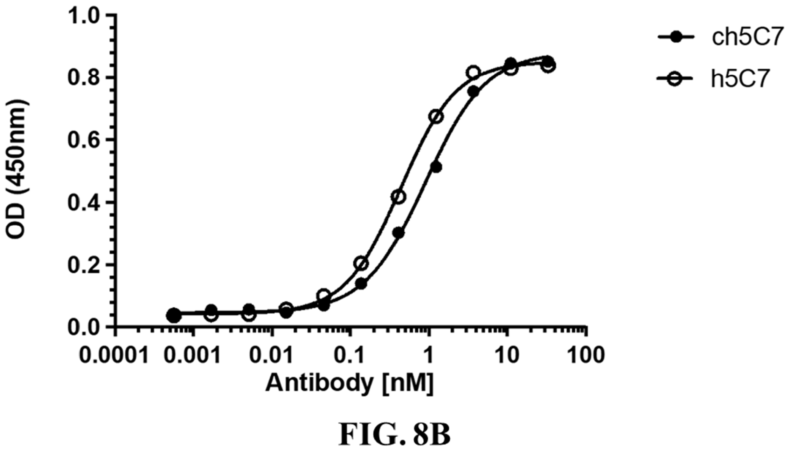

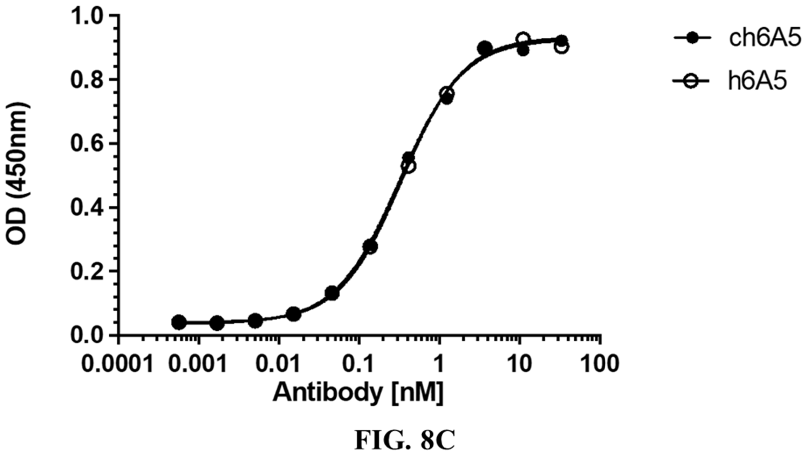

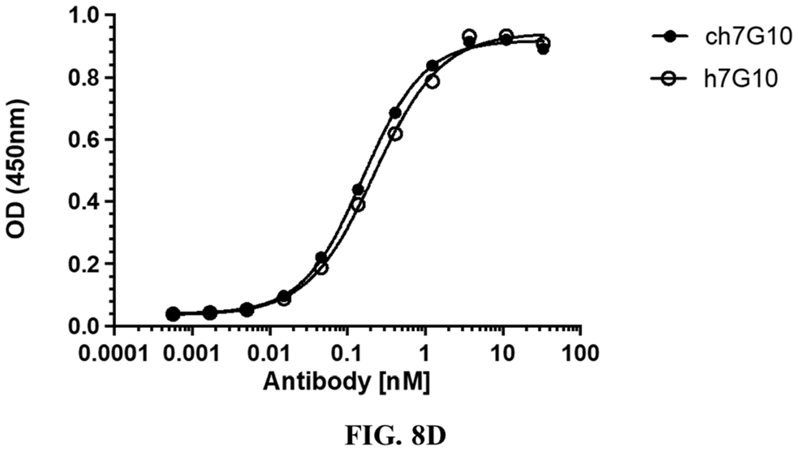

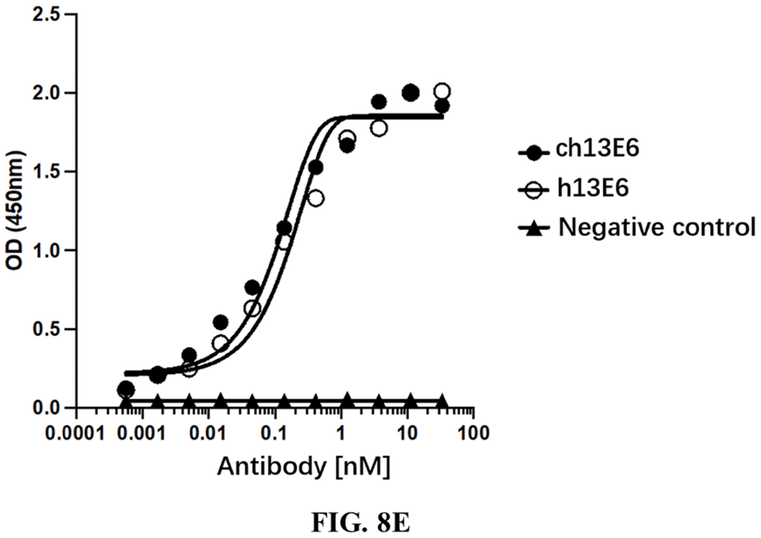

ELISA binding was performed to evaluate if humanized anti-CLEC5A antibodies can bind to recombinant human CLEC5A protein as compared to the corresponding chimeric parental clones. Corning high binding flat bottom 96-well plates (Cat#: 3361) were coated with 1 µg/mL of human CLEC5A (ACROBiosystems, Cat#: CLA-H5243) for 48 hours at 4°C. Protein antigen was aspirated, and wells were washed 3 times with 400 µL of wash buffer (0.05% Tween^{®} 20 in PBS) using a plate washer. Anti-CLEC5A antibodies were prepared as the primary antibody at 5 µg/mL and serially diluted 3-fold through 11 points (diluted concentrations) with reagent buffer (PBS + 0.05% BSA). 100 µL of primary antibody was added to appropriate wells and incubated for 1 hour at room temperature. Primary antibody was aspirated, and wells were washed 3 times with 400 µL of wash buffer (0.05% Tween^{®} 20 in PBS) using a plate washer. Secondary antibody BioLegend donkey anti-human HRP (Cat#: 410902) was diluted 1:10,000 in reagent buffer (PBS + 0.5% BSA), 100 µL was added per well and incubated for 30 minutes at room temperature. Secondary antibody was aspirated, and wells were washed 3 times with 400 µL of wash buffer (0.05% Tween^{®} 20 in PBS) using a plate washer. 100 µL of Thermo 1-step TMB Turbo (Cat#: 34022) was added to each well and incubated for 10 minutes at room temperature in the dark. 100 µL of Fisher 1N Sulfuric Acid (Cat#: SA212-2) stop solution was added to each well and the plate was read in a plate reader at 450 nm. For analysis, OD450 of each antibody was plotted using GraphPad Prism software (Version 9.4.1; GraphPad Software Inc.). As shown in **FIGs. 8A-8E** and the table below, all humanized formats of anti-CLEC5A antibodies showed similar affinities to the human CLEC5A protein as compared to the corresponding chimeric parental clones, suggesting that humanization was successful for the above clones.

**Table 3. Humanized anti-CLEC5A antibodies binding to human CLEC5A by ELISA**

| Antibody clone | Chimeric EC50 (nM) | Humanized EC50 (nM) |
|---|---|---|
| 3A7 | 0.71 | 0.47 |
| 5C7 | 0.90 | 0.44 |
| 6A5 | 0.31 | 0.33 |
| 7G10 | 0.16 | 0.22 |
| 13E6 | 0.61 | 0.12 |

### Example 11. Molecule constructions of TAA/CLEC5A bispecific antibodies

Six different formats of TAA/CLEC5A bispecific antibodies were generated as shown in **FIGs. 9A-9F**. Competent E. coli (NEB^{®} 5-alpha) cells were transformed with the assembled plasmid encoding the clone with correct sequences, based on sequencing results (Elim Biopharm). The transformed cells were further cultured with LB containing carbenicillin (100 µg/ml) for plasmid purification (QIAGEN^{®} Plasmid Plus Kits). Plasmids were eluted in nuclease-free water (Sigma) and stored at -80°C.

Antibodies were expressed with CHO cells (ExpiCHO^{™} Expression System, Gibco) by transfection of both pcDNA3.4-huIgG1-Hc and pcDNA3.4-huKappa-Lc containing paired VH and VL sequence. ExpiCHO^{™} cells were cultured with the ExpiCHO^{™} expression medium and maintained between 0.3 to 6 × 10⁶/ml according to manufacturer's recommendation at 37°C, 125 rpm, 5% CO₂ and 80% humidity. In a 125 ml baffled flask, 25 ml of fresh ExpiCHO^{™} cells (6 × 10⁶/ml and viability > 95%) were prepared from 1 day-long culture seeded at 3 × 10⁶/ml. 1 ml serum-free medium (OptiPRO^{™} SFM, Gibco) containing both pcDNA3.4-huIgG1-Hc and pcDNA3.4-huKappa-Lc (12 µg of each plasmid) was mixed well by pipetting with 1 ml OptiPRO^{™} SFM containing 80 µl transfection reagent (ExpiFectamine^{™} CHO Reagent, Gibco). The transfection mixture was then added to the 25 ml ExpiCHO^{™} cells and cultured at 37°C. On the next day, the transfection culture was transferred to a 32°C incubator after adding 150 µl ExpiFectamine^{™} CHO Enhancer, 6 ml ExpiCHO^{™} Feed and 1× penicillin/streptomycin (Gibco). The cell density and viability of the transfection culture was monitored and the IgG1 antibody titer in the medium was determined using BLI technology with Protein A biosensor (Gator Bio). After about 5 days, the culture medium containing secreted IgG1 antibodies were collected (by centrifugation at 2000 × g, 10 minutes), filtered (Thermo Scientific^{™} Nalgene^{™} Rapid-Flow^{™} Sterile Disposable Filter) and further purified using Protein A resin (TOYOPEARL AF-rProtein A Hc-650F)-packed gravity-flow column (Bio-Rad). IgG1 antibodies were eluted with 3.5 ml Glycine-HCl (100 mM, pH 2.7), immediately neutralized with 1 M Tris-HCl (pH 8.5), dialyzed with Thermo Scientific^{™} Slide-A-Lyzer^{™} G2 Dialysis Cassettes (20K MWCO) in 1× PBS buffer (pH 7.2) and stored at 4°C. The concentration of the purified IgG1 antibody was determined with a NanoDrop^{™} One/OneC Microvolume UV-Vis Spectrophotometer (Thermo Scientific^{™}) and the quality of IgG1 antibody was checked by SDS-PAGE gel under both denaturing and non-denaturing conditions.

### Example 12. Transfection and purification of TAA/CLEC5A bispecific antibodies

All antibody light chain- and heavy chain-coding sequences were generated by direct DNA synthesis and cloned into mammalian expression vectors. The cloned sequences were verified by Sanger sequencing.

Chinese hamster ovary (CHO) cells were grown in CHOgro^{™} medium (Mirus) supplemented with 4 mM L-Glutamine and 0.33% Poloxamer-188, which was diluted to 4 × 10⁶ cells/ml with fresh cell culture media at the time of transfection. Cells were transfected by lipofection with 1 µg of plasmid DNA per 4 × 10⁶ cells. Transfected cells were kept in an incubator with shaking at 125 rpm in 5% CO₂ and 70% humidity environment at 32°C.

Protein expression titers were measured every 5 days. Cell supernatant was collected by centrifugation after 10-14 days post-transfection and filtered through a 0.22 µm PES membrane filter. Expressed antibodies were purified by Protein A chromatography (TOYOPEARL^{®} AF-rProtein A HC-650M resin, Tosoh) and aggregates were removed by anion exchange resins (TOYOPEARL NH2-750F, Tosoh).

Purified antibodies were analyzed by HPLC (Dionex UltiMate^{®} 3000-RS UHPLC, Thermo Fisher) controlled by Chromeleon^{®} software. A size exclusion column TSKgel UP-SW3000, 2 µm, 4.6 mm ID×15 cm (Tosoh) was used to resolve antibodies purity and aggregation.

All purified antibodies had 97% or greater monomer purity, which were sterilized through a 0.22 µm PES membrane filter, and used for binding and functional assays.

### Example 13. Binding of HER2/CLEC5A bispecific antibody to human macrophages

Binding abilities of HER2/CLEC5A bispecific antibodies to human macrophages were evaluated.

CD14+ monocytes (purified from human PBMC donor #441 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 7 days. About 50,000 M0 macrophages were incubated with serially diluted antibodies in FACS buffer (PBS + 0.5% BSA) for 30 minutes on ice. After incubation, cells were washed twice and detected with anti-human IgG PE secondary antibody (Jackson ImmunoResearch, Cat#: 109-116-170). For analysis, the mean fluorescence intensity (MFI of PE) of each antibody was determined and plotted using GraphPad Prism software (Version 9.4.1; GraphPad Software Inc.).

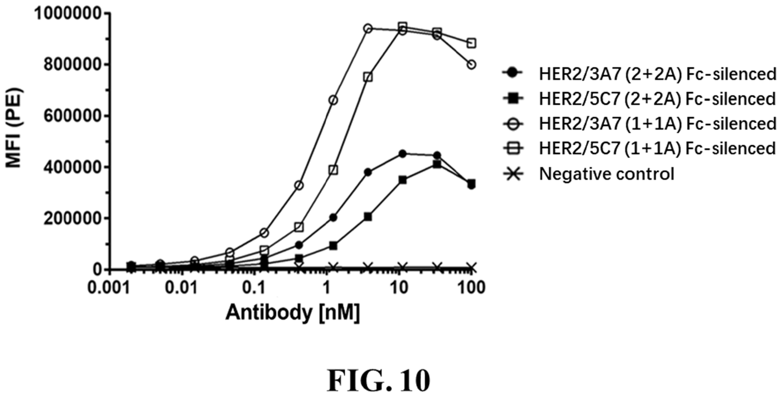

As shown **FIG. 10** and the table below, both 2+2 A format and 1+1 A format of HER2/CLEC5A antibodies bound to M0 macrophages (CLEC5A+) cells with differing affinities. However, binding affinities of 2+2 format HER2/CLEC5A antibodies were lower compared to 1+1 format antibodies. Sequences of the rabbit-human chimeric antibodies 3A7 and 5C7 were used.

**Table 4. HER2/CLEC5A bispecific antibody binding to human macrophages**

| Antibody | EC50 (nM) |
|---|---|
| HER2/3A7 (2+2 A) Fc-silenced | 1.183 |
| HER2/5C7 (2+2 A) Fc-silenced | 3.115 |
| HER2/3A7 (1+1 A) Fc-silenced | 0.6112 |
| HER2/5C7 (1+1 A) Fc-silenced | 1.494 |
| Negative control | N/A |

### Example 14. Binding of TAA/CLEC5A bispecific antibodies to human macrophages

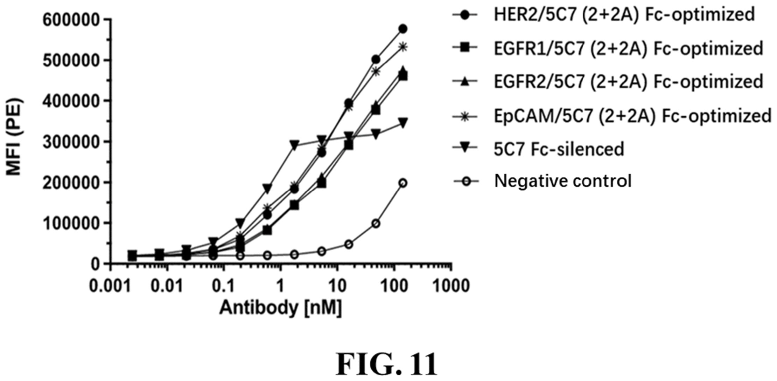

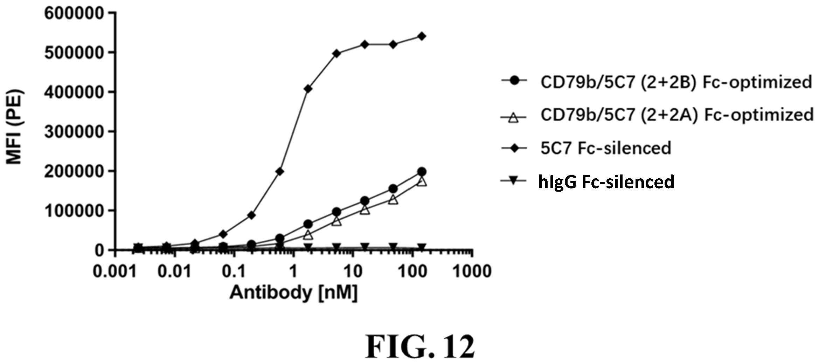

Binding abilities of different TAA/CLEC5A bispecific antibodies to human macrophages were evaluated. CD14+ monocytes (purified from human PBMC donor #900 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 7 days. About 50,000 M0 macrophages were incubated with serially diluted antibodies in FACS buffer (PBS + 0.5% BSA) for 30 minutes on ice. After incubation, cells were washed twice and detected with anti-human IgG PE secondary antibody (Jackson ImmunoResearch, Cat#: 109-116-170). For analysis, the mean fluorescence intensity (MFI of PE) of each antibody was determined and plotted using GraphPad Prism software (Version 9.4.1; GraphPad Software Inc.). As shown **FIGs. 11-12** and the table below, different TAA/CLEC5A bispecific antibodies can bind M0 macrophages (CLEC5A+) cells.

**Table 5. TAA/CLEC5A bispecific antibodies binding to human macrophages**

| Antibody | EC50 (nM) |
|---|---|
| HER2/5C7 (2+2 A) Fc-optimized | 10.76 |
| EGFR1/5C7 (2+2 A) Fc-optimized | 18.98 |
| EGFR2/5C7 (2+2 A) Fc-optimized | 19.49 |
| EpCAM/5C7 (2+2 A) Fc-optimized | 7.35 |
| 5C7 Fc-silenced | 0.49 |
| Negative control | N/A |

### Example 15. Binding of TAA/CLEC5A bispecific antibodies to human CLEC5A

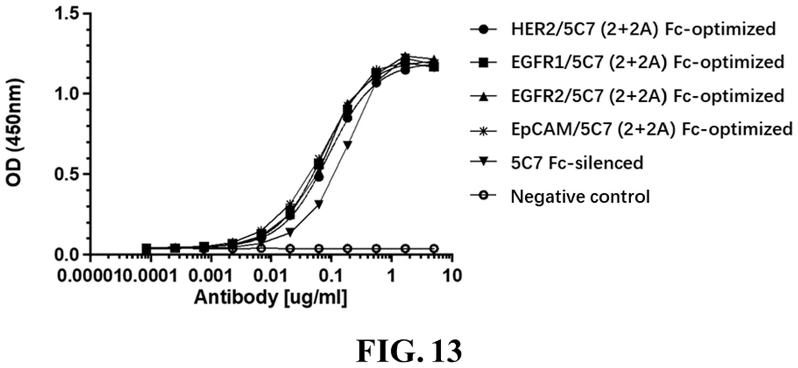

ELISA binding was performed to assess affinities of different TAA/CLEC5A bispecific antibodies for recombinant human CLEC5A protein. Corning high binding flat bottom 96-well plates (Cat#: 3361) were coated with 1 µg/mL of human CLEC5A (ACROBiosystems, Cat#: CLA-H5243) for 48 hours at 4°C. Protein antigen was aspirated, and wells were washed 3 times with 400 µL of wash buffer (0.05% Tween^{®} 20 in PBS) using a plate washer. TAA/CLEC5A bispecific antibodies were prepared as the primary antibody at 5 µg/mL and serially diluted 3-fold through 11 points (diluted concentrations) with reagent buffer (PBS + 0.05% BSA). 100 µL of primary antibody was added to appropriate wells and incubated for 1 hour at room temperature. Primary antibody was aspirated, and wells were washed 3 times with 400 µL of wash buffer (0.05% Tween^{®} 20 in PBS) using a plate washer. Secondary antibody BioLegend donkey anti-human HRP (Cat#: 410902) was diluted 1:10,000 in reagent buffer (PBS + 0.5% BSA), and 100 µL was added per well, followed by an incubation for 30 minutes at room temperature. Secondary antibody was aspirated, and wells were washed 3 times with 400 µL of wash buffer (0.05% Tween^{®} 20 in PBS) using a plate washer. 100 µL of Thermo 1-step TMB Turbo (Cat#: 34022) was added to each well and incubated for 10 minutes at room temperature in the dark. 100 µL of Fisher 1N Sulfuric Acid (Cat#: SA212-2) stop solution was added to each well and the plate was read in a plate reader at 450 nm. For analysis, OD450 of each antibody was plotted using GraphPad Prism software (Version 9.4.1; GraphPad Software Inc.). As shown **FIG. 13** and the table below, all TAA/CLEC5A bispecific antibodies had similar affinities for human CLEC5A.

**Table 6. TAA/CLEC5A bispecific antibodies binding to human CLEC5A**

| Antibody | EC50 (µg/mL) |
|---|---|
| HER2/5C7 (2+2 A) Fc-optimized | 0.089 |
| EGFR1/5C7 (2+2 A) Fc-optimized | 0.072 |
| EGFR2/5C7 (2+2 A) Fc-optimized | 0.081 |
| EpCAM/5C7 (2+2 A) Fc-optimized | 0.064 |
| 5C7 Fc-silenced | 0.158 |
| Negative control | N/A |

### Example 16. Binding of HER2/CLEC5A bispecific antibodies to human HER2

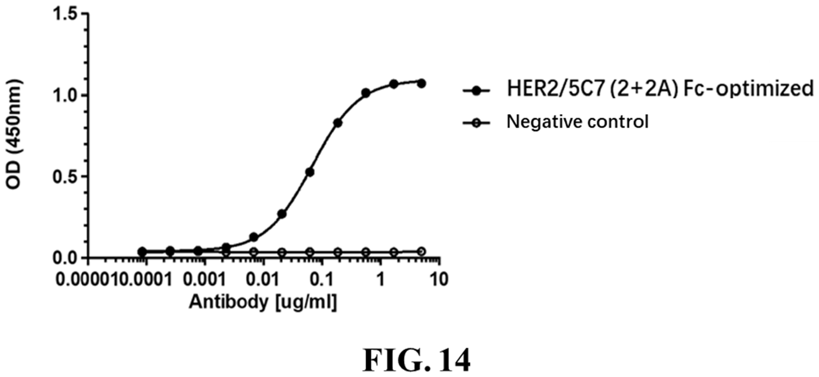

ELISA binding was performed to determine affinity of HER2/CLEC5A bispecific antibodies for recombinant human HER2 protein. Corning high binding flat bottom 96-well plates (Cat#: 3361) were coated with 1 µg/mL of human HER2 (ACROBiosystems, Cat#: HE2-H52R8) for 48 hours at 4°C. Protein antigen was aspirated, and wells were washed 3 times with 400 µL of wash buffer (0.05% Tween^{®} 20 in PBS) using a plate washer. HER2/CLEC5A (2+2 A) Fc-optimized antibody was prepared as the primary antibody at 5 µg/mL and serially diluted 3-fold through 11 points (diluted concentrations) with reagent buffer (PBS + 0.05% BSA). 100 µL of primary antibody was added to appropriate wells and incubated for 1 hour at room temperature. Primary antibody was aspirated, and wells were washed 3 times with 400 µL of wash buffer (0.05% Tween^{®} 20 in PBS) using a plate washer. Secondary antibody BioLegend donkey anti-human HRP (Cat#: 410902) was diluted 1:10,000 in reagent buffer (PBS + 0.5% BSA), and 100 µL was added per well, followed by an incubation for 30 minutes at room temperature. Secondary antibody was aspirated, and wells were washed 3 times with 400 µL of wash buffer (0.05% Tween^{®} 20 in PBS) using a plate washer. 100 µL of Thermo 1-step TMB Turbo (Cat#: 34022) was added to each well and incubated for 10 minutes at room temperature in the dark. 100 µL of Fisher 1N Sulfuric Acid (Cat#: SA212-2) stop solution was added to each well and the plate was read in a plate reader at 450 nm. For analysis, OD450 of each antibody was plotted using GraphPad Prism software (Version 9.4.1; GraphPad Software Inc.). As shown in **FIG. 14** and the table below, HER2/CLEC5A bispecific antibody bound human HER2 with a high affinity.

**Table 7: HER2/CLEC5A bispecific antibodies binding to human HER2**

| Antibody | EC50 (µg/mL) |
|---|---|
| HER2/5C7 (2+2 A) Fc-optimized | 0.068 |
| Negative control | N/A |

### Example 17. Binding of EGFR/CLEC5A bispecific antibodies to human EGFR

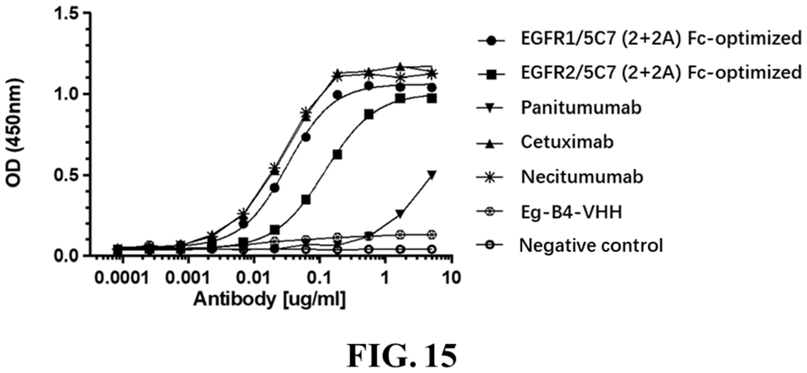

ELISA binding was performed to determine affinities of different anti-EGFR hIgG1 (Cetuximab, Panitumumab, Necitumumab, Eg-B4-VHH) and EGFR/CLEC5A (2+2 A) Fc-optimized antibodies with EGFR portion of Amivantamab (EGFR1) and Nimotuzumab (EGFR2) for recombinant human EGFR protein. Specifically, Corning high binding flat bottom 96-well plates (Cat#: 3361) were coated with 1 µg/mL of human EGFR (ACROBiosystems, Cat#: EGR-H5222) for 48 hours at 4°C. Protein antigen was aspirated, and wells were washed 3 times with 400 µL of wash buffer (0.05% Tween^{®} 20 in PBS) using a plate washer. EGFR hIgG1 and EGFR/CLEC5A (2+2A) Fc-optimized antibodies were prepared as the primary antibody at 5 µg/mL and serially diluted 3-fold through 11 points (diluted concentrations) with reagent buffer (PBS + 0.05% BSA). 100 µL of primary antibody was added to appropriate wells and incubated for 1 hour at room temperature. Primary antibody was aspirated, and wells were washed 3 times with 400 µL of wash buffer (0.05% Tween^{®} 20 in PBS) using a plate washer. Secondary antibody BioLegend donkey anti-human HRP (Cat#: 410902) was diluted 1:10,000 in reagent buffer (PBS + 0.5% BSA), and 100 µL was added per well, followed by an incubation for 30 minutes at room temperature. Secondary antibody was aspirated, and wells were washed 3 times with 400 µL of wash buffer (0.05% Tween^{®} 20 in PBS) using a plate washer. 100 µL of Thermo 1-step TMB Turbo (Cat#: 34022) was added to each well and incubated for 10 minutes at room temperature in the dark. 100 µL of Fisher 1N Sulfuric Acid (Cat#: SA212-2) stop solution was added to each well and the plate was read in a plate reader at 450 nm. For analysis, OD450 of each antibody was plotted using GraphPad Prism software (Version 9.4.1; GraphPad Software Inc.). As shown in **FIG. 15** and the table below, EGFR/CLEC5A (2+2A) bispecific antibodies showed lower binding affinities to human EGFR compared to Cetuximab and Necitumumab.

**Table 8: EGFR/CLEC5A bispecific antibodies binding to human EGFR**

| Antibody | EC50 (µg/mL) |
|---|---|
| EGFR1/5C7 (2+2 A) Fc-optimized | 0.031 |
| EGFR2/5C7 (2+2 A) Fc-optimized | 0.120 |
| Cetuximab | 0.025 |
| Necitumumab | 0.022 |
| Panitumumab | 6.64 |
| Eg-B4-VHH | 0.03 |
| Negative control | N/A |

### Example 18. Binding of EpCAM/CLEC5A bispecific antibodies to DLD-1

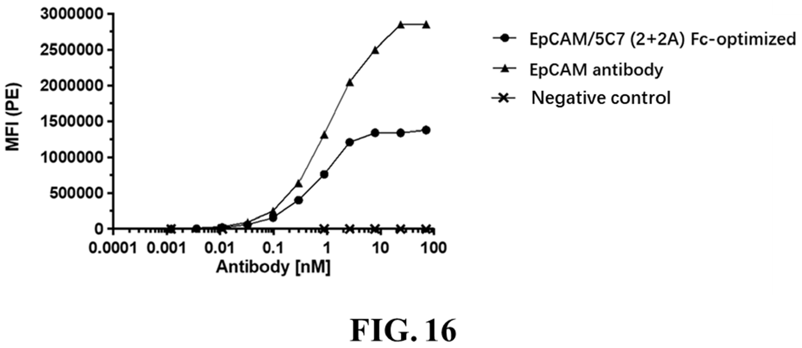

Binding abilities of an anti-EpCAM antibody (Solitomab EpCAM binding part IgG form) and EpCAM/CLEC5A (2+2 A) Fc-optimized antibodies to DLD-1 cells (EPCAM+) were evaluated. About 50,000 DLD-1 cells (ATCC, CCL-221) were incubated with serially diluted antibodies in FACS buffer (PBS + 0.5% BSA) for 30 minutes on ice. After incubation, cells were washed twice and detected with anti-human IgG PE secondary antibody (Jackson ImmunoResearch, Cat#: 109-116-170). For analysis, the mean fluorescence intensity (MFI of PE) of each antibody was determined and plotted using GraphPad Prism software (Version 9.4.1; GraphPad Software Inc.). As shown in **FIG. 16** and the table below, the anti-EpCAM antibody showed a higher binding affinity compared to EpCAM/CLEC5A bispecific antibody to DLD-1 cells.

**Table 9: EpCAM/CLEC5A bispecific antibodies binding to DLD-1**

| Antibody | EC50 (nM) |
|---|---|
| EpCAM/5C7 (2+2 A) Fc-optimized | 1.1 |
| Anti-EpCAM antibody | 0.67 |
| Negative control | N/A |

### Example 19. Binding of CD79b/CLEC5A bispecific antibodies to Ramos cells

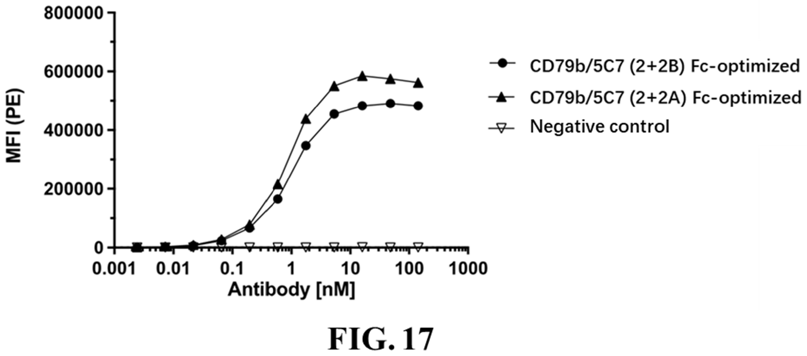

Binding abilities of CD79b/CLEC5A bispecific antibodies to Ramos cells (CD79b+) were evaluated. About 50,000 Ramos cells (ATCC, CRL-1596) were incubated with serially diluted antibodies in FACS buffer (PBS + 0.5% BSA) for 30 minutes on ice. After incubation, cells were washed twice and detected with anti-human IgG PE secondary antibody (Jackson ImmunoResearch, Cat#: 109-116-170). For analysis, the mean fluorescence intensity (MFI of PE) of each antibody was determined and plotted using GraphPad Prism software (Version 9.4.1; GraphPad Software Inc.). As shown in **FIG. 17**, the results suggest that both 2+2 A and 2+2 B formats of CD79b/CLEC5A bispecific antibodies had similar affinity for Ramos cells.

### Example 20. HER2/CLEC5A bispecific antibodies mediated SK-BR-3 cell killing by M0 macrophages

The ability of HER2/CLEC5A bispecific antibodies to kill target cancer SK-BR-3 cells (HER2+) by effector macrophages (CLEC5A+) M0 cells through both phagocytosis and trogocytosis mechanism was evaluated. Specifically, CD14+ monocytes (purified from human PBMC donor #031 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 7 days. SK-BR-3 cells were labelled with CFSE (ThermoFisher, Cat#: C34554). About 100,000 macrophages were incubated with 20,000 CFSE+ SK-BR-3 (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. SK-BR-3 cells were gated as CFSE+ by FACS, and absolute cell count of CFSE+ cells were obtained by collecting a fixed volume for all treatment conditions. Percentage of target cell killing was calculated as: (Absolute number of CFSE+ SK-BR-3 cell number in non-treatment group - Absolute number of CFSE+ SYTOX- SK-BR-3 cell number in treatment group)/Absolute number of CFSE+ SK-BR-3 cell number in non-treatment group × 100.

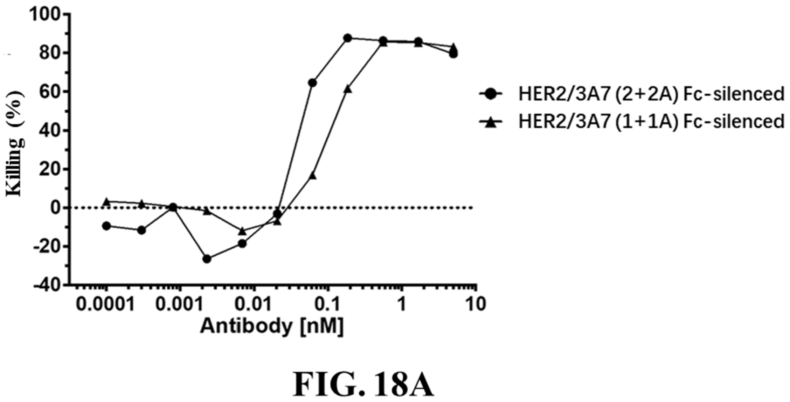

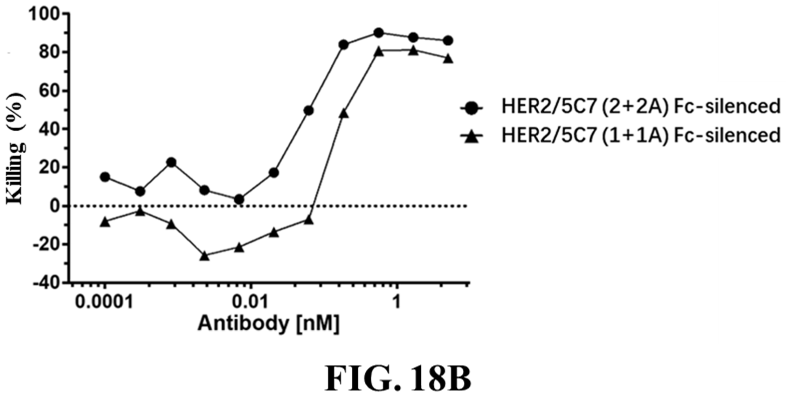

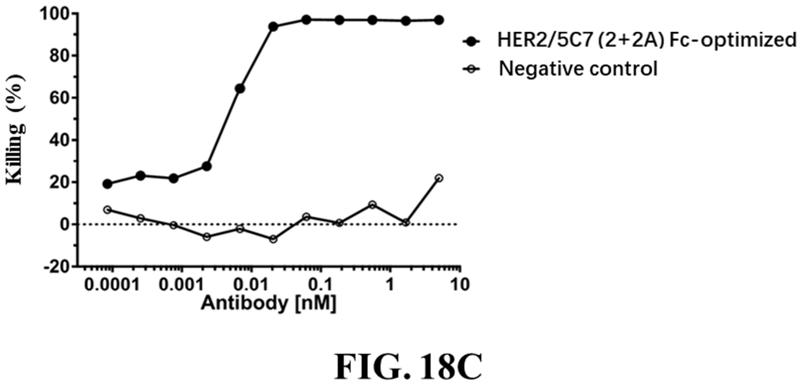

As shown in **FIGs. 18A-18C** and the table below, HER2/CLEC5A bispecific antibodies efficiently killed HER2+ cancer cell SK-BR-3, indicating that tumor cells expressing target antigen HER2 can be killed with HER2/CLEC5A bispecific antibodies by recruiting macrophages.

**Table 10. HER2/CLEC5A bispecific antibodies mediated SK-BR-3 cell killing by M0 macrophages**

| Antibody | IC50 (nM) |
|---|---|
| HER2/3A7 (2+2 A) Fc-silenced | 0.0408 |
| HER2/5C7 (2+2 A) Fc-silenced | 0.0614 |
| HER2/3A7 (1+1 A) Fc-silenced | 0.114 |
| HER2/5C7 (1+1 A) Fc-silenced | 0.148 |
| HER2/5C7 (2+2 A) Fc-optimized | 0.006 |

### Example 21. HER2/CLEC5A bispecific antibodies mediated SK-BR-3 cell killing by M1 and M2 macrophages

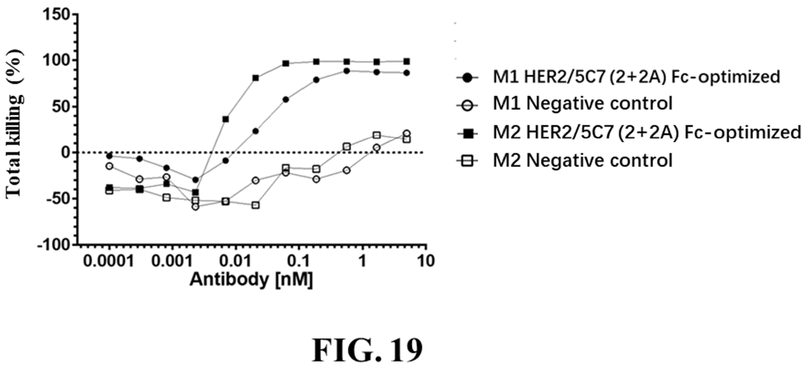

The ability of HER2/CLEC5A bispecific antibodies to kill target cancer SK-BR-3 cells (HER2+) by effector macrophages (CLEC5A+) cells polarized to either M1 or M2 states through both phagocytosis and trogocytosis mechanisms was evaluated. CD14+ monocytes (purified from human PBMC donor #900 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 6 days. On Day 6, M0 macrophages were polarized with 50 ng/mL of IFN-γ (StemCell Technologies, Cat#: 78020) to M1 or 25 ng/mL of IL-10 (StemCell Technologies, Cat#: 78024), to M2 for another 24 hours (Day 7). SK-BR-3 cells were labelled with CFSE (ThermoFisher, Cat#: C34554). About 100,000 macrophages were incubated with 20,000 CFSE+ SK-BR-3 (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. SK-BR-3 cells were gated as CFSE+ by FACS, and absolute cell count of CFSE+ cells were obtained by collecting a fixed volume for all treatment conditions. Percentage of target cell killing was calculated as: (Absolute number of CFSE+ SK-BR-3 cell number in non-treatment group - Absolute number of CFSE+ SYTOX- SK-BR-3 cell number in treatment group)/Absolute number of CFSE+ SK-BR-3 cell number in non-treatment group × 100. As shown in **FIG. 19** and the table below, surprisingly, HER2/CLEC5A bispecific antibodies can efficiently kill HER2+ cancer cells SK-BR-3 by recruiting both M1 and M2 macrophages.

**Table 11: HER2/CLEC5A bispecific antibodies mediated SK-BR-3 cell killing by M1 and M2 macrophages**

| Effector cell | Antibody | IC50 (nM) |
|---|---|---|
| M1 | HER2/5C7 (2+2 A) Fc-optimized | 0.03226 |
| | M1 Negative control | 1.474 |
| M2 | HER2/5C7 (2+2 A) Fc- optimized | 0.006553 |
| | M2 Negative control | 0.1491 |

### Example 22. Cytokine production by HER2/CLEC5A bispecific antibodies

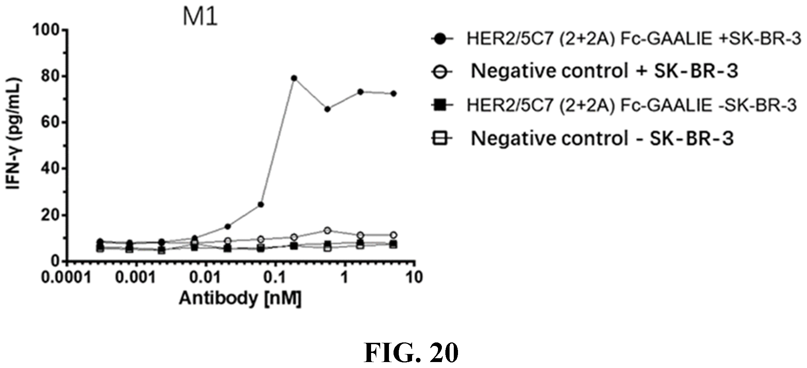

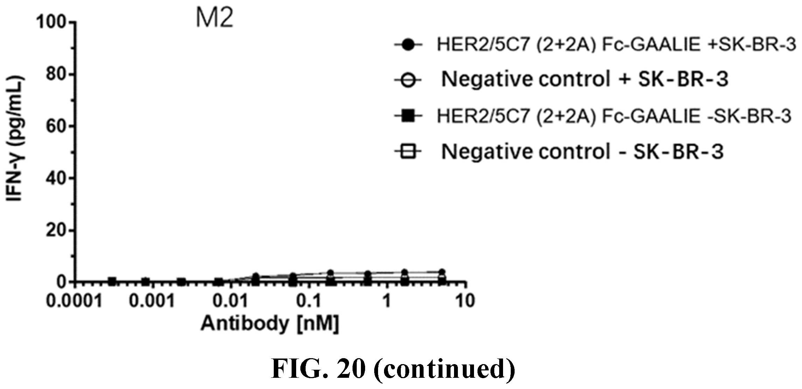

The ability of HER2/CLEC5A bispecific antibodies to activate M1 and M2 polarized macrophages in the presence or absence of target SK-BR-3 cells (HER2+) was evaluated. CD14+ monocytes (purified from human PBMC donor #900 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 6 days. On Day 6, M0 macrophages were polarized with 50 ng/mL of IFN-γ (StemCell Technologies, Cat#: 78020) to M1 or 25 ng/mL of IL-10 (StemCell Technologies, Cat#: 78024), to M2 for another 24 hours (Day 7). About 100,000 macrophages were incubated with 20,000 SK-BR-3 (E:T ratio of 5:1) in the presence of serially diluted antibodies in complete RPMI media (with 10% heat-inactivated FBS and 5% penicillin/streptomycin) for 24 hours at 37°C. After incubation, supernatant was collected for cytokine analysis. Cytokine IFN-γ was measured with V-PLEX Plus pro-inflammatory Panel 1 Human Kit (Meso Scale Discovery, Cat#: K15049G-1). As shown in **FIG. 20**, the HER2/CLEC5A bispecific antibody activated both M1 and M2 macrophages only in the presence of SK-BR-3, with low level of cytokine release.

### Example 23. Effects of plasma and hIgG1 on HER2/CLEC5A bispecific antibody-mediated target cell killing

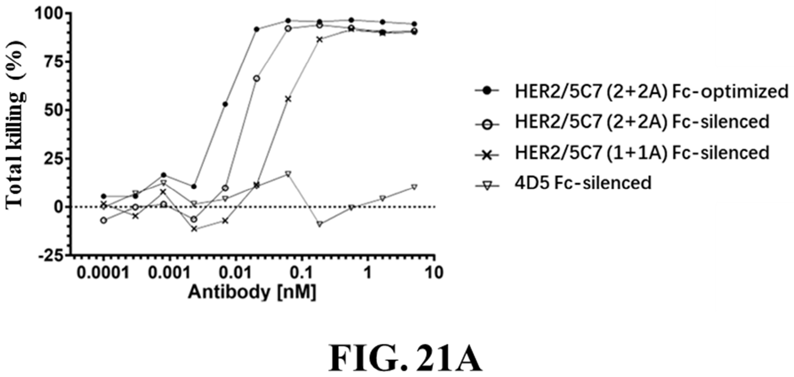

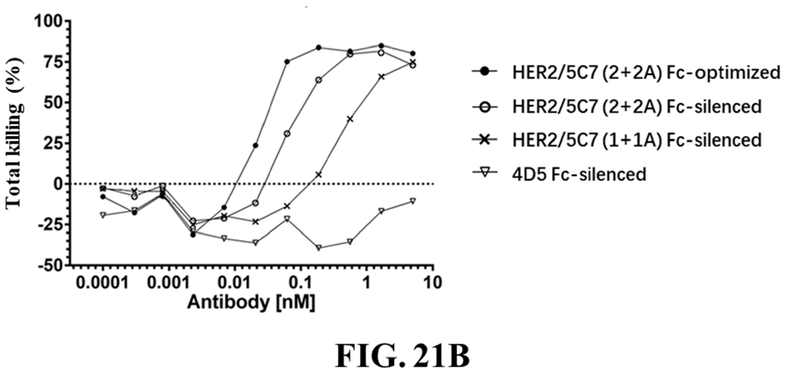

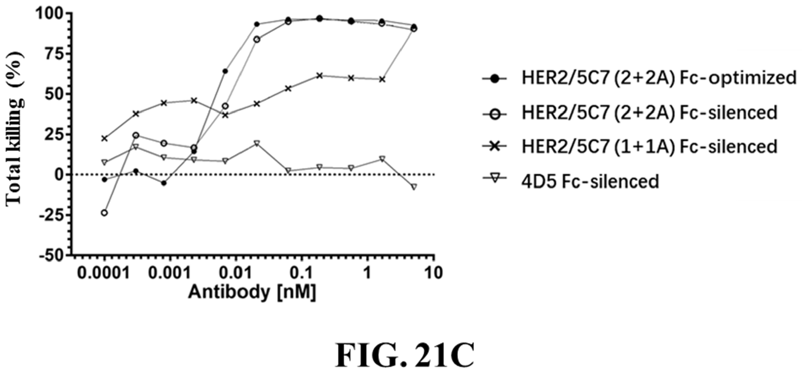

The effects of human plasma and purified hIgG1 antibody on HER2/CLEC5A antibody-mediated killing of target cancer SK-BR-3 cells (HER2+) by effector macrophages (CLEC5A+) was evaluated. CD14+ monocytes (purified from human PBMC donor #308 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 7 days. SK-BR-3 cells were labelled with CFSE (Thermofisher, Cat#: C34554). About 100,000 macrophages were incubated with 20,000 CFSE+ SK-BR-3 (E:T ratio of 5:1) in the presence of serially diluted antibodies in complete RPMI media (with 10% heat-inactivated FBS and 5% penicillin/streptomycin) for 24 hours at 37°C in the presence of media (control), 50% plasma (heparin), or hIgG1 (BioLegend, Cat#: 403502). After incubation, the cells were resuspended in 100 µl of FACS buffer with SYTOX^{™} Blue Dead Cell Stain (ThermoFisher, Cat#: S34857) and analyzed with a Cytek^{®} cytometer. SK-BR-3 cells were gated as CFSE+ by FACS, and absolute cell count of CFSE+ cells were obtained by collecting a fixed volume for all treatment conditions. Percentage of target cell killing was calculated as: (Absolute number of CFSE+ SK-BR-3 cell number in nontreatment group - Absolute number of CFSE+ SYTOX- SK-BR-3 cell number in treatment group)/Absolute number of CFSE+ SK-BR-3 cell number in nontreatment group × 100. As shown in **FIGs. 21A-21C** and the table below, there was no significant reduction in the activity of different HER2/CLEC5A bispecific antibodies even in the presence of plasma or hIgG1.

**Table 12. HER2/CLEC5A bispecific antibody-mediated target cell killing in the presence of media, plasma, or hIgG1**

| Presence | Antibody | IC50 (nM) |
|---|---|---|
| media | HER2/5C7 (2+2A) Fc-optimized | 0.006835 |
| | HER2/5C7 (2+2A) Fc-silenced | 0.01416 |
| | HER2/5C7 (1+1 A) Fc-silenced | 0.04809 |
| | 4D5 Fc-silenced | N/A |
| plasma | HER2/5C7 (2+2A) Fc-optimized | 0.02378 |
| | HER2/5C7 (2+2A) Fc-silenced | 0.06766 |
| | HER2/5C7 (1+1 A) Fc-silenced | 0.4332 |
| | 4D5 Fc-silenced | N/A |
| hIgG1 | HER2/5C7 (2+2A) Fc-optimized | 0.004859 |
| | HER2/5C7 (2+2A) Fc-silenced | 0.00761 |
| | HER2/5C7 (1+1 A) Fc-silenced | 0.00632 |
| | 4D5 Fc-silenced | N/A |

### Example 24. EGFR/CLEC5A bispecific antibodies mediated DLD-1 cell killing by macrophages

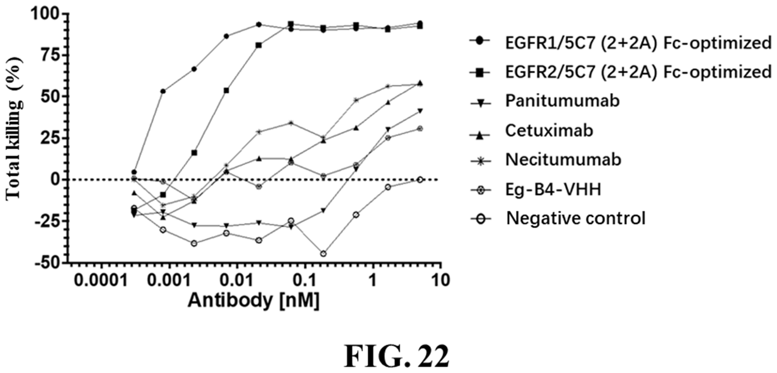

The ability of different EGFR hIgG1 (Cetuximab, Panitumumab, Necitumumab, Eg-B4-VHH) and EGFR/CLEC5A (2+2 A) Fc-optimized antibodies with EGFR portion of Amivantamab (EGFR1) and Nimotuzumab (EGFR2) antibodies to kill target cancer DLD-1 cells (EGFR+) by effector macrophages (CLEC5A+) through both phagocytosis and trogocytosis mechanisms was evaluated. Specifically, CD14+ monocytes (purified from human PBMC donor #900 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 7 days. DLD-1 cells were labelled with CFSE (ThermoFisher, Cat#: C34554). About 100,000 macrophages were incubated with 20,000 CFSE+ DLD-1 (E:T ratio of 5:1) in the presence of serially diluted antibodies in complete RPMI media (with 10% heat-inactivated FBS and 5% penicillin/streptomycin) for 24 hours at 37 °C. After incubation, the cells were resuspended in 100 µl of FACS buffer with SYTOX^{™} Blue Dead Cell Stain (ThermoFisher, Cat#: S34857) and analyzed with a Cytek^{®} cytometer. DLD-1 cells were gated as CFSE+ by FACS, and absolute cell count of CFSE+ cells were obtained by collecting a fixed volume for all treatment conditions. Percentage of target cell killing was calculated as: (Absolute number of CFSE+ DLD-1 cell number in nontreatment group - Absolute number of CFSE+ SYTOX- DLD-1 cell number in treatment group)/Absolute number of CFSE+ DLD-1 cell number in nontreatment group × 100. As shown in **FIG. 22** and the table below, EGFR/CLEC5A bispecific antibodies had a high potency of killing EGFR+ target cells.

**Table 13: EGFR/CLEC5A BsAbs mediated DLD-1 cell killing by macrophages**

| Antibody | IC50 (nM) |
|---|---|
| EGFR1/5C7 (2+2 A) Fc-optimized | 0.001483 |
| EGFR2/5C7 (2+2 A) Fc-optimized | 0.004711 |
| Panitumumab | 0.6415 |
| Cetuximab | 0.7918 |
| Necitumumab | 0.05742 |
| Eg-B4-VHH | 3.861 |
| Negative control | N/A |

### Example 25. EpCAM/CLEC5A bispecific antibodies mediated DLD-1 cell killing by macrophages

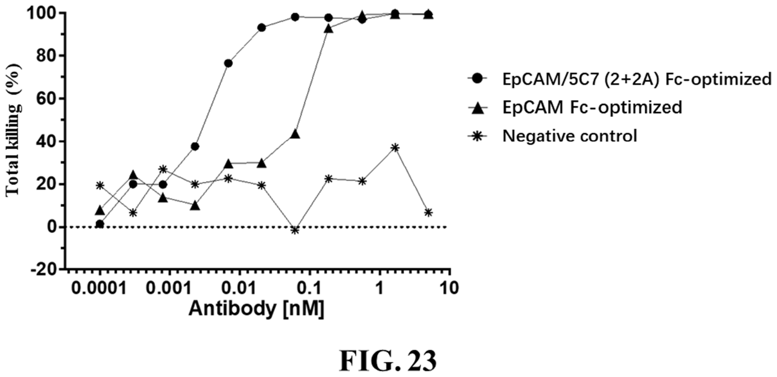

The ability of an anti-EpCAM antibody (Solitomab EpCAM binding part in IgG form) and EpCAM/CLEC5A (2+2 A) Fc-optimized antibodies to kill target cancer DLD-1 cells (EpCAM+) by effector macrophages (CLEC5A+) cells through both phagocytosis and trogocytosis mechanisms was evaluated. CD14+ monocytes (purified from human PBMC donor #900 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 7 days. DLD-1 cells were labelled with CFSE (ThermoFisher, Cat#: C34554). About 100,000 macrophages were incubated with 20,000 CFSE+ DLD-1 (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. DLD-1 cells were gated as CFSE+ by FACS, and absolute cell count of CFSE+ cells were obtained by collecting a fixed volume for all treatment conditions. Percentage of target cell killing was calculated as: (Absolute number of CFSE+ DLD-1 cell number in nontreatment group - Absolute number of CFSE+ SYTOX- DLD-1 cell number in treatment group)/Absolute number of CFSE+ DLD-1 cell number in nontreatment group × 100. As shown in **FIG. 23** and the table below, the EpCAM/CLEC5A bispecific antibody had a high potency of killing EpCAM+ target cells.

**Table 14: EpCAM/CLEC5A BsAbs mediated DLD-1 cell killing by macrophages**

| Antibody | IC50 (nM) |
|---|---|
| EpCAM/5C7 (2+2 A) Fc-optimized | 0.003504 |
| Anti-EpCAM antibody | 0.0812 |
| Negative control | 0.101 |

### Example 26. EpCAM/CLEC5A bispecific antibodies mediated various cancer cell killing by macrophages

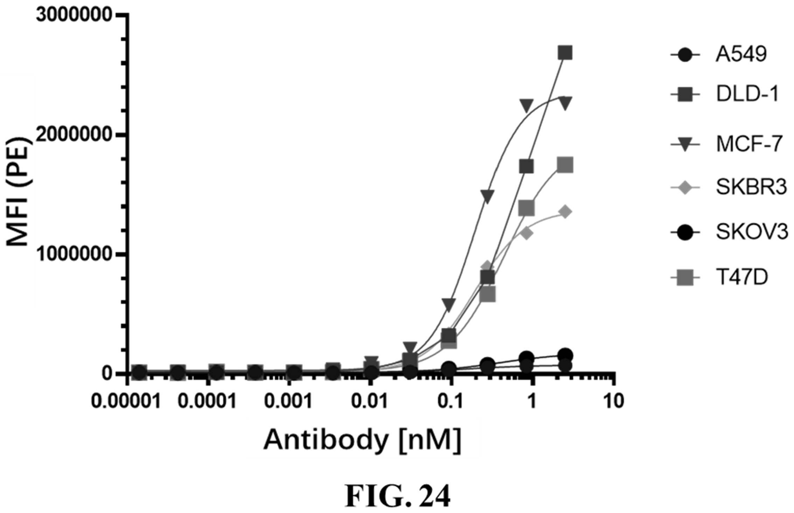

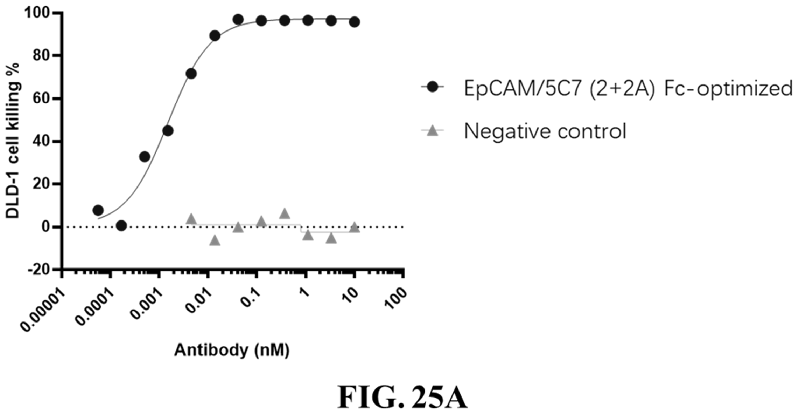

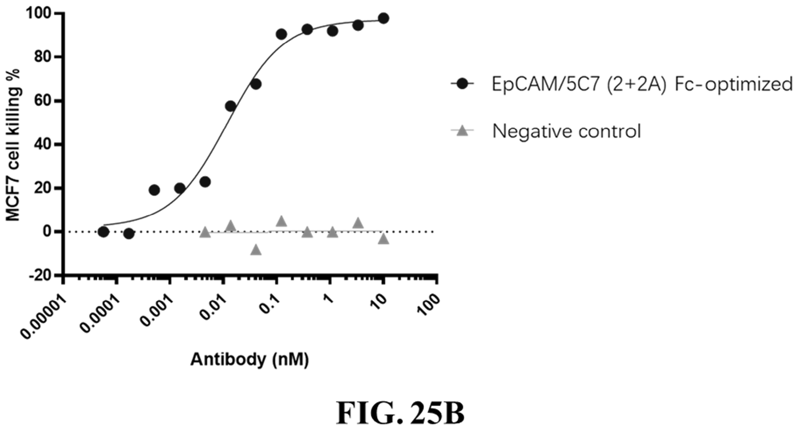

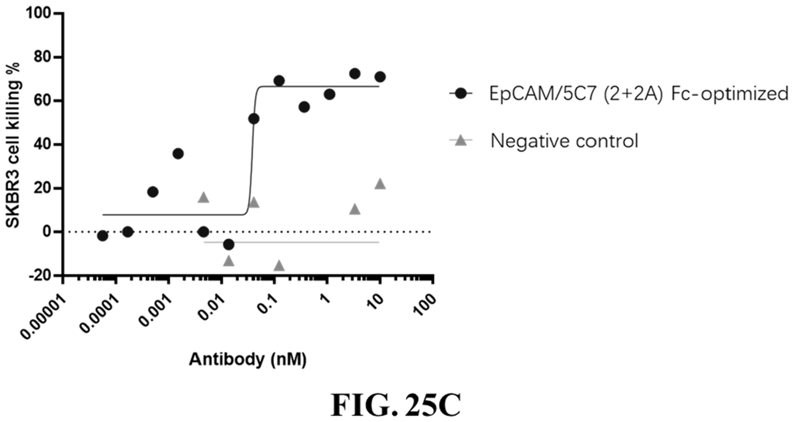

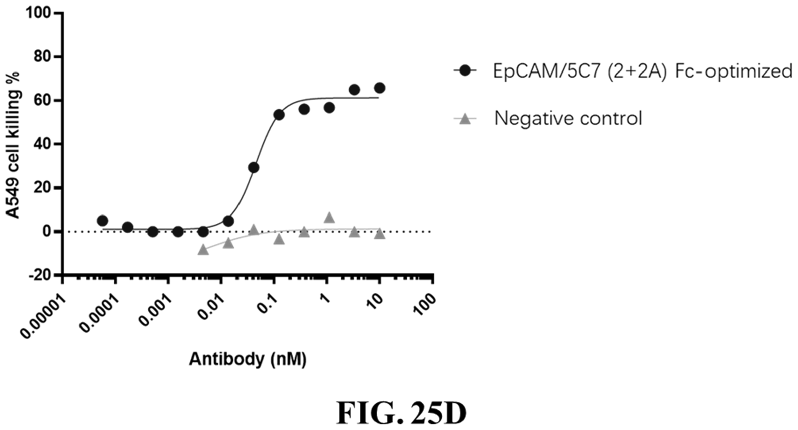

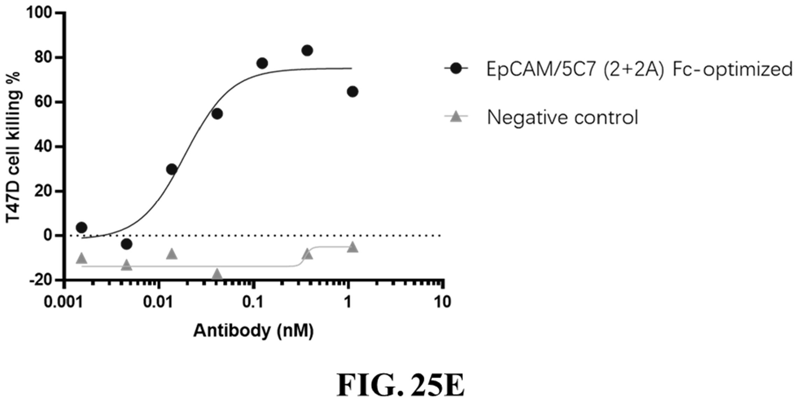

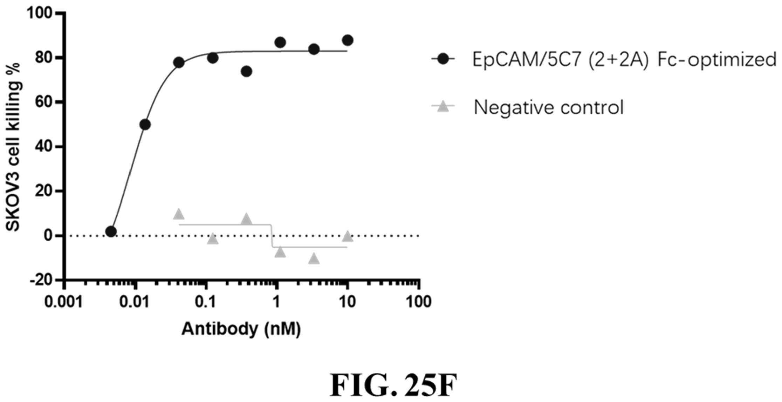

hEpCAM expression level on various cancer cell lines was evaluated. Various cancer cell lines A549, DLD-1, MCF-7, SKPR3, SKOV3 and T47D were used. 50,000 cancer cells were stained with 0.5 nM anti-EpCAM antibody-AF488 (1:200 dilution, BioLegend, Cat#: 302208) for 30 minutes on ice. The cells were washed with PBS twice, and then stained with DAPI (1:5000 dilution from 1 mg/ml, Invitrogen, Cat#:D1306) for 5 minutes at room temperature before tested by flow cytometry (Cytek^{®}, Northen LightsTM). The raw data were analyzed by FlowJoTM Portal 10 and graphed using GraphPad Prism 10. As shown in FIG. 24, these cell lines showed distinct EpCAM density.The effects of EpCAM/CLEC5A bispecific antibodies to kill target cell lines of distinct EpCAM density by effector M0 macrophages were evaluated. Various cancer cell lines A549, DLD-1, MCF-7, SKPR3, SKOV3 and T47D were used. CD14+ monocytes (purified from human PBMC with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 7 days. Cancer cell cells were labeled with CFSE (ThermoFisher, Cat#: C34554). About 100,000 macrophages were incubated with 20,000 CFSE+ cancer cells (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. Cancer cells were gated as CFSE+ by FACS, and the absolute cell count of CFSE+ cells was obtained by collecting a fixed volume for all treatment conditions. Percentage of target cell killing was calculated as: (absolute number of CFSE+ cancer cell number in non-treatment group - absolute number of CFSE+ SYTOX- cancer cell number in treatment group)/absolute number of CFSE+ cancer cell number in non-treatment group × 100. As shown in **FIGs. 25A-25F**, the EpCAM/CLEC5A bispecific antibody had a high potency of killing target cells with distinct EpCAM density.

### Example 27. Different myeloid cell engagers mediated multiple myeloma cancer cell killing by PBMCs

The killing efficacy of multiple myeloma cancer cells (NCI-H929) in healthy human PBMCs by myeloid cell engagers was evaluated. Specifically, the myeloid cell engagers (e.g., GPRC5D/5C7 (2+2 A) Fc-optimized, BCMA/5C7 (2+2 A) Fc-optimized, CD38/5C7 (2+2 A) Fc-optimized) were serially diluted. NCI-H929 cells (as target cells) were stained with CFSE first, then mixed with healthy human PBMCs (E:T=10:1) in a 96-well-round-bottom plate, which was further incubated with serial diluted bispecific antibodies, (1 to 3 dilution from 100 nM) for 48 hours. After the incubation, the plate was spun down, and cells were stained by live/dead staining dye. The stained cells were then processed using a CytoFLEX LX flowcytometry. Percentage of cell killing was calculated as: (live tumor cell number in nontreatment group-live tumor cell number in treatment group)/live tumor cell number in nontreatment group × 100.

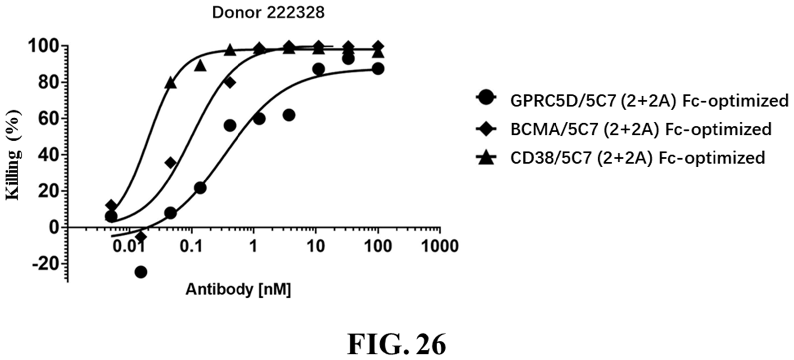

The results are shown in **FIG 26**. The maximum killing percentage was about 99% for BCMA/5C7 (2+2 A) Fc-optimized and CD38/5C7 (2+2 A) Fc-optimized) bispecific antibodies in NCI-H929 killing; and the maximum killing percentage was about 92% for GPRC5D/5C7 (2+2 A) Fc-optimized bispecific antibody. The results indicate that myeloid engagers can efficiently kill multiple myeloid cancer cells with distinct targets.

### Example 28. CD79b expression level on malignant lymphoma cell lines and B cell in healthy PBMC donors

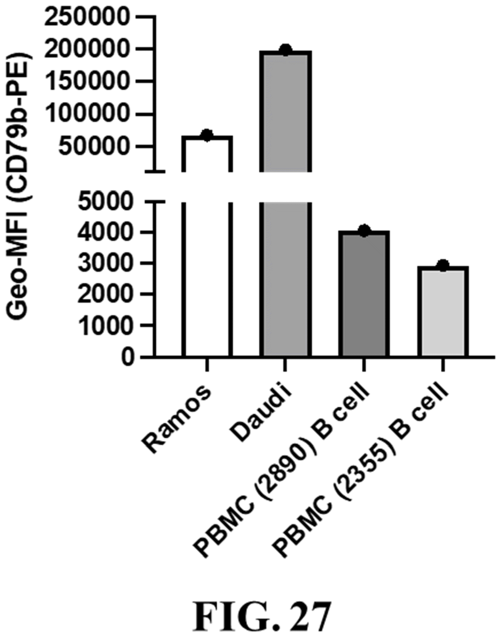

hCD79b expression level on malignant lymphoma cell lines and B cells in healthy PBMC donors was evaluated. Malignant lymphoma cell lines (Ramos and Daudi) were obtained from ATCC, and frozen PBMCs were obtained from healthy donors (Stanford Blood Center). 50,000 Ramos or Daudi cells and 20,0000 PBMCs were stained with 0.5 nM anti-CD79b antibody-AF488 and anti-CD19 antibody-PE (1:200 dilution, BioLegend, Cat#: 302208) for 30 minutes on ice. The cells were washed with PBS twice, and then stained with DAPI (1:5000 dilution from 1 mg/ml, Invitrogen, Cat#:D1306) for 5 minutes at room temperature before tested by flow cytometry (Cytek^{®}, Northen LightsTM). The raw data were analyzed by FlowJoTM Portal 10 and graphed using GraphPad Prism 10. As shown in **FIG. 27**, hCD79b expression levels of Ramos and Daudi cells were more than about 4-10 folds on healthy B cells in PBMCs.

### Example 29. PBMC immuno-phenotyping

To know the ratio of various cell populations in PBMCs, immuno-phenotyping of cells was conducted. The healthy human blood came from Stanford Blood Center. PBMCs were isolated with EasySep^{™} Human Monocyte Enrichment Kit without CD16 Depletion (StemCell Technologies, Cat#: 19058). PBMCs were washed twice with FACS buffer (1× PBS + 2% BSA + 2 mM EDTA). 1 × 10⁵ PBMCs were seeded in each well of a V-bottom 96-well plate. Then, 50 µl of antibody cocktail (BV421 CD19 (BD Biosciences, Cat#: 562440), BV605 CD11b (BioLegend, Cat#: 101237), BV711 CD11 (BD Biosciences, Cat#: 563130, FITC CD56 (BD Biosciences, Cat#: 340410), PE CD8 (BioLegend, Cat#: 344706), Percpcy5.5 CD14 (BD Biosciences, Cat#: 550787), PEcy7 CD16 (BD Biosciences, Cat#: 557744), APC CD3 (BD Biosciences, Cat#: 555335), AF700 CD4 (BD Biosciences, Cat#: 566318), AF700 HLA-DR (BD Biosciences, Cat#: 560743)) was added into each well. The mixture was incubated on ice for 30 minutes; then, the cells were washed twice with FACS buffer (after cells were spun down at 1200 rpm for 5 minutes). The cells were resuspended in 100 µl SYTOX^{™} Blue Dead Cell Stain (1:1000) buffer and incubated for 20 minutes on ice in the dark. Cells were analyzed with a Cytek^{®} cytometer. The ratio of various cell population was calculated based on the various surface marker positive cells (CD19+ B cells, CD56+ NK cells, CD14+ monocytes, CD8+ T cells, CD4+ T cells, CD11c+/HLA-DR+ DC cells).

**Table 15. PBMC immuno-phenotyping of different donors**

| Donors | B | NK | Monocyte | CD8 T | CD4 T | DC |
|---|---|---|---|---|---|---|
| D022 | 25.4 % | 23.1 % | 28.0 % | 2.57 % | 7.65 % | 5.45 % |
| D023 | 6.35 % | 35.4 % | 11.8 % | 13.3 % | 24.9 % | 1.14 % |
| D234 | 13.9 % | 25.6 % | 11.9 % | 7.87 % | 22.5 % | 3.72 % |
| D281 | 3.33 % | 9.60 % | 26.6 % | 6.00 % | 32.0 % | 0.29 % |
| D367 | 13.7 % | 16.3 % | 20.0 % | 29.5 % | 9.49 % | 0.84% |
| D431 | 9.26 % | 12.80 % | 8.81 % | 19.7 % | 39.7 % | 1.59 % |

### Example 30. CD79b/CLEC5A bispecific antibodies mediated killing of Ramos cells by PBMCs

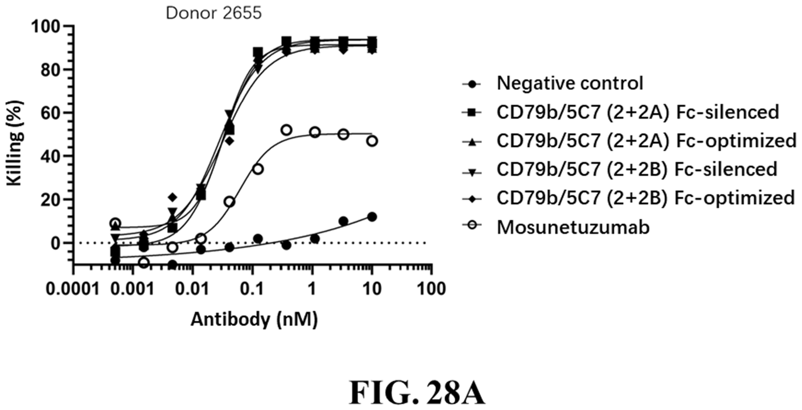

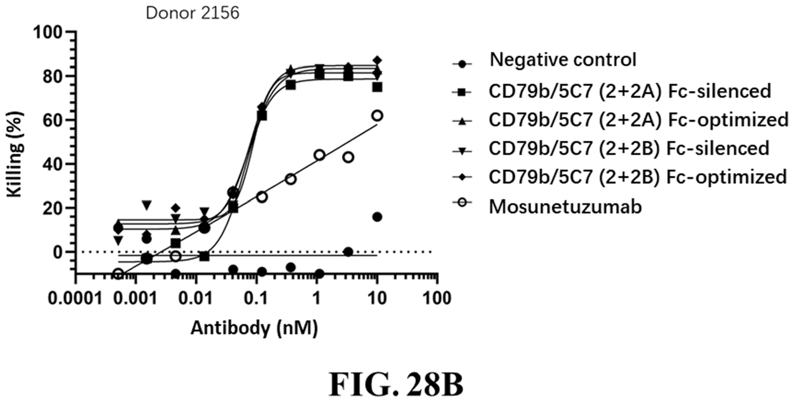

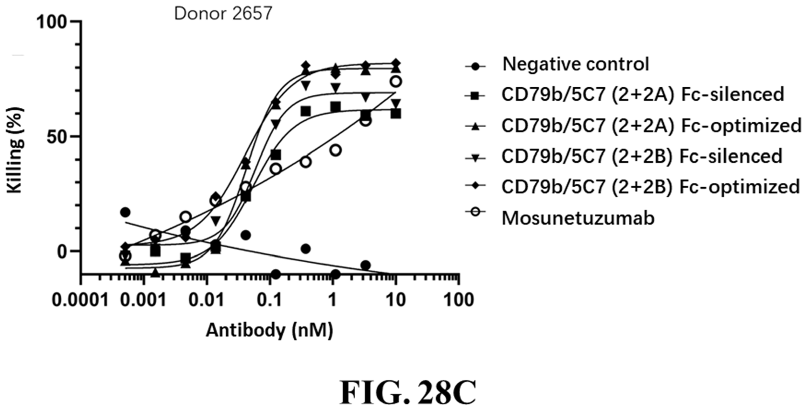

CD79b/CLEC5A bispecific antibody-mediated tumor killing with fresh human PBMCs and Ramos cells was tested. Fresh human PBMCs were isolated from donated health human blood from SBC (Stanford Blood Center) and U-bottom 96 well plates are used. Ramos cells stained with CFSE staining buffer (Invitrogen). 2 × 10⁴ CFSE-stained Ramos cells in 50 µl medium were added into each designated well. 2 × 10⁵ PBMCs in 130 µl medium were added into each designated well. 20 µl of serially diluted testing antibodies in complete culture media were added into each designated well, and total final volume for each well was 200 µl. The plate with well-mixed cells was incubated at 37°C with 5% CO₂. Samples were collected 24 hours after the incubation. Cell pellet was resuspended in FACS buffer and rinsed twice. Cells were stained with SYTOX^{™} Blue Dead Cell Stain and PE-conjugated CD19. Cells are analyzed with a Cytek^{®} cytometer (live Ramos cells gating: CFSE+ SYTOX- or CD19+CFSE+ SYTOX-). Killing efficacy was calculated as: (Killing % = (control live Ramos cells count - treated live Ramos cells count)/control live Ramos count). As shown in **FIGs. 28A-28C**, all the CD79b/CLEC5A bispecific antibodies demonstrated superior Ramos cell killing efficacy than commercial Mosunetuzumab at 24 hours, and CD79b/CLEC5A bispecific antibodies exhibited higher endogenous B killing than Mosunetuzumab in all three donors' PBMCs.

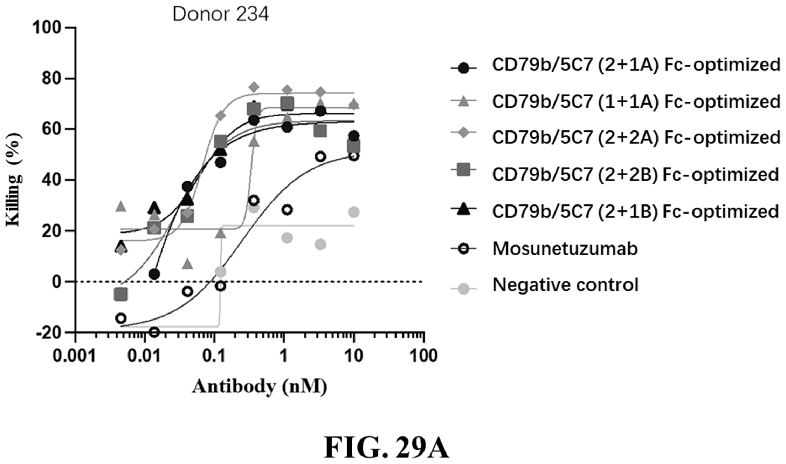

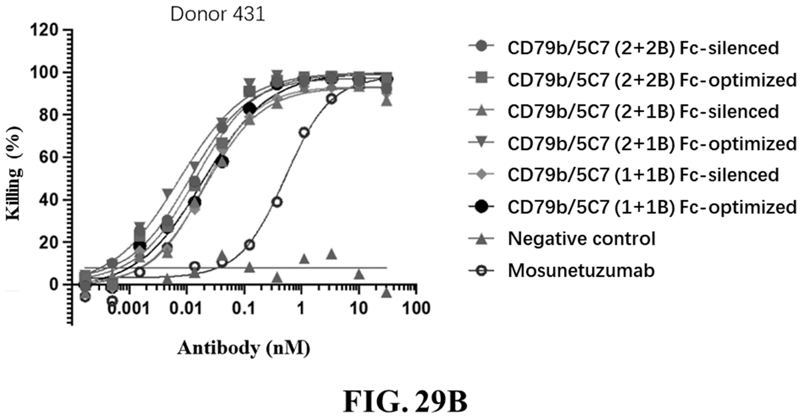

The efficacy of CD79b/CLEC5A bispecific antibody-mediated malignant B cell depletion was evaluated by Ramos spike-in killing assay. Specifically, fresh PBMCs were isolated from healthy blood samples provided by Stanford Blood Center with Ficoll-paque gradient centrifugation. Ramos cells (target) were stained with CFSE first, and then mixed with PBMCs (effector) at E:T=10:1. The cells were then incubated together with titrated antibodies in a round bottom 96-well plate for 24 hours at 37°C with 5% CO₂. The real E:T ratio calculated with monocyte: B (endogenous B cells plus Ramos cell) was 0.5:1, and it was 1.5:1 if included NK cells as the effector cells. After 24 hours, the cells were spun down for flow cytometry analysis, and the supernatant was collected for ELISA analysis of cytokine release. The cell samples were washed once with PBS, and then stained with Zombie Aqua^{™} 1:1000 dilution for 20 minutes at 4°C. After the staining, the cells were washed with PBS once, resuspended with flow buffer, and then analyzed with a Cytek^{®} (Northern light^{™}) flow cytometer. Ramos cell killing % = (Ramos cells in the non-treatment group-Ramos cells in the treatment group)/Ramos cells in the non-treatment group × 100. As shown in **FIGs. 29A-29B** and the table below, with respect to Donor 234, compared to clinical used Mosunetuzumab (IC50 was about 0.25 nM), CD79b/CLEC5A bispecific antibodies with different structures all showed a higher Ramos killing effect (with IC50 of about 60% to 80% higher). For example, IC50 of the CD79b/CLEC5A bispecific antibodies ranged from 0.0047 nM for CD79b/5C7 (2+1 A) Fc-optimized to 0.338 nM for CD79b/5C7 (1+1 A) Fc-optimized. With respect to Donor 431, the real E:T was <1:1 if calculated with monocyte: B cells, and the E:T=2:1 with NK cells included as effector cells. The Ramos spike-into PBMC killing results showed that as compared to Mosunetuzumab (IC50 was 0.0069 nM), CD79b/CLEC5A bispecific antibodies with different structures all showed a higher Ramos killing effect (IC50 of about 80% to nearly 100% higher). For example, IC50 of the CD79b/CLEC5A bispecific antibodies ranged from 0.0062 nM for CD79b/5C7 (2+2 B) Fc-silenced to 0.0329 nM for CD79b/5C7 (1+1 B) Fc-silenced.

**Table 16. CD79b/CLEC5A bispecific antibodies mediated Ramos cell killing by PBMCs (test 2)**

| Donor | Antibodies | IC50 (nM) |
|---|---|---|
| Donor 234 | CD79b/5C7 (2+1 A) Fc-optimized | 0.004718 |
| | CD79b/5C7 (1+1 A) Fc-optimized | 0.338 |
| | CD79b/5C7 (2+2 A) Fc-optimized | 0.06753 |
| | CD79b/5C7 (2+2 B) Fc-optimized | 0.02984 |
| | CD79b/5C7 (2+1 B) Fc-optimized | 0.06394 |
| | Mosunetuzumab | 0.2515 |
| | Negative control | - |
| Donor 431 | CD79b/5C7 (2+2 B) Fc-silenced | 0.0062 |
| | CD79b/5C7 (2+2 B) Fc-optimized | 0.0168 |
| | CD79b/5C7 (2+1 B) Fc-silenced | 0.0155 |
| | CD79b/5C7 (2+1 B) Fc-optimized | 0.0104 |
| | CD79b/5C7 (1+1 B) Fc-silenced | 0.0329 |
| | CD79b/5C7 (1+1 B) Fc-optimized | 0.0253 |
| | Mosunetuzumab | 0.0069 |
| | Negative control | - |

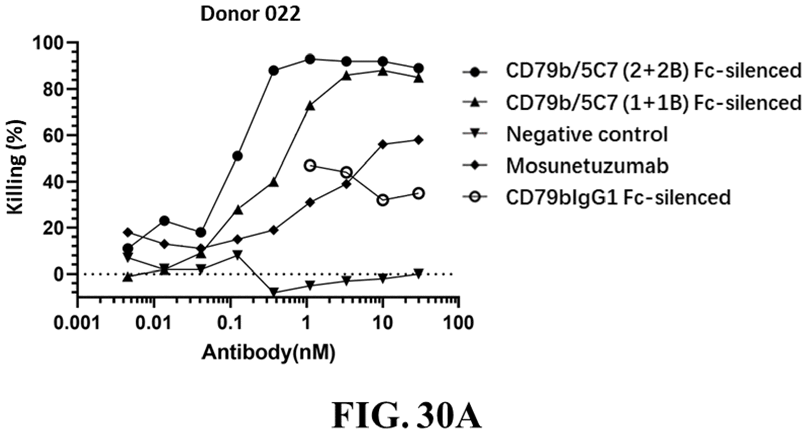

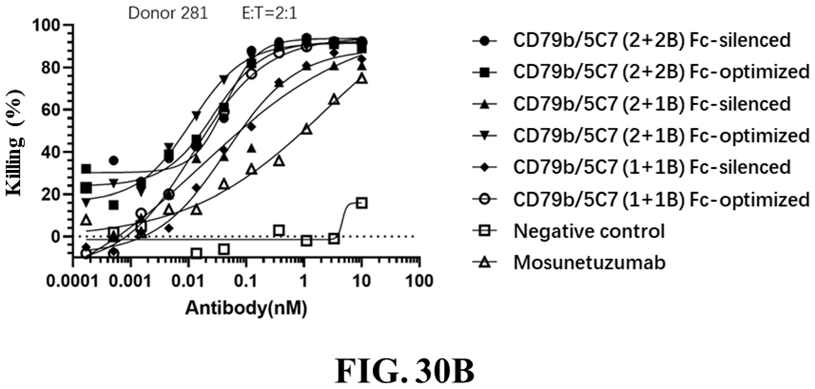

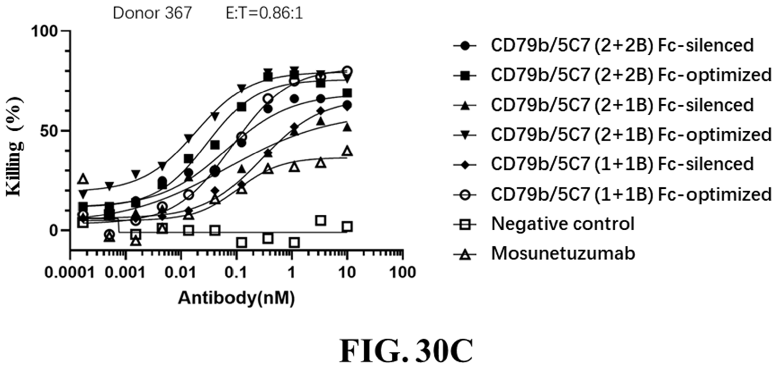

To test the efficacy of CD79b/CLEC5A bispecific antibodies to kill Ramos cells, a coculture system of human PBMCs with Ramos cells was set up. PBMCs were isolated from healthy human blood with with EasySep^{™} Human Monocyte Enrichment Kit without CD16 Depletion (StemCell Technologies, Cat#: 19058). Ramos cells were stained with CFSE (Invitrogen). 20 µl of serially diluted bispecific antibodies in complete media was added into each well of a U-bottom 96 well plate. 2 × 10⁵ PBMCs and 2 × 10⁴ Ramos cells (E:T ratio = 10:1) in 180 µl complete culture media (RPMI1640 + 10% FBS) were added into each well, respectively. The mixture was incubated at 37°C and 5% CO₂ for 24 hours. Cells were spun down at 1500 rpm for 5 minutes. Cells pellet was washed and resuspended in FACS buffer, stained and analyzed with a Cytek^{®} cytometer. The staining panels included SYTOX^{™} Blue Dead Cell Stain and Brilliant Violet 421^{™} anti-human CD19 Antibody (BV421 CD19). Live CFSE+ cells were gated as surviving tumor cells. The killing efficacy is calculated as: Killing = (control tumor cells number-treated tumor cells number)/control tumor cells number. As shown in **FIGs 30A-30C** and the table below, all the CD79b/CLEC5A bispecific antibodies with different structures all showed a higher Ramos killing effect at a very low E:T ratio.

**Table 17. CD79b/CLEC5A bispecific antibodies mediated Ramos cell killing by PBMCs (test 3)**

| Donor | Antibodies | IC50 (nM) |
|---|---|---|
| Donor 022 | CD79b/5C7 (2+2 B) Fc-silenced | 0.1307 |
| | CD79b/5C7 (1+1 B) Fc-silenced | 0.3335 |
| | Mosunetuzumab | 2.386 |
| | Negative control | - |
| Donor 281 | CD79b/5C7 (2+2 B) Fc-silenced | 0.043 |
| | CD79b/5C7 (2+2 B) Fc-optimized | 0.2798 |
| | CD79b/5C7 (2+1 B) Fc-silenced | 0.01654 |
| | CD79b/5C7 (2+1 B) Fc-optimized | 0.01118 |
| | CD79b/5C7 (1+1 B) Fc-silenced | 0.04524 |
| | CD79b/5C7 (1+1 B) Fc-optimized | 0.01231 |
| | Mosunetuzumab | 2.358 |
| | Negative control | - |
| Donor 367 | CD79b/5C7 (2+2 B) Fc-silenced | 0.06427 |
| | CD79b/5C7 (2+2 B) Fc-optimized | 0.02953 |
| | CD79b/5C7 (2+1 B) Fc-silenced | 0.06851 |
| | CD79b/5C7 (2+1 B) Fc-optimized | 0.0186 |
| | CD79b/5C7 (1+1 B) Fc-silenced | 0.2899 |
| | CD79b/5C7 (1+1 B) Fc-optimized | 0.8413 |
| | Mosunetuzumab | 0.1058 |
| | Negative control | - |

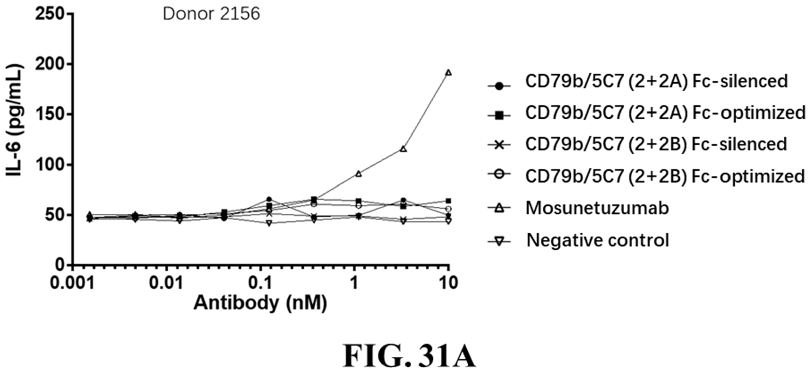

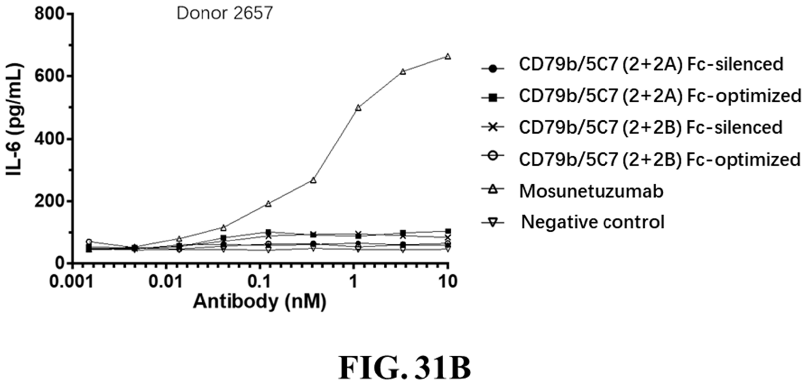

The ability of CD79b/CLEC5A bispecific antibodies to stimulate IL-6 secretion in the presence of target Ramos cells (CD79b+) was evaluated. About 200,000 PBMCs were incubated with 20,000 Ramos cells (E:T ratio of 10:1) in the presence of serially diluted antibodies in complete RPMI media (with 10% heat-inactivated FBS and 5% penicillin/streptomycin) for 24 hours at 37°C. After incubation, supernatant was collected for IL-6 analysis. As shown in **FIGs. 31A-31B**, IL-6 levels did not increase significantly with CD79b/CLEC5A bispecific antibodies in the presence of target Ramos cells. However, IL-6 levels increased in a dose-dependent manner with commercial Mosunetuzumab. The results suggest that CD79b/CLEC5A bispecific antibodies have a better safety profile than Mosunetuzumab.

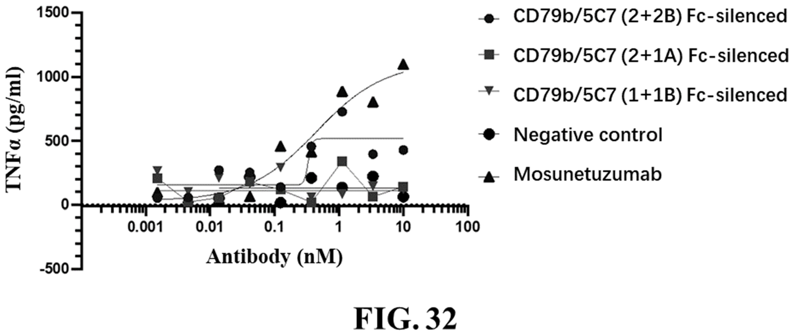

To test the cytokine release in Ramos spike-in assays, supernatant from Ramos spike-in killing assays was collected at 24 hours, TNFα concentration in the supernatant was measured with ELISA kit (TNF alpha Human Uncoated ELISA Kit, Invitrogen, Cat#: 88-7346-88). 96-well high-binding plates coated 24 hours with capture antibody, 25 µL sample to 25 µL of reagent diluent (2× dilution) was tested. As shown in **FIG 32**, there was higher concentration of TNFα released in Mosunetuzumab treated group, but in CD79b/CLEC5A bispecific antibodies treated group, the TNFα concentration was at low or background level.

### Example 31. CD79b/CLEC5A bispecific antibodies mediated killing of endogenous B cells by PBMCs

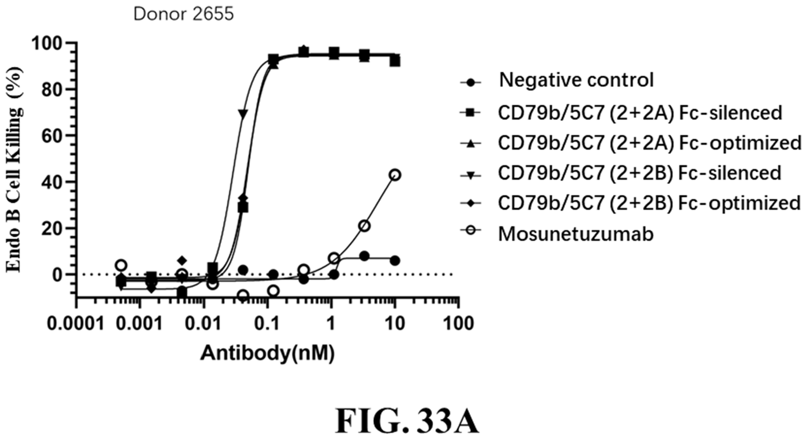

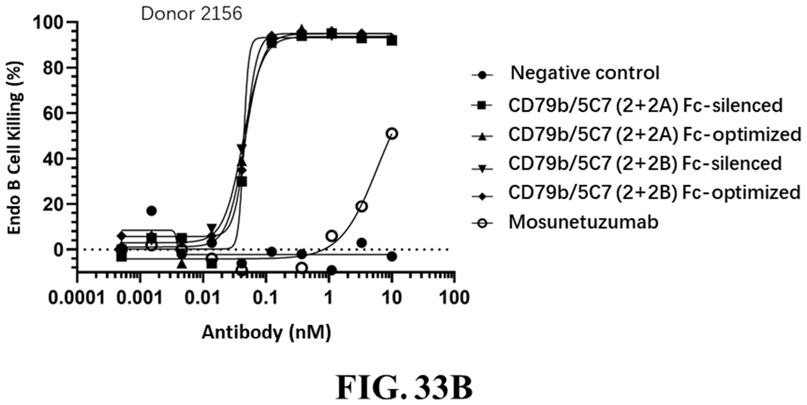

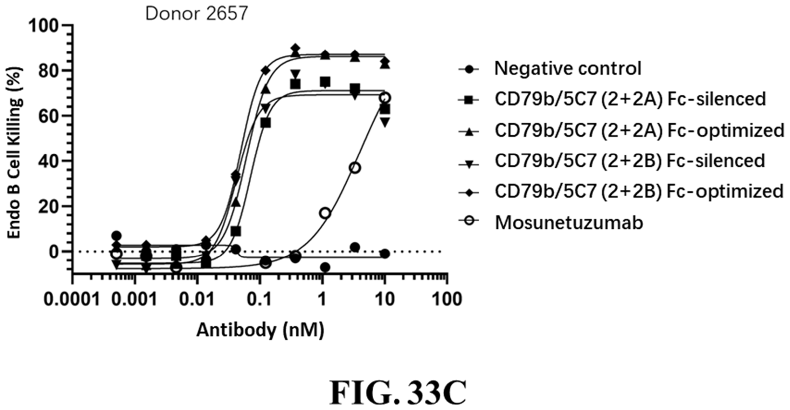

CD79b/CLEC5A bispecific antibody-mediated tumor killing of endogenous B cells was tested. Specifically, fresh human PBMCs were isolated from donated health human blood from SBC (Stanford Blood Center) and U-bottom 96 well plates are used. 2 × 10⁵ PBMCs in 130 µl medium were added into each designated well. 20 µl of serially diluted testing antibodies in complete culture media was added into each designated well, and total final volume for each well was 200 µl. The plate with well-mixed cells was incubated at 37°C and 5% CO₂. Samples were collected 24 hours after incubation. Cells pellet was resuspended in FACS buffer and rinsed twice. Cells were stained with SYTOX^{™} Blue Dead Cell Stain and PE-conjugated anti-CD19 antibody. Cells are analyzed with a Cytek^{®} cytometer. Endogenous B cell killing % = (Endogenous B cells in the non-treatment group-Endogenous B cells in the treatment group)/Endogenous B cells in the non-treatment group × 100. As shown in **FIGs. 33A-33C**, CD79b/CLEC5A bispecific antibodies exhibited higher endogenous B cell killing than Mosunetuzumab in all three donors' PBMCs.

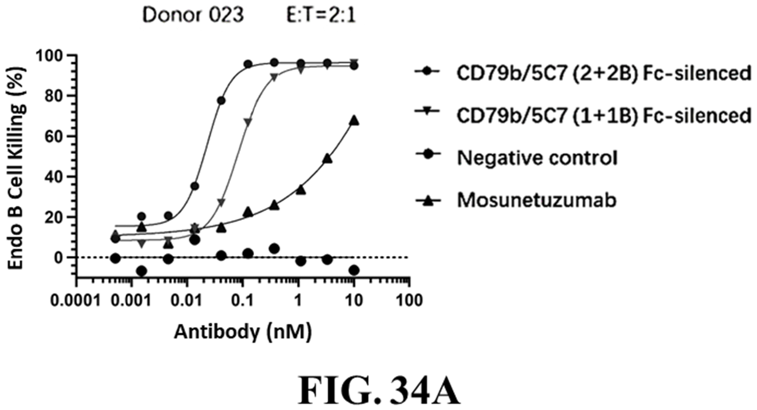

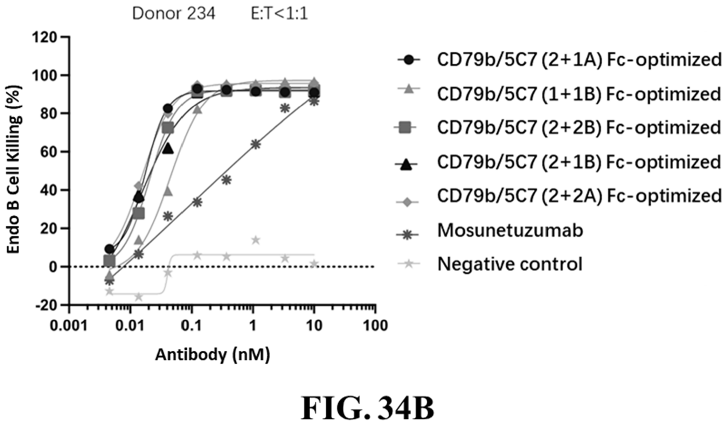

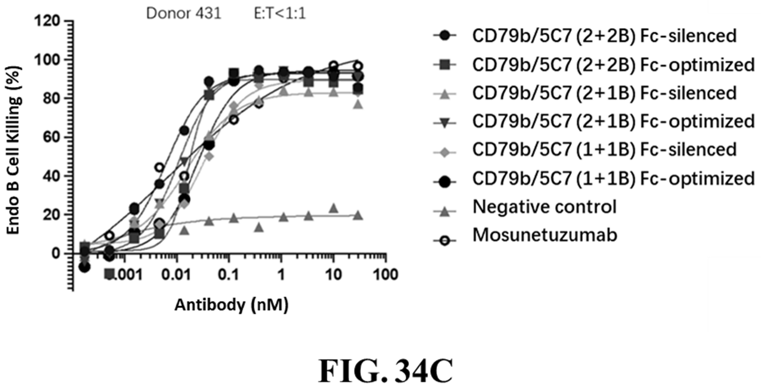

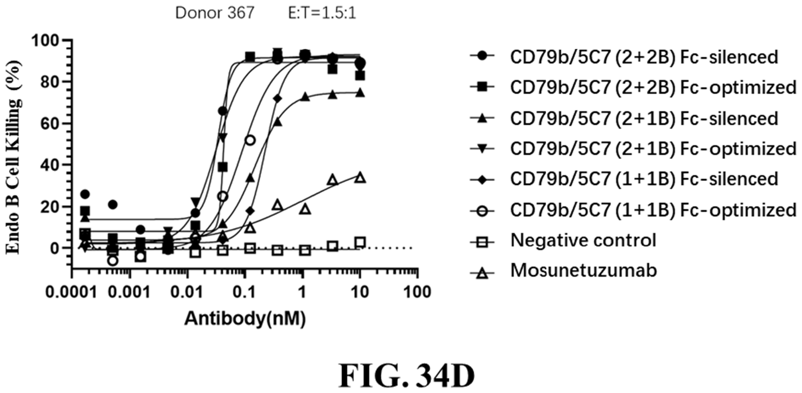

To test the efficacy of CD79b/CLEC5A bispecific antibodies to deplete endogenous B cells, healthy human PBMCs were used. Fresh PBMCs were isolated from healthy blood samples provided by Stanford Blood Center with Ficoll-paque gradient centrifugation. 2 × 10⁵/well PBMCs were incubated together with titrated antibodies in a round bottom 96-well plate for 24 hours at 37°C with 5% CO₂. The real E:T calculated with monocyte to endogenous B cells or monocyte plus NK cells to endogenous B cells varied with donors. After 24 hours, the cells were spun down for flow cytometry analysis, and the supernatant was collected from ELISA analysis of cytokine release. The cell samples were washed once with PBS, and then stained with Zombie Aqua^{™} 1:1000 dilution for 20 minutes at 4°C. After the staining, the cells were washed with PBS once, and then stained with CD19-BV421 (BioLegend, Cat#:302234) for 30 minutes at 4°C. The stained cells were washed with PBS once, resuspended with flow buffer, and then analyzed with a Cytek^{®} (Northern light^{™}) flow cytometer. Endogenous B cell killing %= (Endogenous B cells in the non-treatment group-Endogenous B cells in the treatment group)/Endogenous B cells in the non-treatment group × 100. As shown in **FIGs. 34A-34D** and the table below, for Donor 023, the E:T was 2:1 for monocyte: Endo B, and the number was 7:1 if NK cells were included as the effector cells. Clinically used Mosunetuzumab only had about 65% efficacy at 10 nM, whereas CD79b/CLEC5A bispecific antibodies with different structures all showed a higher endogenous B cell killing effect of around 80% to 96%. With respect to Donor 234, monocyte: Endo B cells (E:T) was <1:1, and the E:T=3:1 if NK cells were included as the effector cells. As compared to clinically used Mosunetuzumab (IC50 was 0.086 nM), CD79b/CLEC5A bispecific antibodies with different structures all showed higher endogenous B cell killing effect of about 90%. For Donor 431, monocyte: Endo B cells (E:T) was <1:1, and the E:T=2:1 if calculated as (monocyte + NK): Endo B. CD79b/CLEC5A bispecific antibodies with different structures all showed a higher Endogenous B cell killing effect (from about 80% to 95% higher). Similar results were observed in Donor 367.

**Table 18. Different myeloid cell engagers mediated killing of endogenous B cells by PBMCs**

| Donor | Antibodies | IC50 (nM) |
|---|---|---|
| Donor 023 | CD79b/5C7 (2+2 B) Fc-silenced | 0.02306 |
| | CD79b/5C7 (1+1 B) Fc-silenced | 0.08329 |
| | Mosunetuzumab | 229.7 |
| | Negative control | - |
| Donor 234 | CD79b/5C7 (2+1 A) Fc-optimized | 0.01779 |
| | CD79b/5C7 (1+1 B) Fc-optimized | 004532 |
| | CD79b/5C7 (2+2 B) Fc-optimized | 0.01559 |
| | CD79b/5C7 (2+1 B) Fc-optimized | 0.02071 |
| | CD79b/5C7 (2+2 A) Fc-optimized | 0.01544 |
| | Mosunetuzumab | 0.8647 |
| | Negative control | - |
| Donor 431 | CD79b/5C7 (2+2 B) Fc-silenced | 0.006174 |
| | CD79b/5C7 (2+2 B) Fc-optimized | 001677 |
| | CD79b/5C7 (2+1 B) Fc-silenced | 0.01546 |
| | CD79b/5C7 (2+1 B) Fc-optimized | 0.1039 |
| | CD79b/5C7 (1+1 B) Fc-silenced | 0.03293 |
| | CD79b/5C7 (1+1B) Fc-optimized | 0.02533 |
| | Mosunetuzumab | - |
| | Negative control | - |
| Donor 367 | CD79b/5C7 (2+2 B) Fc-silenced | 0.006174 |
| | CD79b/5C7 (2+2 B) Fc-optimized | 001677 |
| | CD79b/5C7 (2+1 B) Fc-silenced | 0.01546 |
| | CD79b/5C7 (2+1 B) Fc-optimized | 0.1039 |
| | CD79b/5C7 (1+1B) Fc-silenced | 0.03293 |
| | CD79b/5C7 (1+1B) Fc-optimized | 0.02533 |
| | Mosunetuzumab | - |
| | Negative control | - |

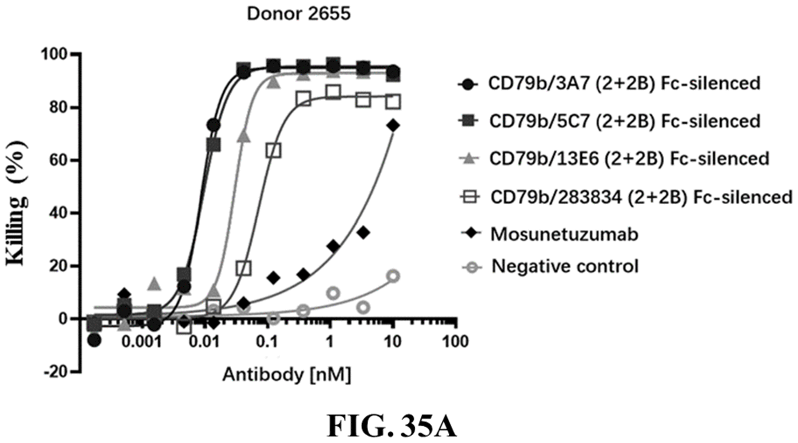

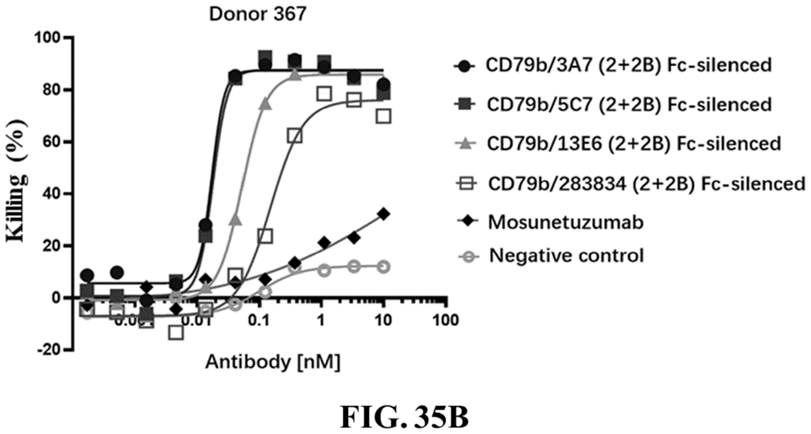

CD79b/CLEC5A bispecific antibody-mediated tumor killing of endogenous B cells was tested. Specifically, fresh human PBMCs were isolated from donated health human blood from SBC (Stanford Blood Center) and U-bottom 96 well plates are used. 2 × 10⁵/well PBMCs were incubated together with titrated antibodies in a round bottom 96-well plate for 24 hours at 37°C with 5% CO₂. Samples were collected 24 hours after incubation. Cells pellet was resuspended in FACS buffer and rinsed twice. Cells were stained with SYTOX^{™} Blue Dead Cell Stain and PE-conjugated anti-CD19 antibody. Endogenous B cell killing % = (Endogenous B cells in the non-treatment group-Endogenous B cells in the treatment group)/Endogenous B cells in the non-treatment group × 100. The results were shown in **FIGs. 35A-35B**.

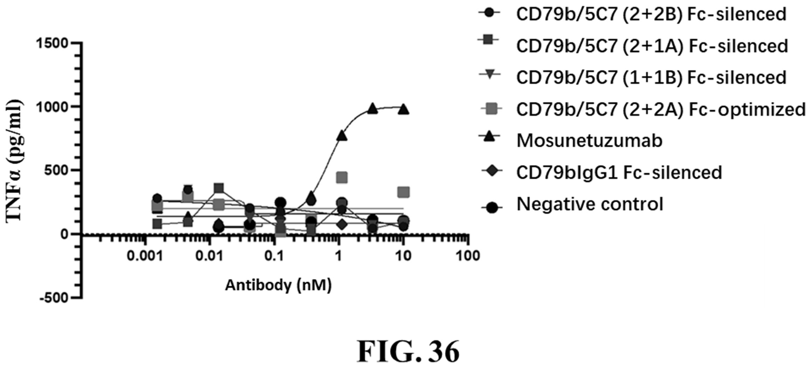

To test the cytokine release of endogenous B cell killing assays, supernatant from endogenous B cell killing assays was collected at 24 hours, TNFα concentration in the supernatant was measured with ELISA kit (TNF alpha Human Uncoated ELISA Kit, Invitrogen, Cat#: 88-7346-88). 96-well high binding plates coated 24 hours with capture antibody, 25 µL sample to 25 µL of reagent diluent (2× dilution) was tested. As shown in **FIG 36**, in CD79b/CLEC5A bispecific antibodies treated group, the TNFα concentration was at low or background level, it suggested that myeloid engagers were safer than bench marker Mosunetuzumab.

### Example 32. CD79b/CLEC5A bispecific antibodies mediated Ramos cell killing by M0 macrophages

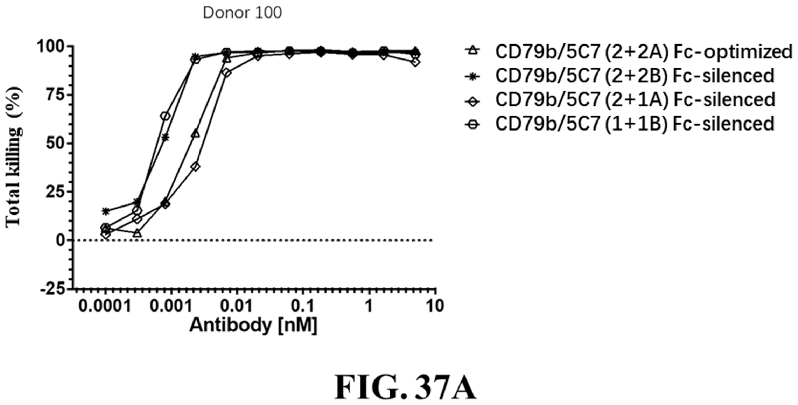

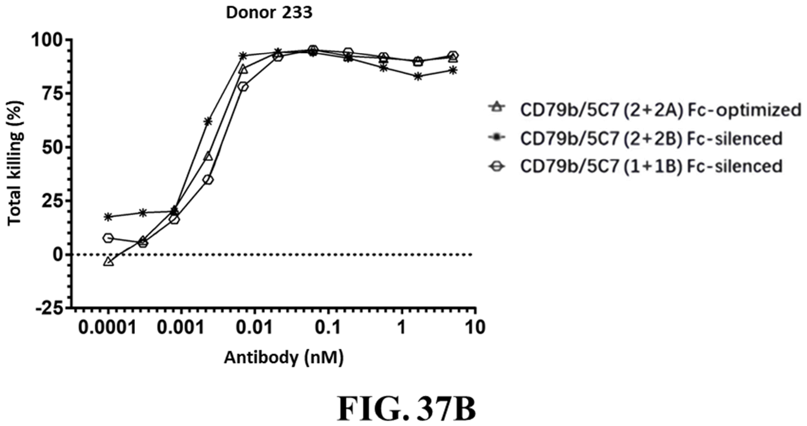

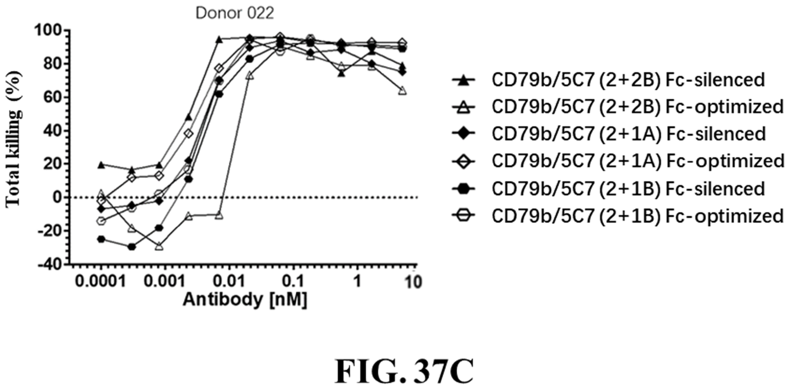

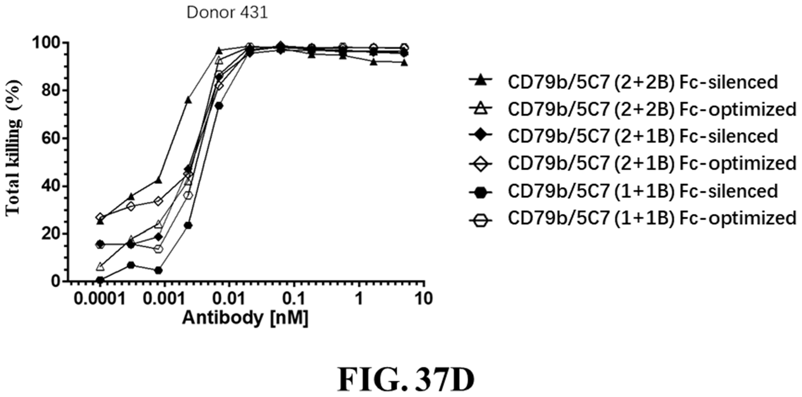

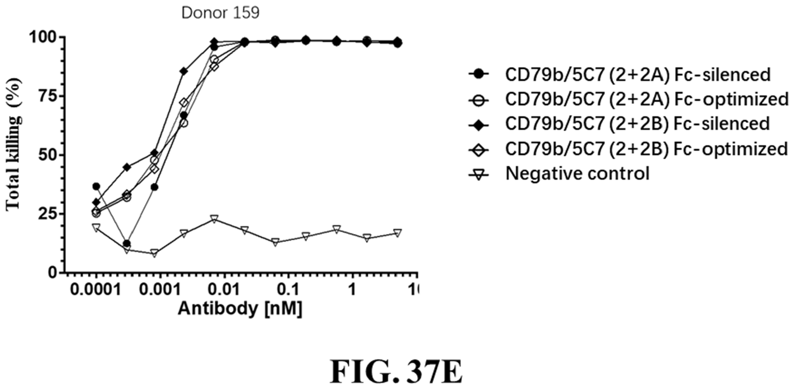

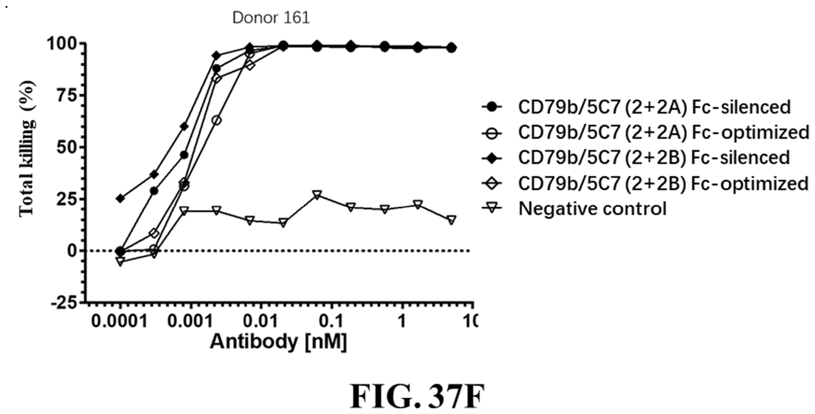

The effects of CD79b/CLEC5A bispecific antibodies with different structures to kill target cancer Ramos cells (CD79b+) by effector macrophages (CLEC5A+) were evaluated. CD14+ monocytes (purified from human PBMC donors #100, #233, #022, #431, #159 and #161 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 7 days. Ramos cells were labeled with CFSE (ThermoFisher, Cat#: C34554). About 100,000 macrophages were incubated with 20,000 CFSE+ Ramos (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. Ramos cells were gated as CFSE+ by FACS, and the absolute cell count of CFSE+ cells was obtained by collecting a fixed volume for all treatment conditions. Percentage of target cell killing was calculated as: (absolute number of CFSE+ Ramos cell number in non-treatment group - absolute number of CFSE+ SYTOX- Ramos cell number in treatment group)/absolute number of CFSE+ Ramos cell number in non-treatment group × 100. As shown in **FIGs. 37A-37F** and the table below, all the CD79b/CLEC5A myeloid engagers can more efficiently kill CD79b+ cancerous Ramos cells.

**Table 19. Different myeloid cell engagers mediated Ramos cell killing by M0 macrophages**

| Donor | Antibodies | IC50 (nM) |
|---|---|---|
| Donor 100 | CD79b/5C7 (2+2 A) Fc-optimized | 0.002 |
| | CD79b/5C7 (2+2 B) Fc-silenced | 0.0008 |
| | CD79b/5C7 (2+1 A) Fc-silenced | 0.002 |
| | CD79b/5C7 (1+1 B) Fc-silenced | 0.006 |
| Donor 233 | CD79b/5C7 (2+2 A) Fc-optimized | 0.002 |
| | CD79b/5C7 (2+2 B) Fc-silenced | 0.002 |
| | CD79b/5C7 (1+1 B) Fc-silenced | 0.003 |
| Donor 022 | CD79b/5C7 (2+2 B) Fc-silenced | 0.002 |
| | CD79b/5C7 (2+2 B) Fc-optimized | 0.012 |
| | CD79b/5C7 (2+1 A) Fc-silenced | 0.003 |
| | CD79b/5C7 (2+1 A) Fc-optimized | 0.002 |
| | CD79b/5C7 (2+1 B) Fc-silenced | 0.003 |
| | CD79b/5C7 (2+1 B) Fc-optimized | 0.003 |
| Donor 431 | CD79b/5C7 (2+2 B) Fc-silenced | 00014 |
| | CD79b/5C7 (2+2 B) Fc-optimized | 0.002 |
| | CD79b/5C7 (2+1 B) Fc-silenced | 0.002 |
| | CD79b/5C7 (2+1 B) Fc-optimized | 0.004 |
| | CD79b/5C7 (1+1 B) Fc-silenced | 0.004 |
| | CD79b/5C7 (1+1 B) Fc-optimized | 0.003 |
| Donor 159 | CD79b/5C7 (2+2 A) Fc-silenced | 0.002 |
| | CD79b/5C7 (2+2 A) Fc-optimized | 0.001 |
| | CD79b/5C7 (2+2 B) Fc-silenced | 0.001 |
| | CD79b/5C7 (2+2 B) Fc-optimized | 0.001 |
| Donor 161 | CD79b/5C7 (2+2 A) Fc-silenced | 0.0006 |
| | CD79b/5C7 (2+2 A) Fc-optimized | 0.0013 |
| | CD79b/5C7 (2+2 B) Fc-silenced | 0.0008 |
| | CD79b/5C7 (2+2 B) Fc-optimized | 0.001 |

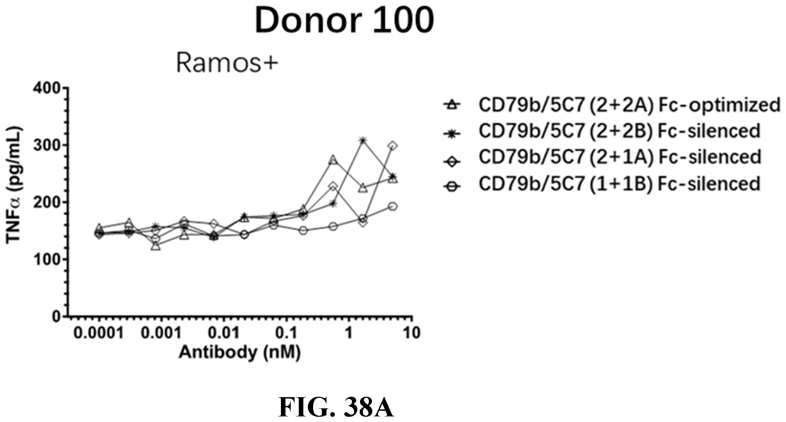

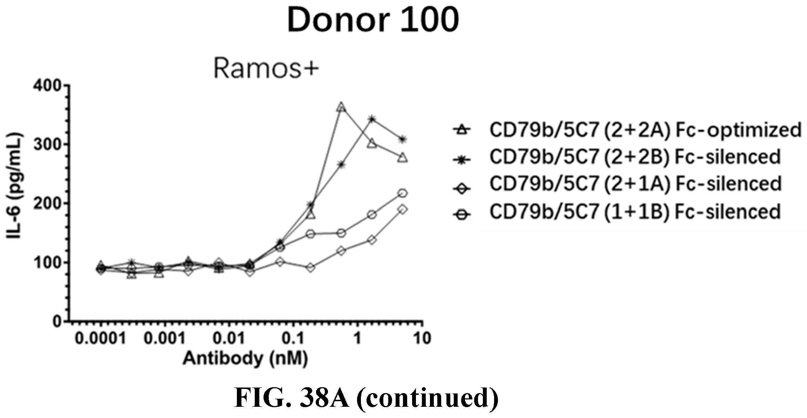

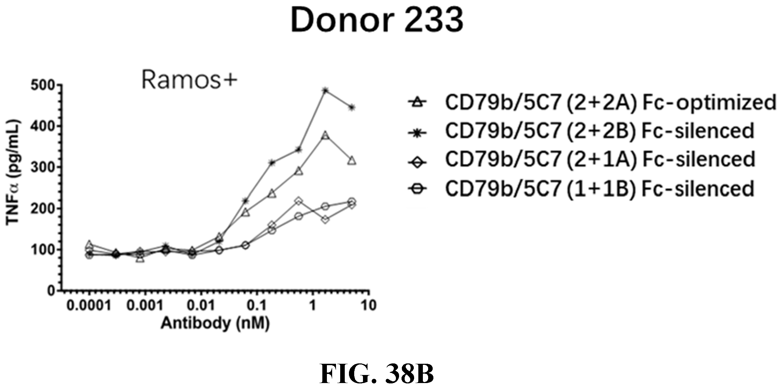

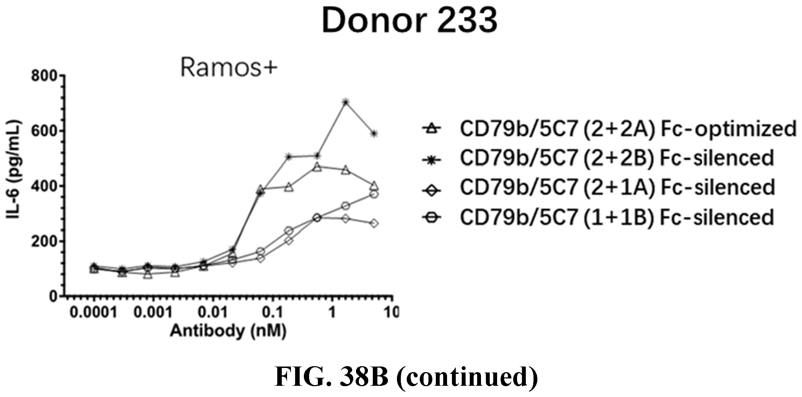

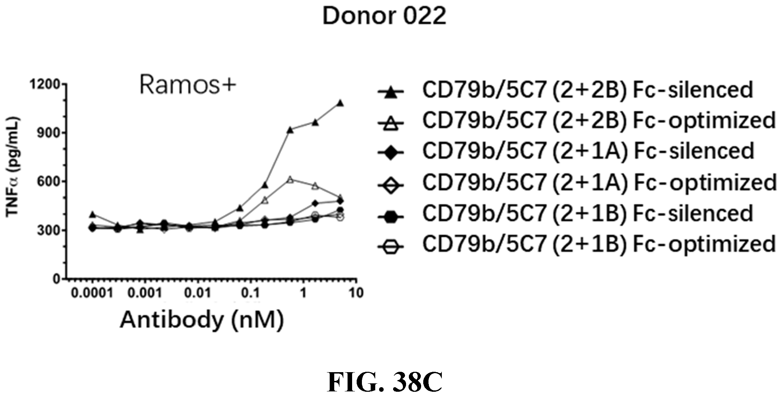

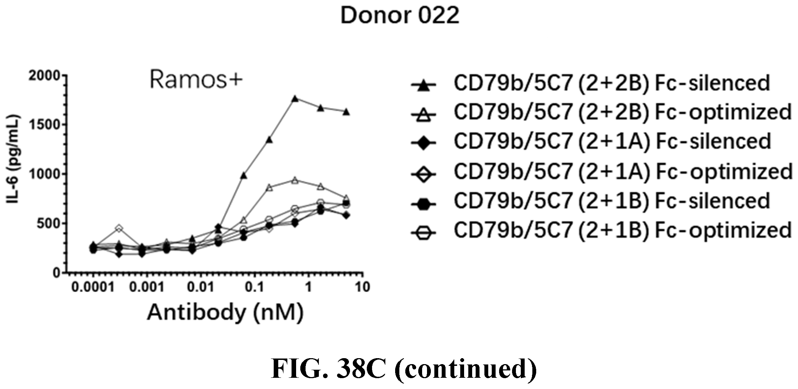

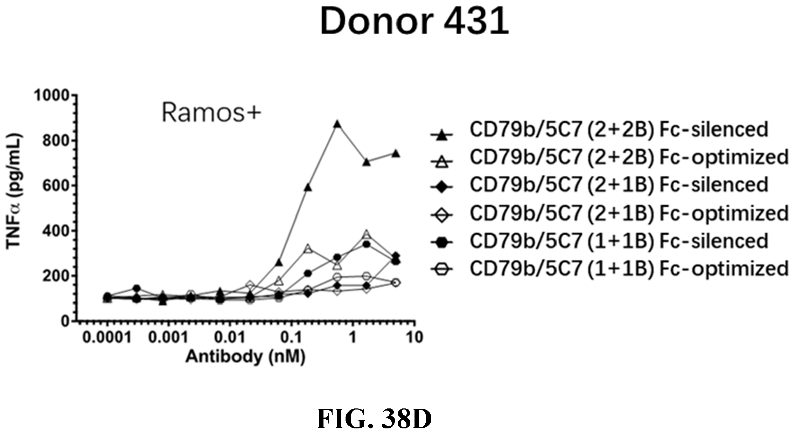

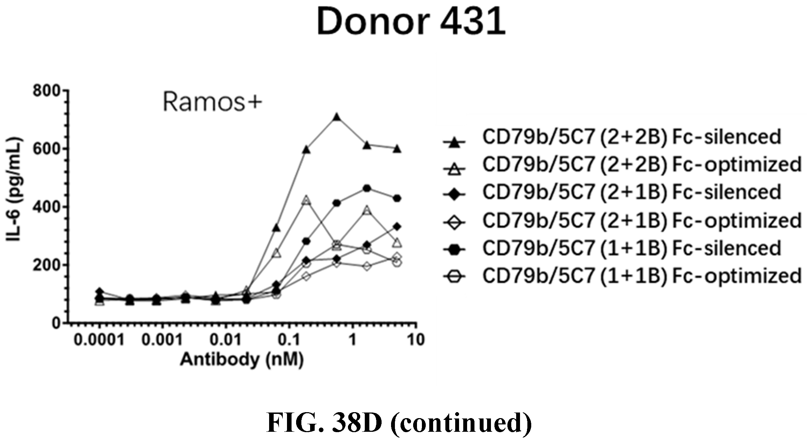

The cytokine release of CD79b/CLEC5A bispecific antibodies with different structures in the presence or absence of target Ramos cells were evaluated. CD14+ monocytes (purified from human PBMC donors #100, #233, #022 and #431 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 7 days. About 100,000 macrophages were incubated with 20,000 Ramos cells (E:T ratio of 5:1) in the presence of serially diluted antibodies in complete RPMI media (with 10% heat-inactivated FBS and 5% penicillin/streptomycin) for 24 hours at 37°C. After incubation, the supernatant was collected for cytokine analysis. TNFα and IL-6 levels in supernatants were measured using ELISA kits (R&D Systems, Cat#: DY206 and DY210, respectively) according to manufacturer's instructions. As shown in **FIGs. 38A-38D**, both IL-6 and TNFα were released from the macrophages only in the presence of target Ramos cells and CD79b/CLEC5A antibodies in a dose-dependent manner and at very low levels, suggesting that the myeloid engagers are safe for clinical use.

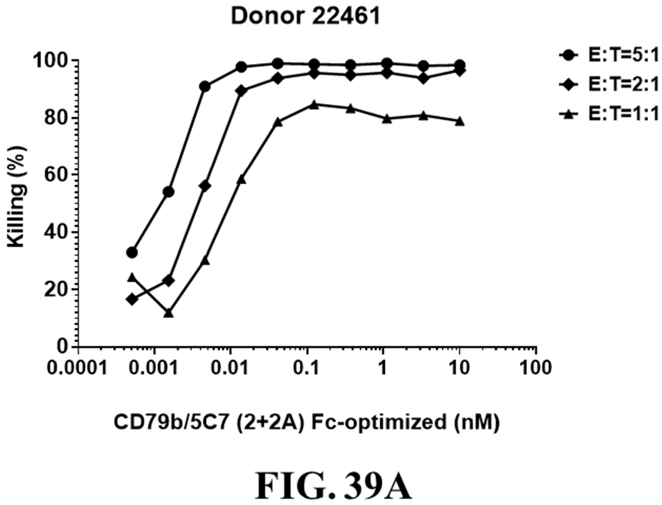

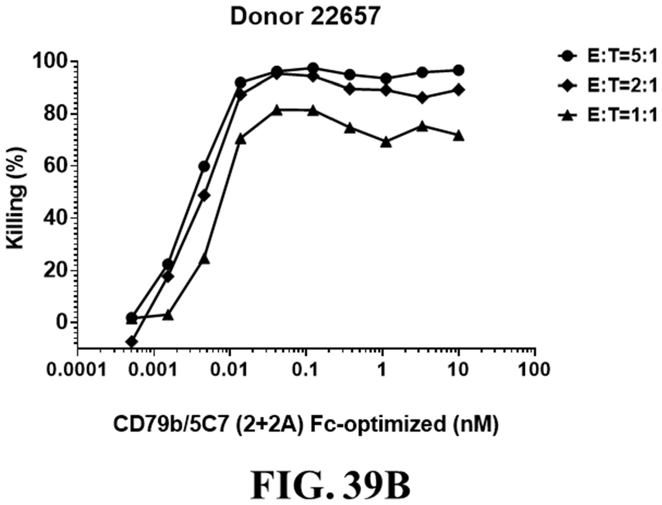

To estimate cancerous B cell (Ramos) killing efficacy by M0 macrophages at different E:T ratios, human monocytes were isolated from PBMCs (donors #22461 and #22657) by EasySep^{™} Human Monocyte Enrichment Kit without CD16 Depletion (StemCell Technologies, Cat#: 19058). Isolated monocytes were induced by 50 ng/mL M-CSF (StemCell Technologies, Cat#: 78057). After 7 days, monocyte-differentiated macrophages (M0) were obtained. Different E:T ratios of M0 macrophages and Ramos cells were incubated with the myeloid cell engagers to test their functions in M0 macrophages. The myeloid cell engager CD79b/CLEC5A bispecific antibody was serially diluted. Ramos cells were stained with CFSE first, and then spiked into M0 macrophages (E:T=5:1, E:T=2:1, or E:T=1:1) in a 96-well-round-bottom plate (Fixed Ramos counts: 2 × 10⁴/well), which were further incubated with serially diluted antibodies (1 to 3 dilutions from 10 nM). After 24 hours of incubation, the plate were spun down, and supernatant were stored at -80°C. Cells were stained by live/dead staining dye, and then processed on a CytoFLEX LX flow cytometer. Percentage of cell killing was calculated as: (live tumor cell number in non-treatment group-live tumor cell number in treatment group)/live tumor cell number in non-treatment group × 100. As shown in **FIGs. 39A-39B**, the myeloid engagers showed potent Ramos cell killing effects at different E:T ratios, even at low E:T ratios (e.g., E:T=1:1) in vitro. The maximum specific tumor cell killing was around 99% (E:T=5:1), 95% (E:T=2:1), 80% (E:T=1:1) for Ramos at 24 hours, indicating that myeloid cell engagers can promote target cell killing efficiently at low E: T ratios of M0 macrophages to target cells.

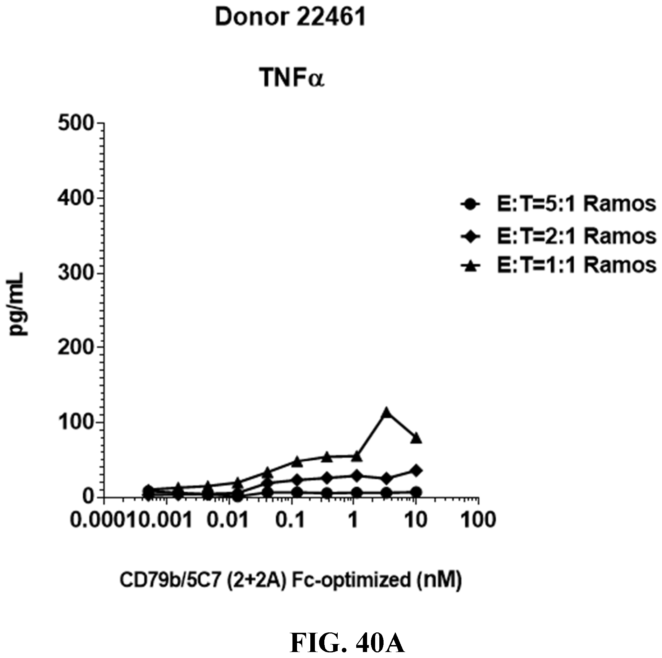

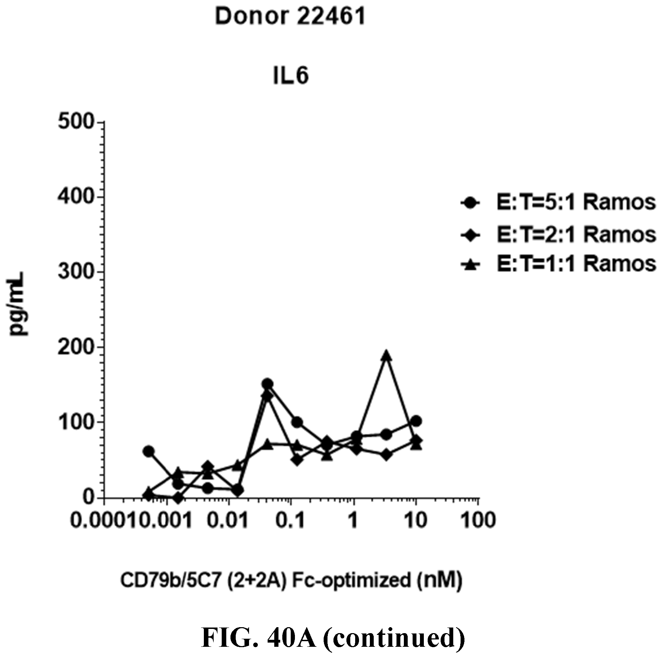

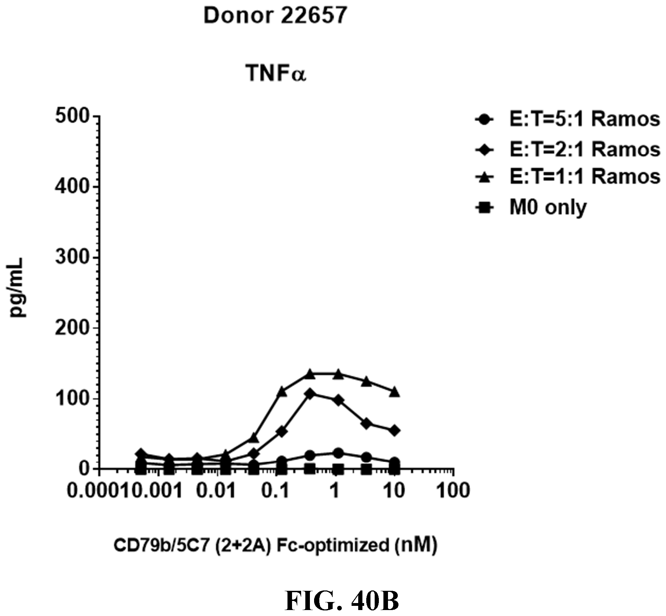

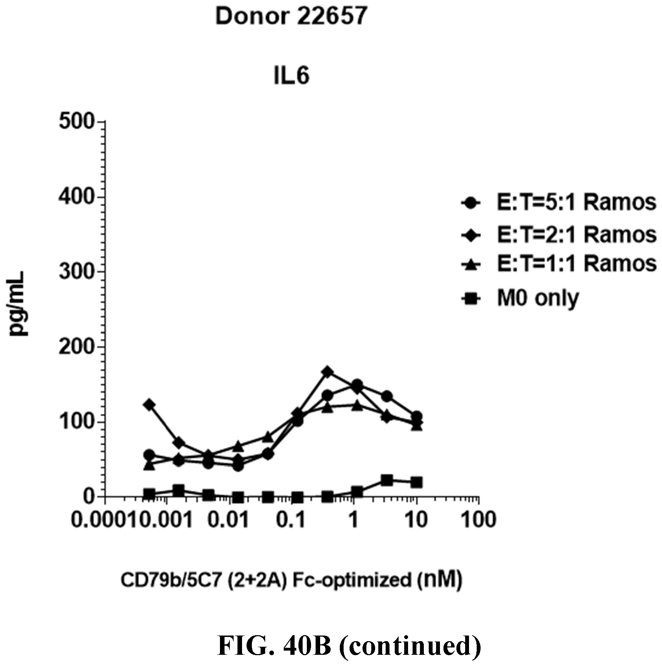

To estimate cytokine release of cancerous B cell (Ramos) killing of M0 macrophages at different E:T ratios, human monocytes were isolated from PBMCs (donors #22461 and #22657) EasySep^{™} Human Monocyte Enrichment Kit without CD16 Depletion (StemCell Technologies, Cat#: 19058). Isolated monocytes were induced by 50 ng/mL M-CSF (StemCell Technologies, Cat#: 78057 ). After 7 days, monocyte-differentiated macrophages (M0) were obtained. Different E:T ratios of M0 macrophages and Ramos cells were incubated with the myeloid cell engagers to test their functions in M0 macrophages. The myeloid cell engager CD79b/CLEC5A bispecific antibody was serially diluted. Ramos cells were stained with CFSE first, and then spiked into M0 macrophages (E:T=5:1, E:T=2:1, or E:T=1:1) in a 96-well-round-bottom plate (Fixed Ramos counts: 2 × 10⁴/well), which were further incubated with serial diluted CD79b/CLEC5A bispecific antibody (1 to 3 dilutions from 10 nM). After 24 hours of incubation, the plate was spun down, and supernatant was recovered for ELISA analysis. ELISA was performed according to instructions of ELISA MAX^{™} Deluxe Set Human TNF-α (BioLegend, Cat#: 430204) and ELISA MAX^{™} Deluxe Set Human IL-6 (BioLegend, Cat#: 430504) kits. Absorbance at 450 nm was measured within 15 minutes by a SpectraMax^{®} Absorbance Reader (MOLECULAR DEVICEs, S/N3052431867). Without target cells (Ramos) spiked in, the myeloid engagers mediated no TNFα or IL-6 release in vitro. As shown in **FIGs. 40A-40B**, with target cells (Ramos) spiked in, the myeloid engagers showed weak TNFα and IL-6 release at different E:T ratios in vitro. The maximum TNFα release was less than 150 pg/mL, whereas the maximum IL-6 release was less than 200 pg/mL at 24 hours, indicating that myeloid cell engagers can promote target cell killing efficiently, but induce very weak cytokine release of M0 macrophages to target cells.

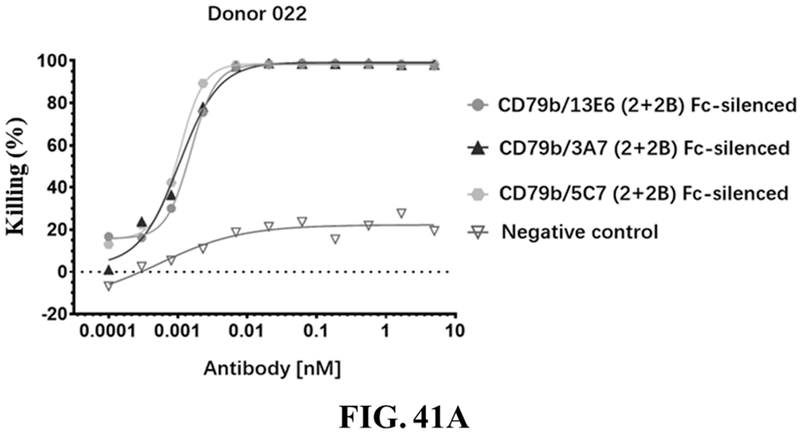

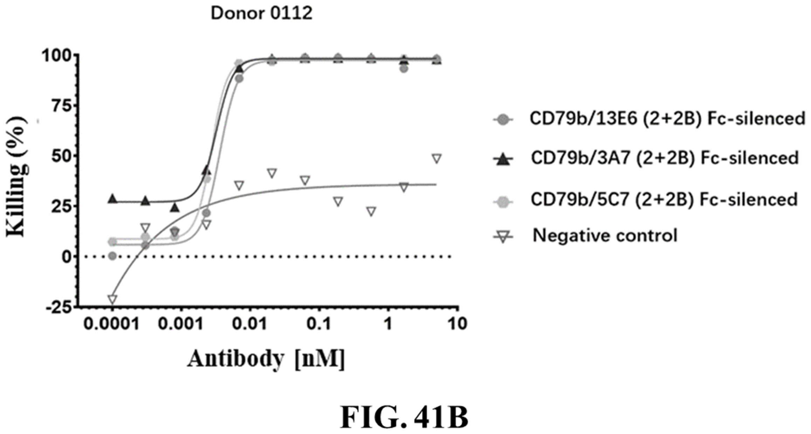

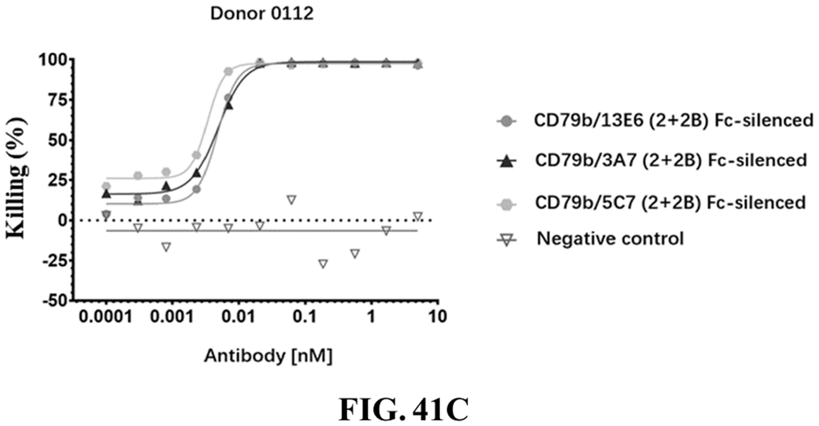

The effects of CD79b/CLEC5A bispecific antibodies with different structures to kill target cancer Ramos cells (CD79b+) by effector macrophages (CLEC5A+) were evaluated. CD14+ monocytes (purified from human PBMC donors #022 and #0112 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 7 days. Ramos cells were labeled with CFSE (ThermoFisher, Cat#: C34554). About 100,000 macrophages were incubated with 20,000 CFSE+ Ramos (E:T ratio of 5:1) in the presence of serially diluted antibodies in complete RPMI media (with 10% heat-inactivated FBS and 5% penicillin/streptomycin) for 24 hours at 37°C. About 80,000 macrophages were incubated with 40,000 CFSE+ Ramos (E:T ratio of 2:1) in the presence of serially diluted antibodies in complete RPMI media (with 10% heat-inactivated FBS and 5% penicillin/streptomycin) for 24 hours at 37°C. About 60,000 macrophages were incubated with 60,000 CFSE+ Ramos (E:T ratio of 1:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. Ramos cells were gated as CFSE+ by FACS, and the absolute cell count of CFSE+ cells was obtained by collecting a fixed volume for all treatment conditions. Percentage of target cell killing was calculated as: (absolute number of CFSE+ Ramos cell number in non-treatment group - absolute number of CFSE+ SYTOX- Ramos cell number in treatment group)/absolute number of CFSE+ Ramos cell number in non-treatment group × 100. As shown in **FIGs. 41A-41C**, all the myeloid engagers can efficiently kill CD79b+ cancerous Ramos cells.

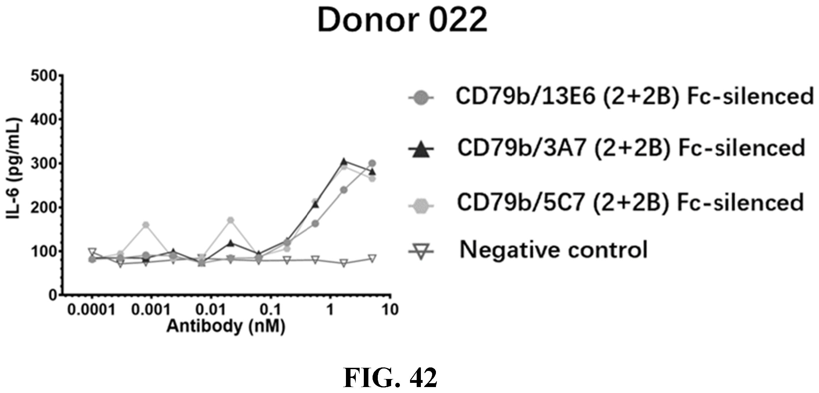

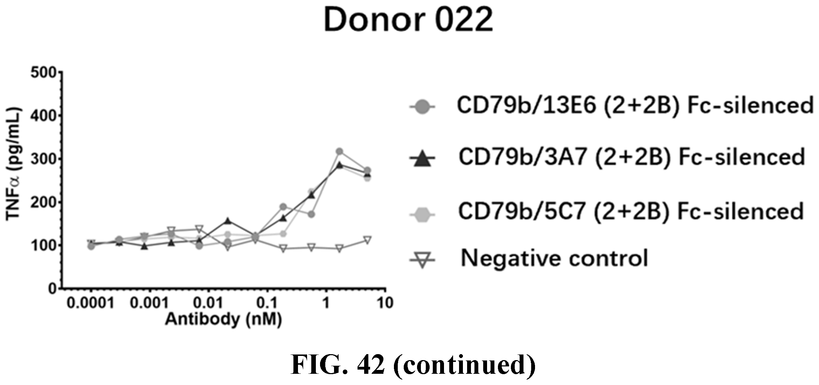

The cytokine release of CD79b/CLEC5A bispecific antibodies with different structures were evaluated. After incubation, the supernatant was collected for cytokine analysis. TNFα and IL-6 levels in supernatants were measured using ELISA kits (R&D Systems, Cat#: DY206 and DY210, respectively) according to manufacturer's instructions. As shown in **FIG. 42**, both IL-6 and TNFα were at very low levels, suggesting that the myeloid engagers are safe for clinical use.

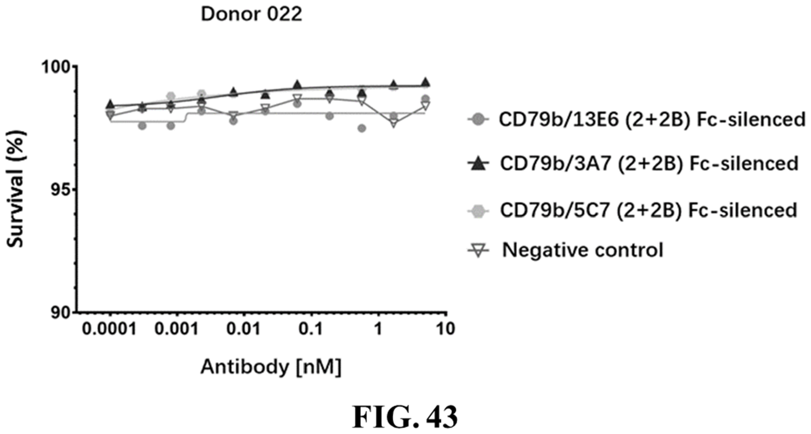

To find out whether the activated M0 macrophages further cause self-killing to reduce its numbers, we analyzed M0 macrophages numbers to test their status. Ramos cells ran through dead cell removal then stained with 0.25 uM CSFE. The number of M0 macrophages was shown in **FIG. 43**, there were no significant changes of macrophages counts.

### Example 33. CD79b/CLEC5A bispecific antibodies mediated Daudi cell killing by M0 macrophages

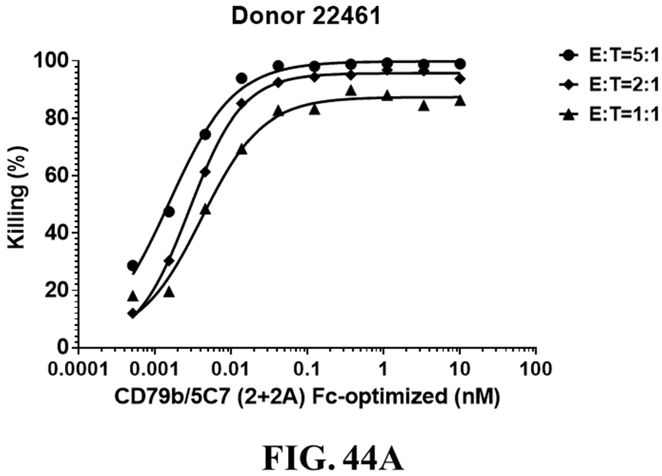

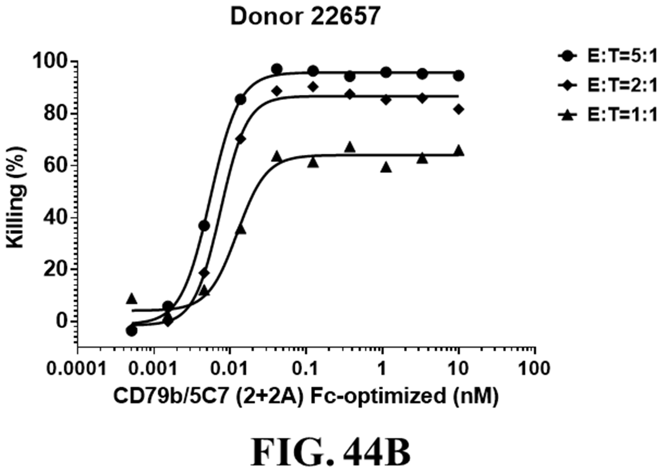

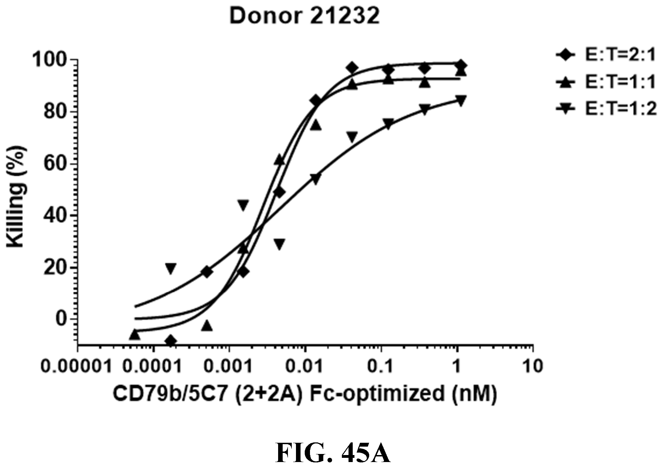

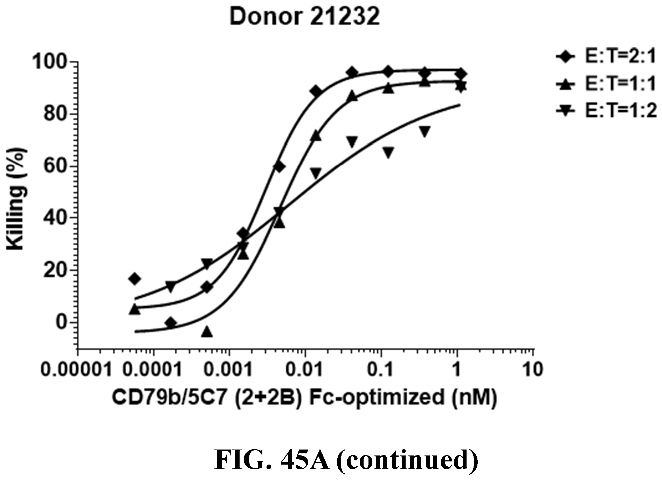

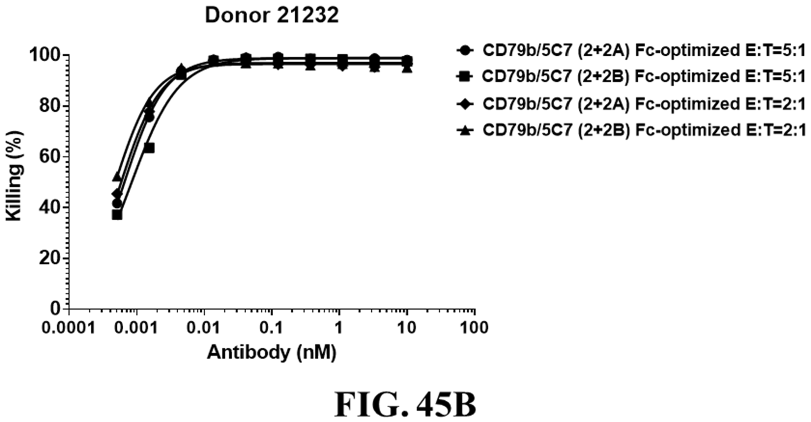

To estimate cancerous B cell (Daudi) killing efficacy of myeloid cell engager CD79b/CLEC5A bispecific antibodies in M0 macrophages at different E:T ratios, human monocytes were isolated from PBMCs (donors #21232, #22657 and #22461) by EasySep^{™} Human Monocyte Enrichment Kit without CD16 Depletion (StemCell Technologies, Cat#: 19058). Isolated monocytes were induced by 50 ng/mL M-CSF (StemCell Technologies, Cat#: 78057). On Day 7, M0 macrophages were obtained. Different E:T ratios of M0 macrophages and Daudi cells were incubated with the myeloid cell engagers to test their functions in M0 macrophages. The myeloid cell engagers (CD79b/CLEC5A bispecific antibodies) were serially diluted. Daudi cells were stained with CFSE first, then spiked into M0 macrophages (E:T=5:1, E:T=2:1, E:T=1:1, or E:T=1:2) in a 96-well-round-bottom plate (Fixed Daudi counts: 2 × 10⁴/well), which were further incubated with serially diluted CD79b/CLEC5A bispecific antibodies (1 to 3 dilutions from 10 nM). After 24 hours of incubation, the plate was spun down, and supernatant was stored at -80°C. Cells were stained with live/dead staining dye, and then processed on a CytoFLEX LX flow cytometer. Percentage of cell killing was calculated as: (live tumor cell number in non-treatment group-live tumor cell number in treatment group)/live tumor cell number in non-treatment group × 100. As shown in **FIGs. 44A-44B**, the myeloid engagers showed potent Daudi cell killing effects in M0 macrophages in vitro at different E:T ratios. The maximum specific tumor cell killing was around 97%-99% at E:T=5:1, 90%-96% at E:T=2:1, and 60%-89% at E:T=1:1 for Daudi cells at 24 hours, indicating that myeloid cell engagers can promote target cell killing efficiently at low E: T ratios of M0 macrophages to target cells. As shown in **FIGs. 45A-45B**, the myeloid engagers showed potent and comparable Daudi cell killing effects in M0 macrophages in vitro at different E:T ratios. The maximum specific tumor cell killing was around 99% (E:T=5:1), 96% (E:T=2:1), 92% (E:T=1:1), and 90% (E:T=1:2) for Daudi cells at 24 hours, indicating that myeloid cell engagers can promote target cell killing efficiently and comparably at low E:T ratios of M0 macrophages to target cells.

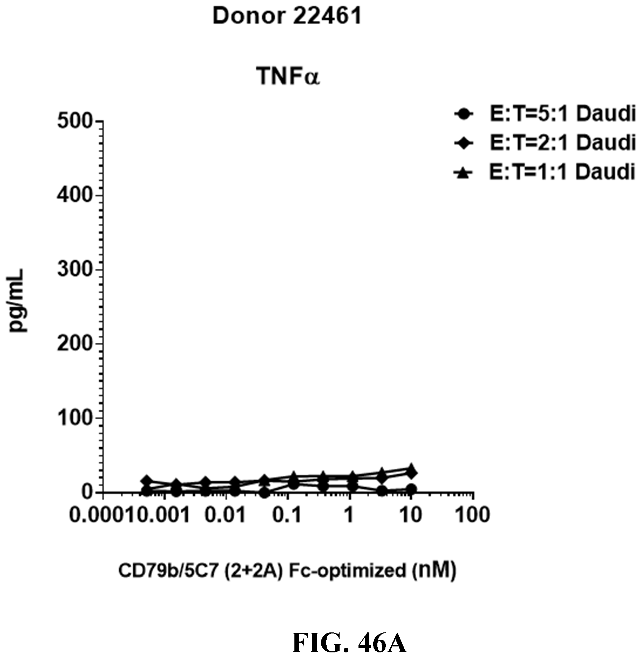

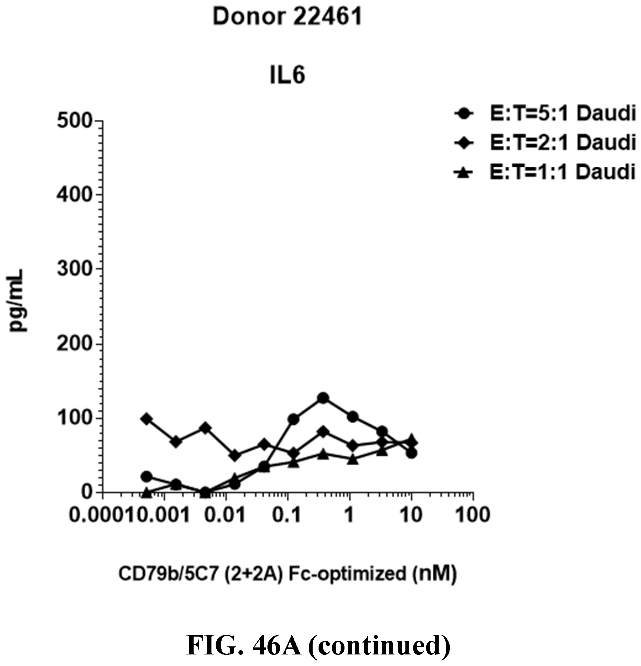

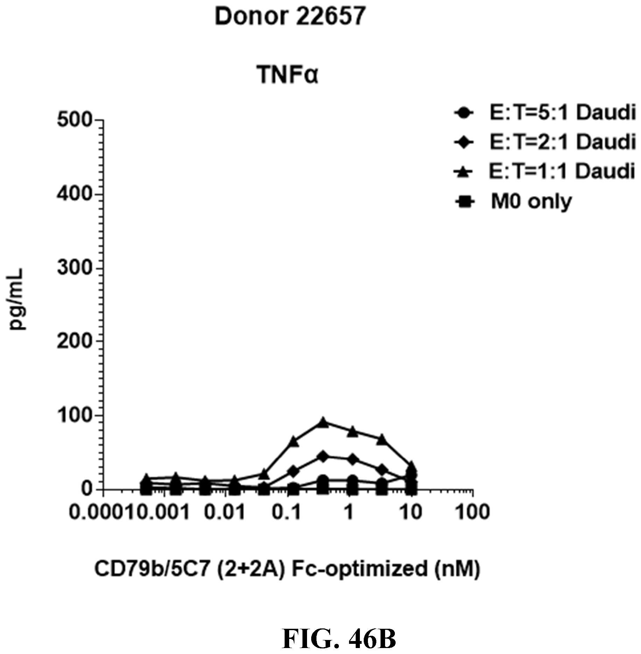

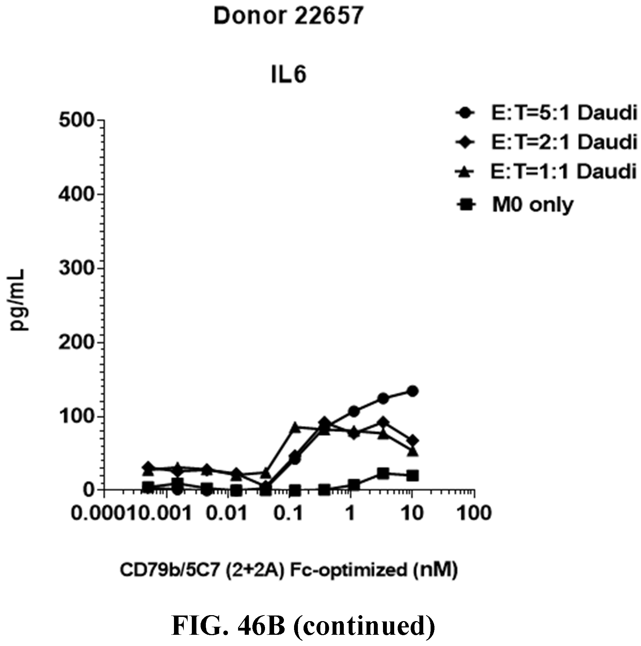

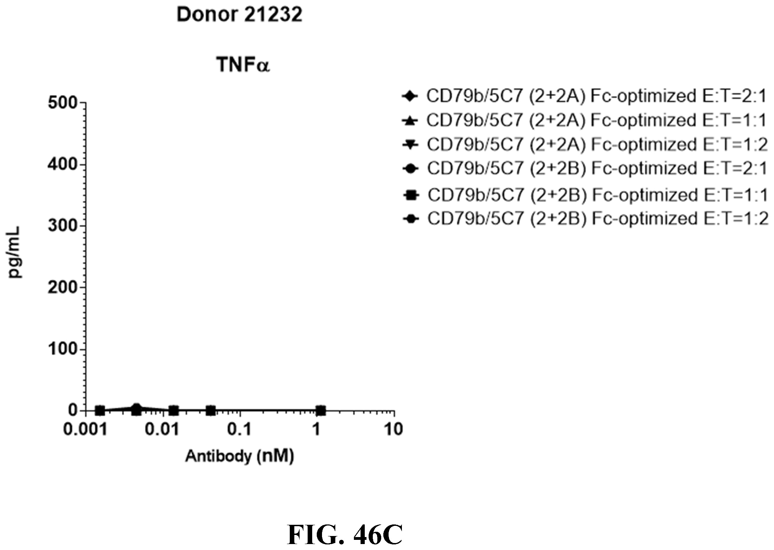

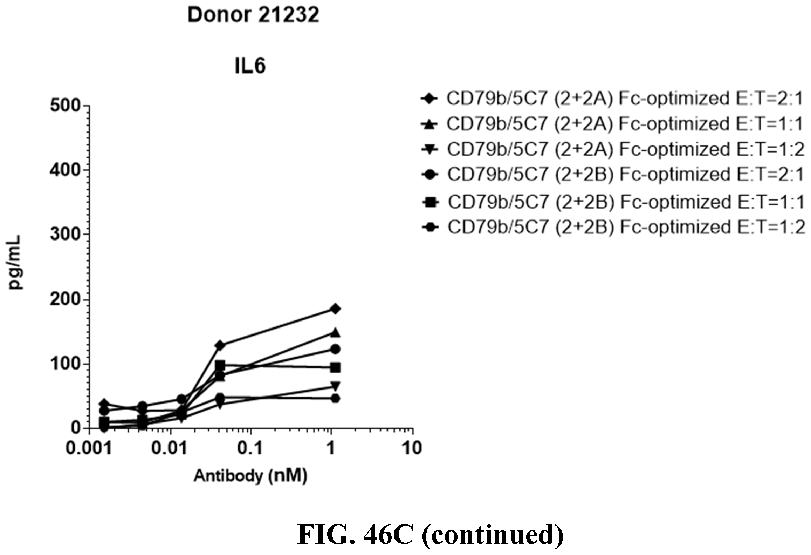

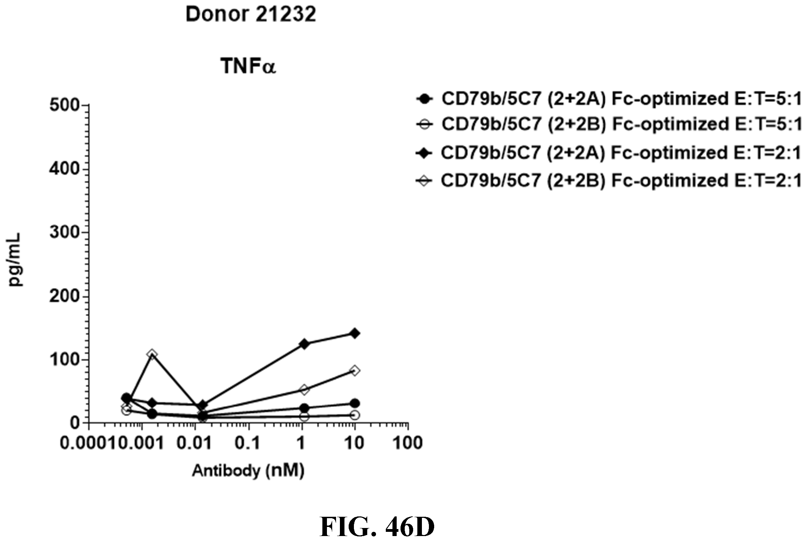

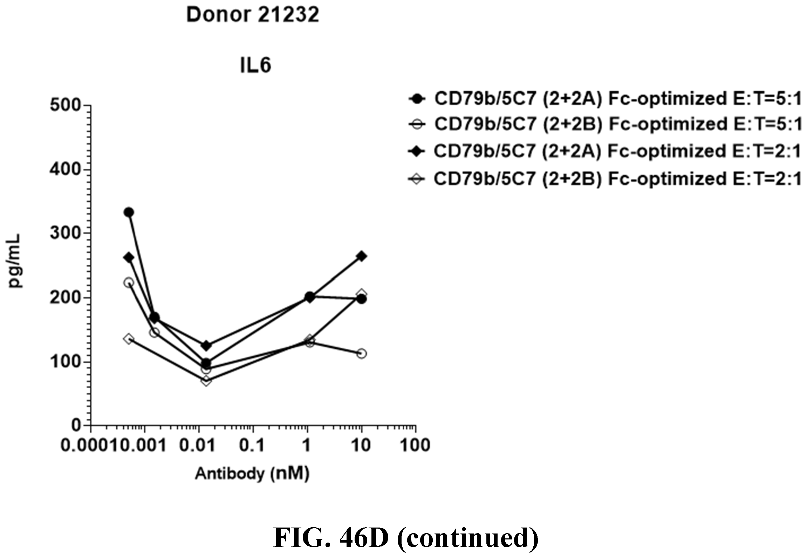

To estimate cytokine release of cancerous B cell (Daudi) killing of myeloid cell engager CD79b/CLEC5A bispecific antibodies in M0 macrophages at different E:T ratios, human monocytes were isolated from PBMCs (donors #21232, #22657 and #22461) by EasySep^{™} Human Monocyte Enrichment Kit without CD16 Depletion (StemCell Technologies, Cat#: 19058). Isolated monocytes were induced by 50 ng/mL M-CSF (StemCell Technologies, Cat#: 78057). On Day 7, M0 macrophages were obtained. Different E:T ratios of M0 macrophages and Daudi cells were incubated with the myeloid cell engager CD79b/CLEC5A bispecific antibodies to test their functions in M0 macrophages. The myeloid cell engagers (CD79b/CLEC5A bispecific antibodies) were serially diluted. Daudi cells were stained with CFSE first, and then spiked into M0 macrophages (E:T=5:1, E:T=2:1, E:T=1:1, or E:T=1:2) in a 96-well-round-bottom plate (Fixed Daudi counts: 2 × 10⁴/well), which were further incubated with serially diluted CD79b/CLEC5A bispecific antibodies (1 to 3 dilutions from 10 nM). After 24 hours of incubation, the plate was spun down, and supernatant was recovered for ELISA analysis. ELISA was performed according to instructions of ELISA MAX^{™} Deluxe Set Human TNF-α (BioLegend, Cat#: 430204) and ELISA MAX^{™} Deluxe Set Human IL-6 (BioLegend, Cat#: 430504) kits. Absorbance at 450 nm was measured within 15 minutes by a SpectraMax^{®} Absorbance Reader (MOLECULAR DEVICEs, S/N3052431867). Without target cells (Daudi) spiked in, the myeloid engagers (CD79b/CLEC5A bispecific antibodies) showed no TNFα or IL-6 release in vitro. As shown in **FIGs. 46A-46D**, with target cells (Daudi) spiked in, the myeloid engagers showed less TNFα and IL-6 release in vitro. The maximum TNFα release was less than 150 pg/mL, whereas the maximum IL-6 release was less than 200 pg/mL at 24 hours, indicating that myeloid cell engagers can promote target cell killing efficiently and induce very weak cytokine release of M0 macrophages to target cells.

### Example 34. CD79b/CLEC5A bispecific antibodies mediated Ramos cell killing by M1 macrophages

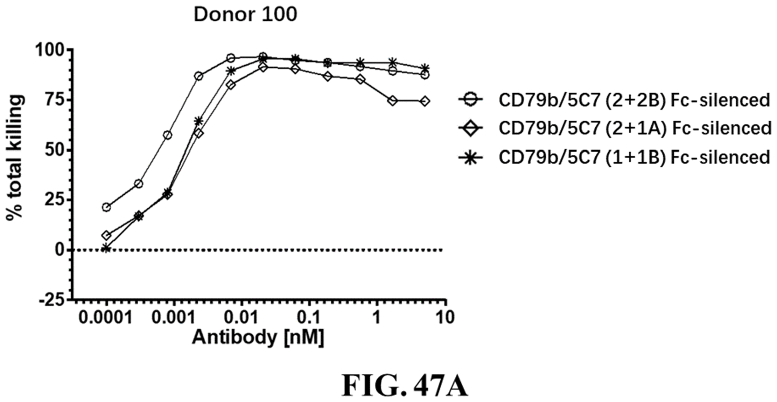

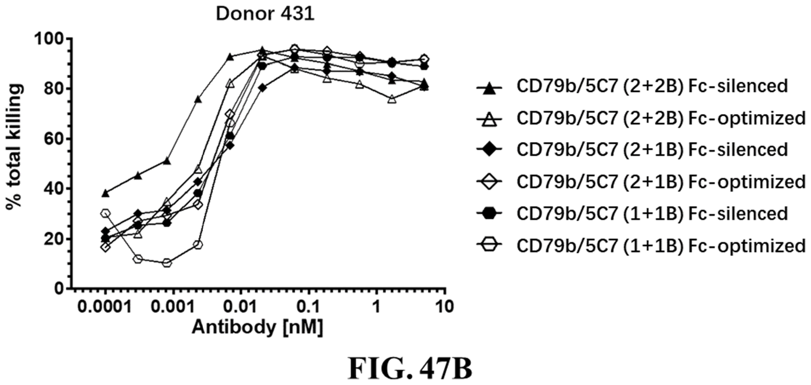

The effects of CD79b/CLEC5A bispecific antibodies with different structures to kill target cancer Ramos cells (CD79b+) by effector macrophages (CLEC5A+) were evaluated. CD14+ monocytes (purified from human PBMC (donors #100 and #431) with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 6 days. On Day 6, M0 macrophages were polarized with 50 ng/mL of IFN-γ (StemCell Technologies, Cat#: 78020) to M1 macrophages for another 24 hours (Day 7). Ramos cells were labeled with CFSE (ThermoFisher, Cat#: C34554). About 100,000 macrophages were incubated with 20,000 CFSE+ Ramos (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. Ramos cells were gated as CFSE+ by FACS, and the absolute cell count of CFSE+ cells was obtained by collecting a fixed volume for all treatment conditions. Percentage of target cell killing was calculated as: (absolute number of CFSE+ Ramos cell number in non-treatment group - absolute number of CFSE+ SYTOX-Ramos cell number in treatment group)/absolute number of CFSE+ Ramos cell number in non-treatment group × 100. As shown in **FIGs. 47A-47B** and the table below, all the myeloid engagers can efficiently kill CD79b+ cancerous Ramos cells.

**Table 20. CD79b/CLEC5A bispecific antibodies mediated Ramos cell killing by M1 macrophages**

| Donor | Antibodies | IC50 (nM) |
|---|---|---|
| Donor 100 | CD79b/5C7 (2+2 B) Fc-silenced | 0.0007 |
| | CD79b/5C7 (2+1 A) Fc-silenced | 0.001 |
| | CD79b/5C7 (1+1 B) Fc-silenced | 0.001 |
| Donor 431 | CD79b/5C7 (2+2 B) Fc-silenced | 0.0014 |
| | CD79b/5C7 (2+2 B) Fc-optimized | 0.002 |
| | CD79b/5C7 (2+1 B) Fc-silenced | 0.005 |
| | CD79b/5C7 (2+1 B) Fc-optimized | 0.005 |
| | CD79b/5C7 (1+1 B) Fc-silenced | 0.005 |
| | CD79b/5C7 (1+1 B) Fc-optimized | 0.006 |

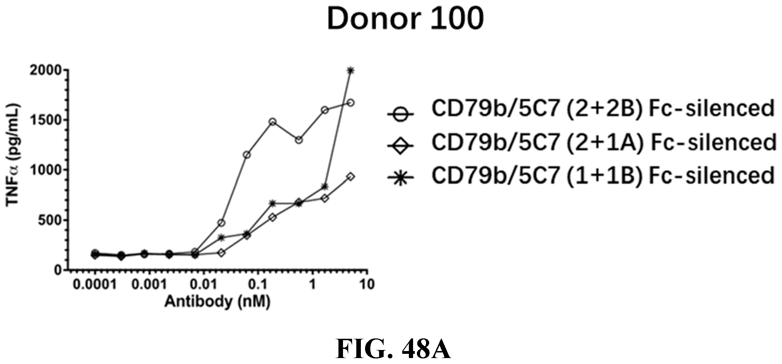

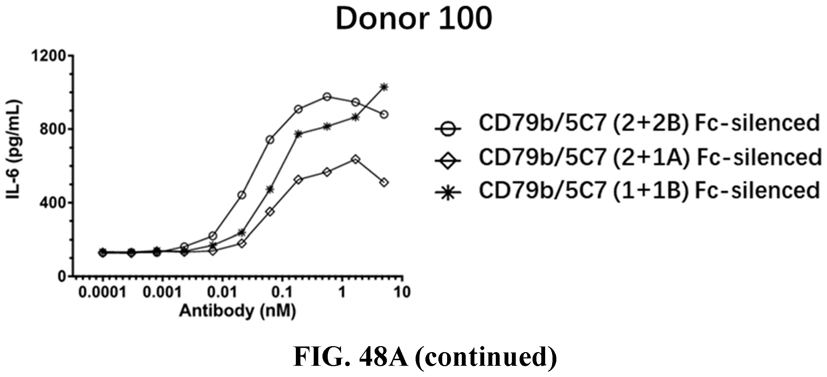

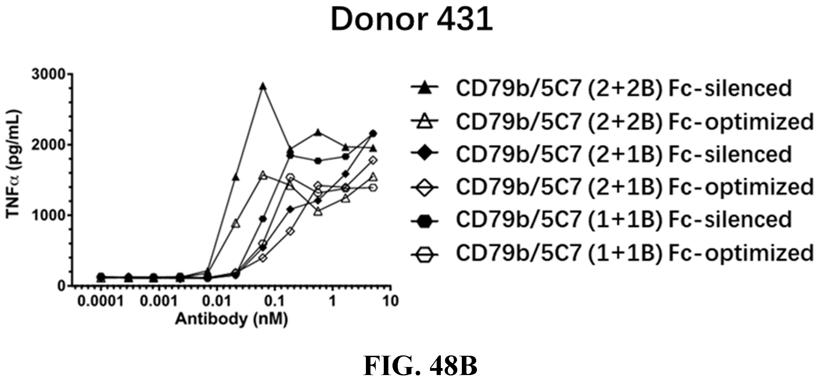

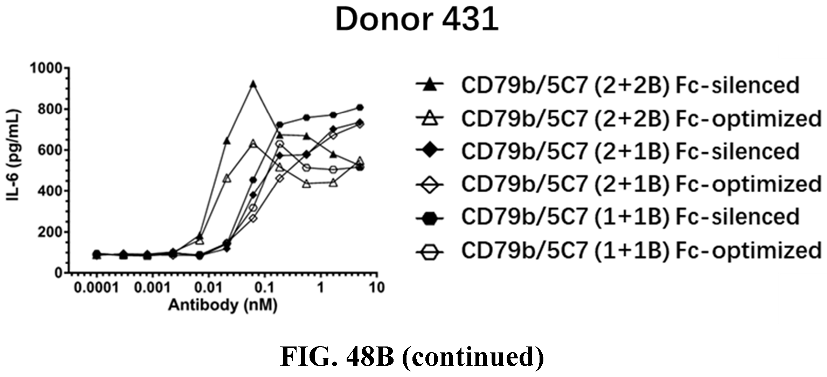

The cytokine release of CD79b/CLEC5A bispecific antibodies with different structures in the presence or absence of target Ramos cells were evaluated. CD14+ monocytes (purified from human PBMC (donors #100 and #431) with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 6 days. On Day 6, M0 macrophages were polarized with 50 ng/mL of IFN-γ (StemCell Technologies, Cat#: 78020) to M1 macrophages for another 24 hours (Day 7). About 100,000 macrophages were incubated with 20,000 Ramos cells (E:T ratio of 5:1) in the presence of serially diluted antibodies in complete RPMI media (with 10% heat-inactivated FBS and 5% penicillin/streptomycin) for 24 hours at 37°C. After incubation, the supernatant was collected for cytokine analysis. TNFα and IL-6 levels in supernatants were measured using ELISA kits (R&D Systems, Cat#: DY206 and DY210, respectively) according to manufacturer's instructions. As shown in **FIGs. 48A-48B**, both IL-6 and TNFα were released from the macrophages only in the presence of target Ramos and CD79b/CLEC5A bispecific antibodies in a dose-dependent manner and at low levels, suggesting that myeloid engagers are safe for clinical use.

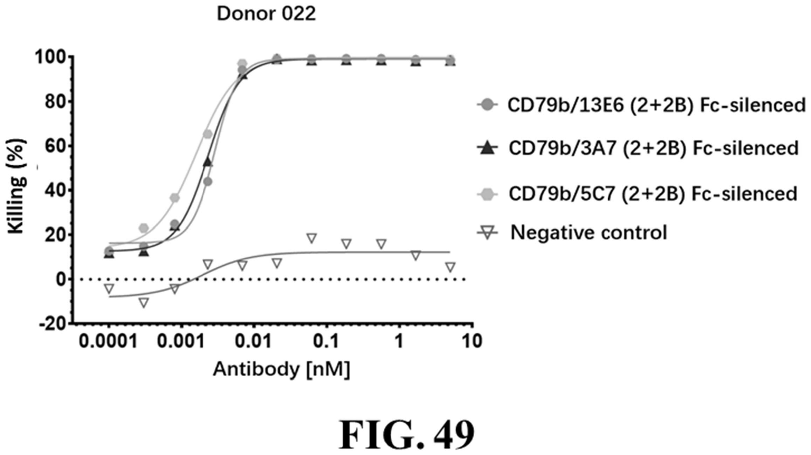

The effects of CD79b/CLEC5A bispecific antibodies with different structures to kill target cancer Ramos cells (CD79b+) by effector macrophages (CLEC5A+) were evaluated. CD14+ monocytes (purified from human PBMC (donors #022) with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 6 days. On Day 6, M0 macrophages were polarized with 50 ng/mL of IFN-γ (StemCell Technologies, Cat#: 78020) to M1 macrophages for another 24 hours (Day 7). Ramos cells were labeled with CFSE (ThermoFisher, Cat#: C34554). About 100,000 macrophages were incubated with 20,000 CFSE+ Ramos (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. Ramos cells were gated as CFSE+ by FACS, and the absolute cell count of CFSE+ cells was obtained by collecting a fixed volume for all treatment conditions. Percentage of target cell killing was calculated as: (absolute number of CFSE+ Ramos cell number in non-treatment group - absolute number of CFSE+ SYTOX-Ramos cell number in treatment group)/absolute number of CFSE+ Ramos cell number in non-treatment group × 100. As shown in **FIG. 49****,** all the myeloid engagers can efficiently kill CD79b+ cancerous Ramos cells.

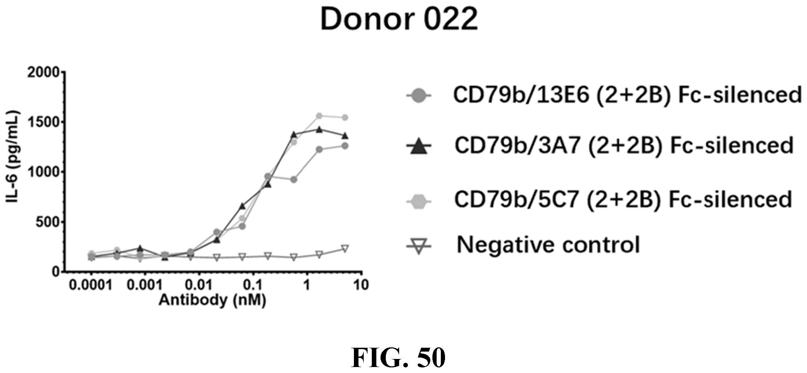

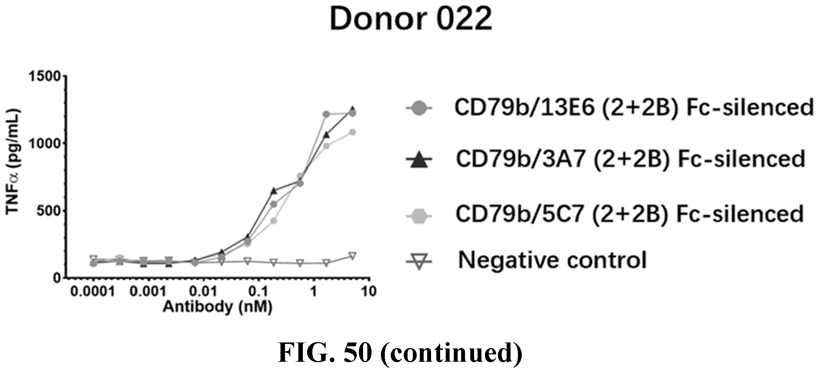

The cytokine release of CD79b/CLEC5A bispecific antibodies with different structures were evaluated. After incubation, the supernatant was collected for cytokine analysis. TNFα and IL-6 levels in supernatants were measured using ELISA kits (R&D Systems, Cat#: DY206 and DY210, respectively) according to manufacturer's instructions. The results were shown in **FIG. 50****.**

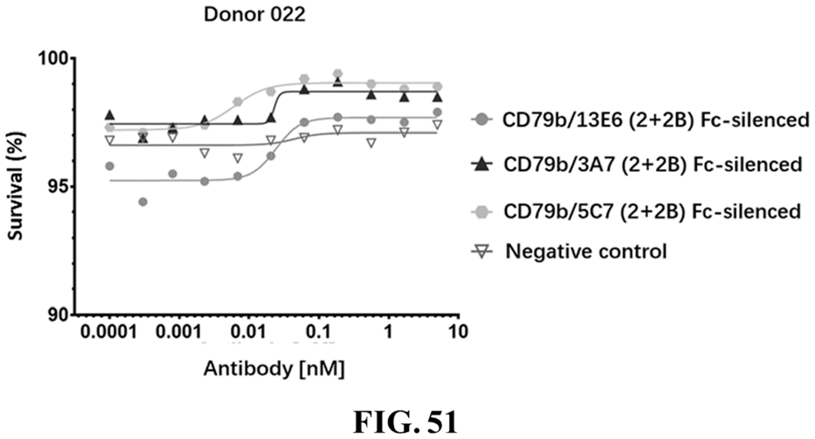

To find out whether the activated M1 macrophages further cause self-killing to reduce its numbers, we analyzed M1 macrophages numbers to test their status. Ramos cells ran through dead cell removal then stained with 0.25 uM CSFE. The number of macrophages was shown in **FIG. 51****,** there were no significant changes of M1 macrophages counts.

### Example 35. CD79b/CLEC5A bispecific antibodies mediated Daudi cell killing by M1 macrophages

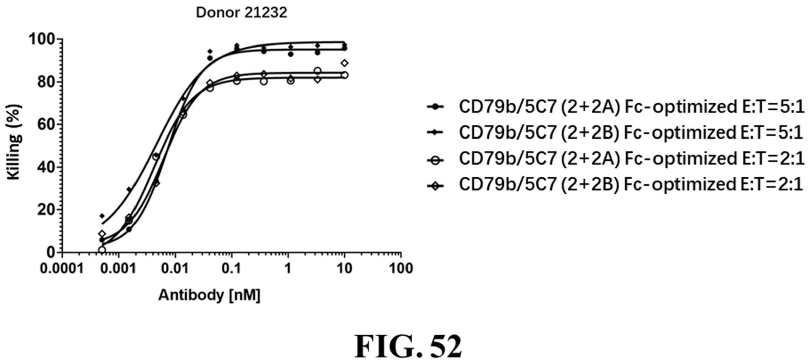

To estimate cancerous B cell (Daudi) killing efficacy of myeloid cell engager CD79b/CLEC5A bispecific antibodies in M1 macrophages at different E:T ratios, human monocytes were isolated from PBMCs by EasySep^{™} Human Monocyte Enrichment Kit without CD16 Depletion (StemCell Technologies, Cat#: 19058). Isolated monocytes were induced by 50 ng/mL M-CSF (StemCell, Cat#: 78057). On Day 6, complete media with 50 ng/mL M-CSF, and 50 ng/mL IFN-γ (StemCell, Cat#: 78020) were added. On Day 7, M1 macrophages were obtained. Different E:T ratios of M1 macrophages and Daudi cells were incubated with the myeloid cell engagers to test their functions in M1 macrophages. The myeloid cell engagers (CD79b/CLEC5A bispecific antibodies) were serially diluted. Daudi cells were stained with CFSE first, and then spiked into M1 macrophages (E:T=5:1 or E:T=2:1) in a 96-well-round-bottom plate (Fixed Daudi counts: 2 × 10⁴/well), which were further incubated with serially diluted CD79b/hCLEC5A bispecific antibodies (1 to 3 dilutions from 10 nM). After 24 hours of incubation, the plate was spun down, and supernatant was stored at -80°C. Cells were stained with live/dead staining dye, and then processed on a CytoFLEX LX flow cytometer. Percentage of cell killing was calculated as: (live tumor cell number in non-treatment group-live tumor cell number in treatment group)/live tumor cell number in non-treatment group × 100. As shown in FIG 52, the myeloid engagers showed potent Daudi cell killing effects at different E:T ratios, and the myeloid engagers showed comparable Daudi cell killing potency in M1 macrophages in vitro. The maximum specific tumor cell killing was around 96% (E:T=5:1) and 85% (E:T=2:1) for Daudi cells at 24 hours, indicating that myeloid cell engagers can promote target cell killing efficiently at different E:T ratios of M1 macrophages to target cells.

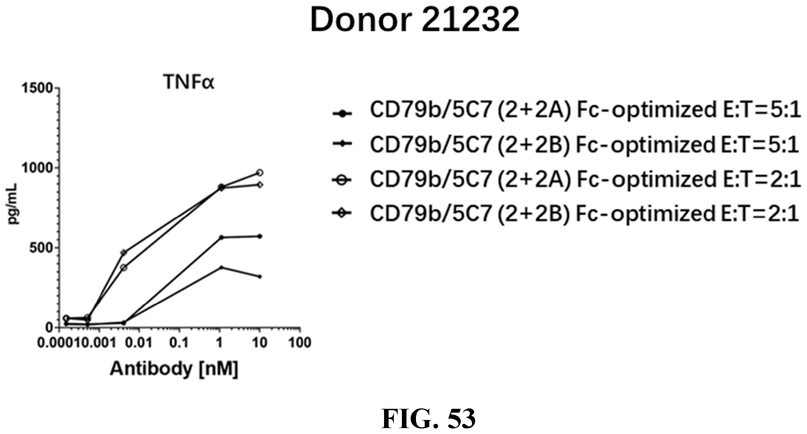

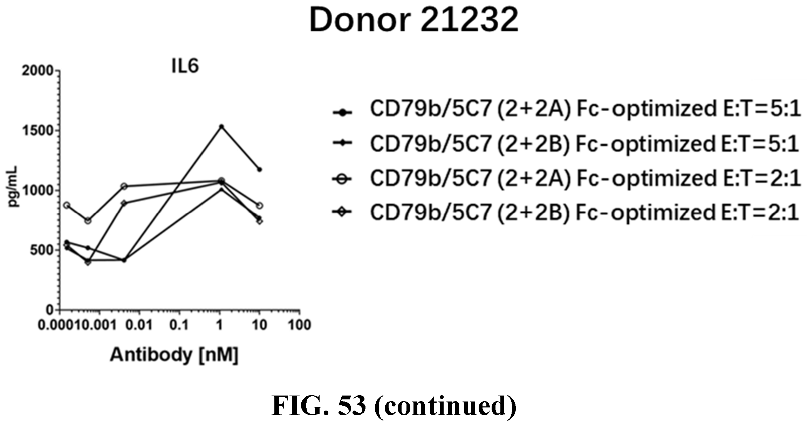

To estimate cytokine release of cancerous B cell (Daudi) killing of myeloid cell engager CD79b/CLEC5A bispecific antibodies in M1 at different E:T ratios, human monocytes were isolated from PBMCs by EasySep^{™} Human Monocyte Enrichment Kit without CD16 Depletion (StemCell Technologies, Cat#: 19058). Isolated monocytes were induced by 50 ng/mL M-CSF (StemCell Technologies, Cat#: 78057). On Day 6, complete media with 50 ng/mL M-CSF and 50 ng/mL IFN-γ (StemCell Technologies, Cat#: 78020). On Day 7, M1 macrophages were obtained. Different E:T ratios of M1 macrophages and Daudi cells were incubated with the myeloid cell engagers to test their functions in M1 macrophages. The myeloid cell engagers (CD79b/CLEC5A bispecific antibodies) were serially diluted. Daudi cells were stained with CFSE first, and then spiked into M1 macrophages (E:T=5:1, or E:T=2:1) in a 96-well-round-bottom plate (Fixed Daudi counts: 2 × 10⁴/well), which were further incubated with serially diluted CD79b/CLEC5A bispecific antibodies (1 to 3 dilutions from 10 nM). After 24 hours of incubation, the plate was spun down, and supernatant was recovered for ELISA analysis. ELISA was performed according to instructions of ELISA MAX^{™} Deluxe Set Human TNF-α (BioLegend, Cat#: 430204) and ELISA MAX^{™} Deluxe Set Human IL-6 (BioLegend, Cat#: 430504) kits. Absorbance at 450 nm was measured within 15 minutes by a SpectraMax^{®} Absorbance Reader (MOLECULAR DEVICEs, S/N3052431867). Without target cells (Daudi) spiked in, the myeloid engagers showed no TNFα and IL-6 release in vitro. As shown in **FIG. 53**, with target cells (Daudi) spiked in, the myeloid engagers showed comparable TNFα and IL-6 release in vitro. The maximum TNFα release was less than 1000 pg/mL, whereas the maximum IL-6 release was less than 1600 pg/mL at 24 hours, indicating that myeloid cell engagers can promote target cell killing efficiently and induce cytokine release of M1 macrophages to target cells.

### Example 36. CD79b/CLEC5A bispecific antibodies mediated Ramos cell killing by M2 macrophages

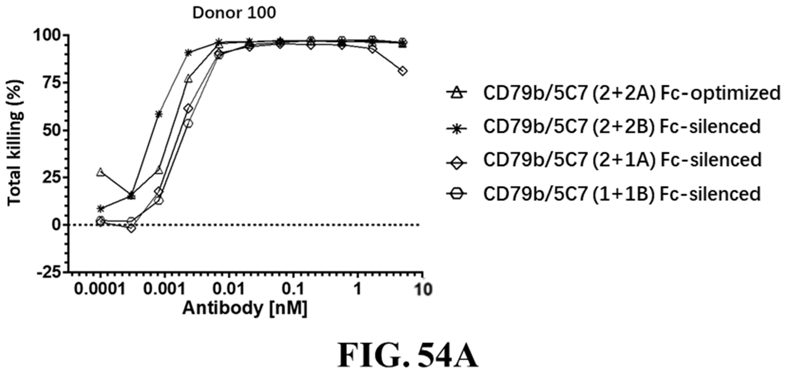

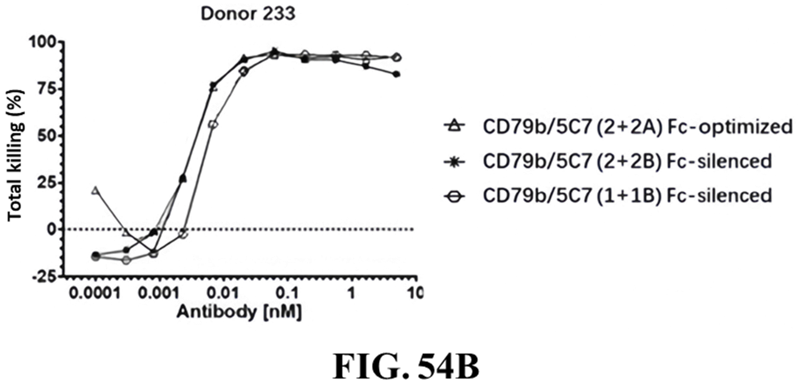

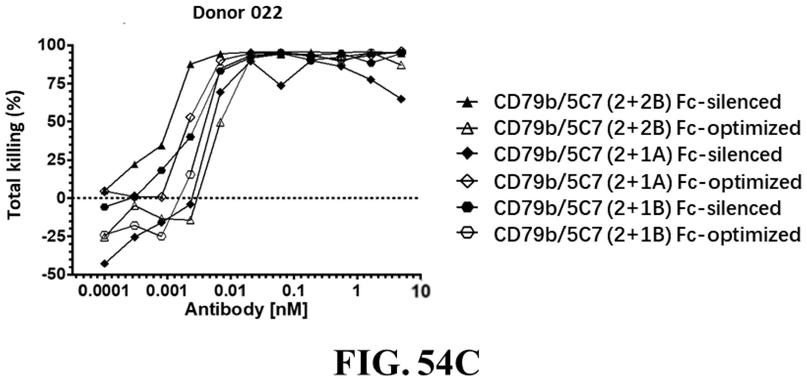

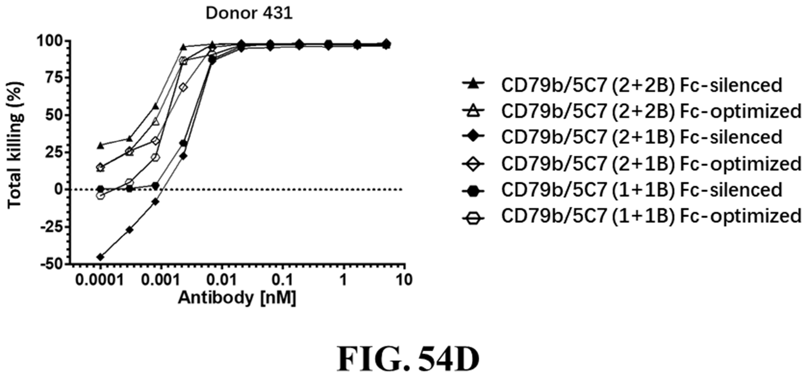

The effects of the valency of myeloid engagers CD79b/CLEC5A antibodies to kill target cancer Ramos cells (CD79b+) by effector M2 macrophages (CLEC5A+) cells were assessed. CD14+ monocytes (purified from human PBMC donors #100, #233, #022, and #431 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 6 days. On Day 6, M0 macrophages were polarized with 25 ng/mL of IL-10 (StemCell Technologies, Cat#: 78024) to M2 macrophages for another 24 hours (Day 7). Ramos cells were labeled with CFSE (ThermoFisher, Cat#: C34554). About 100,000 macrophages were incubated with 20,000 CFSE+ Ramos (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. Ramos cells were gated as CFSE+ by FACS, and the absolute cell count of CFSE+ cells was obtained by collecting a fixed volume for all treatment conditions. Percentage of target cell killing was calculated as: (absolute number of CFSE+ Ramos cell number in non-treatment group - absolute number of CFSE+ SYTOX-Ramos cell number in treatment group)/absolute number of CFSE+ Ramos cell number in non-treatment group × 100. As shown in **FIGs. 54A-54D** and the table below, all the myeloid engagers with different structures can efficiently kill CD79b+ cancerous Ramos cells.

**Table 21. CD79b/CLEC5A bispecific antibodies mediated Ramos cell killing by M2 macrophages**

| Donor | Antibodies | IC50 (nM) |
|---|---|---|
| Donor 100 | CD79b/5C7 (2+2 A) Fc-optimized | 0.001 |
| | CD79b/5C7 (2+2 B) Fc-silenced | 0.0004 |
| | CD79b/5C7 (2+1 A) Fc-silenced | 0.001 |
| | CD79b/5C7 (1+1 B) Fc-silenced | 0.002 |
| Donor 233 | CD79b/5C7 (2+2 A) Fc-optimized | 0.003 |
| | CD79b/5C7 (2+2 B) Fc-silenced | 0.002 |
| | CD79b/5C7 (1+1 B) Fc-silenced | 0.005 |
| Donor 022 | CD79b/5C7 (2+2 B) Fc-silenced | 0.001 |
| | CD79b/5C7 (2+2 B) Fc-optimized | 0.006 |
| | CD79b/5C7 (2+1 A) Fc-silenced | 0.003 |
| | CD79b/5C7 (2+1 A) Fc-optimized | 0.002 |
| | CD79b/5C7 (2+1 B) Fc-silenced | 0.002 |
| | CD79b/5C7 (2+1 B) Fc-optimized | 0.002 |
| Donor 431 | CD79b/5C7 (2+2 B) Fc-silenced | 0.0009 |
| | CD79b/5C7 (2+2 B) Fc-optimized | 0.001 |
| | CD79b/5C7 (2+1 B) Fc-silenced | 0.002 |
| | CD79b/5C7 (2+1 B) Fc-optimized | 0.001 |
| | CD79b/5C7 (1+1 B) Fc-silenced | 0.003 |
| | CD79b/5C7 (1+1 B) Fc-optimized | 0.001 |

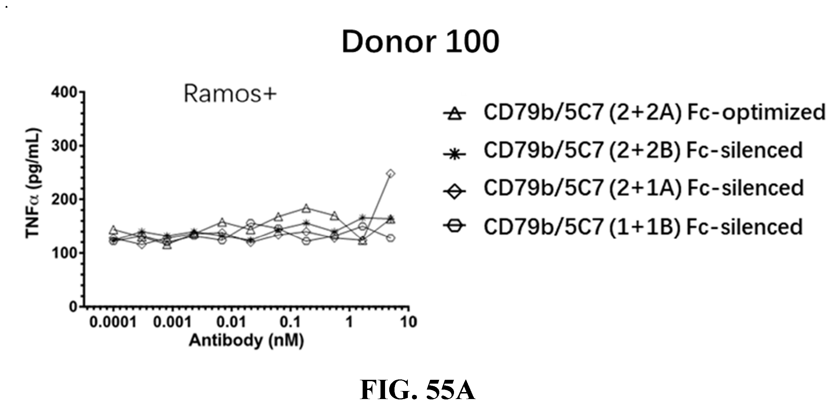

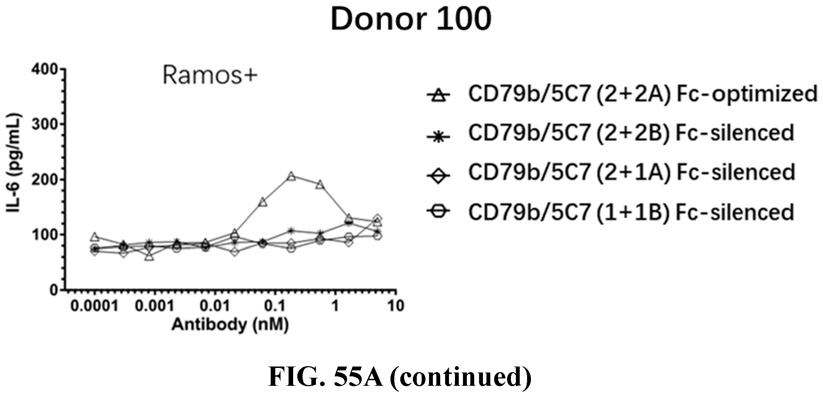

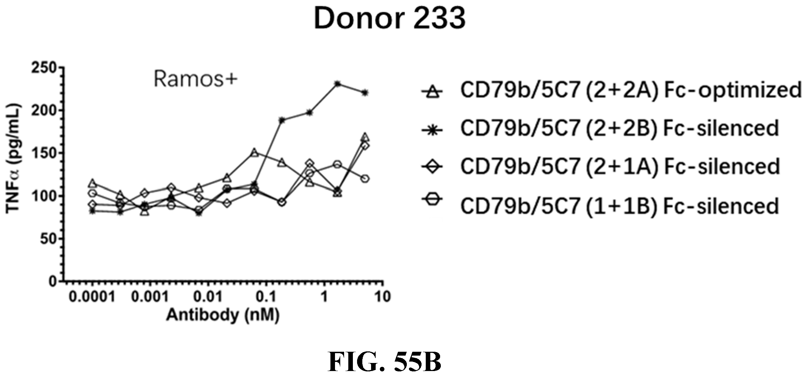

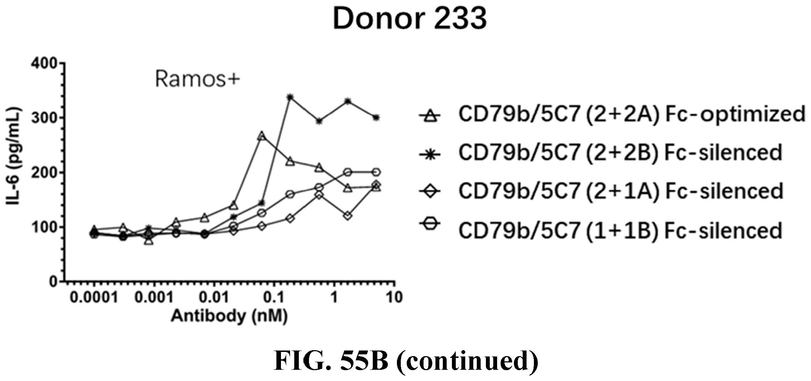

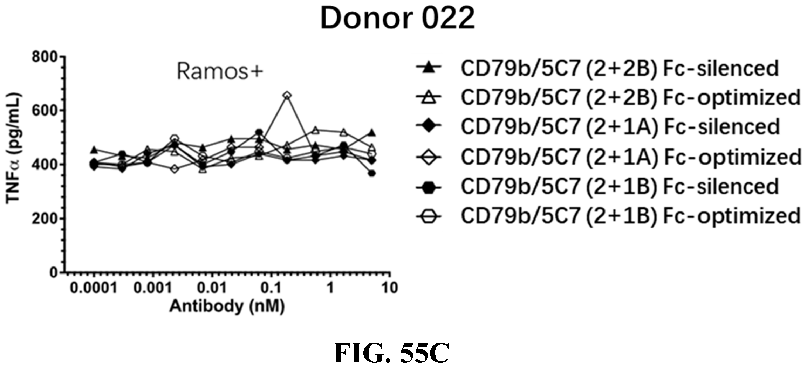

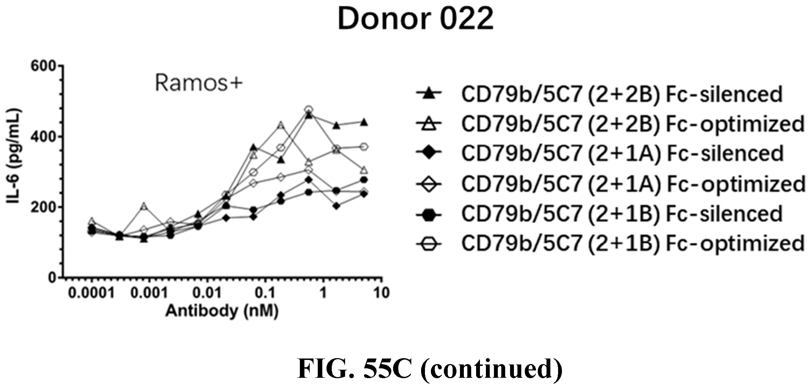

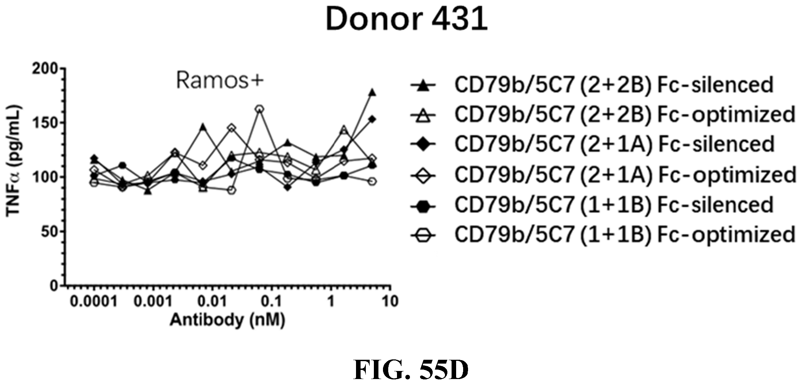

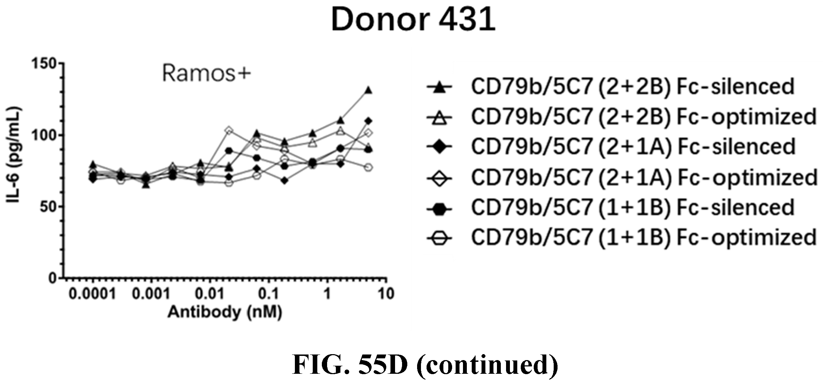

The cytokine release of CD79b/CLEC5A bispecific antibodies with different structures in the presence or absence of target Ramos cells were evaluated. CD14+ monocytes (purified from human PBMC donors #100, #233, #022, and #431 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 6 days. On Day 6, M0 macrophages were polarized with 25 ng/mL of IL-10 (StemCell Technologies, Cat#: 78024) to M2 macrophages for another 24 hours (Day 7). About 100,000 macrophages were incubated with 20,000 Ramos cells (E:T ratio of 5:1) in the presence of serially diluted antibodies in complete RPMI media (with 10% heat-inactivated FBS and 5% penicillin/streptomycin) for 24 hours at 37°C. After incubation, the supernatant was collected for cytokine analysis. TNFα and IL-6 levels in supernatants were measured using ELISA kits (R&D Systems, Cat#: DY206 and DY210, respectively) according to manufacturer's instructions. As shown in **FIGs. 55A-55D**, a small amount of IL-6 was released from the M2 macrophages only in the presence of target Ramos cells and CD79b/CLEC5A bispecific antibodies in a dose-dependent manner. There was no change in TNFα levels either in the presence or absence of target Ramos cells, suggesting that the myeloid engagers are safe for clinical use.

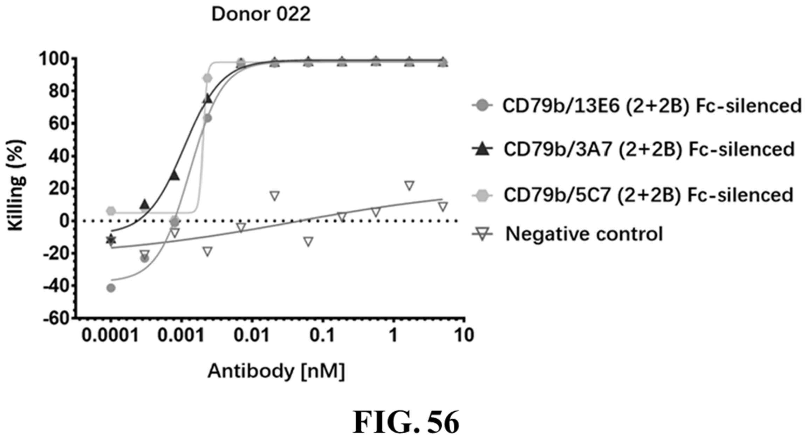

The effects of the valency of myeloid engagers CD79b/CLEC5A antibodies to kill target cancer Ramos cells (CD79b+) by effector M2 macrophages (CLEC5A+) cells were assessed. CD14+ monocytes (purified from human PBMC donors#022 with EasySep^{™} Human Monocyte Enrichment Kit without CD16 Depletion (StemCell Technologies, Cat#: 19058)) were differentiated to M0 macrophages with 50 ng/mL of macrophage colony-stimulating factor (M-CSF, StemCell Technologies, Cat#: 78057) for 6 days. On Day 6, M0 macrophages were polarized with 25 ng/mL of IL-10 (StemCell Technologies, Cat#: 78024) to M2 macrophages for another 24 hours (Day 7). Ramos cells were labeled with CFSE (ThermoFisher, Cat#: C34554). About 100,000 macrophages were incubated with 20,000 CFSE+ Ramos (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. Ramos cells were gated as CFSE+ by FACS, and the absolute cell count of CFSE+ cells was obtained by collecting a fixed volume for all treatment conditions. Percentage of target cell killing was calculated as: (absolute number of CFSE+ Ramos cell number in non-treatment group - absolute number of CFSE+ SYTOX-Ramos cell number in treatment group)/absolute number of CFSE+ Ramos cell number in non-treatment group × 100. As shown in **FIG. 56**, all the myeloid engagers can efficiently kill CD79b+ cancerous Ramos cells.

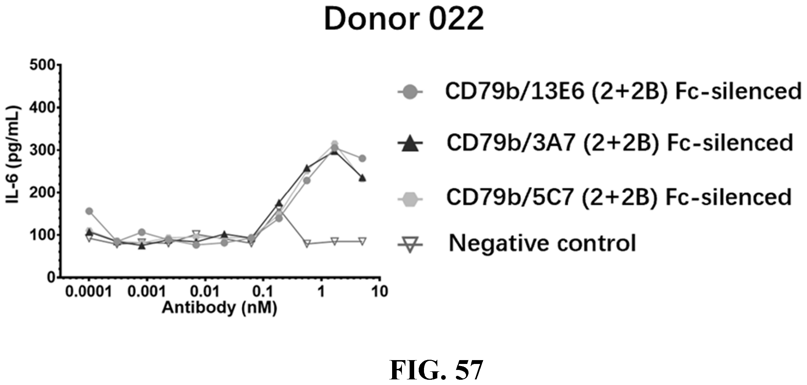

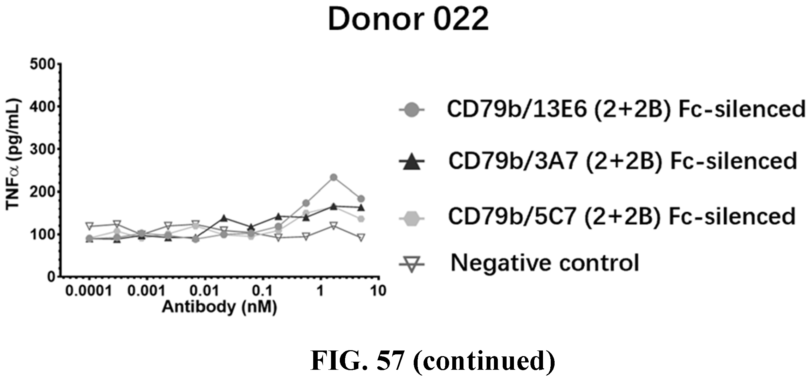

The cytokine release of CD79b/CLEC5A bispecific antibodies with different structures were evaluated. After incubation, the supernatant was collected for cytokine analysis. TNFα and IL-6 levels in supernatants were measured using ELISA kits (R&D Systems, Cat#: DY206 and DY210, respectively) according to manufacturer's instructions. The results were shown in **FIG. 57**.

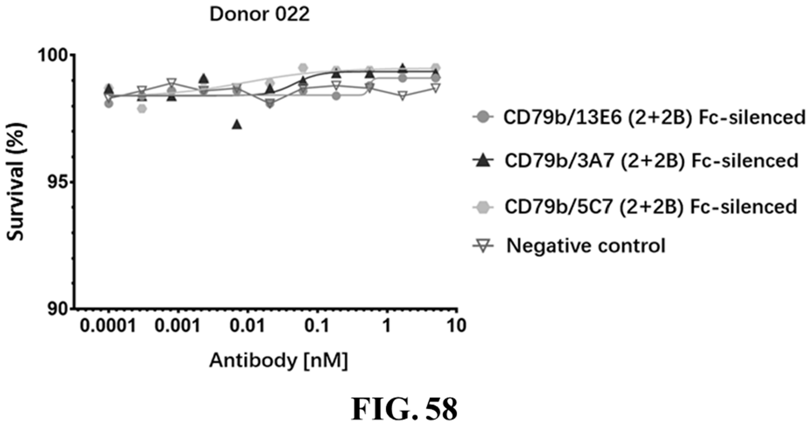

To find out whether the activated M2 macrophages further cause self-killing to reduce its numbers, we analyzed M2 macrophages numbers to test their status. Ramos cells ran through dead cell removal then stained with 0.25 uM CSFE. The number of macrophages was shown in **FIG. 58**, there were no significant changes of M2 macrophages counts.

### Example 37. CD79b/CLEC5A bispecific antibodies mediated Daudi cell killing by M2 macrophages

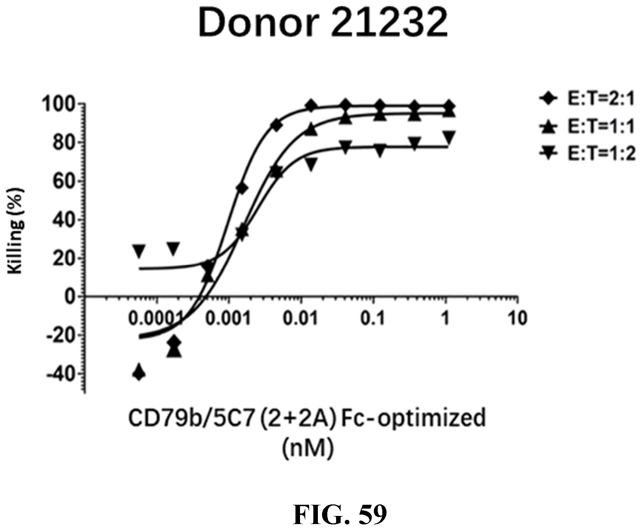

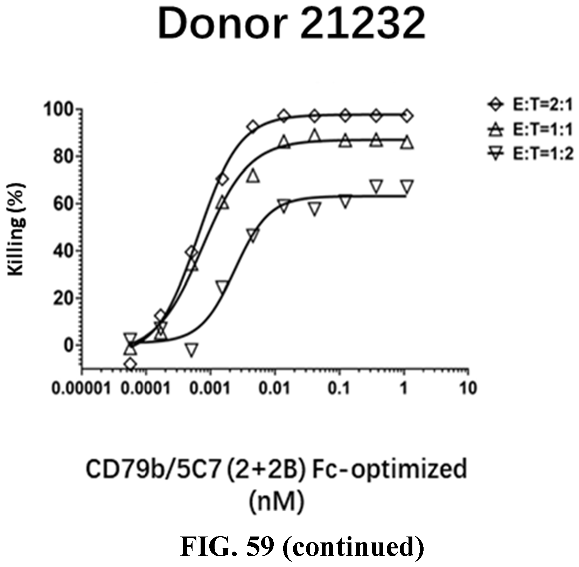

To estimate cancerous B cell (Daudi) killing efficacy of myeloid cell engager CD79b/CLEC5A bispecific antibodies in M2 macrophages at different E:T ratios, human monocytes were isolated from PBMCs by EasySep^{™} Human Monocyte Enrichment Kit without CD16 Depletion (StemCell Technologies, Cat#: 19058). Isolated monocytes were induced by 50 ng/mL M-CSF (StemCell Technologies, Cat#: 78057 ). On Day 6, complete Media with 50 ng/mL M-CSF and 25 ng/mL IL-10 (StemCell Technologies, Cat#: 78024) were added. On Day 7, M2 macrophages were obtained. Different E:T ratios of M2 macrophages and Daudi cells were incubated with the myeloid cell engagers to test their functions in M2 macrophages. The myeloid cell engagers (CD79b/CLEC5A bispecific antibodies) were serially diluted. Daudi cells were stained with CFSE first, and then spiked into M2 macrophages (E:T=2:1, E:T=1:1, or E:T=1:2) in a 96-well-round-bottom plate (Fixed Daudi counts: 2 × 10⁴/well), which were further incubated with serially diluted CD79b/CLEC5A bispecific antibodies (1 to 3 dilutions from 10 nM). After 24 hours of incubation, the plate was spun down, and supernatant was stored at -80°C. Cells were stained with live/dead staining dye, and then processed on a CytoFLEX LX flow cytometer. Percentage of cell killing was calculated as: (live tumor cell number in non-treatment group-live tumor cell number in treatment group)/live tumor cell number in non-treatment group × 100. As shown in **FIG. 59**, the myeloid engagers showed potent Daudi cell killing effects at different E:T ratios, and CD79b/CLEC5A bispecific antibodies showed comparable Daudi cell killing potency in M2 macrophages in vitro. The maximum specific tumor cell killing was around 97-99% (E:T=2:1), 88-96% (E:T=1:1), and 67-82% (E:T=1:2) for Daudi cells at 24 hours, indicating that myeloid cell engagers can promote target cell killing efficiently at different E:T ratios of M2 macrophages to target cells.

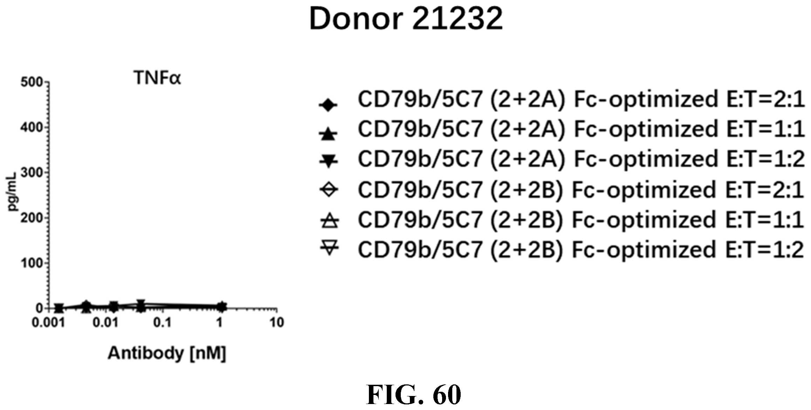

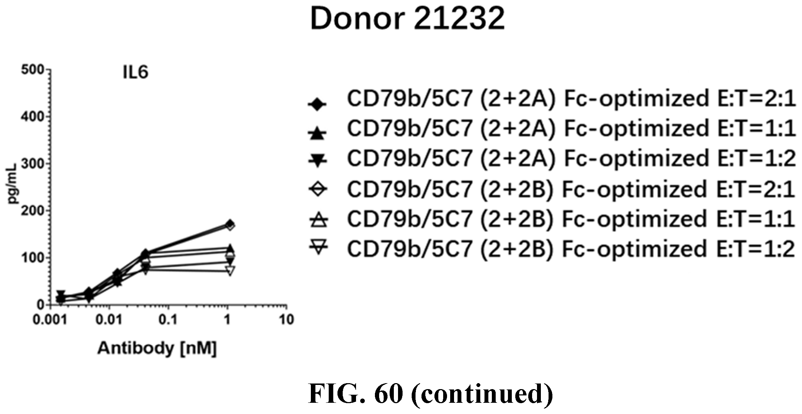

To estimate cytokine release of cancerous B cell (Daudi) killing of myeloid cell engager CD79b/CLEC5A bispecific antibodies in M2 macrophages at different E:T ratios, human monocytes were isolated from PBMCs by EasySep^{™} Human Monocyte Enrichment Kit without CD16 Depletion (StemCell Technologies, Cat#: 19058). Isolated monocytes were induced by 50 ng/mL M-CSF (StemCell Technologies, Cat#: 78057). On Day 6, complete media with 50 ng/mL M-CSF and 25 ng/mL IL-10 (StemCell Technologies, Cat#: 78024) were added. On Day 7, M2 macrophages were obtained. Different E:T ratios of M2 macrophages and Daudi cells were incubated with the myeloid cell engagers (CD79b/CLEC5A bispecific antibodies) to test their functions in M2 macrophages. The myeloid cell engagers were serially diluted. Daudi cells were stained with CFSE first, and then spiked into M2 macrophages (E:T=2:1, E:T=1:1, or E:T=1:2) in a 96-well-round-bottom plate (Fixed Daudi counts: 2 × 10⁴/well), which were further incubated with serially diluted CD79b/CLEC5A bispecific antibodies (1 to 3 dilutions from 10 nM). After 24 hours of incubation, the plate was spun down, and supernatant was recovered for ELISA analysis. ELISA was performed according to instructions of ELISA MAX^{™} Deluxe Set Human TNF-α (BioLegend, Cat#: 430204) and ELISA MAX^{™} Deluxe Set Human IL-6 (BioLegend, Cat#: 430504) kits. Absorbance at 450 nm was measured within 15 minutes by a SpectraMax^{®} Absorbance Reader (MOLECULAR DEVICEs, S/N3052431867). As shown in **FIG. 60**, with target cells (Daudi) spiked in, the myeloid engagers showed comparable TNFα and IL-6 release in vitro. The maximum TNFα release was less than 150 pg/mL, whereas the maximum IL-6 release was less than 200 pg/mL at 24 hours, indicating that the myeloid cell engagers can promote target cell killing efficiently and induce very low cytokine release of M2 macrophages to target cells.

### Example 38. CD79b/CLEC5A bispecific antibodies mediated B cell killing by monocytes

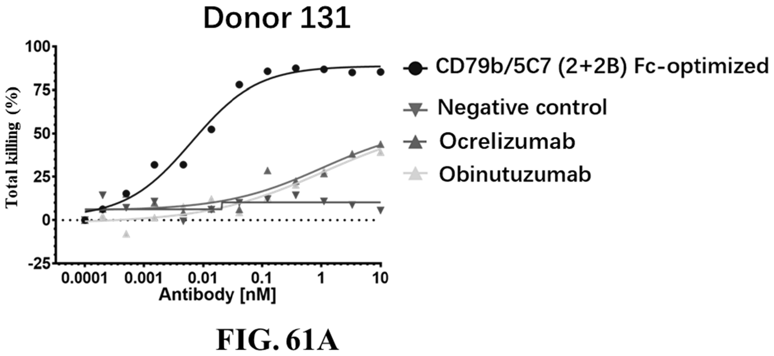

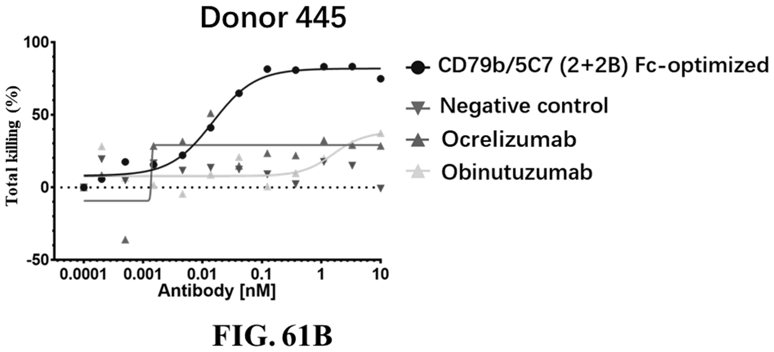

The effects of the valency of myeloid engagers CD79b/CLEC5A antibodies to kill target cancer B cells (CD79b+) by effector monocytes (CLEC5A+) cells were assessed. Fresh monocyte donors #131 and #445 isolated from PBMCs using StemCell human monocyte enrichment kit without CD16 depletion, allowed to rest for 1 hour before setting up assay. About 100,000 momocytes were incubated with 20,000 CFSE+ B cells (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. B cells were gated as CFSE+ by FACS, and the absolute cell count of CFSE+ cells was obtained by collecting a fixed volume for all treatment conditions. Percentage of target cell killing was calculated as: (absolute number of CFSE+ B cell number in non-treatment group - absolute number of CFSE+ SYTOX-B cell number in treatment group)/absolute number of CFSE+ B cell number in non-treatment group × 100. As shown in **FIGs. 61A-61B**, all the myeloid engagers can efficiently kill CD79b+ cancerous Ramos cells.

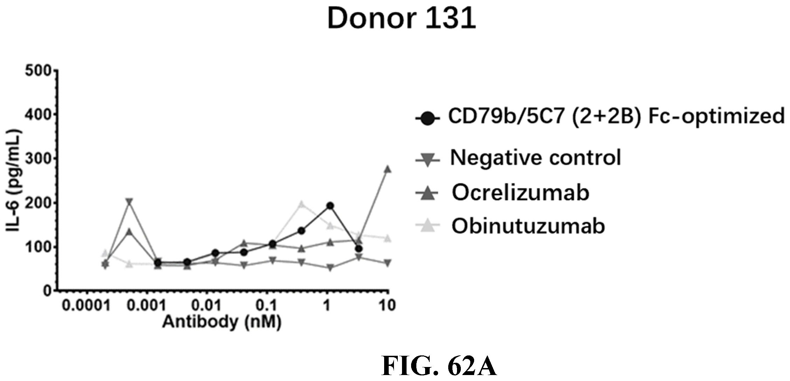

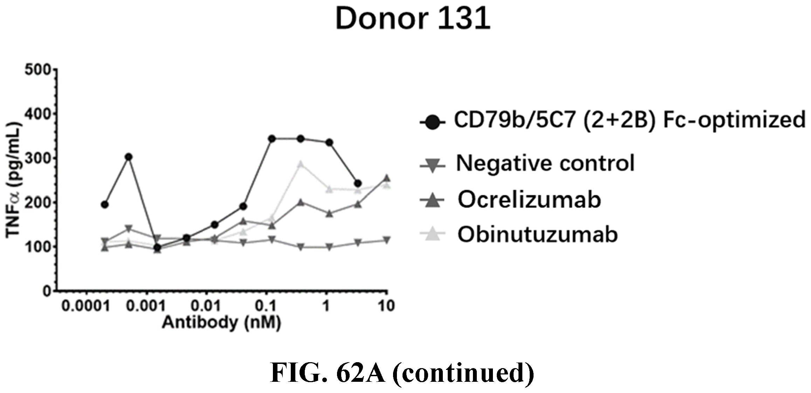

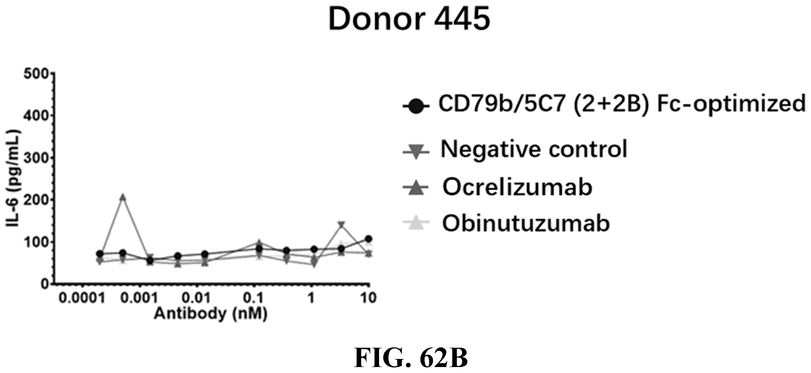

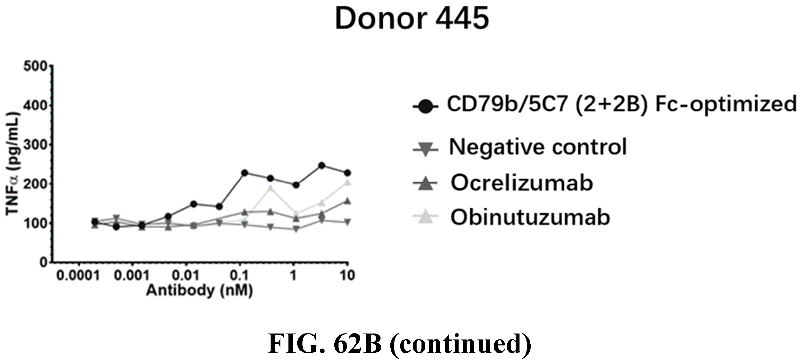

The cytokine release of CD79b/CLEC5A bispecific antibodies with different structures were evaluated. After incubation, the supernatant was collected for cytokine analysis. TNFα and IL-6 levels in supernatants were measured using ELISA kits (R&D Systems, Cat#: DY206 and DY210, respectively) according to manufacturer's instructions. The results were shown in **FIGs. 62A-62B**.

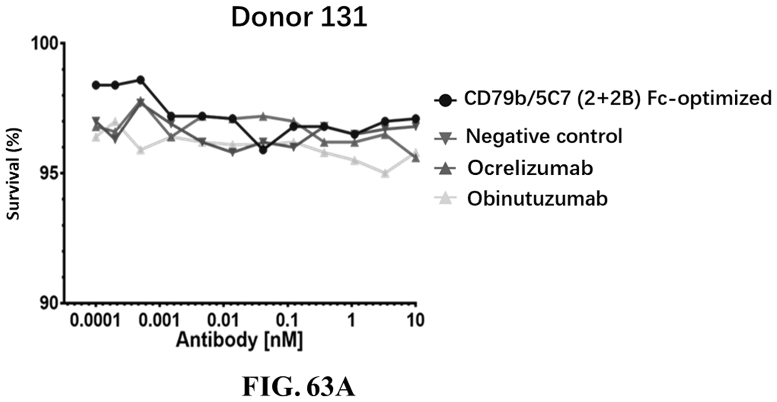

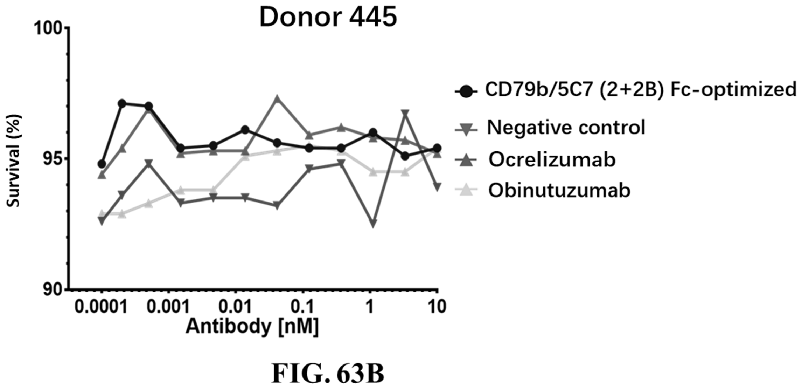

To find out whether the activated monocytes further cause self-killing to reduce its numbers, we analyzed monocytes numbers to test their status. B cells ran through dead cell removal then stained with 0.25 uM CSFE. The number of monocytes was shown in **FIGs. 63A-63B****,** there were no significant changes of monocytes counts.

### Example 39. CD79b/CLEC5A bispecific antibodies mediated B cell killing by monocytes

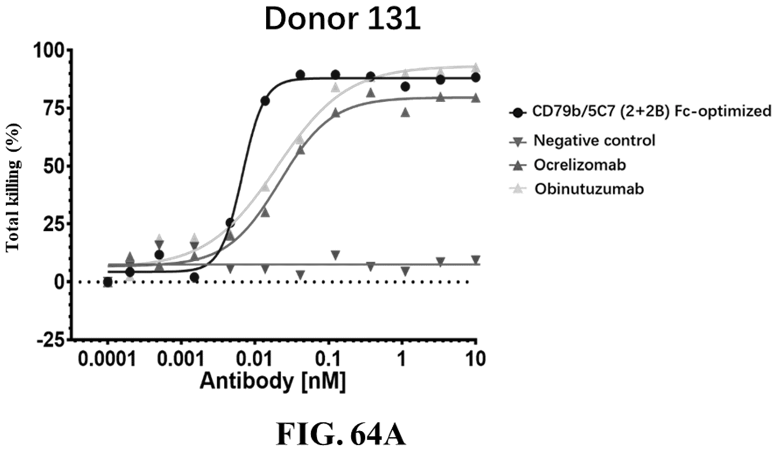

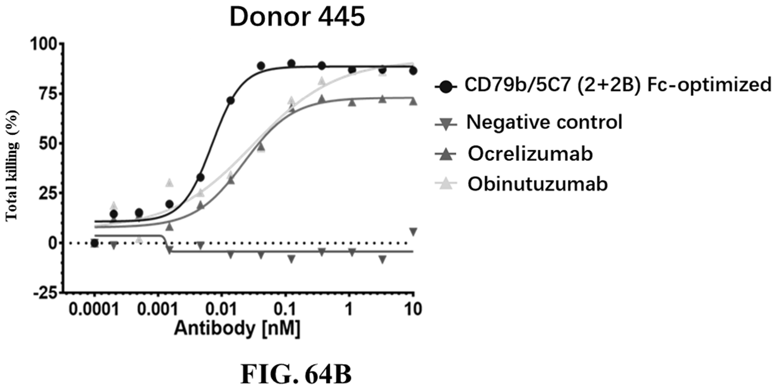

The effects of the valency of myeloid engagers CD79b/CLEC5A antibodies to kill target cancer B cells (CD79b+) by effector monocytes (CLEC5A+) cells were assessed. Fresh monocyte donors #131 and #445 isolated from PBMCs using StemCell human monocyte enrichment kit without CD16 depletion, allowed to rest for 1 hour before setting up assay. About 100,000 momocytes were incubated with 20,000 CFSE+ B cells (E:T ratio of 5:1) in the presence of serially diluted antibodies in complete RPMI media (with 10% heat-inactivated FBS and 5% penicillin/streptomycin) for 24 hours at 37°C. After incubation, the cells were resuspended in 100 µl of FACS buffer with SYTOX^{™} Blue Dead Cell Stain (ThermoFisher, Cat#: S34857) and analyzed with a Cytek^{®} cytometer. B cells were gated as CFSE+ by FACS, and the absolute cell count of CFSE+ cells was obtained by collecting a fixed volume for all treatment conditions. Percentage of target cell killing was calculated as: (absolute number of CFSE+ B cell number in non-treatment group - absolute number of CFSE+ SYTOX-B cell number in treatment group)/absolute number of CFSE+ B cell number in non-treatment group × 100. The results were shown in **FIGs. 64A-64B****.**

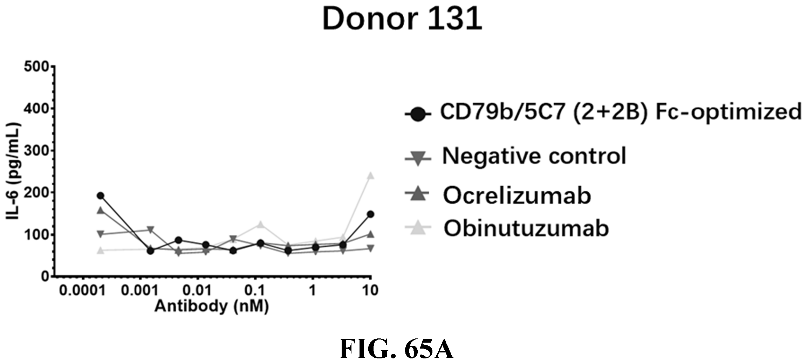

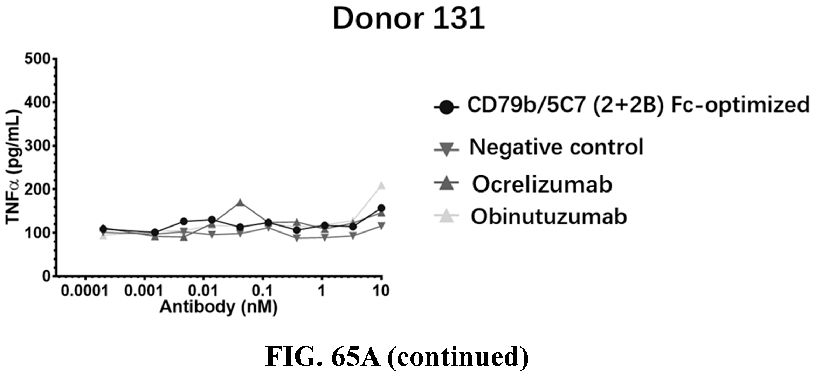

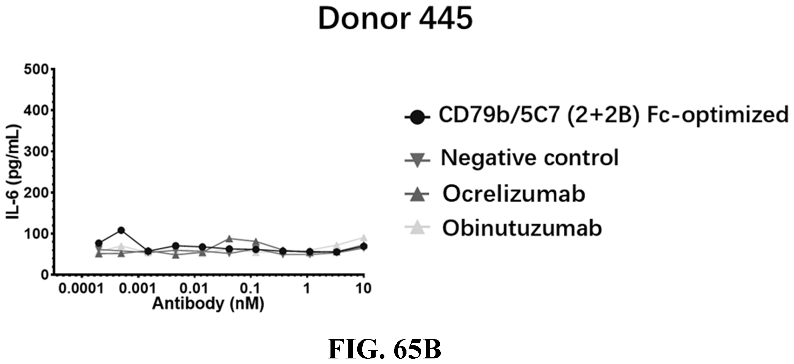

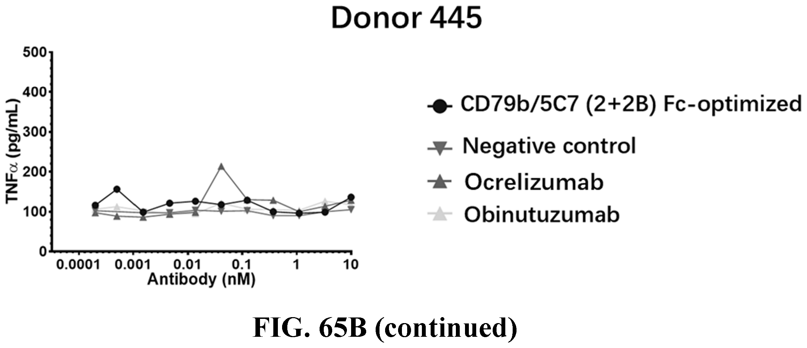

The cytokine release of CD79b/CLEC5A bispecific antibodies with different structures were evaluated. After incubation, the supernatant was collected for cytokine analysis. TNFα and IL-6 levels in supernatants were measured using ELISA kits (R&D Systems, Cat#: DY206 and DY210, respectively) according to manufacturer's instructions. The results were shown in **FIGs. 65A-65B****.**

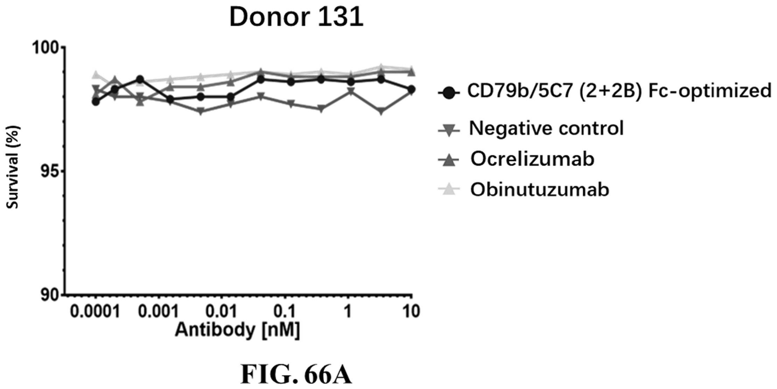

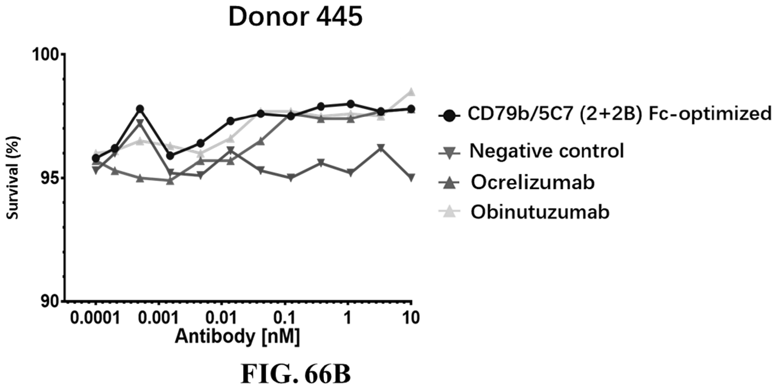

To find out whether the activated monocytes further cause self-killing to reduce its numbers, we analyzed monocytes numbers to test their status. B cells ran through dead cell removal then stained with 0.25 uM CSFE. The number of monocytes was shown in **FIGs. 66A-66B****,** there were no significant changes of monocytes counts.

Sequences discussed in the disclosure are listed in the table below.

**Table 22. Some of the amino acid sequences described in the present disclosure.**

| SEQ ID NO | Description | Sequence |
|---|---|---|
| 1 | Human CLEC5A | MNWHMIISGLIVVVLKVVGMTLFLLYFPQIFNKSNDGFTTTRSYGTVCPK DWEFYQARCFFLSTSESSWNESRDFCKGKGSTLAIVNTPEKLKFLQDITD AEKYFIGLIYHREEKRWRWINNSVFNGNVTNQNQNFNCATIGLTKTFDAA SCDISYRRICEKNAK |
| 2 | Mouse CLEC5A | MNWHMIISGLIVVVIKVVGMTFFLLYFPQVFGKSNDGFVPTESYGTTSVQ NVSQIFGRNDESTMPTRSYGTVCPRNWDFHQGKCFFFSFSESPWKDSMDY CATQGSTLAIVNTPEKLKYLQDIAGIENYFIGLVRQPGEKKWRWINNSVF NGNVTNQDQNFDCVTIGLTKTYDAASCEVSYRWICEMNAK |
| 3 | 3A7 HCDR1 | GMYWIC |
| | (Kabat) | |
| 4 | 3A7 HCDR1 | GFSFSGMY |
| | (Chothia) | |
| 5 | 3A7 HCDR2 | CIYTGSSTTGYASWAKG |
| | (Kabat) | |
| 6 | 3A7 HCDR2 | YTGSST |
| | (Chothia) | |
| 7 | 3A7 HCDR3 | PYGDSSHYRGLNL |
| | (Kabat) | |
| | (Chothia) | |
| 8 | 3A7 LCDR1 | QASQRINNYLS |
| | (Kabat) | |
| | (Chothia) | |
| 9 | 3A7 LCDR2 | GAATLAS |
| | (Kabat) | |
| | (Chothia) | |
| 10 | 3A7 LCDR3 | QCTYGIPSSNYG |
| | (Kabat) | |
| | (Chothia) | |
| 11 | ch3A7 VH | QSLEESGGDLVKPGASLTLTCTASGFSFSGMYWICWVRQAPGKGLEWIAC IYTGSSTTGYASWAKGRFTISKTSSTTVTLQMTSLTAADTATHFCASPYG DSSHYRGLNLWGPGTLVTVSS |
| 12 | ch3A7 VL | AVVMTQTPASVEAAVGGTVTINCQASQRINNYLSWFQQKPGQPPKLLIYG AATLASGVPSRFKGSGSGTEYTLTISDLEPADAATYYCQCTYGIPSSNYG FGGGTEVVVK |
| 13 | 5C7 HCDR1 | RSSYMC |
| | (Kabat) | |
| 14 | 5C7 HCDR1 | GFSFSRSS |
| | (Chothia) | |
| 15 | 5C7 HCDR2 | CLSVGDGHTYYASWAKG |
| | (Kabat) | |
| 16 | 5C7 HCDR2 | SVGDGH |
| | (Chothia) | |
| 17 | 5C7 HCDR3 | APSYSGDGGHGYDL |
| | (Kabat) | |
| | (Chothia) | |
| 18 | 5C7 LCDR1 | QASESVDRNNRLA |
| | (Kabat) | |
| | (Chothia) | |
| 19 | 5C7 LCDR2 | TTSTLAS |
| | (Kabat) | |
| | (Chothia) | |
| 20 | 5C7 LCDR3 | AGYRGAHTDGDA |
| | (Kabat) | |
| | (Chothia) | |
| 21 | ch5C7 VH | QSLEESGGDLVKPGASLTLTCTASGFSFSRSSYMCWVRQAPGKGLEWIGC LSVGDGHTYYASWAKGRFTISKTSSTSVTLQMTRLTAADTATYFCARAPS YSGDGGHGYDLWGPGTLVTVSS |
| 22 | ch5C7 VL | AIVMTQTPSSKSVPVGDTVTINCQASESVDRNNRLAWFQQKPGQPPKLLI YTTSTLASGVPSRFKGSGSGTHFTLTISDVVPDDAATYYCAGYRGAHTDG DAFGGGTEVVVK |
| 23 | 6A5 HCDR1 | SSYYMC |
| | (Kabat) | |
| 24 | 6A5 HCDR1 | GFSFSSSY |
| | (Chothia) | |
| 25 | 6A5 HCDR2 | CIYSSSGRTWYATWVNG |
| | (Kabat) | |
| 26 | 6A5 HCDR2 | YSSSGR |
| | (Chothia) | |
| 27 | 6A5 HCDR3 | SYAGYGGYGYAYFNL |
| | (Kabat) | |
| | (Chothia) | |
| 28 | 6A5 LCDR1 | QASEDIYSSLA |
| | (Kabat) | |
| | (Chothia) | |
| 29 | 6A5 LCDR2 | DASTLAS |
| | (Kabat) | |
| | (Chothia) | |
| 30 | 6A5 LCDR3 | QNYYDSTGSGYGAA |
| | (Kabat) | |
| | (Chothia) | |
| 31 | ch6A5 VH | QQQLEESGGGLVKPGGTLTLTCTASGFSFSSSYYMCWVRQAPGKGLELIA CIYSSSGRTWYATWVNGRFTISRSTSLNTVDLKMTSLTAADTATYFCARS YAGYGGYGYAYFNLWGPGTLVTVSS |
| 32 | ch6A5 VL | DIVMTQTPASVEAGVGGTVTIKCQASEDIYSSLAWYQQKAGQRPKLLIYD ASTLASGVPSRFKGSGSGTEYTLTISGVQPDDAATYYCQNYYDSTGSGYG AAFGGGTEVVVK |
| 33 | 6G9 HCDR1 | SNKYMC |
| | (Kabat) | |
| 34 | 6G9 HCDR1 | GFSFTSNK |
| | (Chothia) | |
| 35 | 6G9 HCDR2 | CIYVGDGNTYYASWAKG |
| | (Kabat) | |
| 36 | 6G9 HCDR2 | YVGDGN |
| | (Chothia) | |
| 37 | 6G9 HCDR3 | GGDYSIGGGGFASNL |
| | (Kabat) | |
| | (Chothia) | |
| 38 | 6G9 LCDR1 | QASESISNFLA |
| | (Kabat) | |
| | (Chothia) | |
| 39 | 6G9 LCDR2 | RASTLAS |
| | (Kabat) | |
| | (Chothia) | |
| 40 | 6G9 LCDR3 | QSYYGTSSTGHNV |
| | (Kabat) | |
| | (Chothia) | |
| 41 | ch6G9 VH | QSLEESGGDLVKPGASLTLTCTASGFSFTSNKYMCWVRQAPGKGPEWIAC IYVGDGNTYYASWAKGRFTISKTSSTTVTLQMTSLTVADTATYFCARGGD YSIGGGGGFASNLWGPGTLVTVSS |
| 42 | ch6G9 VL | AFELTQTPSSVEAAVGGTVTINCQASESISNFLAWYRQKPGQPPKLLIYR ASTLASGVSSRFKGSGSGTEFTLTISDLEPADGASYYCQSYYGTSSTGHN VFGGGTEVVVK |
| 43 | 7G10 HCDR1 | SGYDMC |
| | (Kabat) | |
| 44 | 7G10 HCDR1 | GFSFSSGY |
| | (Chothia) | |
| 45 | 7G10 HCDR2 | CIYGGRSDNVYYARWAKG |
| | (Kabat) | |
| 46 | 7G10 HCDR2 | YGGRSDN |
| | (Chothia) | |
| 47 | 7G10 HCDR3 | DYLYRGYIGGGYGRSNL |
| | (Kabat) | |
| | (Chothia) | |
| 48 | 7G10 LCDR1 | RASEDIENCLA |
| | (Kabat) | |
| | (Chothia) | |
| 49 | 7G10 LCDR2 | QASKLPS |
| | (Kabat) | |
| | (Chothia) | |
| 50 | 7G10 LCDR3 | QNPDYMRNTDNL |
| | (Kabat) | |
| | (Chothia) | |
| 51 | ch7G10 VH | QQQLEESGGGLVKPGGTLTLTCKASGFSFSSGYDMCWVRQAPGKGLEWIA CIYGGRSDNVYYARWAKGRFTISKTSSTTVTLQMTSLTAADTATYFCARD YLYRGYIGGGYGRSNLWGPGTLVTVSS |
| 52 | ch7G10 VL | DVVMTQTPASVSAAVGGTVTINCRASEDIENCLAWYQQKPGQPPKLLIYQ ASKLPSGVPSRFSGSGSGTEYTLTISDLEPDDGATYYCQNPDYMRNTDNL FGGGTEVVVK |
| 53 | 9E11 HCDR1 | NYYYMC |
| | (Kabat) | |
| 54 | 9E11 HCDR1 | GFDLNNYY |
| | (Chothia) | |
| 55 | 9E11 HCDR2 | CTHAGSSSSSYYARWAKG |
| | (Kabat) | |
| 56 | 9E11 HCDR2 | HAGSSSS |
| | (Chothia) | |
| 57 | 9E11 HCDR3 | HDRDYDDMDL |
| | (Kabat) | |
| | (Chothia) | |
| 58 | 9E11 LCDR1 | QASQSIEYGYLS |
| | (Kabat) | |
| | (Chothia) | |
| 59 | 9E11 LCDR2 | YASKLAS |
| | (Kabat) | |
| | (Chothia) | |
| 60 | 9E11 LCDR3 | LYGYFDDYSGAA |
| | (Kabat) | |
| | (Chothia) | |
| 61 | ch9E11 VH | QSLEESGGGLVQPEGSLTLTCTASGFDLNNYYYMCWVRQAPGKGLEWIAC THAGSSSSSYYARWAKGRFTISKTSSTTVTLQMTSLTAADTATYFCARHD RDYDDMDLWGPGTLVTVSS |
| 62 | ch9E11 VL | NIVMTQTTSPVSAVVGGTITINCQASQSIEYGYLSWYQQKPGQPPKLLIF YASKLASGVPSRFSGSGSGTQFTLTISGVQPDDAATYFCLYGYFDDYSGA AFGGGTEVVVK |
| 63 | 13E6 HCDR1 | SSYWLC |
| | (Kabat) | |
| 64 | 13E6 HCDR1 | GFSFSSSY |
| | (Chothia) | |
| 65 | 13E6 HCDR2 | CIYGGSSRGTYYASWAKG |
| | (Kabat) | |
| 66 | 13E6 HCDR2 | YGGSSRG |
| | (Chothia) | |
| 67 | 13E6 HCDR3 | HTYTYPRFSPYFNL |
| | (Kabat) | |
| | (Chothia) | |
| 68 | 13E6 LCDR1 | QASEDIESSLA |
| | (Kabat) | |
| | (Chothia) | |
| 69 | 13E6 LCDR2 | DASDLES |
| | (Kabat) | |
| | (Chothia) | |
| 70 | 13E6 LCDR3 | QSAYYTGSADNT |
| | (Kabat) | |
| | (Chothia) | |
| 71 | ch13E6 VH | QSLEESGGDLVKPGGTLTLTCTASGFSFSSSYWLCWVRQAPGKGLEWIAC IYGGSSRGTYYASWAKGRFTISKTSSTTVTLQMTSLTAADTATYFCASHT YTYPRFSPYFNLWGPGTLVSVSS |
| 72 | ch13E6 VL | AFEMTQTPASVSPAVGGTVSINCQASEDIESSLAWYQQKPGQPPKLLIYD ASDLESGVPSRFKGSGSGTEYTLTISGVQPADAATYFCQSAYYTGSADNT FGGGTEVVVK |
| 73 | 14A2 HCDR1 | SYYYMC |
| | (Kabat) | |
| 74 | 14A2 HCDR1 | GFTLSSYY |
| | (Chothia) | |
| 75 | 14A2 HCDR2 | CIYAGSSGTTVYASWAKG |
| | (Kabat) | |
| 76 | 14A2 HCDR2 | YAGSSGT |
| | (Chothia) | |
| 77 | 14A2 HCDR3 | KLGDVGYGHTTYFNL |
| | (Kabat) | |
| | (Chothia) | |
| 78 | 14A2 LCDR1 | QASQNIYSTLA |
| | (Kabat) | |
| | (Chothia) | |
| 79 | 14A2 LCDR2 | DASDLAS |
| | (Kabat) | |
| | (Chothia) | |
| 80 | 14A2 LCDR3 | QTYYDSGSDDLNA |
| | (Kabat) | |
| | (Chothia) | |
| 81 | ch14A2 VH | QSLEESGGGLVKPEGSLTLTCKASGFTLSSYYYMCWVRQAPGKGLEWIAC IYAGSSGTTVYASWAKGRFTISKTSSTTVTLQMTSLTAADTATYLCARKL GDVGYGHTTYFNLWGPGTLVTVSS |
| 82 | ch14A2 VL | ALVMTQTPSSVSAAVGGTVTIKCQASQNIYSTLAWYQQKPGQPPKLLMFD ASDLASGVPSRFKGSGSGTEYTLTISDLEPADAATYFCQTYYDSGSDDLN AFGGGTEVVVK |
| 83 | h3A7 VH | EQQLLESGGGLVQPGGSLRLSCAASGFSFSGMYWICWVRQAPGKGLEWIA CIYTGSSTTGYASWAKGRFTISKDSSKNTVYLQMNSLRAEDTAVYFCASP YGDSSHYRGLNLWGPGTLVTVSS |
| 84 | h3A7 VL | DVQMTQSPSSLSASVGDRVTITCQASQRINNYLSWFQQKPGKPPKLLIYG AATLASGVPSRFSGSGSGTDYTLTISSLQPEDVATYYCQCTYGIPSSNYG FGGGTKVEIK |
| 85 | h5C7 VH | EQQLVESGGGLVKPGGSLRLSCAASGFSFSRSSYMCWVRQAPGKCLEWIG CLSVGDGHTYYASWAKGRFTISKDSAKNSVYLQMNSLRAEDTAVYFCARA PSYSGDGGHGYDLWGPGTLVTVSS |
| 86 | h5C7 VL | DIQMTQSPSSLSASVGDRVTITCQASESVDRNNRLAWFQQKPGKPPKLLI YTTSTLASGVPSRFSGSGSGTDFTLTISSLQPEDVATYYCAGYRGAHTDG DAFGCGTKVEIK |
| 87 | h6A5 VH | EQQLVESGGGLVQPGGSLRLSCSASGFSFSSSYYMCWVRQAPGKGLELIA CIYSSSGRTWYATWVNGRFTISRDTSKNTVYLQMNSLRAEDTAVYFCARS YAGYGGYGYAYFNLWGPGTLVTVSS |
| 88 | h6A5 VL | EIVMTQSPATLSVSPGERATLSCQASEDIYSSLAWYQQKPGQRPRLLIYD ASTLASGVPARFSGSGSGTEYTLTISSLQSEDFAVYYCQNYYDSTGSGYG AAFGGGTKVEIK |
| 89 | h7G10 VH | EQQLVESGGGVVQPGRSLRLSCAASGFSFSSGYDMCWVRQAPGKGLEWIA CIYGGRSDNVYYARWAKGRFTISKDSSTNTVFLQMNSLRAEDTAVYFCAR DYLYRGYIGGGYGRSNLWGPGTLVTVSS |
| 90 | h7G10 VL | DVQMTQSPSSVSASVGDRVTITCRASEDIENCLAWYQQKPGKPPKLLIYQ ASKLPSGVPSRFSGSGSGTDYTLTISSLQPEDFATYYCQNPDYMRNTDNL FGGGTKVEIK |
| 91 | h13E6 VH | EQQLLESGGGLVQPGGSLRLSCAASGFSFSSSYWLCWVRQAPGKGLEWIA CIYGGSSRGTYYASWAKGRFTISKDSSKNTVYLQMNSLRAEDTAVYFCAS HTYTYPRFSPYFNLWGPGTLVTVSS |
| 92 | h13E6 VL | EFVMTQSPATLSVSPGERATLSCQASEDIESSLAWYQQKPGQPPRLLIYD ASDLESGVPARFSGSGSGTEYTLTISSLQSEDFAVYFCQSAYYTGSADNT FGGGTKVEIK |
| 93 | DX244 VH | QIQLVQSGPELKKPGETVKISCKASGYTFTIYAMNWVKQAPGKGLEWMGW INTDTGEPTYADDFKGRFAFSLETSASTAYLQINNLKNEDMATYFCARSS EVFYDYRFDYWGQGTALTVSS |
| 94 | DX244 VL | DIQMTQSPSSLSASLGERVSLTCRASQDIGSSLNWLQQEPDGTIKRLIYA TSNLDSGVPKRFSGSRSGSDYSLTISSLESEDFVHYYCLQYASSPFTFGS GTKLEIKR |
| 95 | human IgG1 Fc-wild-type | |
| 96 | human IgG1 Fc-silenced | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEP KSCDKTHTCPPCPAPEAAGGPSVFLFPPKPKDTLMISRTPEVTCVVVDVS HEDPEVKFNWYVDGVEVHNAKTKPREEQYNSTYRVVSVLTVLHQDWLNGK EYKCKVSNKALGAPIEKTISKAKGQPREPQVYTLPPSRDELTKNQVSLTC LVKGFYPSDIAVEWESNGQPENNYKTTPPVLDSDGSFFLYSKLTVDKSRW QQGNVFSCSVMHEALHNHYTQKSLSLSPGK |
| 97 | human IgG1 Fc-optimized | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEP KSCDKTHTCPPCPAPELLAGPSVFLFPPKPKDTLMISRTPEVTCVVVDVS HEDPEVKFNWYVDGVEVHNAKTKPREEQYNSTYRVVSVLTVLHQDWLNGK EYKCKVSNKALPLPEEKTISKAKGQPREPQVYTLPPSRDELTKNQVSLTC |
| | | LVKGFYPSDIAVEWESNGQPENNYKTTPPVLDSDGSFFLYSKLTVDKSRW QQGNVFSCSVMHEALHNHYTQKSLSLSPGK |
| 98 | CL kappa | |
| 99 | linker | (GGGGS)ₙ |
| 100 | HER2 HCDR1 | DTYIH |
| | (Kabat) | |
| 101 | HER2 HCDR1 | GFNIKDT |
| | (Chothia) | |
| 102 | HER2 HCDR2 | RIYPTNGYTRYADSVKG |
| | (Kabat) | |
| 103 | HER2 HCDR2 | YPTNGY |
| | (Chothia) | |
| 104 | HER2 HCDR3 | WGGDGFYAMDY |
| | (Kabat) | |
| | (Chothia) | |
| 105 | HER2 LCDR1 | RASQDVNTAVA |
| | (Kabat) | |
| | (Chothia) | |
| 106 | HER2 LCDR2 | SASFLYS |
| | (Kabat) | |
| | (Chothia) | |
| 107 | HER2 LCDR3 | QQHYTTPPT |
| | (Kabat) | |
| | (Chothia) | |
| 108 | HER2 VH | |
| 109 | HER2 VL | |
| 110 | HER2/3A7 (1+1A) Fc-silenced H1 | EVQLVESGGGLVQPGGSLRLSCAASGFNIKDTYIHWVRQAPGKGLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKVEPKSCDKTHTCPPCPAPEAAGGPSVFLFPPKP KDTLMISRTPEVTCVVVDVSHEDPEVKFNWYVDGVEVHNAKTKPREEQYN STYRVVSVLTVLHQDWLNGKEYKCKVSNKALGAPIEKTISKAKGQPREPQ VCTLPPSRDELTKNQVSLSCAVKGFYPSDIAVEWESNGQPENNYKTTPPV LDSDGSFFLVSKLTVDKSRWQQGNVFSCSVMHEALHNRFTQKSLSLSPGK |
| | (HER2 hole) | |
| 111 | HER2/3A7 (1+1A) Fc-silenced H2 | QSLEESGGDLVKPGASLTLTCTASGFSFSGMYWICWVRQAPGKGLEWIAC IYTGSSTTGYASWAKGRFTISKTSSTTVTLQMTSLTAADTATHFCASPYG DSSHYRGLNLWGPGTLVTVSSRTVAAPSVFIFPPSDEQLKSGTASVVCLL NNFYPREAKVQWKVDNALQSGNSQESVTEQDSKDSTYSLSSTLTLSKADY EKHKVYACEVTHQGLSSPVTKSFNRGECDKTHTCPPCPAPEAAGGPSVFL FPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQ PREPQVYTLPPCRDELTKNQVSLWCLVKGFYPSDIAVEWESNGQPENNYK TTPPVLDSDGSFFLYSKLTVDKSRWQQGNVFSCSVMHEALHNHYTQKSLS LSPGK |
| | (3A7 knob) | |
| 112 | HER2/3A7 (1+1A) Fc-silenced L1 | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGQ GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC |
| | (HER2) | |
| 113 | HER2/3A7 (1+1A) Fc-silenced L2 | AVVMTQTPASVEAAVGGTVTINCQASQRINNYLSWFQQKPGQPPKLLIYG AATLASGVPSRFKGSGSGTEYTLTISDLEPADAATYYCQCTYGIPSSNYG FGGGTEVVVKSSASTKGPSVFPLAPSSKSTSGGTAALGCLVKDYFPEPVT VSWNSGALTSGVHTFPAVLQSSGLYSLSSVVTVPSSSLGTQTYICNVNHK PSNTKVDKKVEPKSC |
| | (3A7) | |
| 114 | HER2/5C7 (1+1A) Fc-silenced H1 | EVQLVESGGGLVQPGGSLRLSCAASGFNIKDTYIHWVRQAPGKGLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKVEPKSCDKTHTCPPCPAPEAAGGPSVFLFPPKP KDTLMISRTPEVTCVVVDVSHEDPEVKFNWYVDGVEVHNAKTKPREEQYN STYRVVSVLTVLHQDWLNGKEYKCKVSNKALGAPIEKTISKAKGQPREPQ VCTLPPSRDELTKNQVSLSCAVKGFYPSDIAVEWESNGQPENNYKTTPPV LDSDGSFFLVSKLTVDKSRWQQGNVFSCSVMHEALHNRFTQKSLSLSPGK |
| | (HER2 hole) | |
| 115 | HER2/5C7 (1+1A) Fc-silenced H2 | QSLEESGGDLVKPGASLTLTCTASGFSFSRSSYMCWVRQAPGKGLEWIGC LSVGDGHTYYASWAKGRFTISKTSSTSVTLQMTRLTAADTATYFCARAPS YSGDGGHGYDLWGPGTLVTVSSRTVAAPSVFIFPPSDEQLKSGTASVVCL LNNFYPREAKVQWKVDNALQSGNSQESVTEQDSKDSTYSLSSTLTLSKAD YEKHKVYACEVTHQGLSSPVTKSFNRGECDKTHTCPPCPAPEAAGGPSVF LFPPKPKDTLMISRTPEVTCVVVDVSHEDPEVKFNWYVDGVEVHNAKTKP REEQYNSTYRVVSVLTVLHQDWLNGKEYKCKVSNKALGAPIEKTISKAKG QPREPQVYTLPPCRDELTKNQVSLWCLVKGFYPSDIAVEWESNGQPENNY KTTPPVLDSDGSFFLYSKLTVDKSRWQQGNVFSCSVMHEALHNHYTQKSL SLSPGK |
| | (5C7 knob) | |
| 116 | HER2/5C7 (1+1A) Fc-silenced L1 | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGQ GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC |
| | (HER2) | |
| 117 | HER2/5C7 (1+1A) Fc-silenced L2 | AIVMTQTPSSKSVPVGDTVTINCQASESVDRNNRLAWFQQKPGQPPKLLI YTTSTLASGVPSRFKGSGSGTHFTLTISDVVPDDAATYYCAGYRGAHTDG DAFGGGTEVVVKSSASTKGPSVFPLAPSSKSTSGGTAALGCLVKDYFPEP VTVSWNSGALTSGVHTFPAVLQSSGLYSLSSVVTVPSSSLGTQTYICNVN HKPSNTKVDKKVEPKSC |
| | (5C7) | |
| 118 | HER2/3A7 (2+2A) Fc-silenced H | EVQLVESGGGLVQPGGSLRLSCAASGFNIKDTYIHWVRQAPGKGLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKVEPKSCDKTHTCPPCPAPEAAGGPSVFLFPPKP KDTLMISRTPEVTCVVVDVSHEDPEVKFNWYVDGVEVHNAKTKPREEQYN STYRVVSVLTVLHQDWLNGKEYKCKVSNKALGAPIEKTISKAKGQPREPQ VYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| 119 | HER2/3A7 (2+2A) Fc-silenced L | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGQ GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGECGGGGSGGGGSGGGGSAVVMTQTPASVEAAVGGTVTI NCQASQRINNYLSWFQQKPGQPPKLLIYGAATLASGVPSRFKGSGSGTEY TLTISDLEPADAATYYCQCTYGIPSSNYGFGGGTEVVVKGGGGSGGGGSG GGGSGGGGSQSLEESGGDLVKPGASLTLTCTASGFSFSGMYWICWVRQAP GKGLEWIACIYTGSSTTGYASWAKGRFTISKTSSTTVTLQMTSLTAADTA THFCASPYGDSSHYRGLNLWGPGTLVTVSS |
| 120 | HER2/5C7 (2+2A) Fc-silenced H | EVQLVESGGGLVQPGGSLRLSCAASGFNIKDTYIHWVRQAPGKGLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK |
| | | DYFPEPVTVSWNSGALTSGVHTFPAVLQSSGLYSLSSVVTVPSSSLGTQT YICNVNHKPSNTKVDKKVEPKSCDKTHTCPPCPAPEAAGGPSVFLFPPKP KDTLMISRTPEVTCVVVDVSHEDPEVKFNWYVDGVEVHNAKTKPREEQYN STYRVVSVLTVLHQDWLNGKEYKCKVSNKALGAPIEKTISKAKGQPREPQ VYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| 121 | HER2/5C7 (2+2A) Fc-silenced L | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGQ GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGECGGGGSGGGGSGGGGSAIVMTQTPSSKSVPVGDTVTI NCQASESVDRNNRLAWFQQKPGQPPKLLIYTTSTLASGVPSRFKGSGSGT HFTLTISDVVPDDAATYYCAGYRGAHTDGDAFGGGTEVVVKGGGGSGGGG SGGGGSGGGGSQSLEESGGDLVKPGASLTLTCTASGFSFSRSSYMCWVRQ APGKGLEWIGCLSVGDGHTYYASWAKGRFTISKTSSTSVTLQMTRLTAAD TATYFCARAPSYSGDGGHGYDLWGPGTLVTVSS |
| 122 | HER2/5C7 (2+2A) Fc-optimized H | EVQLVESGGGLVQPGGSLRLSCAASGFNIKDTYIHWVRQAPGKGLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKVEPKSCDKTHTCPPCPAPELLAGPSVFLFPPKP KDTLMISRTPEVTCVVVDVSHEDPEVKFNWYVDGVEVHNAKTKPREEQYN STYRVVSVLTVLHQDWLNGKEYKCKVSNKALPLPEEKTISKAKGQPREPQ VYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| 123 | HER2/5C7 (2+2A) Fc-optimized L | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGQ GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGECGGGGSGGGGSGGGGSAIVMTQTPSSKSVPVGDTVTI NCQASESVDRNNRLAWFQQKPGQPPKLLIYTTSTLASGVPSRFKGSGSGT HFTLTISDVVPDDAATYYCAGYRGAHTDGDAFGCGTEVVVKGGGGSGGGG SGGGGSGGGGSQSLEESGGDLVKPGASLTLTCTASGFSFSRSSYMCWVRQ APGKCLEWIGCLSVGDGHTYYASWAKGRFTISKTSSTSVTLQMTRLTAAD TATYFCARAPSYSGDGGHGYDLWGPGTLVTVSS |
| 124 | HER2 Fc-silenced H | EVQLVESGGGLVQPGGSLRLSCAASGFNIKDTYIHWVRQAPGKGLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKVEPKSCDKTHTCPPCPAPEAAGGPSVFLFPPKP KDTLMISRTPEVTCVVVDVSHEDPEVKFNWYVDGVEVHNAKTKPREEQYN STYRVVSVLTVLHQDWLNGKEYKCKVSNKALGAPIEKTISKAKGQPREPQ VYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| 125 | HER2 Fc-silenced L | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGQ GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC |
| 126 | EGFR1 HCDR1 | TYGMH |
| | (Kabat) | |
| 127 | EGFR1 HCDR1 | GFTFSTY |
| | (Chothia) | |
| 128 | EGFR1 HCDR2 | VIWDDGSYKYYGDSVKG |
| | (Kabat) | |
| 129 | EGFR1 HCDR2 | WDDGSY |
| | (Chothia) | |
| 130 | EGFR1 HCDR3 | DGITMVRGVMKDYFDY |
| | (Kabat) | |
| | (Chothia) | |
| 131 | EGFR1 LCDR1 | RASQDISSALV |
| | (Kabat) | |
| | (Chothia) | |
| 132 | EGFR1 LCDR2 | DASSLES |
| | (Kabat) | |
| | (Chothia) | |
| 133 | EGFR1 LCDR3 | QQFNSYPLT |
| | (Kabat) | |
| | (Chothia) | |
| 134 | EGFR1 VH | |
| 135 | EGFR1 VL | |
| 136 | EGFR2 HCDR1 | NYYIY |
| | (Kabat) | |
| 137 | EGFR2 HCDR1 | GYTFTNY |
| | (Chothia) | |
| 138 | EGFR2 HCDR2 | GINPTSGGSNFNEKFKT |
| | (Kabat) | |
| 139 | EGFR2 HCDR2 | NPTSGG |
| | (Chothia) | |
| 140 | EGFR2 HCDR3 | QGLWFDSDGRGFDF |
| | (Kabat) | |
| | (Chothia) | |
| 141 | EGFR2 LCDR1 | RSSQNIVHSNGNTYLD |
| | (Kabat) | |
| | (Chothia) | |
| 142 | EGFR2 LCDR2 | KVSNRFS |
| | (Kabat) | |
| | (Chothia) | |
| 143 | EGFR2 LCDR3 | FQYSHVPWT |
| | (Kabat) | |
| | (Chothia) | |
| 144 | EGFR2 VH | |
| 145 | EGFR2 VL | DIQMTQSPSSLSASVGDRVTITCRSSQNIVHSNGNTYLDWYQQTPGKAPK LLIYKVSNRFSGVPSRFSGSGSGTDFTFTISSLQPEDIATYYCFQYSHVP WTFGQGTKLEIK |
| 146 | EGFR1/5C7 (2+2A) Fc-optimized H | QVQLVESGGGVVQPGRSLRLSCAASGFTFSTYGMHWVRQAPGKGLEWVAV IWDDGSYKYYGDSVKGRFTISRDNSKNTLYLQMNSLRAEDTAVYYCARDG ITMVRGVMKDYFDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPELLAGPSVFL FPPKPKDTLMISRTPEVTCVVVDVSHEDPEVKFNWYVDGVEVHNAKTKPR EEQYNSTYRVVSVLTVLHQDWLNGKEYKCKVSNKALPLPEEKTISKAKGQ PREPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKSLS LSPGK |
| 147 | EGFR1/5C7 (2+2A) Fc-optimized L | AIQLTQSPSSLSASVGDRVTITCRASQDISSALVWYQQKPGKAPKLLIYD ASSLESGVPSRFSGSESGTDFTLTISSLQPEDFATYYCQQFNSYPLTFGG GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGECGGGGSGGGGSGGGGSAIVMTQTPSSKSVPVGDTVTI NCQASESVDRNNRLAWFQQKPGQPPKLLIYTTSTLASGVPSRFKGSGSGT HFTLTISDVVPDDAATYYCAGYRGAHTDGDAFGCGTEVVVKGGGGSGGGG SGGGGSGGGGSQSLEESGGDLVKPGASLTLTCTASGFSFSRSSYMCWVRQ APGKCLEWIGCLSVGDGHTYYASWAKGRFTISKTSSTSVTLQMTRLTAAD TATYFCARAPSYSGDGGHGYDLWGPGTLVTVSS |
| 148 | EGFR2/5C7 (2+2A) Fc-optimized H | QVQLQQSGAEVKKPGSSVKVSCKASGYTFTNYYIYWVRQAPGQGLEWIGG INPTSGGSNFNEKFKTRVTITADESSTTAYMELSSLRSEDTAFYFCTRQG LWFDSDGRGFDFWGQGTTVTVSSASTKGPSVFPLAPSSKSTSGGTAALGC LVKDYFPEPVTVSWNSGALTSGVHTFPAVLQSSGLYSLSSVVTVPSSSLG TQTYICNVNHKPSNTKVDKKVEPKSCDKTHTCPPCPAPELLAGPSVFLFP PKPKDTLMISRTPEVTCVVVDVSHEDPEVKFNWYVDGVEVHNAKTKPREE QYNSTYRVVSVLTVLHQDWLNGKEYKCKVSNKALPLPEEKTISKAKGQPR EPQVYTLPPSRDELTKNQVSLTCLVKGFYPSDIAVEWESNGQPENNYKTT PPVLDSDGSFFLYSKLTVDKSRWQQGNVFSCSVMHEALHNHYTQKSLSLS PGK |
| 149 | EGFR2/5C7 (2+2A) Fc-optimized L | DIQMTQSPSSLSASVGDRVTITCRSSQNIVHSNGNTYLDWYQQTPGKAPK LLIYKVSNRFSGVPSRFSGSGSGTDFTFTISSLQPEDIATYYCFQYSHVP WTFGQGTKLEIKRTVAAPSVFIFPPSDEQLKSGTASVVCLLNNFYPREAK VQWKVDNALQSGNSQESVTEQDSKDSTYSLSSTLTLSKADYEKHKVYACE VTHQGLSSPVTKSFNRGECGGGGSGGGGSGGGGSAIVMTQTPSSKSVPVG DTVTINCQASESVDRNNRLAWFQQKPGQPPKLLIYTTSTLASGVPSRFKG SGSGTHFTLTISDVVPDDAATYYCAGYRGAHTDGDAFGCGTEVVVKGGGG SGGGGSGGGGSGGGGSQSLEESGGDLVKPGASLTLTCTASGFSFSRSSYM CWVRQAPGKCLEWIGCLSVGDGHTYYASWAKGRFTISKTSSTSVTLQMTR LTAADTATYFCARAPSYSGDGGHGYDLWGPGTLVTVSS |
| 150 | EpCAM HCDR1 | NYLIE |
| | (Kabat) | |
| 151 | EpCAM HCDR1 | GYAFTNY |
| | (Chothia) | |
| 152 | EpCAM HCDR2 | VINPGSGGTNYNEKFKG |
| | (Kabat) | |
| 153 | EpCAM HCDR2 | NPGSGG |
| | (Chothia) | |
| 154 | EpCAM HCDR3 | DGPWFAY |
| | (Kabat) | |
| | (Chothia) | |
| 155 | EpCAM LCDR1 | KASENVVTYVS |
| | (Kabat) | |
| | (Chothia) | |
| 156 | EpCAM LCDR2 | GASNRYT |
| | (Kabat) | |
| | (Chothia) | |
| 157 | EpCAM LCDR3 | GQGYSYPYT |
| | (Kabat) | |
| | (Chothia) | |
| 158 | EpCAM VH | QVQLQQSGAELVRPGTSVKVSCKASGYAFTNYLIEWVKQRPGQGLEWIGV INPGSGGTNYNEKFKGKATLTADKSSSTAYMQLSSLTSDDSAVYFCARDG PWFAYWGQGTLVTVSA |
| 159 | EpCAM VL | NIVMTQSPKSMSMSVGERVTLTCKASENVVTYVSWYQQKPEQSPKLLIYG ASNRYTGVPDRFTGSGSATDFTLTISSVQAEDLADYHCGQGYSYPYTFGG GTKLEIK |
| 160 | EpCAM/5C7 (2+2A) Fc-optimized H | QVQLQQSGAELVRPGTSVKVSCKASGYAFTNYLIEWVKQRPGQGLEWIGV INPGSGGTNYNEKFKGKATLTADKSSSTAYMQLSSLTSDDSAVYFCARDG PWFAYWGQGTLVTVSAASTKGPSVFPLAPSSKSTSGGTAALGCLVKDYFP EPVTVSWNSGALTSGVHTFPAVLQSSGLYSLSSVVTVPSSSLGTQTYICN VNHKPSNTKVDKKVEPKSCDKTHTCPPCPAPELLAGPSVFLFPPKPKDTL MISRTPEVTCVVVDVSHEDPEVKFNWYVDGVEVHNAKTKPREEQYNSTYR VVSVLTVLHQDWLNGKEYKCKVSNKALPLPEEKTISKAKGQPREPQVYTL PPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK |
| 161 | EpCAM/5C7 (2+2A) Fc-optimized L | NIVMTQSPKSMSMSVGERVTLTCKASENVVTYVSWYQQKPEQSPKLLIYG ASNRYTGVPDRFTGSGSATDFTLTISSVQAEDLADYHCGQGYSYPYTFGG GTKLEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGECGGGGSGGGGSGGGGSAIVMTQTPSSKSVPVGDTVTI NCQASESVDRNNRLAWFQQKPGQPPKLLIYTTSTLASGVPSRFKGSGSGT HFTLTISDVVPDDAATYYCAGYRGAHTDGDAFGCGTEVVVKGGGGSGGGG SGGGGSGGGGSQSLEESGGDLVKPGASLTLTCTASGFSFSRSSYMCWVRQ APGKCLEWIGCLSVGDGHTYYASWAKGRFTISKTSSTSVTLQMTRLTAAD TATYFCARAPSYSGDGGHGYDLWGPGTLVTVSS |
| 162 | EpCAM Fc-optimized H | QVQLQQSGAELVRPGTSVKVSCKASGYAFTNYLIEWVKQRPGQGLEWIGV INPGSGGTNYNEKFKGKATLTADKSSSTAYMQLSSLTSDDSAVYFCARDG PWFAYWGQGTLVTVSAASTKGPSVFPLAPSSKSTSGGTAALGCLVKDYFP EPVTVSWNSGALTSGVHTFPAVLQSSGLYSLSSVVTVPSSSLGTQTYICN VNHKPSNTKVDKKVEPKSCDKTHTCPPCPAPELLAGPSVFLFPPKPKDTL MISRTPEVTCVVVDVSHEDPEVKFNWYVDGVEVHNAKTKPREEQYNSTYR VVSVLTVLHQDWLNGKEYKCKVSNKALPLPEEKTISKAKGQPREPQVYTL PPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK |
| 163 | EpCAM Fc-optimized L | NIVMTQSPKSMSMSVGERVTLTCKASENVVTYVSWYQQKPEQSPKLLIYG ASNRYTGVPDRFTGSGSATDFTLTISSVQAEDLADYHCGQGYSYPYTFGG GTKLEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC |
| 164 | GPRC5D HCDR1 | GYTMN |
| | (Kabat) | |
| 165 | GPRC5D HCDR1 | GYSFTGY |
| | (Chothia) | |
| 166 | GPRC5D HCDR2 | LINPYNSDTNYAQKLQG |
| | (Kabat) | |
| 167 | GPRC5D HCDR2 | NPYNSD |
| | (Chothia) | |
| 168 | GPRC5D HCDR3 | VALRVALDY |
| | (Kabat) | |
| | (Chothia) | |
| 169 | GPRC5D LCDR1 | KASQNVATHVG |
| | (Kabat) | |
| | (Chothia) | |
| 170 | GPRC5D LCDR2 | SASYRYS |
| | (Kabat) | |
| | (Chothia) | |
| 171 | GPRC5D LCDR3 | QQYNRYPYT |
| | (Kabat) | |
| | (Chothia) | |
| 172 | GPRC5D VH | QVQLVQSGAEVKKPGASVKVSCKASGYSFTGYTMNWVRQAPGQGLEWMGL INPYNSDTNYAQKLQGRVTMTTDTSTSTAYMELRSLRSDDTAVYYCARVA LRVALDYWGQGTLVTVSS |
| 173 | GPRC5D VL | DIQMTQSPSSLSASVGDRVTITCKASQNVATHVGWYQQKPGKAPKRLIYS ASYRYSGVPSRFSGSGSGTEFTLTISNLQPEDFATYYCQQYNRYPYTFGQ GTKLEIK |
| 174 | GPRC5D/5C7 (2+2A) Fc-optimized H | QVQLVQSGAEVKKPGASVKVSCKASGYSFTGYTMNWVRQAPGQGLEWMGL INPYNSDTNYAQKLQGRVTMTTDTSTSTAYMELRSLRSDDTAVYYCARVA LRVALDYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYI CNVNHKPSNTKVDKKVEPKSCDKTHTCPPCPAPELLAGPSVFLFPPKPKD TLMISRTPEVTCVVVDVSHEDPEVKFNWYVDGVEVHNAKTKPREEQYNST YRVVSVLTVLHQDWLNGKEYKCKVSNKALPLPEEKTISKAKGQPREPQVY TLPPSRDELTKNQVSLTCLVKGFYPSDIAVEWESNGQPENNYKTTPPVLD SDGSFFLYSKLTVDKSRWQQGNVFSCSVMHEALHNHYTQKSLSLSPGK |
| 175 | GPRC5D/5C7 (2+2A) Fc-optimized L | DIQMTQSPSSLSASVGDRVTITCKASQNVATHVGWYQQKPGKAPKRLIYS ASYRYSGVPSRFSGSGSGTEFTLTISNLQPEDFATYYCQQYNRYPYTFGQ GTKLEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGECGGGGSGGGGSGGGGSDIQMTQSPSSLSASVGDRVTI TCQASESVDRNNRLAWFQQKPGKPPKLLIYTTSTLASGVPSRFSGSGSGT DFTLTISSLQPEDVATYYCAGYRGAHTDGDAFGCGTKVEIKGGGGSGGGG SGGGGSGGGGSEQQLVESGGGLVKPGGSLRLSCAASGFSFSRSSYMCWVR QAPGKCLEWIGCLSVGDGHTYYASWAKGRFTISKDSAKNSVYLQMNSLRA EDTAVYFCARAPSYSGDGGHGYDLWGPGTLVTVSS |
| 176 | BCMA HCDR1 | SGSYFWG |
| | (Kabat) | |
| 177 | BCMA HCDR1 | GGSISSGSY |
| | (Chothia) | |
| 178 | BCMA HCDR2 | SIYYSGITYYNPSLKS |
| | (Kabat) | |
| 179 | BCMA HCDR2 | YYSGI |
| | (Chothia) | |
| 180 | BCMA HCDR3 | HDGAVAGLFDY |
| | (Kabat) | |
| | (Chothia) | |
| 181 | BCMA LCDR1 | GGNNIGSKSVH |
| | (Kabat) | |
| | (Chothia) | |
| 182 | BCMA LCDR2 | DDSDRPS |
| | (Kabat) | |
| | (Chothia) | |
| 183 | BCMA LCDR3 | QVWDSSSDHVV |
| | (Kabat) | |
| | (Chothia) | |
| 184 | BCMA VH | QLQLQESGPGLVKPSETLSLTCTVSGGSISSGSYFWGWIRQPPGKGLEWI GSIYYSGITYYNPSLKSRVTISVDTSKNQFSLKLSSVTAADTAVYYCARH DGAVAGLFDYWGQGTLVTVSS |
| 185 | BCMA VL | SYVLTQPPSVSVAPGQTARITCGGNNIGSKSVHWYQQPPGQAPVVVVYDD SDRPSGIPERFSGSNSGNTATLTISRVEAGDEAVYYCQVWDSSSDHVVFG GGTKLTVLGQP |
| 186 | BCMA/5C7 (2+2A) Fc-optimized H | QLQLQESGPGLVKPSETLSLTCTVSGGSISSGSYFWGWIRQPPGKGLEWI GSIYYSGITYYNPSLKSRVTISVDTSKNQFSLKLSSVTAADTAVYYCARH DGAVAGLFDYWGQGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPELLAGPSVFLFPPK PKDTLMISRTPEVTCVVVDVSHEDPEVKFNWYVDGVEVHNAKTKPREEQY NSTYRVVSVLTVLHQDWLNGKEYKCKVSNKALPLPEEKTISKAKGQPREP QVYTLPPSRDELTKNQVSLTCLVKGFYPSDIAVEWESNGQPENNYKTTPP |
| | | VLDSDGSFFLYSKLTVDKSRWQQGNVFSCSVMHEALHNHYTQKSLSLSPG K |
| 187 | BCMA/5C7 (2+2A) Fc-optimized L | SYVLTQPPSVSVAPGQTARITCGGNNIGSKSVHWYQQPPGQAPVVVVYDD SDRPSGIPERFSGSNSGNTATLTISRVEAGDEAVYYCQVWDSSSDHVVFG GGTKLTVLGQPKAAPSVTLFPPSSEELQANKATLVCLISDFYPGAVTVAW KGDSSPVKAGVETTTPSKQSNNKYAASSYLSLTPEQWKSHRSYSCQVTHE GSTVEKTVAPTECSGGGGSGGGGSGGGGSDIQMTQSPSSLSASVGDRVTI TCQASESVDRNNRLAWFQQKPGKPPKLLIYTTSTLASGVPSRFSGSGSGT DFTLTISSLQPEDVATYYCAGYRGAHTDGDAFGCGTKVEIKGGGGSGGGG SGGGGSGGGGSEQQLVESGGGLVKPGGSLRLSCAASGFSFSRSSYMCWVR QAPGKCLEWIGCLSVGDGHTYYASWAKGRFTISKDSAKNSVYLQMNSLRA EDTAVYFCARAPSYSGDGGHGYDLWGPGTLVTVSS |
| 188 | CD38 HCDR1 | SFAMS |
| | (Kabat) | |
| 189 | CD38 HCDR1 | GFTFNSF |
| | (Chothia) | |
| 190 | CD38 HCDR2 | AISGSGGGTYYADSVKG |
| | (Kabat) | |
| 191 | CD38 HCDR2 | SGSGGG |
| | (Chothia) | |
| 192 | CD38 HCDR3 | DKILWFGEPVFDY |
| | (Kabat) | |
| | (Chothia) | |
| 193 | CD38 LCDR1 | RASQSVSSYLA |
| | (Kabat) | |
| | (Chothia) | |
| 194 | CD38 LCDR2 | DASNRAT |
| | (Kabat) | |
| | (Chothia) | |
| 195 | CD38 LCDR3 | QQRSNWPPT |
| | (Kabat) | |
| | (Chothia) | |
| 196 | CD38 VH | |
| 197 | CD38 VL | |
| 198 | CD38/5C7 (2+2A) Fc-optimized H | EVQLLESGGGLVQPGGSLRLSCAVSGFTFNSFAMSWVRQAPGKGLEWVSA ISGSGGGTYYADSVKGRFTISRDNSKNTLYLQMNSLRAEDTAVYFCAKDK ILWFGEPVFDYWGQGTLVTVSSASTKGPSVFPLAPSSKSTSGGTAALGCL VKDYFPEPVTVSWNSGALTSGVHTFPAVLQSSGLYSLSSVVTVPSSSLGT QTYICNVNHKPSNTKVDKKVEPKSCDKTHTCPPCPAPELLAGPSVFLFPP KPKDTLMISRTPEVTCVVVDVSHEDPEVKFNWYVDGVEVHNAKTKPREEQ YNSTYRVVSVLTVLHQDWLNGKEYKCKVSNKALPLPEEKTISKAKGQPRE PQVYTLPPSRDELTKNQVSLTCLVKGFYPSDIAVEWESNGQPENNYKTTP PVLDSDGSFFLYSKLTVDKSRWQQGNVFSCSVMHEALHNHYTQKSLSLSP GK |
| 199 | CD38/5C7 (2+2A) Fc-optimized L | EIVLTQSPATLSLSPGERATLSCRASQSVSSYLAWYQQKPGQAPRLLIYD ASNRATGIPARFSGSGSGTDFTLTISSLEPEDFAVYYCQQRSNWPPTFGQ GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGECGGGGSGGGGSGGGGSDIQMTQSPSSLSASVGDRVTI TCQASESVDRNNRLAWFQQKPGKPPKLLIYTTSTLASGVPSRFSGSGSGT DFTLTISSLQPEDVATYYCAGYRGAHTDGDAFGCGTKVEIKGGGGSGGGG SGGGGSGGGGSEQQLVESGGGLVKPGGSLRLSCAASGFSFSRSSYMCWVR |
| | | QAPGKCLEWIGCLSVGDGHTYYASWAKGRFTISKDSAKNSVYLQMNSLRA EDTAVYFCARAPSYSGDGGHGYDLWGPGTLVTVSS |
| 200 | CD79b HCDR1 | IDYWIS |
| | (Kabat) | |
| 201 | CD79b HCDR1 | GFTINIDY |
| | (Chothia) | |
| 202 | CD79b HCDR2 | IIDPGDGTTYFAIWVSG |
| | (Kabat) | |
| 203 | CD79b HCDR2 | DPGDGT |
| | (Chothia) | |
| 204 | CD79b HCDR3 | EFSASAYQYDL |
| | (Kabat) | |
| | (Chothia) | |
| 205 | CD79b LCDR1 | RASEDIENYLA |
| | (Kabat) | |
| | (Chothia) | |
| 206 | CD79b LCDR2 | RASTLSS |
| | (Kabat) | |
| | (Chothia) | |
| 207 | CD79b LCDR3 | QQGYRNSNVDNP |
| | (Kabat) | |
| | (Chothia) | |
| 208 | CD79b VH | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSS |
| 209 | CD79b VL | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIK |
| 210 | CD79b/5C7 (1+1A) Fc-silenced H1 | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPEAAGGPSVFLFPPK PKDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREP QVCTLPPSRDELTKNQVSLSCAVKGFYPSDIAVEWESNGQPENNYKTTPP VLDSDGSFFLVSKLTVDKSRWQQGNVFSCSVMHEALHNRFTQKSLSLSPG K |
| | (CD79b hole) | |
| 211 | CD79b/5C7 (1+1A) Fc-silenced H2 | EQQLVESGGGLVKPGGSLRLSCAASGFSFSRSSYMCWVRQAPGKGLEWIG CLSVGDGHTYYASWAKGRFTISKDSAKNSVYLQMNSLRAEDTAVYFCARA PSYSGDGGHGYDLWGPGTLVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTCPPCPAPEAAGGPSVFLF PPKPKDTLMISRTPEVTCVVVDVSHEDPEVKFNWYVDGVEVHNAKTKPRE EQYNSTYRVVSVLTVLHQDWLNGKEYKCKVSNKALGAPIEKTISKAKGQP REPQVYTLPPCRDELTKNQVSLWCLVKGFYPSDIAVEWESNGQPENNYKT TPPVLDSDGSFFLYSKLTVDKSRWQQGNVFSCSVMHEALHNHYTQKSLSL SPGK |
| | (5C7 knob) | |
| 212 | CD79b/5C7 (1+1A) Fc-silenced L1 | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC |
| | (CD79b) | |
| 213 | CD79b/5C7 (1+1A) Fc-silenced L2 | DIQMTQSPSSLSASVGDRVTITCQASESVDRNNRLAWFQQKPGKPPKLLI YTTSTLASGVPSRFSGSGSGTDFTLTISSLQPEDVATYYCAGYRGAHTDG DAFGGGTKVEIKRTVAAPSVFIFPPSDEQLKSGTASVVCLLNNFYPREAK |
| | (5C7) | |
| | | VQWKVDNALQSGNSQESVTEQDSKDSTYSLSSTLTLSKADYEKHKVYACE VTHQGLSSPVTKSFNRGEC |
| 214 | CD79b/5C7 (1+1B) Fc-silenced H1 | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPEAAGGPSVFLFPPK PKDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREP QVCTLPPSRDELTKNQVSLSCAVKGFYPSDIAVEWESNGQPENNYKTTPP VLDSDGSFFLVSKLTVDKSRWQQGNVFSCSVMHEALHNRFTQKSLSLSPG K |
| | (CD79b hole) | |
| 215 | CD79b/5C7 (1+1B) Fc-silenced H2 | DIQMTQSPSSLSASVGDRVTITCQASESVDRNNRLAWFQQKPGKPPKLLI YTTSTLASGVPSRFSGSGSGTDFTLTISSLQPEDVATYYCAGYRGAHTDG DAFGGGTKVEIKGGGGSGGGGSGGGGSGGGGSEQQLVESGGGLVKPGGSL RLSCAASGFSFSRSSYMCWVRQAPGKGLEWIGCLSVGDGHTYYASWAKGR FTISKDSAKNSVYLQMNSLRAEDTAVYFCARAPSYSGDGGHGYDLWGPGT LVTVSSDKTHTCPPCPAPEAAGGPSVFLFPPKPKDTLMISRTPEVTCVVV DVSHEDPEVKFNWYVDGVEVHNAKTKPREEQYNSTYRVVSVLTVLHQDWL NGKEYKCKVSNKALGAPIEKTISKAKGQPREPQVYTLPPCRDELTKNQVS LWCLVKGFYPSDIAVEWESNGQPENNYKTTPPVLDSDGSFFLYSKLTVDK SRWQQGNVFSCSVMHEALHNHYTQKSLSLSPGK |
| | (5C7 knob) | |
| 216 | CD79b/5C7 (1+1B) Fc-silenced L1 | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC |
| | (CD79b) | |
| 217 | CD79b/5C7 (2+1A) Fc-silenced H1 | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDGGGGSGGGGSGGGGSEQQLVETGGG LIQPGGSLRLSCAASGFTINIDYWISWVRQAPGKGLEWIGIIDPGDGTTY FAIWVSGRFTISFDNSKNTVYLQMNSLRAEDTAVYFCVREFSASAYQYDL WGPGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKP SNTKVDKKVEPKSCDKTHTCPPCPAPEAAGGPSVFLFPPKPKDTLMISRT PEVTCVVVDVSHEDPEVKFNWYVDGVEVHNAKTKPREEQYNSTYRVVSVL TVLHQDWLNGKEYKCKVSNKALGAPIEKTISKAKGQPREPQVCTLPPSRD ELTKNQVSLSCAVKGFYPSDIAVEWESNGQPENNYKTTPPVLDSDGSFFL VSKLTVDKSRWQQGNVFSCSVMHEALHNRFTQKSLSLSPGK |
| | (CD79b hole) | |
| 218 | CD79b/5C7 (2+1A) Fc-silenced H2 | DIQMTQSPSSLSASVGDRVTITCQASESVDRNNRLAWFQQKPGKPPKLLI YTTSTLASGVPSRFSGSGSGTDFTLTISSLQPEDVATYYCAGYRGAHTDG DAFGGGTKVEIKGGGGSGGGGSGGGGSGGGGSEQQLVESGGGLVKPGGSL RLSCAASGFSFSRSSYMCWVRQAPGKGLEWIGCLSVGDGHTYYASWAKGR FTISKDSAKNSVYLQMNSLRAEDTAVYFCARAPSYSGDGGHGYDLWGPGT LVTVSSDKTHTCPPCPAPEAAGGPSVFLFPPKPKDTLMISRTPEVTCVVV DVSHEDPEVKFNWYVDGVEVHNAKTKPREEQYNSTYRVVSVLTVLHQDWL NGKEYKCKVSNKALGAPIEKTISKAKGQPREPQVYTLPPCRDELTKNQVS LWCLVKGFYPSDIAVEWESNGQPENNYKTTPPVLDSDGSFFLYSKLTVDK SRWQQGNVFSCSVMHEALHNHYTQKSLSLSPGK |
| | (5C7 knob) | |
| 219 | CD79b/5C7 (2+1A) Fc-silenced L1 | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ |
| | (CD79b) | |
| | | WKVDNALQSGNSQESVTEQDSKDSTYSLSSTLTLSKADYEKHKVYACEVT HQGLSSPVTKSFNRGEC |
| 220 | CD79b/5C7 (2+1B) Fc-silenced H1 | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPEAAGGPSVFLFPPK PKDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREP QVCTLPPSRDELTKNQVSLSCAVKGFYPSDIAVEWESNGQPENNYKTTPP VLDSDGSFFLVSKLTVDKSRWQQGNVFSCSVMHEALHNRFTQKSLSLSPG K |
| | (CD79b hole) | |
| 221 | CD79b/5C7 (2+1B) Fc-silenced H2 | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPEAAGGPSVFLFPPK PKDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREP QVYTLPPCRDELTKNQVSLWCLVKGFYPSDIAVEWESNGQPENNYKTTPP VLDSDGSFFLYSKLTVDKSRWQQGNVFSCSVMHEALHNHYTQKSLSLSPG KGGGGSGGGGSGGGGSDIQMTQSPSSLSASVGDRVTITCQASESVDRNNR LAWFQQKPGKPPKLLIYTTSTLASGVPSRFSGSGSGTDFTLTISSLQPED VATYYCAGYRGAHTDGDAFGGGTKVEIKGGGGSGGGGSGGGGSGGGGSEQ QLVESGGGLVKPGGSLRLSCAASGFSFSRSSYMCWVRQAPGKGLEWIGCL SVGDGHTYYASWAKGRFTISKDSAKNSVYLQMNSLRAEDTAVYFCARAPS YSGDGGHGYDLWGPGTLVTVSS |
| | (CD79b-5C7 knob) | |
| 222 | CD79b/5C7 (2+1B) Fc-silenced L1 | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC |
| | (CD79b) | |
| 223 | CD79b/5C7 (2+2A) Fc-silenced H | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPEAAGGPSVFLFPPK PKDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREP QVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG K |
| 224 | CD79b/5C7 (2+2A) Fc-silenced L | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGECGGGGSGGGGSGGGGSDIQMTQSPSSLSASVGDR VTITCQASESVDRNNRLAWFQQKPGKPPKLLIYTTSTLASGVPSRFSGSG SGTDFTLTISSLQPEDVATYYCAGYRGAHTDGDAFGGGTKVEIKGGGGSG GGGSGGGGSGGGGSEQQLVESGGGLVKPGGSLRLSCAASGFSFSRSSYMC WVRQAPGKGLEWIGCLSVGDGHTYYASWAKGRFTISKDSAKNSVYLQMNS LRAEDTAVYFCARAPSYSGDGGHGYDLWGPGTLVTVSS |
| 225 | CD79b/5C7 (2+2B) Fc-silenced H | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ |
| | | TYICNVNHKPSNTKVDKKVEPKSCDKTHTCPPCPAPEAAGGPSVFLFPPK PKDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREP QVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG KGGGGSGGGGSGGGGSDIQMTQSPSSLSASVGDRVTITCQASESVDRNNR LAWFQQKPGKPPKLLIYTTSTLASGVPSRFSGSGSGTDFTLTISSLQPED VATYYCAGYRGAHTDGDAFGGGTKVEIKGGGGSGGGGSGGGGSGGGGSEQ QLVESGGGLVKPGGSLRLSCAASGFSFSRSSYMCWVRQAPGKGLEWIGCL SVGDGHTYYASWAKGRFTISKDSAKNSVYLQMNSLRAEDTAVYFCARAPS YSGDGGHGYDLWGPGTLVTVSS |
| 226 | CD79b/5C7 (2+2B) Fc-silenced L | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC |
| 227 | CD79b/5C7 (1+1A) Fc-optimized H1 | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSS ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEP KSCDKTHTCPPCPAPELLAGPSVFLFPPKPKDTLMISRTPEVTCVVVDVS HEDPEVKFNWYVDGVEVHNAKTKPREEQYNSTYRVVSVLTVLHQDWLNGK EYKCKVSNKALPLPEEKTISKAKGQPREPQVYTLPPSRDELTKNQVSLSC AVKGFYPSDIAVEWESNGQPENNYKTTPPVLDSDGSFFLVSKLTVDKSRW QQGNVFSCSVMHEALHNHYTQKSLSLSPGK |
| | (CD79b hole) | |
| 228 | CD79b/5C7 (1+1A) Fc-optimized H2 | EQQLVESGGGLVKPGGSLRLSCAASGFSFSRSSYMCWVRQAPGKGLEWIG CLSVGDGHTYYASWAKGRFTISKDSAKNSVYLQMNSLRAEDTAVYFCARA PSYSGDGGHGYDLWGPGTLVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTCPPCPAPELLAGPSVFLF PPKPKDTLMISRTPEVTCVVVDVSHEDPEVKFNWYVDGVEVHNAKTKPRE EQYNSTYRVVSVLTVLHQDWLNGKEYKCKVSNKALPLPEEKTISKAKGQP REPQVYTLPPSRDELTKNQVSLWCLVKGFYPSDIAVEWESNGQPENNYKT TPPVLDSDGSFFLYSKLTVDKSRWQQGNVFSCSVMHEALHNHYTQKSLSL SPGK |
| | (5C7 knob) | |
| 229 | CD79b/5C7 (1+1A) Fc-optimized L1 | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC |
| | (CD79b) | |
| 230 | CD79b/5C7 (1+1A) Fc-optimized L2 | DIQMTQSPSSLSASVGDRVTITCQASESVDRNNRLAWFQQKPGKPPKLLI YTTSTLASGVPSRFSGSGSGTDFTLTISSLQPEDVATYYCAGYRGAHTDG DAFGGGTKVEIKRTVAAPSVFIFPPSDEQLKSGTASVVCLLNNFYPREAK VQWKVDNALQSGNSQESVTEQDSKDSTYSLSSTLTLSKADYEKHKVYACE VTHQGLSSPVTKSFNRGEC |
| | (5C7) | |
| 231 | CD79b/5C7 (1+1B) Fc-optimized H1 | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPELLAGPSVFLFPPK PKDTLMISRTPEVTCVVVDVSHEDPEVKFNWYVDGVEVHNAKTKPREEQY NSTYRVVSVLTVLHQDWLNGKEYKCKVSNKALPLPEEKTISKAKGQPREP QVYTLPPSRDELTKNQVSLSCAVKGFYPSDIAVEWESNGQPENNYKTTPP VLDSDGSFFLVSKLTVDKSRWQQGNVFSCSVMHEALHNHYTQKSLSLSPG K |
| | (CD79b hole) | |
| 232 | CD79b/5C7 (1+1B) Fc-optimized H2 | DIQMTQSPSSLSASVGDRVTITCQASESVDRNNRLAWFQQKPGKPPKLLI YTTSTLASGVPSRFSGSGSGTDFTLTISSLQPEDVATYYCAGYRGAHTDG DAFGGGTKVEIKGGGGSGGGGSGGGGSGGGGSEQQLVESGGGLVKPGGSL RLSCAASGFSFSRSSYMCWVRQAPGKGLEWIGCLSVGDGHTYYASWAKGR FTISKDSAKNSVYLQMNSLRAEDTAVYFCARAPSYSGDGGHGYDLWGPGT LVTVSSDKTHTCPPCPAPELLAGPSVFLFPPKPKDTLMISRTPEVTCVVV DVSHEDPEVKFNWYVDGVEVHNAKTKPREEQYNSTYRVVSVLTVLHQDWL NGKEYKCKVSNKALPLPEEKTISKAKGQPREPQVYTLPPSRDELTKNQVS LWCLVKGFYPSDIAVEWESNGQPENNYKTTPPVLDSDGSFFLYSKLTVDK SRWQQGNVFSCSVMHEALHNHYTQKSLSLSPGK |
| | (5C7 knob) | |
| 233 | CD79b/5C7 (1+1B) Fc-optimized L1 | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC |
| | (CD79b) | |
| 234 | CD79b/5C7 (2+1A) Fc-optimized H1 | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDGGGGSGGGGSGGGGSEQQLVETGGG LIQPGGSLRLSCAASGFTINIDYWISWVRQAPGKGLEWIGIIDPGDGTTY FAIWVSGRFTISFDNSKNTVYLQMNSLRAEDTAVYFCVREFSASAYQYDL WGPGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKP SNTKVDKKVEPKSCDKTHTCPPCPAPELLAGPSVFLFPPKPKDTLMISRT PEVTCVVVDVSHEDPEVKFNWYVDGVEVHNAKTKPREEQYNSTYRVVSVL TVLHQDWLNGKEYKCKVSNKALPLPEEKTISKAKGQPREPQVYTLPPSRD ELTKNQVSLSCAVKGFYPSDIAVEWESNGQPENNYKTTPPVLDSDGSFFL VSKLTVDKSRWQQGNVFSCSVMHEALHNHYTQKSLSLSPGK |
| | (CD79b hole) | |
| 235 | CD79b/5C7 (2+1A) Fc-optimized H2 | DIQMTQSPSSLSASVGDRVTITCQASESVDRNNRLAWFQQKPGKPPKLLI YTTSTLASGVPSRFSGSGSGTDFTLTISSLQPEDVATYYCAGYRGAHTDG DAFGGGTKVEIKGGGGSGGGGSGGGGSGGGGSEQQLVESGGGLVKPGGSL RLSCAASGFSFSRSSYMCWVRQAPGKGLEWIGCLSVGDGHTYYASWAKGR FTISKDSAKNSVYLQMNSLRAEDTAVYFCARAPSYSGDGGHGYDLWGPGT LVTVSSDKTHTCPPCPAPELLAGPSVFLFPPKPKDTLMISRTPEVTCVVV DVSHEDPEVKFNWYVDGVEVHNAKTKPREEQYNSTYRVVSVLTVLHQDWL NGKEYKCKVSNKALPLPEEKTISKAKGQPREPQVYTLPPSRDELTKNQVS LWCLVKGFYPSDIAVEWESNGQPENNYKTTPPVLDSDGSFFLYSKLTVDK SRWQQGNVFSCSVMHEALHNHYTQKSLSLSPGK |
| | (5C7 knob) | |
| 236 | CD79b/5C7 (2+1A) Fc-optimized L1 | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC |
| | (CD79b) | |
| 237 | CD79b/5C7 (2+1B) Fc-optimized H1 | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPELLAGPSVFLFPPK PKDTLMISRTPEVTCVVVDVSHEDPEVKFNWYVDGVEVHNAKTKPREEQY NSTYRVVSVLTVLHQDWLNGKEYKCKVSNKALPLPEEKTISKAKGQPREP QVYTLPPSRDELTKNQVSLSCAVKGFYPSDIAVEWESNGQPENNYKTTPP VLDSDGSFFLVSKLTVDKSRWQQGNVFSCSVMHEALHNHYTQKSLSLSPG K |
| | (CD79b hole) | |
| 238 | CD79b/5C7 (2+1B) Fc-optimized H2 | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE |
| | (CD79b-5C7 knob) | |
| | | FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPELLAGPSVFLFPPK PKDTLMISRTPEVTCVVVDVSHEDPEVKFNWYVDGVEVHNAKTKPREEQY NSTYRVVSVLTVLHQDWLNGKEYKCKVSNKALPLPEEKTISKAKGQPREP QVYTLPPSRDELTKNQVSLWCLVKGFYPSDIAVEWESNGQPENNYKTTPP VLDSDGSFFLYSKLTVDKSRWQQGNVFSCSVMHEALHNHYTQKSLSLSPG KGGGGSGGGGSGGGGSDIQMTQSPSSLSASVGDRVTITCQASESVDRNNR LAWFQQKPGKPPKLLIYTTSTLASGVPSRFSGSGSGTDFTLTISSLQPED VATYYCAGYRGAHTDGDAFGGGTKVEIKGGGGSGGGGSGGGGSGGGGSEQ QLVESGGGLVKPGGSLRLSCAASGFSFSRSSYMCWVRQAPGKGLEWIGCL SVGDGHTYYASWAKGRFTISKDSAKNSVYLQMNSLRAEDTAVYFCARAPS YSGDGGHGYDLWGPGTLVTVSS |
| 239 | CD79b/5C7 (2+1B) Fc-optimized L1 | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC |
| | (CD79b) | |
| 240 | CD79b/5C7 (2+2A) Fc-optimized H | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPELLAGPSVFLFPPK PKDTLMISRTPEVTCVVVDVSHEDPEVKFNWYVDGVEVHNAKTKPREEQY NSTYRVVSVLTVLHQDWLNGKEYKCKVSNKALPLPEEKTISKAKGQPREP QVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG K |
| 241 | CD79b/5C7 (2+2A) Fc-optimized L | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGECGGGGSGGGGSGGGGSDIQMTQSPSSLSASVGDR VTITCQASESVDRNNRLAWFQQKPGKPPKLLIYTTSTLASGVPSRFSGSG SGTDFTLTISSLQPEDVATYYCAGYRGAHTDGDAFGCGTKVEIKGGGGSG GGGSGGGGSGGGGSEQQLVESGGGLVKPGGSLRLSCAASGFSFSRSSYMC WVRQAPGKCLEWIGCLSVGDGHTYYASWAKGRFTISKDSAKNSVYLQMNS LRAEDTAVYFCARAPSYSGDGGHGYDLWGPGTLVTVSS |
| 242 | CD79b/5C7 (2+2B) Fc-optimized H | EQQLVETGGGLIQPGGSLRLSCAASGFTINIDYWISWVRQAPGKGLEWIG IIDPGDGTTYFAIWVSGRFTISFDNSKNTVYLQMNSLRAEDTAVYFCVRE FSASAYQYDLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPELLAGPSVFLFPPK PKDTLMISRTPEVTCVVVDVSHEDPEVKFNWYVDGVEVHNAKTKPREEQY NSTYRVVSVLTVLHQDWLNGKEYKCKVSNKALPLPEEKTISKAKGQPREP QVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG GGGGSGGGGSGGGGSDIQMTQSPSSLSASVGDRVTITCQASESVDRNNRL AWFQQKPGKPPKLLIYTTSTLASGVPSRFSGSGSGTDFTLTISSLQPEDV ATYYCAGYRGAHTDGDAFGCGTKVEIKGGGGSGGGGSGGGGSGGGGSEQQ LVESGGGLVKPGGSLRLSCAASGFSFSRSSYMCWVRQAPGKCLEWIGCLS VGDGHTYYASWAKGRFTISKDSAKNSVYLQMNSLRAEDTAVYFCARAPSY SGDGGHGYDLWGPGTLVTVSS |
| 243 | CD79b/5C7 (2+2B) Fc-optimized L | ELVMTQSPATLSVSPGERATLSCRASEDIENYLAWYQQKPGQPPRLLIYR ASTLSSGVPARFSGSGSGTEFTLTISSLQSEDFAVYYCQQGYRNSNVDNP FGGGTKVEIKRTVAAPSVFIFPPSDEQLKSGTASVVCLLNNFYPREAKVQ |
| | | WKVDNALQSGNSQESVTEQDSKDSTYSLSSTLTLSKADYEKHKVYACEVT HQGLSSPVTKSFNRGEC |
| 244 | CD79b/3A7 (2+2B) Fc-silenced H | QSLEESGGDLVKPGASLTLTCTASGFSFSGMYWICWVRQAPGKGLEWIAC IYTGSSTTGYASWAKGRFTISKTSSTTVTLQMTSLTAADTATHFCASPYG DSSHYRGLNLWGPGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTCPPCPAPEAAGGPSVFLFPPK PKDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREP QVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG KGGGGSGGGGSGGGGSDIQMTQSPSSLSASVGDRVTITCQASESVDRNNR LAWFQQKPGKPPKLLIYTTSTLASGVPSRFSGSGSGTDFTLTISSLQPED VATYYCAGYRGAHTDGDAFGGGTKVEIKGGGGSGGGGSGGGGSGGGGSEQ QLVESGGGLVKPGGSLRLSCAASGFSFSRSSYMCWVRQAPGKGLEWIGCL SVGDGHTYYASWAKGRFTISKDSAKNSVYLQMNSLRAEDTAVYFCARAPS YSGDGGHGYDLWGPGTLVTVSS |
| 245 | CD79b/3A7 (2+2B) Fc-silenced L | AVVMTQTPASVEAAVGGTVTINCQASQRINNYLSWFQQKPGQPPKLLIYG AATLASGVPSRFKGSGSGTEYTLTISDLEPADAATYYCQCTYGIPSSNYG FGGGTEVVVKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC |
| 246 | CD79b/13E6 (2+2B) Fc-silenced H | QSLEESGGDLVKPGGTLTLTCTASGFSFSSSYWLCWVRQAPGKGLEWIAC IYGGSSRGTYYASWAKGRFTISKTSSTTVTLQMTSLTAADTATYFCASHT YTYPRFSPYFNLWGPGTLVSVSSASTKGPSVFPLAPSSKSTSGGTAALGC LVKDYFPEPVTVSWNSGALTSGVHTFPAVLQSSGLYSLSSVVTVPSSSLG TQTYICNVNHKPSNTKVDKKVEPKSCDKTHTCPPCPAPEAAGGPSVFLFP PKPKDTLMISRTPEVTCVVVDVSHEDPEVKFNWYVDGVEVHNAKTKPREE QYNSTYRVVSVLTVLHQDWLNGKEYKCKVSNKALGAPIEKTISKAKGQPR EPQVYTLPPSRDELTKNQVSLTCLVKGFYPSDIAVEWESNGQPENNYKTT PPVLDSDGSFFLYSKLTVDKSRWQQGNVFSCSVMHEALHNHYTQKSLSLS PGKGGGGSGGGGSGGGGSDIQMTQSPSSLSASVGDRVTITCQASESVDRN NRLAWFQQKPGKPPKLLIYTTSTLASGVPSRFSGSGSGTDFTLTISSLQP EDVATYYCAGYRGAHTDGDAFGGGTKVEIKGGGGSGGGGSGGGGSGGGGS EQQLVESGGGLVKPGGSLRLSCAASGFSFSRSSYMCWVRQAPGKGLEWIG CLSVGDGHTYYASWAKGRFTISKDSAKNSVYLQMNSLRAEDTAVYFCARA PSYSGDGGHGYDLWGPGTLVTVSS |
| 247 | CD79b/13E6 (2+2B) Fc-silenced L | AFEMTQTPASVSPAVGGTVSINCQASEDIESSLAWYQQKPGQPPKLLIYD ASDLESGVPSRFKGSGSGTEYTLTISGVQPADAATYFCQSAYYTGSADNT FGGGTEVVVKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC |

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### Embodiments

Although the present invention is defined in the claims, it should be understood that the present invention can also (alternatively) be defined in accordance with the following embodiments:
1. An antibody or antigen-binding fragment thereof that binds to CLEC5A (C-type lectin domain family 5 member A) comprising:
   a heavy chain variable region (VH) comprising complementarity determining regions (CDRs) 1, 2, and 3, wherein the VH CDR1 region comprises an amino acid sequence that is at least 80% identical to a selected VH CDR1 amino acid sequence, the VH CDR2 region comprises an amino acid sequence that is at least 80% identical to a selected VH CDR2 amino acid sequence, and the VH CDR3 region comprises an amino acid sequence that is at least 80% identical to a selected VH CDR3 amino acid sequence; and
   a light chain variable region (VL) comprising CDRs 1, 2, and 3, wherein the VL CDR1 region comprises an amino acid sequence that is at least 80% identical to a selected VL CDR1 amino acid sequence, the VL CDR2 region comprises an amino acid sequence that is at least 80% identical to a selected VL CDR2 amino acid sequence, and the VL CDR3 region comprises an amino acid sequence that is at least 80% identical to a selected VL CDR3 amino acid sequence,
   wherein the selected VH CDRs 1, 2, and 3 amino acid sequences and the selected VL CDRs, 1, 2, and 3 amino acid sequences are one of the following:
      (1) the selected VH CDRs 1, 2, 3 amino acid sequences are set forth in SEQ ID NOs: 3, 5, 7, respectively, and the selected VL CDRs 1, 2, 3 amino acid sequences are set forth in SEQ ID NOs: 8-10, respectively;
      (2) the selected VH CDRs 1, 2, 3 amino acid sequences are set forth in SEQ ID NOs: 4, 6, 7, respectively, and the selected VL CDRs 1, 2, 3 amino acid sequences are set forth in SEQ ID NOs: 8-10, respectively;
      (3) the selected VH CDRs 1, 2, 3 amino acid sequences are set forth in SEQ ID NOs: 13, 15, 17, respectively, and the selected VL CDRs 1, 2, 3 amino acid sequences are set forth in SEQ ID NOs: 18-20, respectively;
      (4) the selected VH CDRs 1, 2, 3 amino acid sequences are set forth in SEQ ID NOs: 14, 16, 17, respectively, and the selected VL CDRs 1, 2, 3 amino acid sequences are set forth in SEQ ID NOs: 18-20, respectively;
      (5) the selected VH CDRs 1, 2, 3 amino acid sequences are set forth in SEQ ID NOs: 23, 25, 27, respectively, and the selected VL CDRs 1, 2, 3 amino acid sequences are set forth in SEQ ID NOs: 28-30, respectively;
      (6) the selected VH CDRs 1, 2, 3 amino acid sequences are set forth in SEQ ID NOs: 24, 26, 27, respectively, and the selected VL CDRs 1, 2, 3 amino acid sequences are set forth in SEQ ID NOs: 28-30, respectively;
      (7) the selected VH CDRs 1, 2, 3 amino acid sequences are set forth in SEQ ID NOs: 33, 35, 37, respectively, and the selected VL CDRs 1, 2, 3 amino acid sequences are set forth in SEQ ID NOs: 38-40, respectively;
      (8) the selected VH CDRs 1, 2, 3 amino acid sequences are set forth in SEQ ID NOs: 34, 36, 37, respectively, and the selected VL CDRs 1, 2, 3 amino acid sequences are set forth in SEQ ID NOs: 38-40, respectively;
      (9) the selected VH CDRs 1, 2, 3 amino acid sequences are set forth in SEQ ID NOs: 43, 45, 47, respectively, and the selected VL CDRs 1, 2, 3 amino acid sequences are set forth in SEQ ID NOs: 48-50, respectively;
      (10) the selected VH CDRs 1, 2, 3 amino acid sequences are set forth in SEQ ID NOs: 44, 46, 47, respectively, and the selected VL CDRs 1, 2, 3 amino acid sequences are set forth in SEQ ID NOs: 48-50, respectively;
      (11) the selected VH CDRs 1, 2, 3 amino acid sequences are set forth in SEQ ID NOs: 53, 55, 57, respectively, and the selected VL CDRs 1, 2, 3 amino acid sequences are set forth in SEQ ID NOs: 58-60, respectively;
      (12) the selected VH CDRs 1, 2, 3 amino acid sequences are set forth in SEQ ID NOs: 54, 56, 57, respectively, and the selected VL CDRs 1, 2, 3 amino acid sequences are set forth in SEQ ID NOs: 58-60, respectively;
      (13) the selected VH CDRs 1, 2, 3 amino acid sequences are set forth in SEQ ID NOs: 63, 65, 67, respectively, and the selected VL CDRs 1, 2, 3 amino acid sequences are set forth in SEQ ID NOs: 68-70, respectively;
      (14) the selected VH CDRs 1, 2, 3 amino acid sequences are set forth in SEQ ID NOs: 64, 66, 67, respectively, and the selected VL CDRs 1, 2, 3 amino acid sequences are set forth in SEQ ID NOs: 68-70, respectively;
      (15) the selected VH CDRs 1, 2, 3 amino acid sequences are set forth in SEQ ID NOs: 73, 75, 77, respectively, and the selected VL CDRs 1, 2, 3 amino acid sequences are set forth in SEQ ID NOs: 78-80, respectively; and
      (16) the selected VH CDRs 1, 2, 3 amino acid sequences are set forth in SEQ ID NOs: 74, 76, 77, respectively, and the selected VL CDRs 1, 2, 3 amino acid sequences are set forth in SEQ ID NOs: 78-80, respectively.
2. The antibody or antigen-binding fragment thereof of embodiment 1, wherein the VH comprises CDRs 1, 2, 3 with the amino acid sequences set forth in SEQ ID NOs: 3, 5, 7, respectively, and the VL comprises CDRs 1, 2, 3 with the amino acid sequences set forth in SEQ ID NOs: 8-10, respectively, according to Kabat definition.
3. The antibody or antigen-binding fragment thereof of embodiment 1, wherein the VH comprises CDRs 1, 2, 3 with the amino acid sequences set forth in SEQ ID NOs: 13, 15, 17, respectively, and the VL comprises CDRs 1, 2, 3 with the amino acid sequences set forth in SEQ ID NOs: 18-20, respectively, according to Kabat definition.
4. The antibody or antigen-binding fragment thereof of embodiment 1, wherein the VH comprises CDRs 1, 2, 3 with the amino acid sequences set forth in SEQ ID NOs: 23, 25, 27, respectively, and the VL comprises CDRs 1, 2, 3 with the amino acid sequences set forth in SEQ ID NOs: 28-30, respectively, according to Kabat definition.
5. The antibody or antigen-binding fragment thereof of embodiment 1, wherein the VH comprises CDRs 1, 2, 3 with the amino acid sequences set forth in SEQ ID NOs: 33, 35, 37, respectively, and the VL comprises CDRs 1, 2, 3 with the amino acid sequences set forth in SEQ ID NOs: 38-40, respectively, according to Kabat definition.
6. The antibody or antigen-binding fragment thereof of embodiment 1, wherein the VH comprises CDRs 1, 2, 3 with the amino acid sequences set forth in SEQ ID NOs: 43, 45, 47, respectively, and the VL comprises CDRs 1, 2, 3 with the amino acid sequences set forth in SEQ ID NOs: 48-50, respectively, according to Kabat definition.
7. The antibody or antigen-binding fragment thereof of embodiment 1, wherein the VH comprises CDRs 1, 2, 3 with the amino acid sequences set forth in SEQ ID NOs: 53, 55, 57, respectively, and the VL comprises CDRs 1, 2, 3 with the amino acid sequences set forth in SEQ ID NOs: 58-60, respectively, according to Kabat definition.
8. The antibody or antigen-binding fragment thereof of embodiment 1, wherein the VH comprises CDRs 1, 2, 3 with the amino acid sequences set forth in SEQ ID NOs: 63, 65, 67, respectively, and the VL comprises CDRs 1, 2, 3 with the amino acid sequences set forth in SEQ ID NOs: 68-70, respectively, according to Kabat definition.
9. The antibody or antigen-binding fragment thereof of embodiment 1, wherein the VH comprises CDRs 1, 2, 3 with the amino acid sequences set forth in SEQ ID NOs: 73, 75, 77, respectively, and the VL comprises CDRs 1, 2, 3 with the amino acid sequences set forth in SEQ ID NOs: 78-80, respectively, according to Kabat definition.
10. The antibody or antigen-binding fragment thereof of embodiment 1, wherein the VH comprises CDRs 1, 2, 3 with the amino acid sequences set forth in SEQ ID NOs: 4, 6, 7, respectively, and the VL comprises CDRs 1, 2, 3 with the amino acid sequences set forth in SEQ ID NOs: 8-10, respectively, according to Chothia definition.
11. The antibody or antigen-binding fragment thereof of embodiment 1, wherein the VH comprises CDRs 1, 2, 3 with the amino acid sequences set forth in SEQ ID NOs: 14, 16, 17, respectively, and the VL comprises CDRs 1, 2, 3 with the amino acid sequences set forth in SEQ ID NOs: 18-20, respectively, according to Chothia definition.
12. The antibody or antigen-binding fragment thereof of embodiment 1, wherein the VH comprises CDRs 1, 2, 3 with the amino acid sequences set forth in SEQ ID NOs: 24, 26, 27, respectively, and the VL comprises CDRs 1, 2, 3 with the amino acid sequences set forth in SEQ ID NOs: 28-30, respectively, according to Chothia definition.
13. The antibody or antigen-binding fragment thereof of embodiment 1, wherein the VH comprises CDRs 1, 2, 3 with the amino acid sequences set forth in SEQ ID NOs: 34, 36, 37, respectively, and the VL comprises CDRs 1, 2, 3 with the amino acid sequences set forth in SEQ ID NOs: 38-40, respectively, according to Chothia definition.
14. The antibody or antigen-binding fragment thereof of embodiment 1, wherein the VH comprises CDRs 1, 2, 3 with the amino acid sequences set forth in SEQ ID NOs: 44, 46, 47, respectively, and the VL comprises CDRs 1, 2, 3 with the amino acid sequences set forth in SEQ ID NOs: 48-50, respectively, according to Chothia definition.
15. The antibody or antigen-binding fragment thereof of embodiment 1, wherein the VH comprises CDRs 1, 2, 3 with the amino acid sequences set forth in SEQ ID NOs: 54, 56, 57, respectively, and the VL comprises CDRs 1, 2, 3 with the amino acid sequences set forth in SEQ ID NOs: 58-60, respectively, according to Chothia definition.
16. The antibody or antigen-binding fragment thereof of embodiment 1, wherein the VH comprises CDRs 1, 2, 3 with the amino acid sequences set forth in SEQ ID NOs: 64, 66, 67, respectively, and the VL comprises CDRs 1, 2, 3 with the amino acid sequences set forth in SEQ ID NOs: 68-70, respectively, according to Chothia definition.
17. The antibody or antigen-binding fragment thereof of embodiment 1, wherein the VH comprises CDRs 1, 2, 3 with the amino acid sequences set forth in SEQ ID NOs: 74, 76, 77, respectively, and the VL comprises CDRs 1, 2, 3 with the amino acid sequences set forth in SEQ ID NOs: 78-80, respectively, according to Chothia definition.
18. An antibody or antigen-binding fragment thereof that binds to CLEC5A comprising
   a heavy chain variable region (VH) comprising an amino acid sequence that is at least 90% identical to a selected VH sequence, and a light chain variable region (VL) comprising an amino acid sequence that is at least 90% identical to a selected VL sequence, wherein the selected VH sequence and the selected VL sequence are one of the following:
   (1) the selected VH sequence is SEQ ID NO: 11, and the selected VL sequence is SEQ ID NO: 12;
   (2) the selected VH sequence is SEQ ID NO: 21, and the selected VL sequence is SEQ ID NO: 22;
   (3) the selected VH sequence is SEQ ID NO: 31, and the selected VL sequence is SEQ ID NO: 32;
   (4) the selected VH sequence is SEQ ID NO: 41, and the selected VL sequence is SEQ ID NO: 42;
   (5) the selected VH sequence is SEQ ID NO: 51, and the selected VL sequence is SEQ ID NO: 52;
   (6) the selected VH sequence is SEQ ID NO: 61, and the selected VL sequence is SEQ ID NO: 62;
   (7) the selected VH sequence is SEQ ID NO: 71, and the selected VL sequence is SEQ ID NO: 72;
   (8) the selected VH sequence is SEQ ID NO: 81, and the selected VL sequence is SEQ ID NO: 82;
   (9) the selected VH sequence is SEQ ID NO: 83, and the selected VL sequence is SEQ ID NO: 84;
   (10) the selected VH sequence is SEQ ID NO: 85, and the selected VL sequence is SEQ ID NO: 86;
   (11) the selected VH sequence is SEQ ID NO: 87, and the selected VL sequence is SEQ ID NO: 88;
   (12) the selected VH sequence is SEQ ID NO: 89, and the selected VL sequence is SEQ ID NO: 90; and
   (13) the selected VH sequence is SEQ ID NO: 91, and the selected VL sequence is SEQ ID NO: 92.
19. The antibody or antigen-binding fragment thereof of embodiment 18, wherein the VH comprises the sequence of SEQ ID NO: 11 and the VL comprises the sequence of SEQ ID NO: 12.
20. The antibody or antigen-binding fragment thereof of embodiment 18, wherein the VH comprises the sequence of SEQ ID NO: 21 and the VL comprises the sequence of SEQ ID NO: 22.
21. The antibody or antigen-binding fragment thereof of embodiment 18, wherein the VH comprises the sequence of SEQ ID NO: 31 and the VL comprises the sequence of SEQ ID NO: 32.
22. The antibody or antigen-binding fragment thereof of embodiment 18, wherein the VH comprises the sequence of SEQ ID NO: 41 and the VL comprises the sequence of SEQ ID NO: 42.
23. The antibody or antigen-binding fragment thereof of embodiment 18, wherein the VH comprises the sequence of SEQ ID NO: 51 and the VL comprises the sequence of SEQ ID NO: 52.
24. The antibody or antigen-binding fragment thereof of embodiment 18, wherein the VH comprises the sequence of SEQ ID NO: 61 and the VL comprises the sequence of SEQ ID NO: 62.
25. The antibody or antigen-binding fragment thereof of embodiment 18, wherein the VH comprises the sequence of SEQ ID NO: 71 and the VL comprises the sequence of SEQ ID NO: 72.
26. The antibody or antigen-binding fragment thereof of embodiment 18, wherein the VH comprises the sequence of SEQ ID NO: 81 and the VL comprises the sequence of SEQ ID NO: 82.
27. The antibody or antigen-binding fragment thereof of embodiment 18, wherein the VH comprises the sequence of SEQ ID NO: 83 and the VL comprises the sequence of SEQ ID NO: 84.
28. The antibody or antigen-binding fragment thereof of embodiment 18, wherein the VH comprises the sequence of SEQ ID NO: 85 and the VL comprises the sequence of SEQ ID NO: 86.
29. The antibody or antigen-binding fragment thereof of embodiment 18, wherein the VH comprises the sequence of SEQ ID NO: 87 and the VL comprises the sequence of SEQ ID NO: 88.
30. The antibody or antigen-binding fragment thereof of embodiment 18, wherein the VH comprises the sequence of SEQ ID NO: 89 and the VL comprises the sequence of SEQ ID NO: 90.
31. The antibody or antigen-binding fragment thereof of embodiment 18, wherein the VH comprises the sequence of SEQ ID NO: 91 and the VL comprises the sequence of SEQ ID NO: 92.
32. The antibody or antigen-binding fragment thereof of any one of embodiments 1-31, wherein the antibody or antigen-binding fragment thereof specifically binds to human, mouse, or monkey CLEC5A.
33. The antibody or antigen-binding fragment thereof of any one of embodiments 1-32, wherein the antibody or antigen-binding fragment thereof can mediate phagocytosis of macrophages (e.g., with a target cell killing efficacy that at least 60%, at least 70%, at least 80%, at least 90%, at least 100%, at least 110%, at least 120%, at least 130%, at least 140%, or at least 150% as compared to that of a reference antibody (e.g., DX244)), and/or wherein the antibody or antigen-binding fragment thereof can induce low levels of cytokine (e.g., IL-6 or TNFα) release (e.g., less than 80%, less than 70%, less than 60%, less than 50%, less than 40%, less than 30%, less than 20%, less than 10%, less than 5%, or less than 1% as compared to that induced by a reference antibody (e.g., DX244)).
34. An antibody or antigen-binding fragment thereof that binds to CLEC5A, wherein the antibody or antigen-binding fragment thereof can mediate phagocytosis of macrophages (e.g., with a target cell killing efficacy that is at least 60%, at least 70%, at least 80%, at least 90%, at least 100%, at least 110%, at least 120%, at least 130%, at least 140%, or at least 150% as compared to that of a reference antibody (e.g., DX244)), and/or wherein the antibody or antigen-binding fragment thereof can induce a low level of cytokine (e.g., IL-6 or TNFα) release (e.g., less than 80%, less than 70%, less than 60%, less than 50%, less than 40%, less than 30%, less than 20%, less than 10%, less than 5%, or less than 1% as compared to that induced by a reference antibody (e.g., DX244)).
35. An antibody or antigen-binding fragment thereof, comprising:
   i) a first antigen-binding domain that specifically binds to a first antigen, wherein the first antigen is a tumor associated antigen (TAA); and
   ii) a second antigen-binding domain that specifically binds to C-type lectin domain family 5 member A (CLEC5A);
      or
   i) a first antigen-binding domain that specifically binds to a first antigen, wherein the first antigen is an autoimmune disease target; and
   ii) a second antigen-binding domain that specifically binds to CLEC5A.
36. The antibody or antigen-binding fragment thereof of embodiment 35, wherein the antibody or antigen-binding fragment thereof has one or more of the following effects:
   i) the antibody or antigen-binding fragment thereof can mediate target cell killing by myeloid cells (e.g., monocytes and/or macrophages (e.g., M0, M1, and/or M2 macrophages));
   ii) the antibody or antigen-binding fragment thereof can mediate target cell killing by myeloid cells (e.g., monocytes and/or macrophages) at a low E:T (effector cell : target cell) ratio, optionally wherein the low E:T ratio is less than 1:10, less than 1:9, less than 1:8, less than 1:7, less than 1:6, less than 1:5, less than 1:4, less than 1:3, less than 1:2, less than 1:1, less than 2:1, less than 3:1, less than 4:1, less than 5:1, less than 6:1, less than 7:1, less than 8:1, less than 9:1, or less than 10:1); and
   iii) the antibody or antigen-binding fragment thereof can induce a low level of cytokine (e.g., IL-6 or TNFα) release of myeloid cells (e.g., monocytes and/or macrophages), e.g., less than 80%, less than 70%, less than 60%, less than 50%, less than 40%, less than 30%, less than 20%, less than 10%, less than 5%, or less than 1% as compared to that induced by a reference antibody.
37. The antibody or antigen-binding fragment thereof of embodiment 35 or 36, wherein the first antigen-binding domain comprises a first heavy chain variable region (VH1) and a first light chain variable region (VL1); and the second antigen-binding domain comprises a second heavy chain variable region (VH2) and a second light chain variable region (VL2).
38. The antibody or antigen-binding fragment thereof of any one of embodiments 35-37, wherein the second antigen-binding domain is a single-chain fragment variable (scFv) domain, wherein the VH2 and VL2 are linked by a first linker.
39. The antibody or antigen-binding fragment thereof of embodiment 38, wherein the second antigen-binding domain is connected to the C-terminus of a light chain through a second linker.
40. The antibody or antigen-binding fragment thereof of any one of embodiments 35-39, further comprising an Fc region.
41. The antibody or antigen-binding fragment thereof of embodiment 40, wherein the C-terminus of the VH1 of the first antigen-binding domain is connected to the Fc region, optionally through a CH1 domain.
42. The antibody or antigen-binding fragment thereof of embodiment 40, wherein the C-terminus of the VH1 of the first antigen-binding domain is connected to the N-terminus of a Fc region, and the N-terminus of the second antigen-binding domain is connected to the C-terminus of the Fc region.
43. The antibody or antigen-binding fragment thereof of embodiment 37, wherein the antibody comprises a first heavy chain comprising the VH1 and a first light chain comprising the VL1; and a second heavy chain comprising the VH2 and a second light chain comprising the VL2.
44. The antibody or antigen-binding fragment thereof of embodiment 43, wherein:
   the first heavy chain comprises one or more knob mutations; and
   the second heavy chain comprises one or more hole mutations.
45. The antibody or antigen-binding fragment thereof of embodiment 43, wherein:
   the first heavy chain comprises one or more hole mutations; and
   the second heavy chain comprises one or more knob mutations.
46. The antibody or antigen-binding fragment thereof of any one of embodiments 35-45, wherein the Fc region is an Fc region of human IgG1, IgG2, IgG3, or IgG4.
47. The antibody or antigen-binding fragment thereof of embodiment 46, wherein the Fc region is an Fc region of human IgG1.
48. The antibody or antigen-binding fragment thereof of embodiment 46 or 47, wherein the Fc region comprises one or more the following amino acid residues (all numbering is according to EU numbering):
   (1) an Alanine (A) at position 236; a Leucine (L) at position 330 and a Glutamic acid (E) at position 332;
   (2) an Alanine (A) at position 236; an Aspartic acid (D) at position 293; a Leucine (L) at position 330 and a Glutamic acid (E) at position 332;
   (3) an Alanine (A) at position 236;
   (4) an Alanine (A) at position 236; an Aspartic acid (D) at position 293; and a Glutamic acid (E) at position 332;
   (5) an Aspartic acid (D) at position 293 and a Glutamic acid (E) at position 332;
   (6) an Aspartic acid (D) at position 293; a Leucine (L) at position 330 and a Glutamic acid (E) at position 332; and
   (7) an Alanine (A) at position 234; an Alanine (A) at position 235 and a Glycine (G) at position 329;
   optionally, the Fc region is afucosylated.
49. The antibody or antigen-binding fragment thereof of any one of embodiments 35-48, wherein the tumor associated antigen (TAA) is HER2, CD79b, EGFR, EpCAM, BCMA, CD38, GPRC5D, DLL3, CD70, GPC3, Fas ligand (FasL), CD1d, Membrane glycolipids, globotriaosyl-ceramide (Gb3Cer/CD77), gangliosides (GD2, GD3, and GM2), , CD34, CD45, human leukocyte antigen-DR (HLA-DR), CD123, CLL1, CD105, CD71, SSC, MAGE, MUC16, CD19, WT-1, B7H3, TEM8, CD22, LI-CAM, ROR-1, CEA, 4-1BB, ETA, 5T4, adenocarcinoma antigen, alpha-fetoprotein (AFP), BAFF, B- lymphoma cell, CA242 antigen, CA-125, carbonic anhydrase 9 (CA-IX), C-MET, CCR4, CD133, CD152, CD20, CD125, CD200, CD221, CD23 (IgE receptor), CD28, CD30 (TNFRSF8), CD33, CD4, CD40, CD44 v6, CD51, CD52, CD56, CD74, CD80, CEA, CNT0888, CTLA-4, DR5, CD3, FAP, fibronectin extra domain-B, folate receptor 1, GD2, GD3 ganglioside, glycoprotein 75, GPNMB, , HGF, human scatter factor receptor kinase, IGF-l receptor, IGF-I, IgG1, IL-5, IL-13, IL-6, IL-15, insulin-like growth factor I receptor, integrin a5b1, integrin avb3, MSLN, MS4A1, MUC1, mucin CanAg, N-glycolylneuraminic acid, NPC-1C, PD-1, PD-L1, PDGF-R a, TWEAK, phosphatidylserine, prostatic carcinoma cells, RANKL, RON, SCH 900105, SDC1, SLAMF7, TAG-72, tenascin C, TGF beta 2, TGF-b, TRAIL-R1, TRAIL-R2, tumor antigen CTAA16.88, VEGF-A, VEGFR-1, VEGFR2, or vimentin;
   wherein the autoimmune disease target is CD79b, CD38, ACHE, BAFF, BTK, CCL2, CD19, CD20, CD25, CD40, CD52, CD80, CD86, ETAR, ETBR, FCGRT, GM-CSF, JAK1, IFNAR, IFNB1, IFNG, IgE, IgG Fc, IL1A, IL1B, IL-2, IL-4, IL-5, IL-6, IL6R, IL7, IL-12, IL-13, IL-17, IL-17R, IL-18, IL-21, IL-22, IL-23, Integrin, ITG-A4B1, ITG-A4B7, ITG-AVB6, TL1A, TNF-α, TNF-β, TNFSF13B, TSLP, TYK2, or VEGFR.
50. The antibody or antigen-binding fragment thereof of any one of embodiments 35-49, wherein the TAA is HER2.
51. The antibody or antigen-binding fragment thereof of embodiment 50, wherein
   (1) the selected VH1 CDRs 1, 2, 3 amino acid sequences are set forth in SEQ ID NOs: 100, 102, 104, respectively, and the selected VL1 CDRs 1, 2, 3 amino acid sequences are set forth in SEQ ID NOs: 105-107, respectively; and
      the selected VH2 CDRs 1, 2, 3 amino acid sequences and the selected VL2 CDRs 1, 2, 3 amino acid sequences are selected from one of the sequences set forth in any one of embodiments 1-17; or
   (2) the selected VH1 CDRs 1, 2, 3 amino acid sequences are set forth in SEQ ID NOs: 101, 103, 104, respectively, and the selected VL1 CDRs 1, 2, 3 amino acid sequences are set forth in SEQ ID NOs: 105-107, respectively; and
      the selected VH2 CDRs 1, 2, 3 amino acid sequences and the selected VL2 CDRs 1, 2, 3 amino acid sequences are selected from one of the sequences set forth in any one of embodiments 1-17.
52. The antibody or antigen-binding fragment thereof of any one of embodiments 35-49, wherein the TAA or autoimmune disease target is CD79b.
53. The antibody or antigen-binding fragment thereof of embodiment 52, wherein
   (1) the selected VH1 CDRs 1, 2, 3 amino acid sequences are set forth in SEQ ID NOs: 200, 202, 204, respectively, and the selected VL1 CDRs 1, 2, 3 amino acid sequences are set forth in SEQ ID NOs: 205-207, respectively; and
      the selected VH2 CDRs 1, 2, 3 amino acid sequences and the selected VL2 CDRs 1, 2, 3 amino acid sequences are selected from one of the sequences set forth in any one of embodiments 1-17; or
   (2) the selected VH1 CDRs 1, 2, 3 amino acid sequences are set forth in SEQ ID NOs: 201, 203, 204, respectively, and the selected VL1 CDRs 1, 2, 3 amino acid sequences are set forth in SEQ ID NOs: 205-207, respectively; and
      the selected VH2 CDRs 1, 2, 3 amino acid sequences and the selected VL2 CDRs 1, 2, 3 amino acid sequences are selected from one of the sequences set forth in any one of embodiments 1-17.
54. The antibody or antigen-binding fragment thereof of any one of embodiments 35-49, wherein the TAA is EGFR.
55. The antibody or antigen-binding fragment thereof of embodiment 54, wherein
   (1) the selected VH1 CDRs 1, 2, 3 amino acid sequences are set forth in SEQ ID NOs: 126, 128, 130, respectively, and the selected VL1 CDRs 1, 2, 3 amino acid sequences are set forth in SEQ ID NOs: 131-133, respectively; and
      the selected VH2 CDRs 1, 2, 3 amino acid sequences and the selected VL2 CDRs 1, 2, 3 amino acid sequences are selected from one of the sequences set forth in any one of embodiments 1-17;
   (2) the selected VH1 CDRs 1, 2, 3 amino acid sequences are set forth in SEQ ID NOs: 127, 129, 130, respectively, and the selected VL1 CDRs 1, 2, 3 amino acid sequences are set forth in SEQ ID NOs: 131-133, respectively; and
      the selected VH2 CDRs 1, 2, 3 amino acid sequences and the selected VL2 CDRs 1, 2, 3 amino acid sequences are selected from one of the sequences set forth in any one of embodiments 1-17;
   (3) the selected VH1 CDRs 1, 2, 3 amino acid sequences are set forth in SEQ ID NOs: 136, 138, 140, respectively, and the selected VL1 CDRs 1, 2, 3 amino acid sequences are set forth in SEQ ID NOs: 141-143, respectively; and
      the selected VH2 CDRs 1, 2, 3 amino acid sequences and the selected VL2 CDRs 1, 2, 3 amino acid sequences are selected from one of the sequences set forth in any one of embodiments 1-17; or
   (4) the selected VH1 CDRs 1, 2, 3 amino acid sequences are set forth in SEQ ID NOs: 137, 139, 140, respectively, and the selected VL1 CDRs 1, 2, 3 amino acid sequences are set forth in SEQ ID NOs: 141-143, respectively; and
      the selected VH2 CDRs 1, 2, 3 amino acid sequences and the selected VL2 CDRs 1, 2, 3 amino acid sequences are selected from one of the sequences set forth in any one of embodiments 1-17.
56. The antibody or antigen-binding fragment thereof of any one of embodiments 35-49, wherein the TAA is EpCAM.
57. The antibody or antigen-binding fragment thereof of embodiment 56, wherein
   (1) the selected VH1 CDRs 1, 2, 3 amino acid sequences are set forth in SEQ ID NOs: 150, 152, 154, respectively, and the selected VL1 CDRs 1, 2, 3 amino acid sequences are set forth in SEQ ID NOs: 155-157, respectively; and
      the selected VH2 CDRs 1, 2, 3 amino acid sequences and the selected VL2 CDRs 1, 2, 3 amino acid sequences are selected from one of the sequences set forth in any one of embodiments 1-17; or
   (2) the selected VH1 CDRs 1, 2, 3 amino acid sequences are set forth in SEQ ID NOs: 151, 153, 154, respectively, and the selected VL1 CDRs 1, 2, 3 amino acid sequences are set forth in SEQ ID NOs: 155-157, respectively; and
      the selected VH2 CDRs 1, 2, 3 amino acid sequences and the selected VL2 CDRs 1, 2, 3 amino acid sequences are selected from one of the sequences set forth in any one of embodiments 1-17;
58. The antibody or antigen-binding fragment thereof of any one of embodiments 35-49, wherein the TAA or autoimmune disease target is GPRC5D.
59. The antibody or antigen-binding fragment thereof of embodiment 58, wherein
   (1) the selected VH1 CDRs 1, 2, 3 amino acid sequences are set forth in SEQ ID NOs: 164, 166, 168, respectively, and the selected VL1 CDRs 1, 2, 3 amino acid sequences are set forth in SEQ ID NOs: 169-171, respectively; and
      the selected VH2 CDRs 1, 2, 3 amino acid sequences and the selected VL2 CDRs 1, 2, 3 amino acid sequences are selected from one of the sequences set forth in any one of embodiments 1-17; or
   (2) the selected VH1 CDRs 1, 2, 3 amino acid sequences are set forth in SEQ ID NOs: 165, 167, 168, respectively, and the selected VL1 CDRs 1, 2, 3 amino acid sequences are set forth in SEQ ID NOs: 169-171, respectively; and
      the selected VH2 CDRs 1, 2, 3 amino acid sequences and the selected VL2 CDRs 1, 2, 3 amino acid sequences are selected from one of the sequences set forth in any one of embodiments 1-17.
60. The antibody or antigen-binding fragment thereof of any one of embodiments 35-49, wherein the TAA or autoimmune disease target is BCMA.
61. The antibody or antigen-binding fragment thereof of embodiment 60, wherein
   (1) the selected VH1 CDRs 1, 2, 3 amino acid sequences are set forth in SEQ ID NOs: 176, 178, 180, respectively, and the selected VL1 CDRs 1, 2, 3 amino acid sequences are set forth in SEQ ID NOs: 181-183, respectively; and
      the selected VH2 CDRs 1, 2, 3 amino acid sequences and the selected VL2 CDRs 1, 2, 3 amino acid sequences are selected from one of the sequences set forth in any one of embodiments 1-17; or
   (2) the selected VH1 CDRs 1, 2, 3 amino acid sequences are set forth in SEQ ID NOs: 177, 179, 180, respectively, and the selected VL1 CDRs 1, 2, 3 amino acid sequences are set forth in SEQ ID NOs: 181-183, respectively; and
      the selected VH2 CDRs 1, 2, 3 amino acid sequences and the selected VL2 CDRs 1, 2, 3 amino acid sequences are selected from one of the sequences set forth in any one of embodiments 1-17.
62. The antibody or antigen-binding fragment thereof of any one of embodiments 35-49, wherein the TAA or autoimmune disease target is CD38.
63. The antibody or antigen-binding fragment thereof of embodiment 62, wherein
   (1) the selected VH1 CDRs 1, 2, 3 amino acid sequences are set forth in SEQ ID NOs: 188, 190, 192, respectively, and the selected VL1 CDRs 1, 2, 3 amino acid sequences are set forth in SEQ ID NOs: 193-195, respectively; and
      the selected VH2 CDRs 1, 2, 3 amino acid sequences and the selected VL2 CDRs 1, 2, 3 amino acid sequences are selected from one of the sequences set forth in any one of embodiments 1-17; or
   (2) the selected VH1 CDRs 1, 2, 3 amino acid sequences are set forth in SEQ ID NOs: 189, 191, 192, respectively, and the selected VL1 CDRs 1, 2, 3 amino acid sequences are set forth in SEQ ID NOs: 193-195, respectively; and
      the selected VH2 CDRs 1, 2, 3 amino acid sequences and the selected VL2 CDRs 1, 2, 3 amino acid sequences are selected from one of the sequences set forth in any one of embodiments 1-17.
64. An antibody or antigen-binding fragment thereof that cross-competes with the antibody or antigen-binding fragment thereof of any one of embodiments 1-63.
65. A nucleic acid comprising a polynucleotide encoding an antibody or antigen-binding fragment thereof set forth in any one of embodiments 1-64.
66. A vector comprising the nucleic acid of embodiment 65.
67. A cell comprising the vector of embodiment 66.
68. A method of decreasing the rate of tumor growth, the method comprising contacting a tumor cell with an effective amount of a composition comprising the antibody or antigen-binding fragment thereof of any one of embodiments 1-64.
69. A method of killing a tumor cell, the method comprising
   contacting a tumor cell with an effective amount of a composition comprising the antibody or antigen-binding fragment thereof of any one of embodiments 1-64.
70. A method of increasing immune response in a subject, the method comprising administering to the subject an effective amount of a composition comprising the antibody or antigen-binding fragment thereof of any one of embodiments 1-64.
71. A method of treating a subject having cancer, the method comprising administering a therapeutically effective amount of a composition comprising the antibody or antigen-binding fragment thereof of any one of embodiments 1-64 to the subject.
72. The method of embodiment 71, wherein the cancer is a solid tumor or a blood tumor.
73. The method of embodiment 71 or 72, wherein the cancer is breast cancer, lung cancer, stomach cancer, colorectal cancer, prostate cancer, ovarian cancer, colon cancer, esophageal cancer, tracheal cancer, gastric cancer bladder cancer, uterine cancer, rectal cancer, cancer of the small intestine, pancreatic cancer and/or liver cancer.
74. The method of embodiment 71 or 72, wherein the cancer is multiple myeloma, B-cell lymphoma, diffuse Large B-Cell Lymphoma, acute B-cell leukemia, chronic lymphocytic leukemia, B-cell prelymphocytic leukemia, spleen with villous lymphocytes Lymphoma, hairy cell leukemia, follicular lymphoma, and/or mantle cell lymphoma.
75. The method of any one of embodiments 70-74, wherein the subject is further treated with an effective amount of an anti-4-1BB antibody, an anti-OX40 antibody, an anti-PD-1 antibody, an anti-CTLA4 antibody, an anti-CD40 antibody, and/or an anti-PD-L1 antibody.
76. A method of treating a subject having an autoimmune disease, the method comprising administering a therapeutically effective amount of a composition comprising the antibody or antigen-binding fragment thereof of any one of embodiments 1-64 to the subject.
77. The method of embodiment 76, wherein the autoimmune disease is selected from rheumatoid arthritis, psoriasis, multiple sclerosis, immune thrombocytopenic purpura, myasthenia gravis, neuromyelitis optica, IgG4-related diseases, systemic Lupus Erythematosus, lupus nephritis, giant cell arteritis, takayasu disease, cold agglutinin disease, warm autoimmune hemolytic anemia, and anti-neutrophil cytoplasmic antibody (ANCA) associated vasculitides including for example, tranulomatosis with polyangiitis (GPA) (Wegener's Granulomatosis) and Microscopic Polyangiitis (MPA).
78. The method of embodiment 77, wherein the autoimmune disease is multiple sclerosis, systemic lupus erythematosus and/or rheumatoid arthritis.
79. A pharmaceutical composition comprising the antibody or antigen-binding fragment thereof of any one of embodiments 1-64, and a pharmaceutically acceptable carrier.

## Claims

1. An antibody or antigen-binding fragment thereof, comprising:
i) a first antigen-binding domain that specifically binds to a first antigen, wherein the first antigen is cluster of differentiation 79b (CD79b); and
ii) a second antigen-binding domain that specifically binds to C-type lectin domain family 5 member A (CLEC5A).

2. The antibody or antigen-binding fragment thereof of claim 1, wherein the first antigen-binding domain comprises a first heavy chain variable region (VH1) comprising CDRs 1, 2, and 3, and a first light chain variable region (VL1) comprising CDRs 1, 2, and 3; and the second antigen-binding domain comprises a second heavy chain variable region (VH2) comprising CDRs 1, 2, and 3, and a second light chain variable region (VL2) comprising CDRs 1, 2, and 3, wherein:
(1) the VH1 CDRs 1, 2, 3 amino acid sequences are set forth in SEQ ID NOs: 200, 202, 204, respectively, and the VL1 CDRs 1, 2, 3 amino acid sequences are set forth in SEQ ID NOs: 205-207, respectively; or
(2) the VH1 CDRs 1, 2, 3 amino acid sequences are set forth in SEQ ID NOs: 201, 203, 204, respectively, and the VL1 CDRs 1, 2, 3 amino acid sequences are set forth in SEQ ID NOs: 205-207, respectively, and wherein
(1) the VH2 CDRs 1, 2, 3 amino acid sequences are set forth in SEQ ID NOs: 13, 15, 17, respectively, and the VL2 CDRs 1, 2, 3 amino acid sequences are set forth in SEQ ID NOs: 18-20, respectively;
(2) the VH2 CDRs 1, 2, 3 amino acid sequences are set forth in SEQ ID NOs: 14, 16, 17, respectively, and the VL2 CDRs 1, 2, 3 amino acid sequences are set forth in SEQ ID NOs: 18-20, respectively;
(3) the VH2 CDRs 1, 2, 3 amino acid sequences are set forth in SEQ ID NOs: 63, 65, 67, respectively, and the VL2 CDRs 1, 2, 3 amino acid sequences are set forth in SEQ ID NOs: 68-70, respectively;
(4) the VH2 CDRs 1, 2, 3 amino acid sequences are set forth in SEQ ID NOs: 64, 66, 67, respectively, and the VL2 CDRs 1, 2, 3 amino acid sequences are set forth in SEQ ID NOs: 68-70, respectively;
(5) the VH2 CDRs 1, 2, 3 amino acid sequences are set forth in SEQ ID NOs: 3, 5, 7, respectively, and the VL2 CDRs 1, 2, 3 amino acid sequences are set forth in SEQ ID NOs: 8-10, respectively;
(6) the VH2 CDRs 1, 2, 3 amino acid sequences are set forth in SEQ ID NOs: 4, 6, 7, respectively, and the VL2 CDRs 1, 2, 3 amino acid sequences are set forth in SEQ ID NOs: 8-10, respectively;
(7) the VH2 CDRs 1, 2, 3 amino acid sequences are set forth in SEQ ID NOs: 23, 25, 27, respectively, and the VL2 CDRs 1, 2, 3 amino acid sequences are set forth in SEQ ID NOs: 28-30, respectively;
(8) the VH2 CDRs 1, 2, 3 amino acid sequences are set forth in SEQ ID NOs: 24, 26, 27, respectively, and the VL2 CDRs 1, 2, 3 amino acid sequences are set forth in SEQ ID NOs: 28-30, respectively;
(9) the VH2 CDRs 1, 2, 3 amino acid sequences are set forth in SEQ ID NOs: 33, 35, 37, respectively, and the VL2 CDRs 1, 2, 3 amino acid sequences are set forth in SEQ ID NOs: 38-40, respectively;
(10) the VH2 CDRs 1, 2, 3 amino acid sequences are set forth in SEQ ID NOs: 34, 36, 37, respectively, and the VL2 CDRs 1, 2, 3 amino acid sequences are set forth in SEQ ID NOs: 38-40, respectively;
(11) the VH2 CDRs 1, 2, 3 amino acid sequences are set forth in SEQ ID NOs: 43, 45, 47, respectively, and the VL2 CDRs 1, 2, 3 amino acid sequences are set forth in SEQ ID NOs: 48-50, respectively;
(12) the VH2 CDRs 1, 2, 3 amino acid sequences are set forth in SEQ ID NOs: 44, 46, 47, respectively, and the VL2 CDRs 1, 2, 3 amino acid sequences are set forth in SEQ ID NOs: 48-50, respectively;
(13) the VH2 CDRs 1, 2, 3 amino acid sequences are set forth in SEQ ID NOs: 53, 55, 57, respectively, and the VL2 CDRs 1, 2, 3 amino acid sequences are set forth in SEQ ID NOs: 58-60, respectively;
(14) the VH2 CDRs 1, 2, 3 amino acid sequences are set forth in SEQ ID NOs: 54, 56, 57, respectively, and the VL2 CDRs 1, 2, 3 amino acid sequences are set forth in SEQ ID NOs: 58-60, respectively;
(15) the VH2 CDRs 1, 2, 3 amino acid sequences are set forth in SEQ ID NOs: 73, 75, 77, respectively, and the VL2 CDRs 1, 2, 3 amino acid sequences are set forth in SEQ ID NOs: 78-80, respectively; or
(16) the VH2 CDRs 1, 2, 3 amino acid sequences are set forth in SEQ ID NOs: 74, 76, 77, respectively, and the VL2 CDRs 1, 2, 3 amino acid sequences are set forth in SEQ ID NOs: 78-80, respectively.

3. The antibody or antigen-binding fragment thereof of claim 1 or 2, wherein the first antigen-binding domain comprises a first heavy chain variable region (VH1) and a first light chain variable region (VL1), wherein the VH1 comprises an amino acid sequence that is at least 90% identical to SEQ ID NO: 208, and the VL1 comprises an amino acid sequence that is at least 90% identical to SEQ ID NO: 209.

4. The antibody or antigen-binding fragment thereof of any one of claims 1-3, wherein the second antigen-binding domain comprises a second heavy chain variable region (VH2) and a second light chain variable region (VL2), wherein the VH2 comprises an amino acid sequence that is at least 90% identical to a selected VH2 sequence, and the VL2 comprises an amino acid sequence that is at least 90% identical to a selected VL2 sequence, wherein the selected VH2 sequence and the selected VL2 sequence are one of the following:
(1) the selected VH2 sequence is SEQ ID NO: 85, and the selected VL2 sequence is SEQ ID NO: 86;
(2) the selected VH2 sequence is SEQ ID NO: 71, and the selected VL2 sequence is SEQ ID NO: 72;
(3) the selected VH2 sequence is SEQ ID NO: 11, and the selected VL2 sequence is SEQ ID NO: 12;
(4) the selected VH2 sequence is SEQ ID NO: 21, and the selected VL2 sequence is SEQ ID NO: 22;
(5) the selected VH2 sequence is SEQ ID NO: 31, and the selected VL2 sequence is SEQ ID NO: 32;
(6) the selected VH2 sequence is SEQ ID NO: 41, and the selected VL2 sequence is SEQ ID NO: 42;
(7) the selected VH2 sequence is SEQ ID NO: 51, and the selected VL2 sequence is SEQ ID NO: 52;
(8) the selected VH2 sequence is SEQ ID NO: 61, and the selected VL2 sequence is SEQ ID NO: 62;
(9) the selected VH2 sequence is SEQ ID NO: 81, and the selected VL2 sequence is SEQ ID NO: 82;
(10) the selected VH2 sequence is SEQ ID NO: 83, and the selected VL2 sequence is SEQ ID NO: 84;
(11) the selected VH2 sequence is SEQ ID NO: 87, and the selected VL2 sequence is SEQ ID NO: 88;
(12) the selected VH2 sequence is SEQ ID NO: 89, and the selected VL2 sequence is SEQ ID NO: 90; and
(13) the selected VH2 sequence is SEQ ID NO: 91, and the selected VL2 sequence is SEQ ID NO: 92.

5. The antibody or antigen-binding fragment thereof of any one of claims 1-4, wherein the first antigen-binding domain comprises a first heavy chain variable region (VH1) comprising CDRs 1, 2, and 3, and a first light chain variable region (VL1) comprising CDRs 1, 2, and 3, and the second antigen-binding domain comprises a second heavy chain variable region (VH2) comprising CDRs 1, 2, and 3, and a second light chain variable region (VL2) comprising CDRs 1, 2, and 3, wherein:
(1) the VH1 CDRs 1, 2, 3 amino acid sequences are set forth in SEQ ID NOs: 200, 202, 204, respectively, the VL1 CDRs 1, 2, 3 amino acid sequences are set forth in SEQ ID NOs: 205-207, respectively, the VH2 CDRs 1, 2, 3 amino acid sequences are set forth in SEQ ID NOs: 13, 15, 17, respectively, and the VL2 CDRs 1, 2, 3 amino acid sequences are set forth in SEQ ID NOs: 18-20, respectively, according to Kabat definition; or
(2) the VH1 CDRs 1, 2, 3 amino acid sequences are set forth in SEQ ID NOs: 201, 203, 204, respectively, the VL1 CDRs 1, 2, 3 amino acid sequences are set forth in SEQ ID NOs: 205-207, respectively, the VH2 CDRs 1, 2, 3 amino acid sequences are set forth in SEQ ID NOs: 14, 16, 17, respectively, and the VL2 CDRs 1, 2, 3 amino acid sequences are set forth in SEQ ID NOs: 18-20, respectively, according to Chothia definition.

6. The antibody or antigen-binding fragment thereof of any one of claims 1-4, wherein the first antigen-binding domain comprises a first heavy chain variable region (VH1) comprising CDRs 1, 2, and 3, and a first light chain variable region (VL1) comprising CDRs 1, 2, and 3, and the second antigen-binding domain comprises a second heavy chain variable region (VH2) comprising CDRs 1, 2, and 3, and a second light chain variable region (VL2) comprising CDRs 1, 2, and 3, wherein:
(1) the VH1 CDRs 1, 2, 3 amino acid sequences are set forth in SEQ ID NOs: 200, 202, 204, respectively, the VL1 CDRs 1, 2, 3 amino acid sequences are set forth in SEQ ID NOs: 205-207, respectively, the VH2 CDRs 1, 2, 3 amino acid sequences are set forth in SEQ ID NOs: 63, 65, 67, respectively, and the VL2 CDRs 1, 2, 3 amino acid sequences are set forth in SEQ ID NOs: 68-70, respectively, according to Kabat definition; or
(2) the VH1 CDRs 1, 2, 3 amino acid sequences are set forth in SEQ ID NOs: 201, 203, 204, respectively, the VL1 CDRs 1, 2, 3 amino acid sequences are set forth in SEQ ID NOs: 205-207, respectively, the VH2 CDRs 1, 2, 3 amino acid sequences are set forth in SEQ ID NOs: 64, 66, 67, respectively, and the VL2 CDRs 1, 2, 3 amino acid sequences are set forth in SEQ ID NOs: 68-70, respectively, according to Chothia definition.

7. The antibody or antigen-binding fragment thereof of any one of claims 1-4, wherein the first antigen-binding domain comprises a first heavy chain variable region (VH1) and a first light chain variable region (VL1), and the second antigen-binding domain comprises a second heavy chain variable region (VH2) and a second light chain variable region (VL2), wherein:
(1) the VH1 comprises an amino acid sequence that is at least 90% identical to SEQ ID NO: 208, the VL1 comprises an amino acid sequence that is at least 90% identical to SEQ ID NO: 209, the VH2 comprises an amino acid sequence that is at least 90% identical to SEQ ID NO: 85, the VL2 comprises an amino acid sequence that is at least 90% identical to SEQ ID NO: 86; or
(2) the VH1 comprises an amino acid sequence that is at least 90% identical to SEQ ID NO: 208, the VL1 comprises an amino acid sequence that is at least 90% identical to SEQ ID NO: 209, the VH2 comprises an amino acid sequence that is at least 90% identical to SEQ ID NO: 71, the VL2 comprises an amino acid sequence that is at least 90% identical to SEQ ID NO: 72.

8. The antibody or antigen-binding fragment thereof of any one of claims 2-7, wherein the second antigen-binding domain is a single-chain fragment variable (scFv) domain, wherein the VH2 and VL2 are linked by a first linker; optionally wherein the second antigen-binding domain is connected to the C-terminus of a light chain through a second linker.

9. The antibody or antigen-binding fragment thereof of any one of claims 2-8, further comprising an Fc region, wherein:
i) the C-terminus of the VH1 of the first antigen-binding domain is connected to the Fc region, optionally through a CH1 domain; or
ii) the C-terminus of the VH1 of the first antigen-binding domain is connected to the N-terminus of the Fc region, and the N-terminus of the second antigen-binding domain is connected to the C-terminus of the Fc region.

10. The antibody or antigen-binding fragment thereof of any one of claims 2-9, wherein the antibody comprises a first heavy chain comprising the VH1 and a first light chain comprising the VL1; and a second heavy chain comprising the VH2 and a second light chain comprising the VL2, optionally wherein:
(1) the first heavy chain comprises one or more knob mutations; and the second heavy chain comprises one or more hole mutations; or
(2) the first heavy chain comprises one or more hole mutations; and the second heavy chain comprises one or more knob mutations.

11. The antibody or antigen-binding fragment thereof of any one of claims 9-10, wherein the Fc region is an Fc region of human IgG1, IgG2, IgG3, or IgG4; optionally wherein the Fc region is an Fc region of human IgG1.

12. The antibody or antigen-binding fragment thereof of claim 11, wherein the Fc region comprises one or more the following amino acid residues (all numbering is according to EU numbering):
(1) an Alanine (A) at position 236; a Leucine (L) at position 330 and a Glutamic acid (E) at position 332;
(2) an Alanine (A) at position 236; an Aspartic acid (D) at position 293; a Leucine (L) at position 330 and a Glutamic acid (E) at position 332;
(3) an Alanine (A) at position 236;
(4) an Alanine (A) at position 236; an Aspartic acid (D) at position 293; and a Glutamic acid (E) at position 332;
(5) an Aspartic acid (D) at position 293 and a Glutamic acid (E) at position 332;
(6) an Aspartic acid (D) at position 293; a Leucine (L) at position 330 and a Glutamic acid (E) at position 332; and
(7) an Alanine (A) at position 234; an Alanine (A) at position 235 and a Glycine (G) at position 329;
optionally, the Fc region is afucosylated.

13. A nucleic acid comprising a polynucleotide encoding the antibody or antigen-binding fragment thereof set forth in any one of claims 1-12.

14. A composition comprising the antibody or antigen-binding fragment thereof of any one of claims 1-12 for use in a method of treating a cancer or an autoimmune disease in a subject, the method comprising administering a therapeutically effective amount of the composition to the subject.

15. A pharmaceutical composition comprising the antibody or antigen-binding fragment thereof of any one of claims 1-12, and a pharmaceutical acceptable carrier.
